(19) Europäisches Patentamt / European Patent Office / Office européen des brevets

(11) **EP 3 353 202 B1**

(12) **EUROPEAN PATENT SPECIFICATION**

(45) Date of publication and mention
of the grant of the patent:
**04.11.2020  Bulletin 2020/45**

(51) Int Cl.:
***C07K 16/18*** *(2006.01)*    ***A61K 39/395*** *(2006.01)*

(21) Application number: **16778888.4**

(22) Date of filing: **23.09.2016**

(86) International application number:
**PCT/IB2016/055720**

(87) International publication number:
**WO 2017/051385 (30.03.2017 Gazette 2017/13)**

(54) **PREVENTING, TREATING, AND REDUCING (PERSISTENT) POST-TRAUMATIC HEADACHE**

VORBEUGUNG, BEHANDLUNG UND VERRINGERUNG VON (PERSISTENTEN) POSTTRAUMATISCHEN KOPFSCHMERZEN

PRÉVENTION, TRAITEMENT, ET DIMINUTION (PERSISTANCE) D'UNE CÉPHALÉE POST-TRAUMATIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.09.2015  US 201562232343 P**
**16.08.2016  US 201662375825 P**

(43) Date of publication of application:
**01.08.2018  Bulletin 2018/31**

(73) Proprietor: **Teva Pharmaceuticals International GmbH**
**8645 Rapperswil-Jona (CH)**

(72) Inventor: **BIGAL, Marcelo**
**New Hope, PA 18938-1111 (US)**

(74) Representative: **Marshall, Cameron John**
**Carpmaels & Ransford LLP**
**One Southampton Row**
**London WC1B 5HA (GB)**

(56) References cited:
• **MARCELO E. BIGAL ET AL: "Monoclonal Antibodies for Migraine: Preventing Calcitonin Gene-Related Peptide Activity", CNS DRUGS, vol. 28, no. 5, 1 May 2014 (2014-05-01), pages 389-399, XP055328488, AUCKLAND, NZ ISSN: 1172-7047, DOI: 10.1007/s40263-014-0156-4**

• **ABBAS ALIBHOY ET AL: "Evaluation of cardiovascular parameters in cynomolgus monkeys following IV administration of LBR-101, a monoclonal antibody against calcitonin gene-related peptide", MABS, vol. 6, no. 4, 1 July 2014 (2014-07-01), pages 12-11, XP055144805, ISSN: 1942-0862, DOI: 10.4161/mabs.29242**

• **Miriam E Tucker: "New Data on CGRP Monoclonal Antibodies for Migraine Prevention", , 23 July 2015 (2015-07-23), XP055328484, Retrieved from the Internet: URL:http://www.medscape.com/viewarticle/846893_print [retrieved on 2016-12-13]**

• **MARCELO E. BIGAL ET AL: "Therapeutic antibodies against CGRP or its receptor", BRITISH JOURNAL OF CLINICAL PHARMACOLOGY., vol. 79, no. 6, 22 June 2015 (2015-06-22), pages 886-895, XP055328523, GB ISSN: 0306-5251, DOI: 10.1111/bcp.12591**

• **B V Daiutolo ET AL: "Cortical Injury Modulates the Pain Pathway Partially through Inducible Nitric Oxide Synthase", Department of Neurosurgery Faculty Papers. Paper 65., 1 July 2014 (2014-07-01), XP055328792, Retrieved from the Internet: URL:http://jdc.jefferson.edu/cgi/viewcontent.cgi?article=1065&context=neurosurgeryfp [retrieved on 2016-12-13]**

• D. BREE ET AL: "Development of CGRP-dependent pain and headache related behaviours in a rat model of concussion: Implications for mechanisms of post-traumatic headache", CEPHALALGIA, 29 November 2016 (2016-11-29), XP055328775, GB ISSN: 0333-1024, DOI: 10.1177/0333102416681571

**Description**

Background

Calcitonin gene-related peptide (CGRP) is a 37 amino acid neuropeptide, which belongs to a family of peptides that includes calcitonin, adrenomedullin, adrenomedullin 2 (intermedin), and amylin (Russell et al., Physiol Rev 94:1099-1142, 2014). In humans, two forms of CGRP (a-CGRP and β-CGRP) exist and have similar activities. They vary by three amino acids and exhibit differential distribution. At least two CGRP receptor subtypes may also account for differential activities. CGRP is a neurotransmitter in the central nervous system, and has been shown to be a potent vasodilator in the periphery, where CGRP-containing neurons are closely associated with blood vessels. CGRP-mediated vasodilatation is also associated with neurogenic inflammation, as part of a cascade of events that results in extravasation of plasma and vasodilation of the microvasculature.

Headache is a common complication of traumatic brain injury (TBI), especially so after (repetitive) mild TBI (mTBI) or a mild closed head injury (mCHI). These headaches can be challenging to manage. The headaches can be of high frequency, if not daily. Recognizing the complexity of post-traumatic headaches (PTH), the International Headache Society, in the International Classification of Headache Disorders (ICHD), has a separate classification specific for these headaches (Headache Classification Subcommittee of the International Headache Society, 2004 and the updated edition, 2013). See Riechers et al. (Handbook of Clinical Neurology 128:567-78, 2015). The second edition (ICHD-II) and the 3rd edition (ICHD-3 beta) define PTH as a headache developing within 7 days of the trauma event or emergence from comatose state. These headaches can be defined as acute in the first three months following injury; however, if they persist beyond this, they are defined as chronic. The severity of the head injury can be further used to subdivide categories of PTH into PTH resulting from mild head injury (Glasgow Coma Scale score (GCS) 13-15, loss of consciousness less than 30 minutes, or other symptoms of concussion) or PTH resulting from severe head injury (GCS less than 13, loss of consciousness greater than 30 minutes, amnesia greater than 48 hours, or abnormal imaging). An additional diagnostic category added to ICHD-II is that of headache due to intracranial hematoma, with epidural and subdural hematomas being the primary hematomas in question. To meet ICHD criteria, these hematomas must be seen on imaging and in the case of epidural hematoma it must develop within 24 hours of the hematoma and resolve within 3 months following surgical intervention, whereas the subdural hematoma headache should develop within 24-72 hours and no specific resolution criteria exist.

Daiutolo et al., Department of Neurosurgery Faculty Papers, Paper 65 (2014), considers modulation of the pain pathway by cortical injury partially through inducible nitric oxide synthase.

Bigal et al., CNS Drugs 28(5): 389-399 (2014) considers monoclonal antibodies for migraine.

Summary of the invention

The invention provides a composition comprising a monoclonal anti-CGRP antagonist antibody for use in treating or reducing incidence of post-traumatic headache in a subject, wherein the monoclonal antibody has a CDR H1 as set forth in SEQ ID NO:3; a CDR H2 as set forth in SEQ ID NO:4; a CDR H3 as set forth in SEQ ID NO:5; a CDR L1 as set forth in SEQ ID NO:6; a CDR L2 as set forth in SEQ ID NO:7; and a CDR L3 as set forth in SEQ ID NO:8.

Summary

Disclosed herein are anti-CGRP antagonist antibodies and methods of using the same for preventing, treating, or reducing incidence of (persistent) post-traumatic headache. Also disclosed herein are methods of preventing, treating, or reducing incidence of (persistent) post-traumatic headache in a subject comprising administering to the subject a monoclonal antibody that modulates the CGRP pathway.

Suitable administration schedules include, but are not limited to, monthly, quarterly, or a single dose. In some embodiments, the monoclonal antibody can be administered monthly. For example, the monoclonal antibody can be administered monthly for 1, 2, 3, 4, 5, 6, 7, 8, 9, 10, 11, 12, or more months. In some embodiments, the monoclonal antibody can be administered monthly for three or more months. When administered monthly, the dose of the monoclonal antibody administered to the patient can be about 225 mg to about 900 mg.

The monoclonal antibody can be administered as a single dose. When administered as a single dose, the dose of the monoclonal antibody administered to the patient can be about 675 mg to about 1000 mg.

The treating or reducing can comprise reducing the number of headache hours of any severity, reducing the number of monthly headache days of any severity, reducing the use of any acute headache medications, reducing a 6-item Headache Impact Test (HIT-6) disability score, improving 12-Item Short Form Health Survey (SF-12) score (Ware et al., Med Care 4:220-233, 1996), reducing Patient Global Impression of Change (PGIC) score (Hurst et al., J Manipulative Physiol Ther 27:26-35, 2004), improving Sport ConCuSSion ASSeSment tool 3 (SCAT-3) score (McCrory et al. British Journal of Sports Medicine 47:263-266, 2013), or any combination thereof. In some embodiments, the number of monthly headache days can be reduced for at least seven days after a single administration.

In some embodiments, monthly headache hours experienced by the subject after said administering is reduced by 40 or more hours (e.g., 45, 50, 55, 60, 65, 70, 75, 80, or more) from a pre-administration level in the subject. Monthly headache hours may be reduced by more than 60 hours. In some embodiments, monthly headache hours experienced by the subject after said administering are reduced by 25% or more (e.g., 30%, 35%, 40%, 45%, 50%, or more) relative to a pre-administration level in the subject. Monthly headache hours may be reduced by 40% or more. In some embodiments, monthly headache days experienced by the subject after said administering is reduced by three or more days (e.g., 3, 4, 5, 6, 7, 8, 9, 10, 11, 12, 13, 14, 15, 16, 17, 18, 19, 20, or more days) from a pre-administration level in the subject. In some embodiments, the number of monthly headache days can be reduced by at least about 50% from a pre-administration level in the subject. Thus, in some aspects, the number of monthly headache days can be reduced by at least about 50%, at least about 55%, at least about 60%, at least about 65%, at least about 70%, at least about 75%, at least about 80%, or at least about 90%.

In some embodiments, the administering can be subcutaneous administration. In some embodiments, the administering can be intravenous administration. In some embodiments, the administering can comprise utilizing a pre-filled syringe, pre-filled syringe with a needle safety device, injection pen, or auto-injector comprising a dose of the monoclonal antibody. In some embodiments, the monoclonal antibody can be formulated at a concentration of at least 150 mg/mL. In some embodiments, the monoclonal antibody can be administered in a volume of less than 2 mL, e.g., about 1.5 mL.

In some embodiments, the treating or reducing further comprises administering to the subject a second agent simultaneously or sequentially with the monoclonal antibody for use in the invention. The second agent can be any of 5-HT1 agonists, triptans, ergot alkaloids, and non-steroidal anti-inflammatory drugs. In some embodiments, the second agent is an agent taken by the subject prophylactically. In some embodiments, monthly use of the second agent by the subject is decreased by at least about 15%, e.g., at least 16%, 17%, 18%, 20%, 22%, 25%, 28%, 30%, 35%, 40%, 45%, 50%, 55%, 60%, 65%, 70%, 75%, 80%, 85%, 90%, or at least about 95%, after administering the monoclonal antibody. In some embodiments, the second agent is a triptan.

In some embodiments, the subject is a human.

The monoclonal antibody is an anti-CGRP antagonist antibody. In some embodiments, the monoclonal antibody is a human or humanized monoclonal antibody. The monoclonal antibody has a CDR H1 as set forth in SEQ ID NO:3; a CDR H2 as set forth in SEQ ID NO:4; a CDR H3 as set forth in SEQ ID NO:5; a CDR L1 as set forth in SEQ ID NO:6; a CDR L2 as set forth in SEQ ID NO:7; and a CDR L3 as set forth in SEQ ID NO:8.

In one embodiment, the monoclonal antibody for use in the invention is administered to the subject as a single dose in an amount that modulates the CGRP pathway, wherein the amount of the monoclonal antibody is about 225 mg to about 1000 mg, e.g., about 675 mg or about 900 mg.

In a further embodiment, the anti-CGRP antagonist antibody for use in the invention is administered in combination with at least one additional agent useful for treating the post-traumatic headache. Such additional agents include 5-HT1-like agonists (and agonists acting at other 5-HT1 sites), and non-steroidal anti-inflammatory drugs (NSAIDs).

Examples of 5-HT1 agonists that can be used in combination with an anti-CGRP antibody include a class of compounds known as triptans, such as sumatriptan, zolmitriptan, naratriptan, rizatriptan, eletriptan, almotriptan, and frovatriptan. Ergot alkaloids and related compounds are also known to have 5-HT agonist activity. Included among these compounds are ergotamine tartrate, ergonovine maleate, and ergoloid mesylates (e.g., dihydroergocornine, dihydroergocristine, dihydroergocryptine, and dihydroergotamine mesylate (DHE 45)).

Examples of NSAIDs that can be used in combination with an anti-CGRP antibody include aspirin, diclofenac, diflusinal, etodolac, fenbufen, fenoprofen, flufenisal, flurbiprofen, ibuprofen, indomethacin, ketoprofen, ketorolac, meclofenamic acid, mefenamic acid, nabumetone, naproxen, oxaprozin, phenylbutazone, piroxicam, sulindac, tolmetin or zomepirac, cyclooxygenase-2 (COX-2) inhibitors, celecoxib; rofecoxib; meloxicam; JTE-522; L-745,337; NS398; or a pharmaceutically acceptable salt thereof.

In some embodiments, the anti-CGRP antagonist antibody recognizes a human CGRP. In some embodiments, the anti-CGRP antagonist antibody binds to both human $\alpha$-CGRP and $\beta$-CGRP. In some embodiments, the anti-CGRP antagonist antibody binds human and rat CGRP. In some embodiments, the anti-CGRP antagonist antibody binds the C-terminal fragment having amino acids 25-37 of CGRP. In some embodiments, the anti-CGRP antagonist antibody binds a C-terminal epitope within amino acids 25-37 of CGRP.

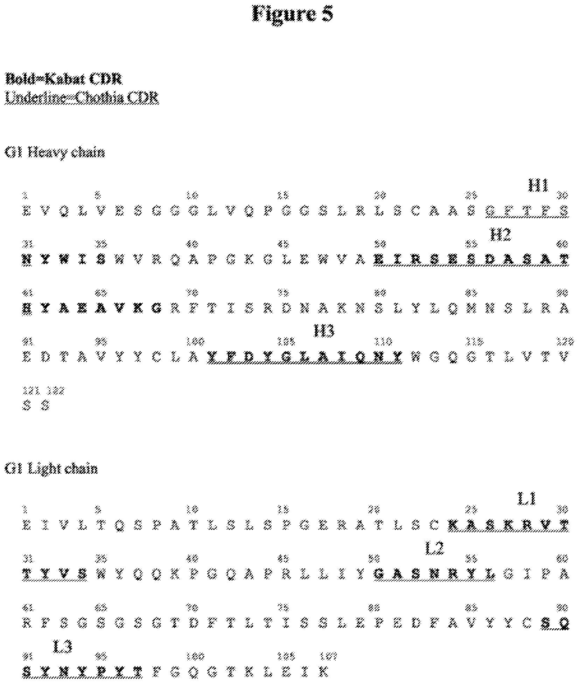

The anti-CGRP antagonist antibody is a monoclonal antibody. In some embodiments, the anti-CGRP antagonist antibody is humanized. In some embodiments, the antibody is human. In some embodiments, the anti-CGRP antagonist antibody is antibody G1 (as described herein). The anti-CGRP antagonist antibody comprises six CDRs of antibody G1. In still other embodiments, the anti-CGRP antagonist antibody comprises the amino acid sequence of the heavy chain variable region shown in Figure 5 (SEQ ID NO:1) and the amino acid sequence of the light chain variable region shown in Figure 5 (SEQ ID NO:2).

In some embodiments, the antibody comprises a modified constant region, such as a constant region that is immunologically inert (including partially immunologically inert), e.g., does not trigger complement mediated lysis, does not stimulate antibody-dependent cell mediated cytotoxicity (ADCC), does not activate microglia, or having reduced one or more of these activities. In some embodiments, the constant region is modified as described in Eur. J. Immunol. (1999) 29:2613-2624; PCT Application No. PCT/GB99/01441; and/or UK Patent Application No. 9809951.8. In other embodiments, the antibody comprises a human heavy chain IgG2 constant region comprising the following mutations: A330P331 to S330S331 (amino acid numbering with reference to the wildtype IgG2 sequence). Eur. J. Immunol. (1999) 29:2613-2624. In some embodiments, the heavy chain constant region of the antibody is a human heavy chain IgG1 with any of the following mutations: 1) A327A330P331 to G327S330S331; 2) E233L234L235G236 (SEQ ID NO:48) to P233V234A235 with G236 deleted; 3) E233L234L235 to P233V234A235; 4) E233L234L235G236A327A330P331 (SEQ ID NO:49) to P233V234A235G327S330S331 (SEQ ID NO:50) with G236 deleted; 5) E233L234L235A327A330P331 (SEQ ID NO:51) to P233V234A235G327S330S331 (SEQ ID NO:50); and 6) N297 to A297 or any other amino acid except N. In some embodiments, the heavy chain constant region of the antibody is a human heavy chain IgG4 with any of the following mutations: E233F234L235G236 (SEQ ID NO:52) to P233V234A235 with G236 deleted; E233F234L235 to P233V234A235; and S228L235 to P228E235.

In still other embodiments, the constant region is aglycosylated for N-linked glycosylation. In some embodiments, the constant region is aglycosylated for N-linked glycosylation by mutating the oligosaccharide attachment residue (such as Asn297) and/or flanking residues that are part of the N-glycosylation recognition sequence in the constant region. In some embodiments, the constant region is aglycosylated for N-linked glycosylation. The constant region may be aglycosylated for N-linked glycosylation enzymatically or by expression in a glycosylation deficient host cell.

The binding affinity ($K_D$) of an anti-CGRP antagonist antibody to CGRP (such as human $\alpha$-CGRP as measured by surface plasmon resonance at an appropriate temperature, such as 25 or 37 °C) can be about 0.02 to about 200 nM. In some embodiments, the binding affinity is any of about 200 nM, about 100 nM, about 50 nM, about 10 nM, about 1 nM, about 500 pM, about 100 pM, about 60 pM, about 50 pM, about 20 pM, about 15 pM, about 10 pM, about 5 pM, or about 2 pM. In some embodiments, the binding affinity is less than any of about 250 nM, about 200 nM, about 100 nM, about 50 nM, about 10 nM, about 1 nM, about 500 pM, about 100 pM, or about 50 pM. In some embodiments, the binding affinity is less than about 50 nM.

The anti-CGRP antagonist antibody may be administered prior to, during and/or after post-traumatic headache. In some embodiments, the anti-CGRP antagonist antibody is administered prior to the attack of post-traumatic headache (e.g., after trauma or injury to the head and/or neck). Administration of an anti-CGRP antagonist antibody can be by any means known in the art, including: orally, intravenously, subcutaneously, intraarterially, intramuscularly, intranasally (e.g., with or without inhalation), intracardially, intraspinally, intrathoracically, intraperitoneally, intraventricularly, sublingually, transdermally, and/or via inhalation. Administration may be systemic, e.g., intravenously, or localized. In some embodiments, an initial dose and one or more additional doses are administered the same way, i.e., subcutaneously or intravenously. In some embodiments, the one or more additional doses are administered in a different way than the initial dose, i.e., the initial dose may be administered intravenously and the one or more additional doses may be administered subcutaneously.

In some embodiments, the anti-CGRP antagonist antibody may be administered in conjunction with another agent, such as another agent for treating post-traumatic headache.

Disclosed herein is a pharmaceutical composition for preventing, treating, or reducing post-traumatic headache comprising an effective amount of an anti-CGRP antagonist antibody, in combination with one or more pharmaceutically acceptable excipients.

Disclosed herein is a kit for use in any of the methods described herein. In some instances, the kit comprises a container, a composition comprising an anti-CGRP antagonist antibody described herein, in combination with a pharmaceutically acceptable carrier, and instructions for using the composition in any of the methods described herein.

Disclosed herein are anti-CGRP antagonist antibodies and polypeptides derived from antibody G1 or its variants shown in Table 6. Accordingly, in one embodiment, the invention can use an antibody G1 (interchangeably termed "G1" and "TEV-48125") that is produced by expression vectors having ATCC Accession Nos. PTA-6866 and PTA-6867. For example, in one embodiment is an antibody comprising a heavy chain produced by the expression vector with ATCC Accession No. PTA-6867. In a further embodiment is an antibody comprising a light chain produced by the expression vector with ATCC Accession No. PTA-6866. The amino acid sequences of the heavy chain and light chain variable regions of G1 are shown in Figure 5. The complementarity determining region (CDR) portions of antibody G1 (including Chothia and Kabat CDRs) are also shown in Figure 5. It is understood that reference to any part of or entire region of G1 encompasses sequences produced by the expression vectors having ATCC Accession Nos. PTA-6866 and PTA-6867, and/or the sequences depicted in Figure 5.

In one embodiment, the invention can use an antibody comprising a $V_H$ domain that is at least 85%, at least 86%, at least 87%, at least 88%, at least 89%, at least 90%, at least 91%, at least 92%, at least 93%, at least 94%, at least 95%, at least 96%, at least 97% at least 98%, at least 99% or 100% identical in amino acid sequence to SEQ ID NO:1.

In another embodiment, the invention can use an antibody comprising a $V_L$ domain that is at least 85%, at least 86%, at least 87%, at least 88%, at least 89%, at least 90%, at least 91%, at least 92%, at least 93%, at least 94%, at least 95%, at least 96%, at least 97% at least 98%, at least 99% or 100% identical in amino acid sequence to SEQ ID NO:2.

In another embodiment, the invention can use an antibody comprising a fragment or a region of the antibody G1. In one embodiment, the fragment is a light chain of the antibody G1. In another embodiment, the fragment is a heavy chain of the antibody G1. In yet another embodiment, the fragment contains one or more variable regions from a light chain and/or a heavy chain of the antibody G1. In yet another embodiment, the fragment contains one or more variable regions from a light chain and/or a heavy chain shown in Figure 5.

In some embodiments, the CDR is a Kabat CDR. In other embodiments, the CDR is a Chothia CDR. In other embodiments, the CDR is a combination of a Kabat and a Chothia CDR (also termed "combined CDR" or "extended CDR"). In other words, for any given embodiment containing more than one CDR, the CDRs may be any of Kabat, Chothia, and/or combined.

In some embodiments, the antibody is a human antibody. In other embodiments, the antibody a humanized antibody. In some embodiments, the antibody is monoclonal. In some embodiments, the antibody (or polypeptide) is isolated. In some embodiments, the antibody (or polypeptide) is substantially pure.

The heavy chain constant region of the antibodies may be from any types of constant region, such as IgG, IgM, IgD, IgA, and IgE; and any isotypes, such as IgG1, IgG2, IgG3, and IgG4.

In some embodiments, the antibody comprises a modified constant region as described herein.

Also disclosed herein is a polynucleotide (which may be isolated) comprising a polynucleotide encoding a fragment or a region of the antibody G1 or its variants shown in Table 6.

Also disclosed herein is a polynucleotide (which may be isolated) comprising a polynucleotide that encodes for antibody G1 or its variants shown in Table 6. The polynucleotide may comprise either or both of the polynucleotides shown in SEQ ID NO:9 and SEQ ID NO:10.

Also disclosed herein are vectors (including expression and cloning vectors) and host cells comprising any of the polynucleotide disclosed herein. In some instances, the vector is pDb.CGRP.hFcGI having ATCC No. PTA-6867. In other instances, the vector is pEb.CGRP.hKGI having ATCC No. PTA-6866.

In one embodiment, the composition for use in the invention is administered to the subject as a single dose in an amount that modulates the CGRP pathway, wherein the amount of the monoclonal antibody administered to the patient is about 675 mg to about 1000 mg.

Brief Description of the Drawings

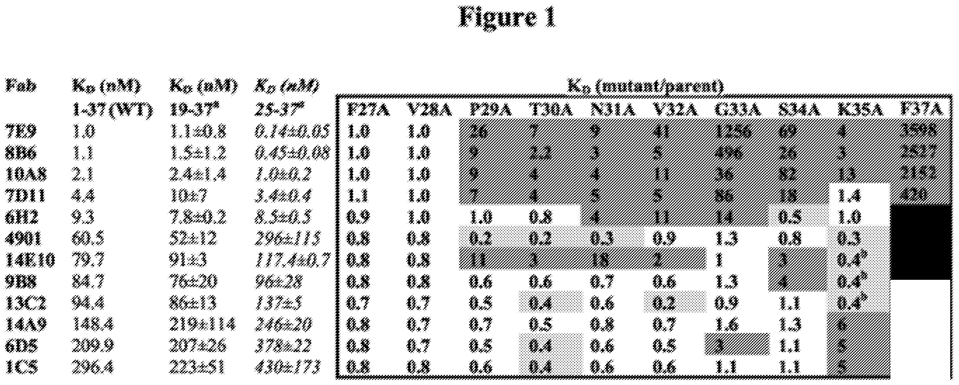

Figure 1 is a table showing binding affinities of 12 murine antibodies for different alanine substituted human α-CGRP fragments. Binding affinities were measured at 25°C using Biacore by flowing Fabs across CGRPs on the chip. The boxed values represent the loss in affinity of alanine mutants relative to parental fragment, 25-37 (italic), except K35A, which was derived from a 19-37 parent. "[a]" indicates affinities for 19-37 and 25-37 fragments are the mean average $\pm$ standard deviation of two independent measurements on different sensor chips. "[b]" indicates these interactions deviated from a simple bimolecular interaction model due to a biphasic offrate, so their affinities were determined using a conformational change model. Grey-scale key: white (1.0) indicates parental affinity; light grey (less than 0.5) indicates higher affinity than parent; dark grey (more than 2) indicates lower affinity than parent; and black indicates that no binding was detected.

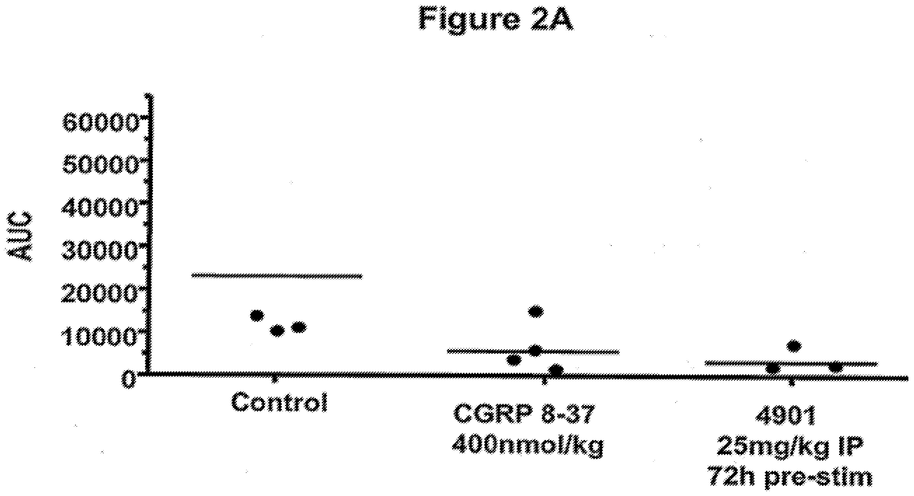

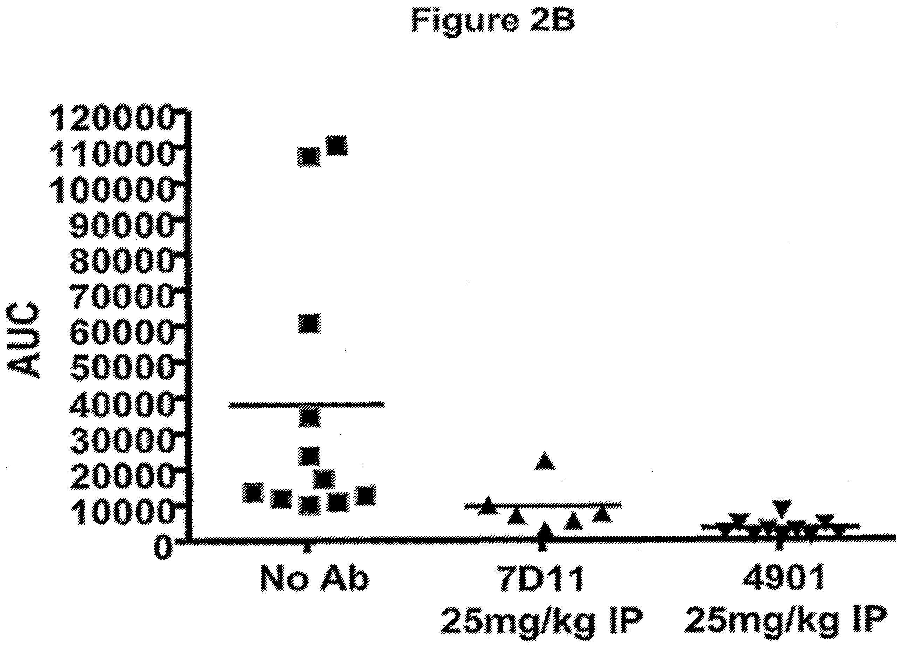

Figures 2A and 2B show the effect of administering CGRP 8-37 (400 nmol/kg), antibody 4901 (25 mg/kg), and antibody 7D11 (25 mg/kg) on skin blood flow measured as blood cell flux after electrical pulse stimulation for 30 seconds. CGRP 8-37 was administered intravenously (iv) 3-5 min before electrical pulse stimulation. Antibodies were administered intraperitoneal (IP) 72 hours before electrical pulse stimulation. Each point in the graphs represents AUC of one rat treated under the conditions as indicated. Each line in the graphs represents average AUC of rats treated under the condition as indicated. AUC (area under the curve) equals to Δflux x Δtime. "Δflux" represents the change of flux units after the electrical pulse stimulation; and "Δtime" represents the time period taken for the blood cell flux level to return to the level before the electrical pulse stimulation.

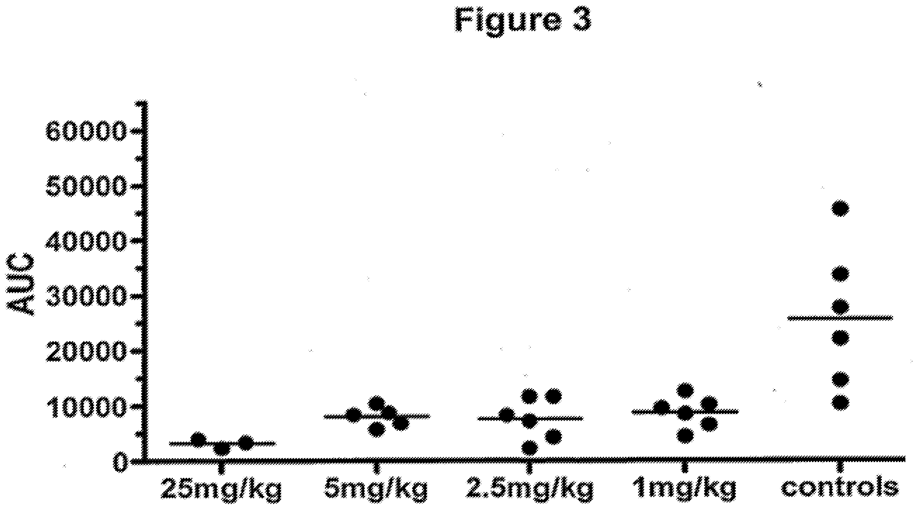

Figure 3 shows the effect of administering different dosage of antibody 4901 (25 mg/kg, 5 mg/kg, 2.5 mg/kg, or 1 mg/kg) on skin blood flow measured as blood cell flux after electrical pulse stimulation for 30 seconds. Antibodies were administered intravenously (IV) 24 hours before electrical pulse stimulation. Each point in the graph represents AUC of one rat treated under the conditions as indicated. The line in the graph represents average AUC of rats treated under the condition as indicated.

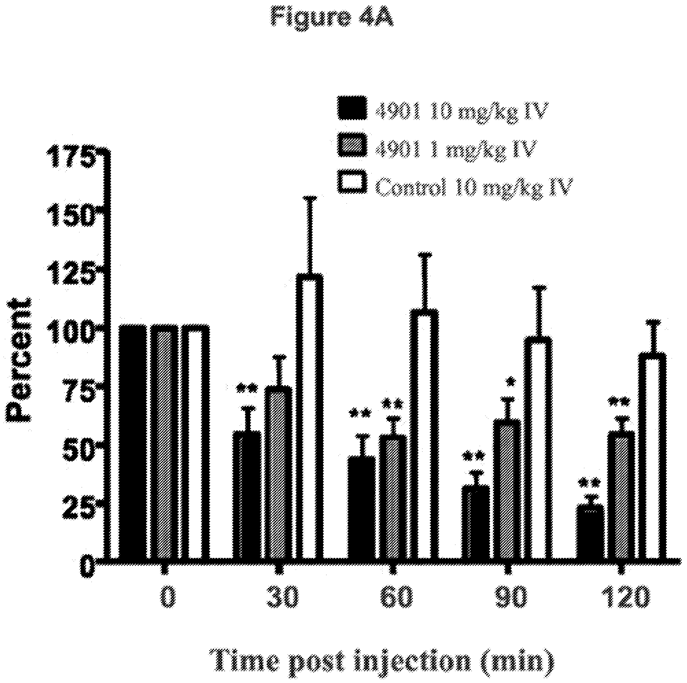

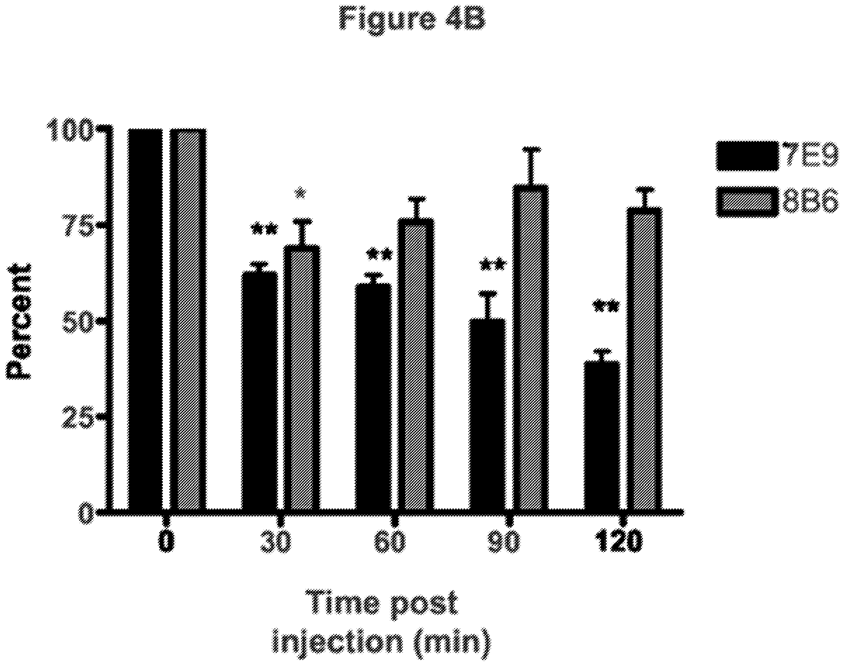

Figures 4A and 4B show the effect of administering antibody 4901 (1 mg/kg or 10 mg/kg, i.v.), antibody 7E9 (10 mg/kg, i.v.), and antibody 8B6 (10 mg/kg, i.v.) on skin blood flow measured as blood cell flux after electrical pulse stimulation for 30 seconds. Antibodies were administered intravenously (i.v.) followed by electrical pulse stimulation at 30 min, 60 min, 90 min, and 120 min after antibody administration. Y axis represents percent of AUC as compared to level of AUC when no antibody was administered (time 0). X axis represents time (minutes) period between the administration of antibodies and electrical pulse stimulation. "*" indicates $P < 0.05$, and "**" indicates $P < 0.01$, as compared to time 0. Data were analyzed using one-way ANOVA with a Dunnett's Multiple comparison test.

Figure 5 shows the amino acid sequence of the heavy chain variable region (SEQ ID NO:1) and light chain variable region (SEQ ID NO:2) of antibody G1. The Kabat CDRs are in bold text, and the Chothia CDRs are underlined. The amino acid residues for the heavy chain and light chain variable region are numbered sequentially.

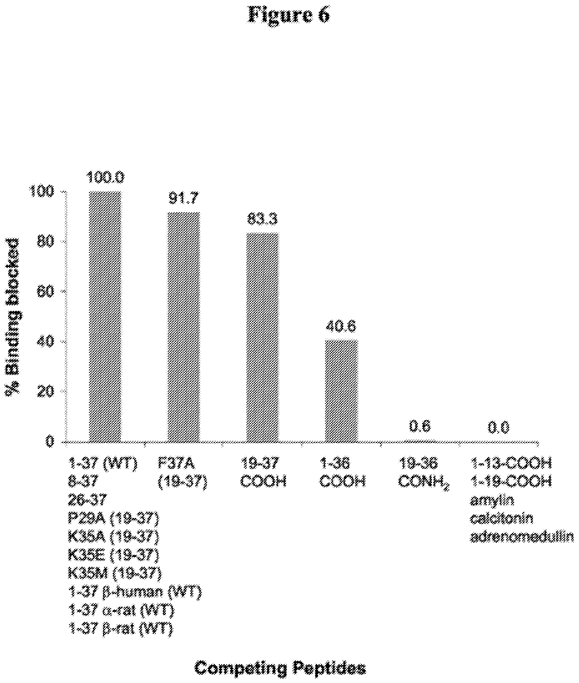

Figure 6 shows epitope mapping of antibody G1 by peptide competition using Biacore. N-biotinylated human $\alpha$-CGRP was captured on SA sensor chip. G1 Fab (50 nM) in the absence of a competing peptide or pre-incubated for 1 hour with 10 $\mu$M of a competing peptide was flowed onto the chip. Binding of G1 Fab to the human $\alpha$-CGRP on the chip was measured. Y axis represents percentage of binding blocked by the presence of the competing peptide compared with the binding in the absence of the competing peptide.

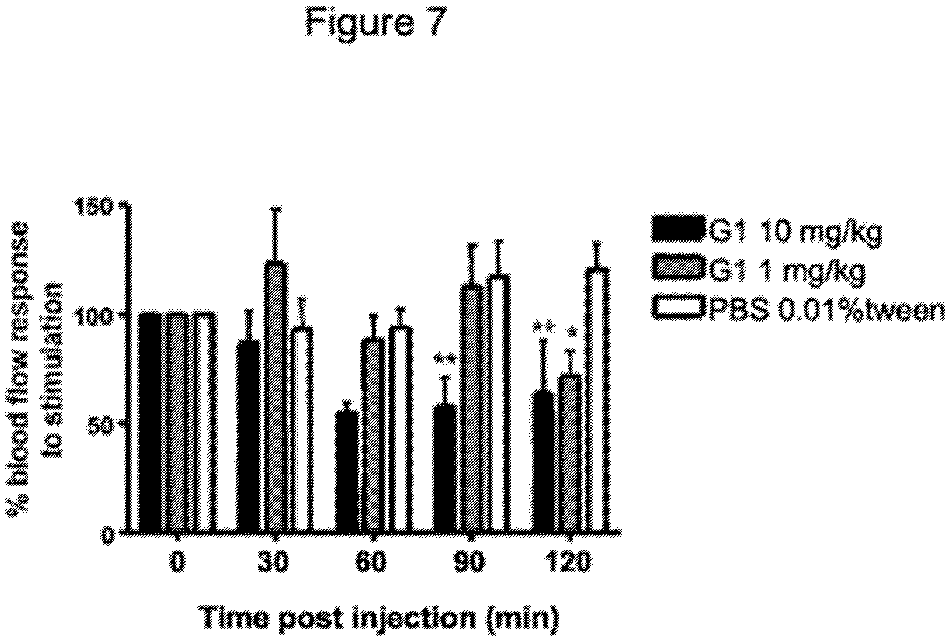

Figure 7 shows the effect of administering antibody G1 (1 mg/kg or 10 mg/kg, i.v.) or vehicle (PBS, 0.01% Tween 20) on skin blood flow measured as blood cell flux after electrical pulse stimulation for 30 seconds. Antibody G1 or vehicle was administered intravenously (i.v.) followed by nerve electrical pulse stimulation at 30 min, 60 min, 90 min, and 120 min after antibody administration. Y axis represents percent of AUC as compared to level of AUC when no antibody or vehicle (defined as 100%) was administered (time 0). X axis represents time (minutes) period between the administration of antibodies and electrical pulse stimulation. "*" indicates $P < 0.05$, and "**" indicates $P < 0.01$, as compared to vehicle. Data were analyzed using two-way ANOVA and Bonferroni post tests.

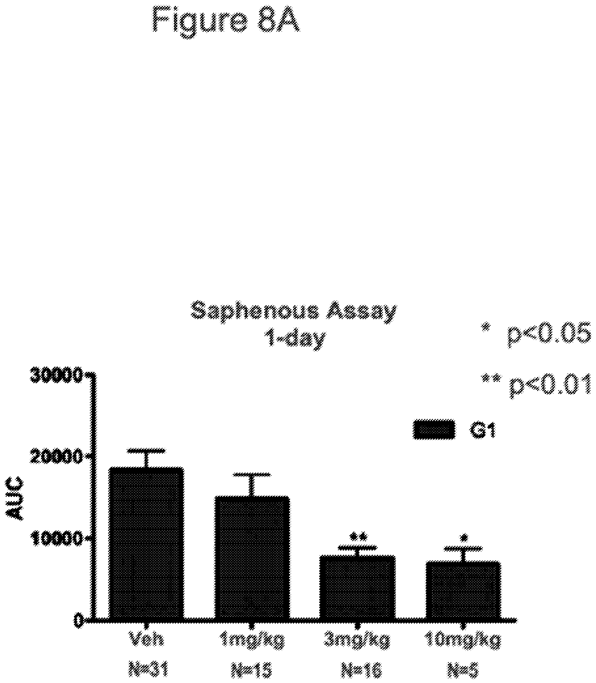

Figure 8A shows the effect of administering antibody G1 (1 mg/kg, 3 mg/kg or 10 mg/kg, i.v.) or vehicle (PBS, 0.01% Tween 20) on skin blood flow measured as blood cell flux after electrical pulse stimulation for 30 seconds 24 hours after dosing. Antibody G1 or vehicle was administered intravenously (i.v.) 24 hours before nerve electrical pulse stimulation. Y axis represents total area under curve (change in blood cell flux multiplied by the change in time from stimulation until flux returns to baseline, AUC). X axis represents varying doses of antibody G1. "*" indicates $P < 0.05$, and "**" indicates $P < 0.01$, as compared to vehicle. Data were analyzed using one-way ANOVA and Dunn's multiple comparison test.

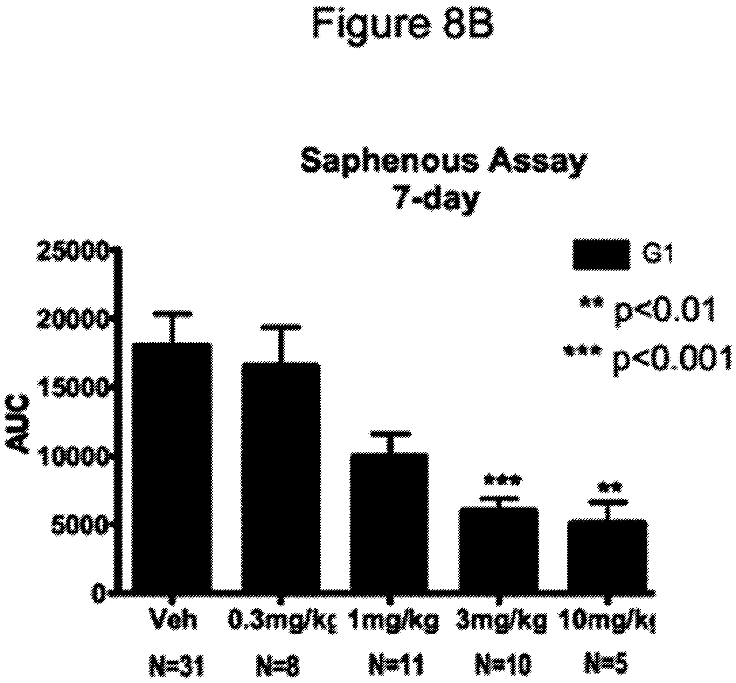

Figure 8B shows the effect of administering antibody G1 (0.3 mg/kg, 1 mg/kg, 3 mg/kg or 10 mg/kg, i.v.) or vehicle (PBS, 0.01% Tween 20) on skin blood flow measured as blood cell flux after electrical pulse stimulation for 30 seconds 7 days after dosing. Antibody G1 or vehicle was administered intravenously (i.v.) 7 days before nerve electrical pulse stimulation. Y axis represents total AUC. X axis represents varying doses of antibody G1. "**" indicates $P < 0.01$, and "***" indicates $P < 0.001$, as compared to vehicle. Data were analyzed using one-way ANOVA and Dunn's multiple comparison test.

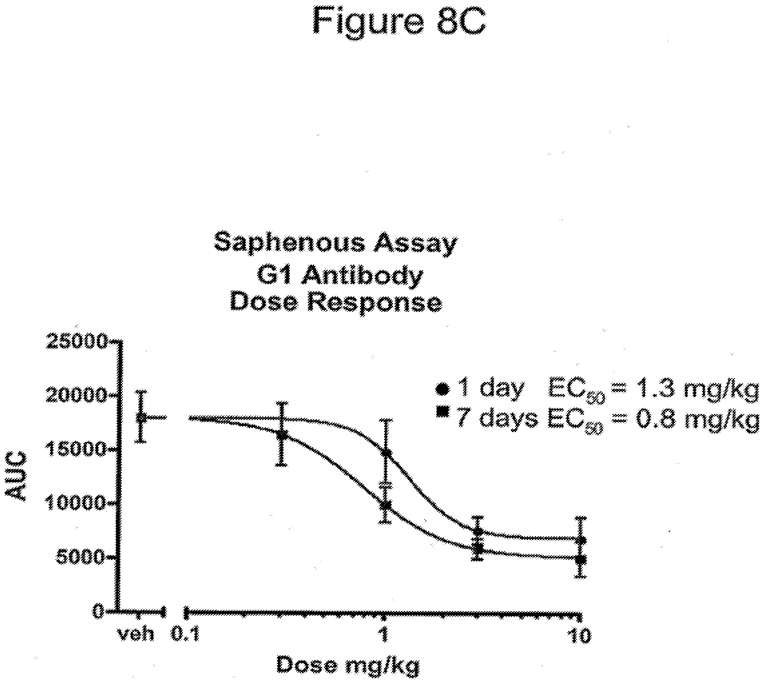

Figure 8C is a curve fit analysis of the data from Figures 8A and 8B. Antibody G1 or vehicle was administered intravenously (i.v.) either 24 hours or 7 days before nerve electrical pulse stimulation. Y axis represents total AUC. X axis represents varying doses of antibody G1 in "mg/kg" on a logarithmic scale to determine $EC_{50}$.

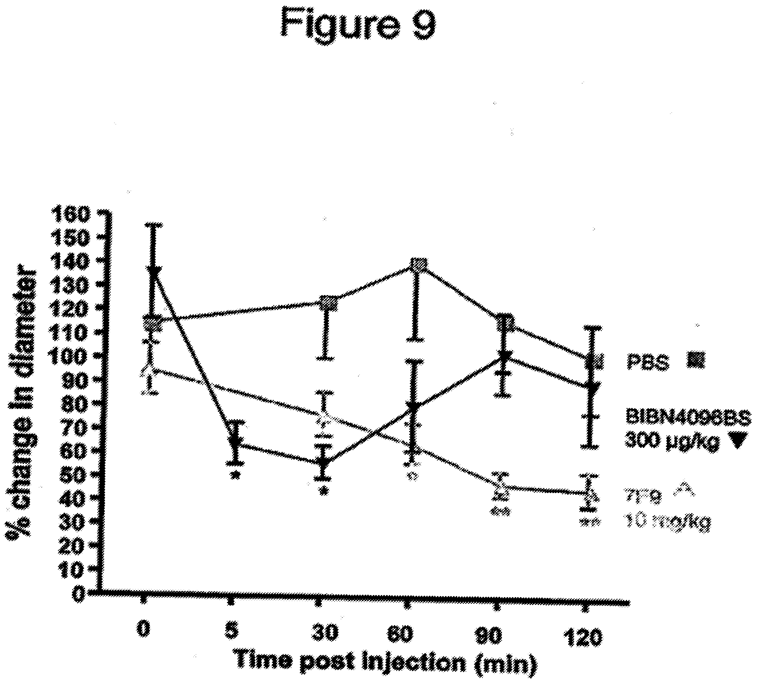

Figure 9 shows the effect of antibody mu7E9 (10 mg/kg), BIBN4096BS or vehicle (PBS, 0.01% Tween 20) on the change in diameter of the middle meningeal artery after electrical field stimulation. Antibody mu7E9, BIBN4096BS or vehicle were administered intravenously (i.v.) at time point 0 minutes after a baseline response to electrical stimulation was established. Y axis represents change in diameter of the middle meningeal artery after electrical field stimulation. Resting diameter corresponds to 0%. X axis represents time (minutes) of electrical pulse stimulation. "*" indicates $P < 0.05$, and "**" indicates $P < 0.01$, as compared to vehicle. Data were analyzed using one-way ANOVA and Dunett's multiple comparison test.

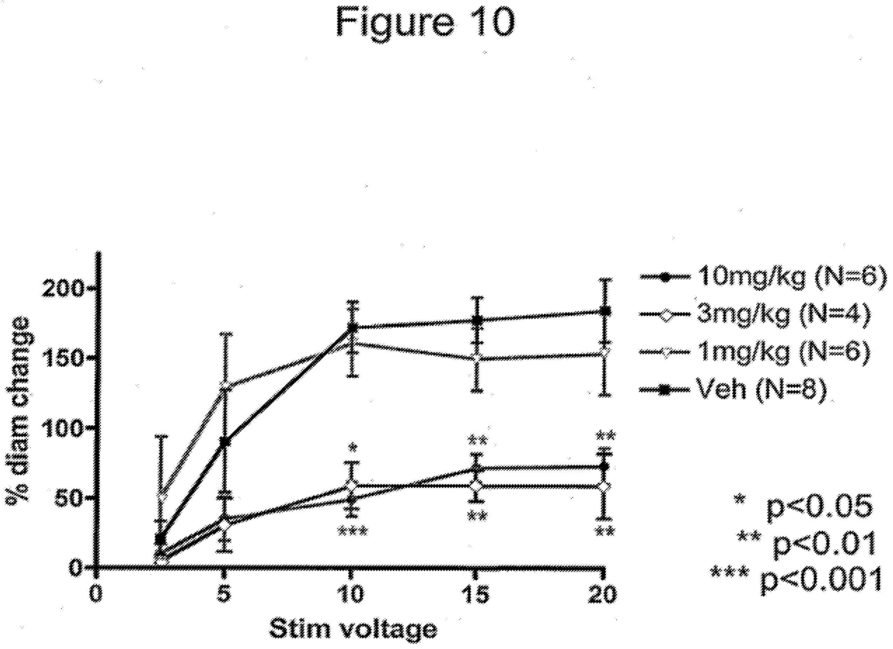

Figure 10 shows the effect of varying doses of antibody G1 (1 mg/kg, 3 mg/kg or 10 mg/kg, i.v.) or vehicle (PBS, 0.01% Tween 20) on the change in diameter of the middle meningeal artery after electrical field stimulation. Antibody G1 or vehicle was administered intravenously (i.v.) 7 days before electrical field stimulation. Y axis represents change in diameter of the middle meningeal artery. Resting diameter corresponds to 0%. X axis represents stimulation voltage. "*" indicates $P < 0.05$, "**" indicates $P < 0.01$, and "***" indicates $P < 0.001$, as compared to vehicle. Data were analyzed using two-way ANOVA and Bonferroni posttests.

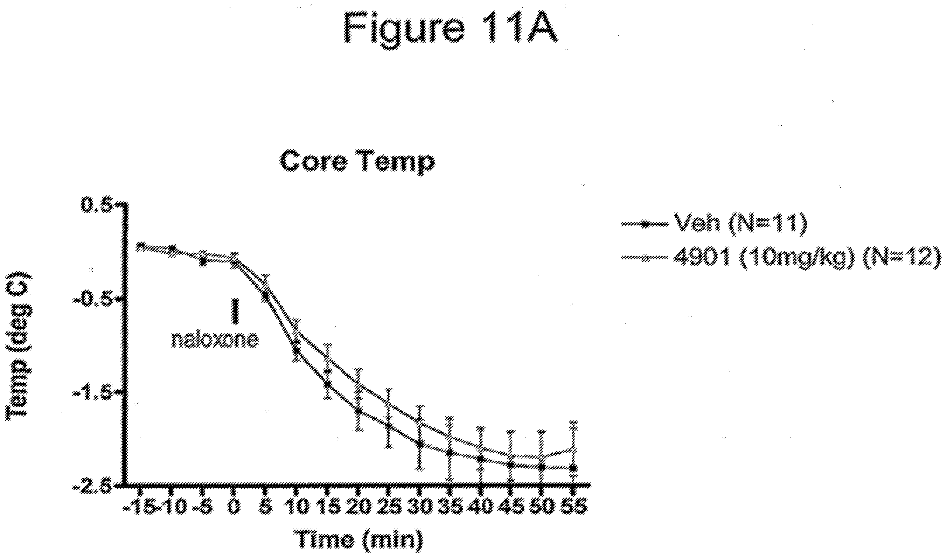

Figure 11A shows the effect of antibody mu4901 (10 mg/kg) or vehicle (PBS, 0.01% Tween 20), administered intravenously (i.v.) 24 hours prior, on the decrease in core temperature induced by subcutaneous injection of naloxone (1 mg/kg) in morphine addicted rats. The Y axis represents temperature difference from baseline. The X axis represents time measured from the point of naloxone injection.

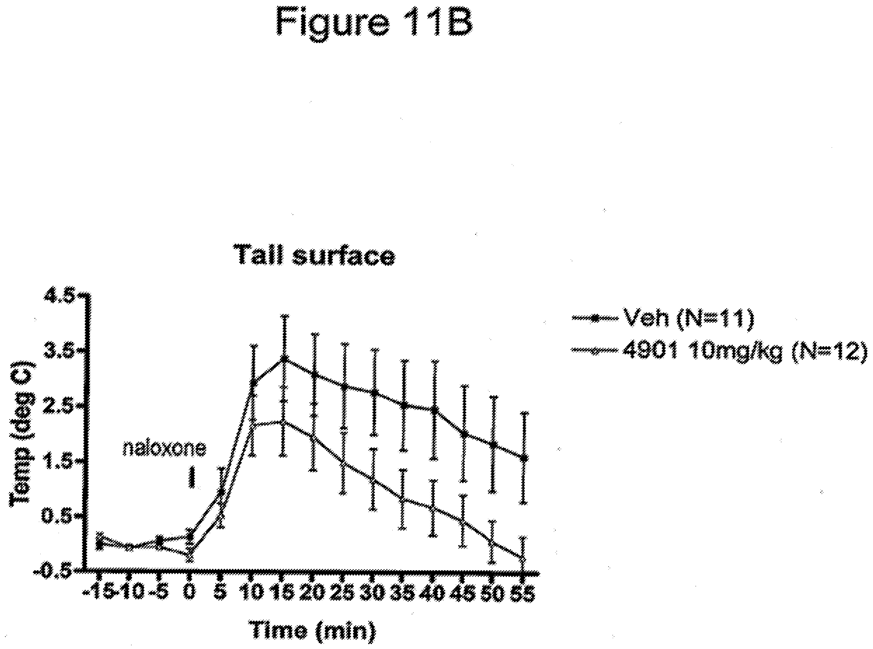

Figure 11B shows the effect of antibody mu4901 (10 mg/kg) or vehicle (PBS, 0.01% Tween 20), administered intravenously (i.v.) 24 hours prior, on the increase in tail surface temperature induced by subcutaneous injection of naloxone (1 mg/kg) in morphine addicted rats. The Y axis represents temperature difference from baseline. The X axis represents time measured from the point of naloxone injection.

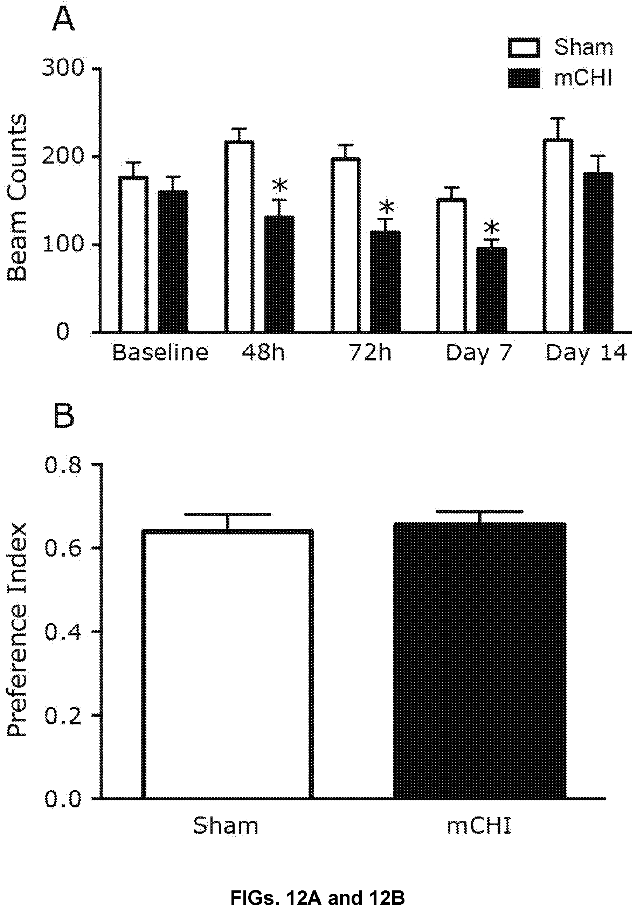

Figures 12A and 12B are two bar graphs that show the effect of mCHI on (12A) vertical exploratory activity and (12B) novel object recognition. Data are mean + SEM (n=8) *p<0.05 vs sham.

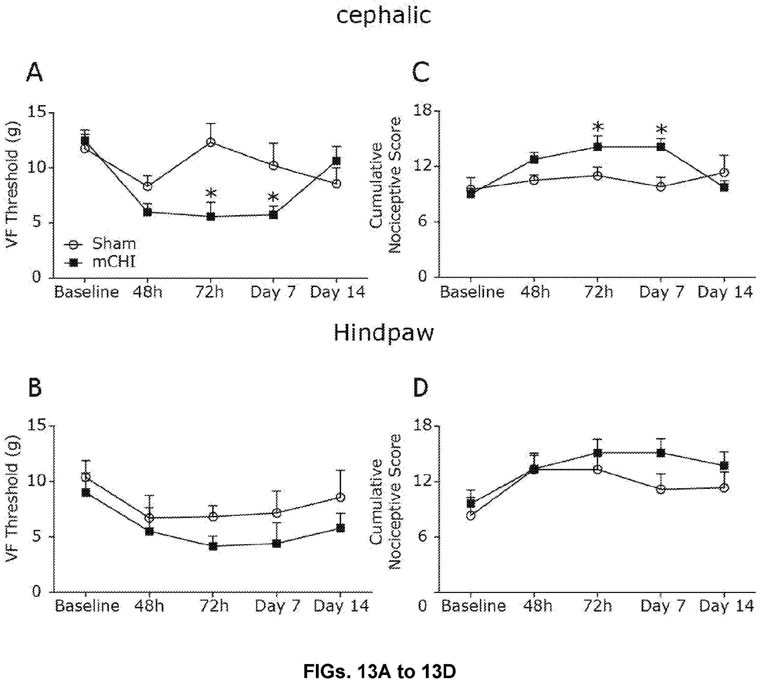

Figures 13A to 13D are four line graphs that show cephalic (13A and 13C) and hindpaw (13B and 13D) response threshold and cumulative nociceptive score to mechanical stimulation of over 14 days following mCHI. Data are mean + SEM (n=6). *p<0.05 vs sham.

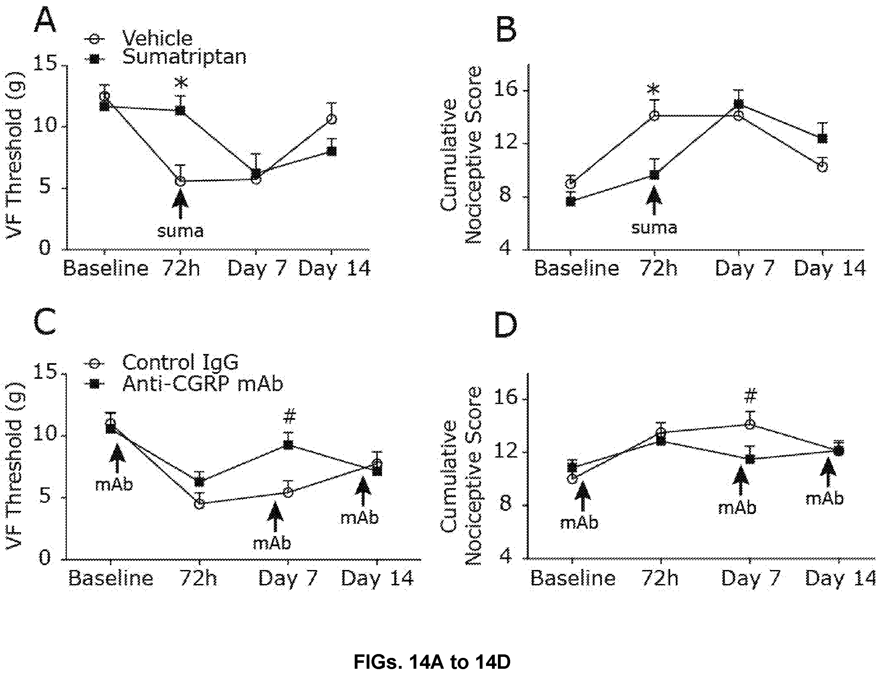

Figures 14A to 14D are four line graphs that show the effect of acute sumatriptan (Figures 14A and 14B) or chronic murine anti-CGRP antibody (Figures 14C and 14D) treatment on response threshold and cumulative nociceptive score to mechanical stimulation following mCHI. Data are mean + SEM (n=6-8). *p<0.05 sham vs mCHI, #p<0.05 mCHI vs. mCHI + Suma +p<0.05 control IgG vs anti-CGRP mAb.

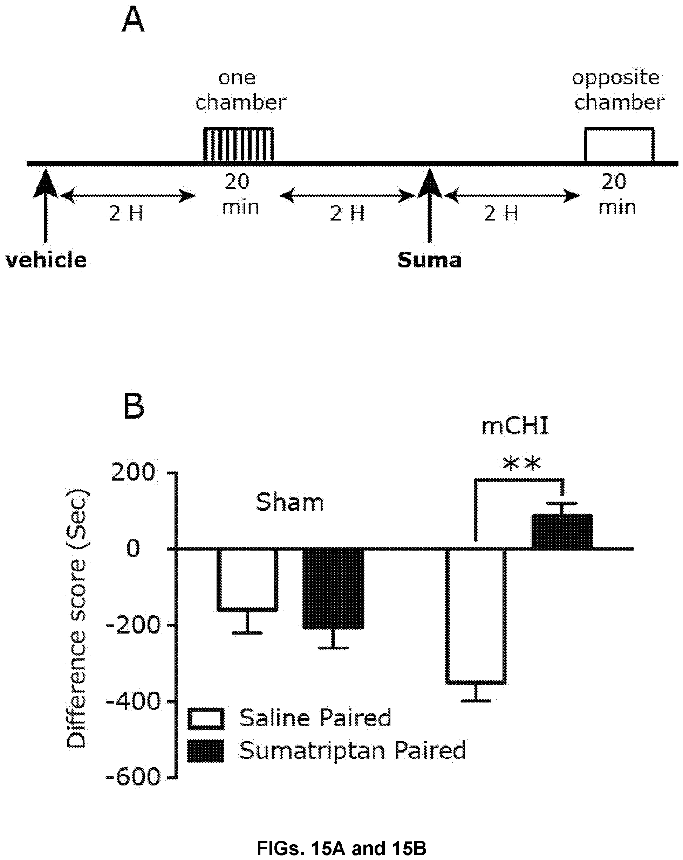

Figures 15A and 15B show treatments protocol for conditioned place preference during conditioning day (Figure 15A) and that sumatriptan produces CPP in animals at 7 days post mCHI but not in sham animals (Figure 15B). Data are mean + SEM (n=8). **p<0.001 saline paired vs. sumatriptan paired.

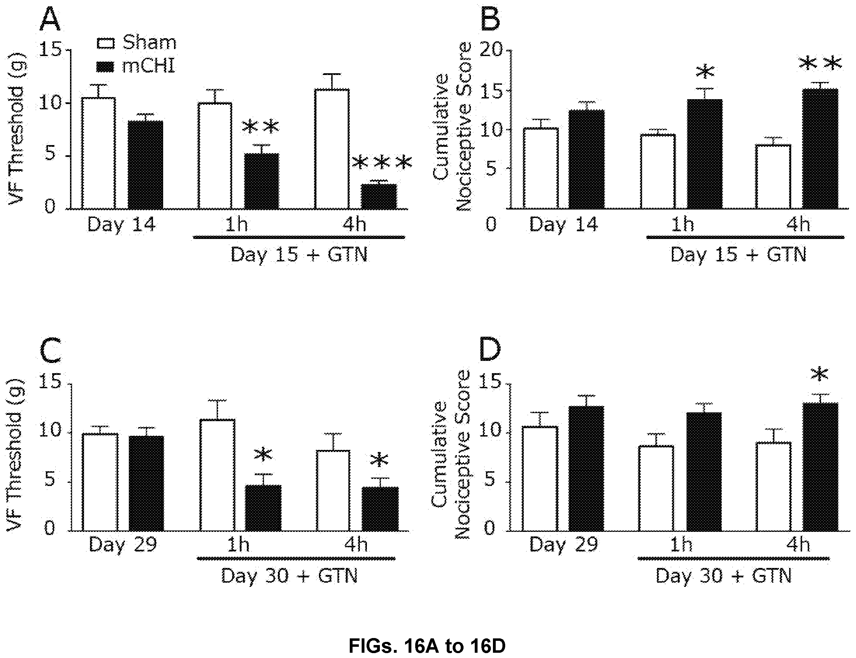

Figures 16A to 16D are four bar graphs that show the effect of GTN administration at Day 15 and Day 30 post mCHI on cephalic mechanical hypersensitivity. Data are mean + SEM (n=8) *p<0.05, **p<0.01 ***p<0.001 vs sham.

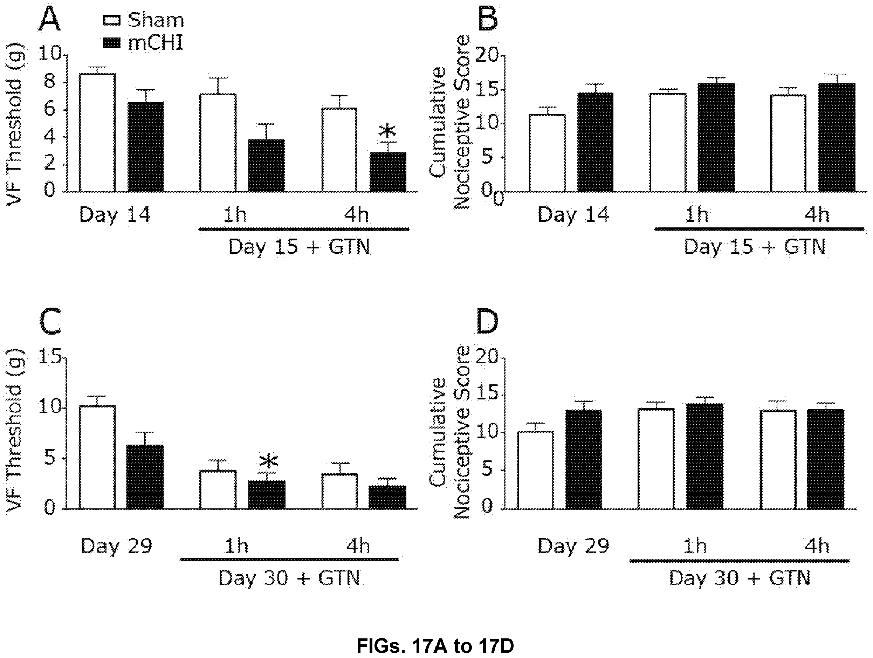

Figures 17A to 17D are four bar graphs that show the effect of GTN administration at Day 15 and Day 30 post mCHI on hindpaw mechanical hypersensitivity. Data are mean + SEM (n=8) *p<0.05 vs sham.

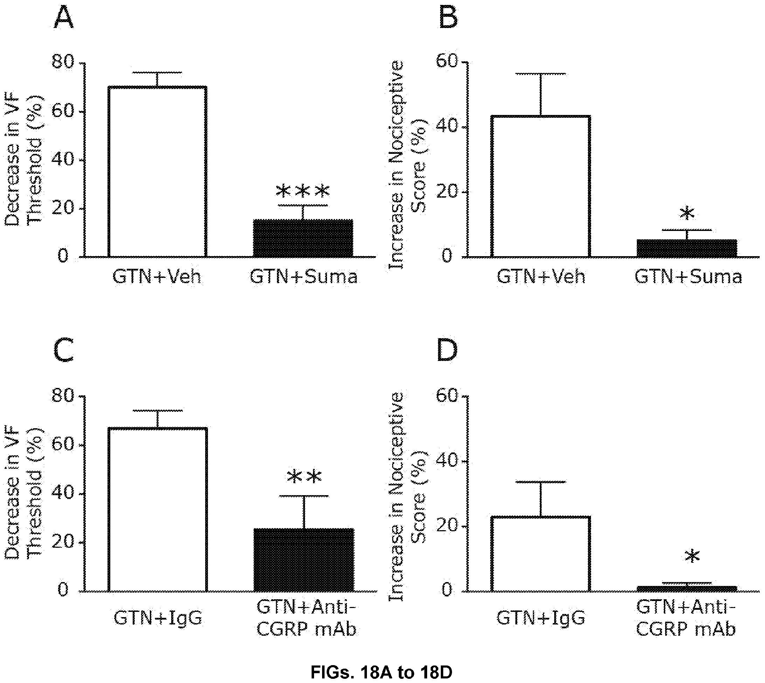

Figures 18A to 18D are four bar graphs that show the effect of acute sumatriptan treatment or chronic administration of anti-CGRP mAb and their control treatments on the % decrease in response thresholds (Figures 18A and 18C) or % increase in nociceptive scores (Figures 18B and 18D) at 4 h post-GTN compared to corresponding pre-GTN Day 14 values. Data are mean + SEM (n=8) *p<0.05, **p<0.01, ***p<0.001 vs GTN + Veh or GTN + IgG.

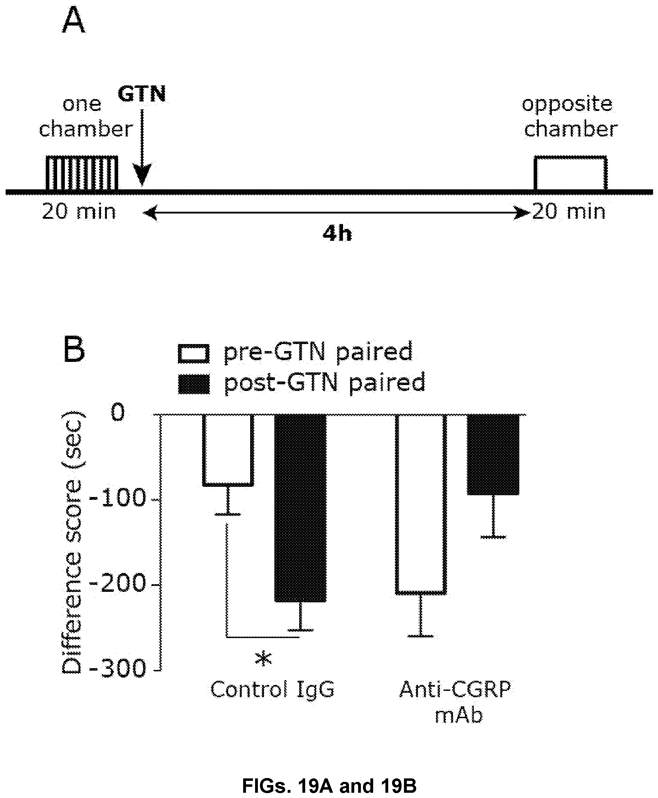

Figures 19A and 19B show treatments protocol for conditioned place aversion during conditioning day (Figure 19A) and that GTN produced placed aversion in the IgG injected mCHI animals on Day 14 post-injury but not in the mCHI animals treated with the anti-CGRP mAb (Figure 19B). Data are mean + SEM (n=8). *p<0.05 pre-GTN-paired chamber vs GTN-paired chamber.

## DETAILED DESCRIPTION

### General Techniques

The practice of the various aspects of the present invention will employ, unless otherwise indicated, conventional techniques of molecular biology (including recombinant techniques), microbiology, cell biology, biochemistry and immunology, which are within the skill of the art. Such techniques are explained fully in the literature, such as, Molecular Cloning: A Laboratory Manual, second edition (Sambrook et al., 1989) Cold Spring Harbor Press; Oligonucleotide Synthesis (M.J. Gait, ed., 1984); Methods in Molecular Biology, Humana Press; Cell Biology: A Laboratory Notebook (J.E. Cellis, ed., 1998) Academic Press; Animal Cell Culture (R.I. Freshney, ed., 1987); Introduction to Cell and Tissue Culture (J.P. Mather and P.E. Roberts, 1998) Plenum Press; Cell and Tissue Culture: Laboratory Procedures (A. Doyle, J.B. Griffiths, and D.G. Newell, eds., 1993-1998) J. Wiley and Sons; Methods in Enzymology (Academic Press, Inc.); Handbook of Experimental Immunology (D.M. Weir and C.C. Blackwell, eds.); Gene Transfer Vectors for Mammalian Cells (J.M. Miller and M.P. Calos, eds., 1987); Current Protocols in Molecular Biology (F.M. Ausubel et al., eds., 1987); PCR: The Polymerase Chain Reaction, (Mullis et al., eds., 1994); Current Protocols in Immunology (J.E. Coligan et al., eds., 1991); Short Protocols in Molecular Biology (Wiley and Sons, 1999); Immunobiology (C.A. Janeway and P. Travers, 1997); Antibodies (P. Finch, 1997); Antibodies: a practical approach (D. Catty., ed., IRL Press, 1988-1989); Monoclonal antibodies: a practical approach (P. Shepherd and C. Dean, eds., Oxford University Press, 2000); Using antibodies: a laboratory manual (E. Harlow and D. Lane (Cold Spring Harbor Laboratory Press, 1999); The Antibodies (M. Zanetti and J.D. Capra, eds., Harwood Academic Publishers, 1995).

### Definitions

As used herein, "about" when used in reference to numerical ranges, cutoffs, or specific values is used to indicate that the recited values may vary by up to as much as 10% from the listed value. Thus, the term "about" is used to encompass variations of $\pm$ 10% or less, variations of $\pm$ 5% or less, variations of $\pm$ 1% or less, variations of $\pm$ 0.5% or less, or variations of $\pm$ 0.1% or less from the specified value.

An "antibody" is an immunoglobulin molecule capable of specific binding to a target, such as a carbohydrate, polynucleotide, lipid, polypeptide, etc., through at least one antigen recognition site, located in the variable region of the immunoglobulin molecule. As used herein, the term encompasses not only intact polyclonal or monoclonal antibodies, but also fragments thereof (such as Fab, Fab', F(ab')$_2$, Fv), single chain (ScFv), mutants thereof, fusion proteins comprising an antibody portion (such as domain antibodies), and any other modified configuration of the immunoglobulin molecule that comprises an antigen recognition site. An antibody includes an antibody of any class, such as IgG, IgA, or IgM (or sub-class thereof), and the antibody need not be of any particular class. Depending on the antibody amino acid sequence of the constant domain of its heavy chains, immunoglobulins can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA1 and IgA2. The heavy-chain constant domains that correspond to the different classes of immunoglobulins are called alpha, delta, epsilon, gamma, and mu, respectively. The subunit structures and three-dimensional configurations of different classes of immunoglobulins are well known.

As used herein, "monoclonal antibody" refers to an antibody obtained from a population of substantially homogeneous antibodies, i.e., the individual antibodies comprising the population are identical except for possible naturally-occurring mutations that may be present in minor amounts. Monoclonal antibodies are highly specific, being directed against a single antigenic site. Furthermore, in contrast to polyclonal antibody preparations, which typically include different antibodies directed against different determinants (epitopes), each monoclonal antibody is directed against a single determinant on the antigen. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler and Milstein, 1975, Nature, 256:495, or may be made by recombinant DNA methods such as described in U.S. Patent No. 4,816,567. The monoclonal antibodies may also be isolated from phage libraries generated using the techniques described in McCafferty et al., 1990, Nature, 348:552-554, for example.

As used herein, "humanized" antibodies refer to forms of non-human (e.g., murine) antibodies that are specific chimeric immunoglobulins, immunoglobulin chains, or fragments thereof (such as Fv, Fab, Fab', F(ab')$_2$ or other antigen-binding subsequences of antibodies) that contain minimal sequence derived from non-human immunoglobulin. For the most part, humanized antibodies are human immunoglobulins (recipient antibody) in which residues from a complementarity determining region (CDR) of the recipient are replaced by residues from a CDR of a non-human species (donor antibody) such as mouse, rat, or rabbit having the desired specificity, affinity, and, biological activity. In some instances, Fv framework region (FR) residues of the human immunoglobulin are replaced by corresponding non-human residues. Furthermore, the humanized antibody may comprise residues that are found neither in the recipient antibody nor in the imported CDR or framework sequences, but are included to further refine and optimize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region or domain (Fc), typically that of a human immunoglobulin. Antibodies may have Fc regions modified as described in WO 99/58572. Other forms of humanized antibodies have one or more CDRs (one, two, three, four, five, six) which are altered with respect to the original antibody, which are also termed one or more CDRs "derived from" one or more CDRs from the original antibody.

As used herein, "human antibody" means an antibody having an amino acid sequence corresponding to that of an antibody produced by a human and/or has been made using any of the techniques for making human antibodies known in the art or disclosed herein. This definition of a human antibody includes antibodies comprising at least one human heavy chain polypeptide or at least one human light chain polypeptide. One such example is an antibody comprising murine light chain and human heavy chain polypeptides. Human antibodies can be produced using various techniques known in the art. In one embodiment, the human antibody is selected from a phage library, where that phage library expresses human antibodies (Vaughan et al., 1996, Nature Biotechnology, 14:309-314; Sheets et al., 1998, PNAS, (USA) 95:6157-6162; Hoogenboom and Winter, 1991, J. Mol. Biol., 227:381; Marks et al., 1991, J. Mol. Biol., 222:581). Human antibodies can also be made by introducing human immunoglobulin loci into transgenic animals, e.g., mice in which the endogenous immunoglobulin genes have been partially or completely inactivated. This approach is described in U.S. Patent Nos. 5,545,807; 5,545,806; 5,569,825; 5,625,126; 5,633,425; and 5,661,016. Alternatively, the human antibody may be prepared by immortalizing human B lymphocytes that produce an antibody directed against a target antigen (such B lymphocytes may be recovered from an individual or may have been immunized in vitro). See, e.g., Cole et al., Monoclonal Antibodies and Cancer Therapy, Alan R. Liss, p. 77 (1985); Boerner et al., 1991, J. Immunol., 147 (1):86-95; and U.S. Patent No. 5,750,373.

As used herein, the term "calcitonin gene-related peptide" and "CGRP" refers to any form of calcitonin gene-related peptide and variants thereof that retain at least part of the activity of CGRP. For example, CGRP may be α-CGRP or β-CGRP. As used herein, CGRP includes all mammalian species of native sequence CGRP, e.g., human, canine, feline, equine, and bovine.

As used herein, an "anti-CGRP antagonist antibody" (interchangeably termed "anti-CGRP antibody") refers to an antibody that is able to bind to CGRP and inhibit CGRP biological activity and/or downstream pathway(s) mediated by CGRP signaling. An anti-CGRP antagonist antibody encompasses antibodies that modulate, block, antagonize, suppress or reduce (including significantly) CGRP biological activity, or otherwise antagonize the CGRP pathway, including downstream pathways mediated by CGRP signaling, such as receptor binding and/or elicitation of a cellular response to CGRP. For purpose of the present invention, it will be explicitly understood that the term "anti-CGRP antagonist antibody" encompasses all the previously identified terms, titles, and functional states and characteristics whereby CGRP itself, CGRP biological activity (including but not limited to its ability to mediate any aspect of headache), or the consequences of the biological activity, are substantially nullified, decreased, or neutralized in any meaningful degree. In some embodiments, an anti-CGRP antagonist antibody binds CGRP and prevents CGRP binding to a CGRP receptor. In other embodiments, an anti-CGRP antibody binds CGRP and prevents activation of a CGRP receptor. Examples of anti-CGRP antagonist antibodies are provided herein.

As used herein, the terms "G1," "antibody G1," and "TEV-48125" are used interchangeably to refer to an anti-CGRP antagonist antibody produced by expression vectors having deposit numbers of ATCC PTA-6867 and ATCC PTA-6866. The amino acid sequence of the heavy chain and light chain variable regions are shown in Figure 5. The CDR portions of antibody G1 (including Chothia and Kabat CDRs) are diagrammatically depicted in Figure 5. The polynucleotides encoding the heavy and light chain variable regions are shown in SEQ ID NO:9 and SEQ ID NO:10. The characterization of G1 is described in the Examples.

The terms "polypeptide", "oligopeptide", "peptide" and "protein" are used interchangeably herein to refer to polymers of amino acids of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other modifications known in the art. It is understood that, because the polypeptides of this invention are based upon an antibody, the polypeptides can occur as single chains or associated chains.

"Polynucleotide," or "nucleic acid," as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after polymerization, such as by conjugation with a labeling component. Other types of modifications include, for example, "caps", substitution of one or more of the naturally occurring nucleotides with an analog, internucleotide modifications such as, for example, those with uncharged linkages (e.g., methyl phosphonates, phosphotriesters, phosphoamidates, carbamates, etc.) and with charged linkages (e.g., phosphorothioates, phosphorodithioates, etc.), those containing pendant moieties, such as, for example, proteins (e.g., nucleases, toxins, antibodies, signal peptides, ply-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl, 2'-fluoro- or 2'-azido-ribose, carbocyclic sugar analogs, $\alpha$-anomeric sugars, epimeric sugars such as arabinose, xyloses or lyxoses, pyranose sugars, furanose sugars, sedoheptuloses, acyclic analogs and abasic nucleoside analogs such as methyl riboside. One or more phosphodiester linkages may be replaced by alternative linking groups. These alternative linking groups include, but are not limited to, embodiments wherein phosphate is replaced by $P(O)S$("thioate"), $P(S)S$ ("dithioate"), $(O)NR_2$ ("amidate"), $P(O)R$, $P(O)OR'$, $CO$ or $CH_2$ ("formacetal"), in which each R or R' is independently H or substituted or unsubstituted alkyl (1-20 C) optionally containing an ether (-O-) linkage, aryl, alkenyl, cycloalkyl, cycloalkenyl or araldyl. Not all linkages in a polynucleotide need be identical. The preceding description applies to all polynucleotides referred to herein, including RNA and DNA.

As used herein, "post-traumatic headache" is a headache attributed to trauma or injury to the head and/or neck, as further described in The International Classification of Headache Disorders, 3rd edition (beta version), Cephalalgia, 33(9): 629-808 (2013). For example, post-traumatic headaches can resemble tension-type headache or migraine. Consequently, their diagnosis is largely dependent on the close temporal relation between the trauma or injury and headache onset. Consistently with those of ICHD-II, the diagnostic criteria of ICHD-3 beta for all subtypes require that headache must be reported to have developed within 7 days of trauma or injury, or within 7 days after regaining consciousness and/or the ability to sense and report pain when these have been lost following trauma or injury. Although this 7-day interval is somewhat arbitrary, and although some experts argue that headache may develop after a longer interval in a minority of patients, there is not enough evidence at this time to change this requirement.

Skilled practitioners will be readily able to recognize a subject with a post-traumatic headache. For example, traumatic injury to the head has occurred, and a headache is reported to have developed within seven days after one of the following: the injury to the head, regaining of consciousness following the injury to the head, or discontinuation of medication(s) that impair ability to sense or report headache following the injury to the head. In some cases, the headache has resolved within 3 months after the injury to the head, or the headache has not yet resolved but 3 months have not yet passed since the injury to the head.

Diagnostic criteria for acute headaches attributed to traumatic injury to the head, which can include headaches of less than 3 months' duration caused by traumatic injury to the head, can include:

A. Any headache fulfilling criteria C and D

B. Traumatic injury to the head has occurred

C. Headache is reported to have developed within 7 days after one of the following:

1. the injury to the head
2. regaining of consciousness following the injury to the head
3. discontinuation of medication(s) that impair ability to sense or report headache following the injury to the head

D. Either of the following:

1. headache has resolved within 3 months after the injury to the head
2. headache has not yet resolved but 3 months have not yet passed since the injury to the head

E. Not better accounted for by another ICHD-3 diagnosis.

Diagnostic criteria for acute headaches attributed to moderate or severe traumatic injury to the head can include injury to the head associated with at least one of the following:

1. loss of consciousness for >30 minutes
2. Glasgow Coma Scale (GCS) score <13
3. post-traumatic amnesia1 lasting >24 hours
4. alteration in level of awareness for >24 hours
5. imaging evidence of a traumatic head injury such as intracranial haemorrhage and/or brain contusion.

Diagnostic criteria for post-traumatic headache attributed to mild traumatic injury to the head can include injury to the head fulfilling both of the following:

1. associated with none of the following:

a) loss of consciousness for >30 minutes
b) Glasgow Coma Scale (GCS) score <13
c) post-traumatic amnesia lasting >24 hours
d) altered level of awareness for >24 hours
e) imaging evidence of a traumatic head injury such as intracranial haemorrhage and/or brain contusion

2. associated, immediately following the head injury, with one or more of the following symptoms and/or signs:

a) transient confusion, disorientation, or impaired consciousness
b) loss of memory for events immediately before or after the head injury
c) two or more other symptoms suggestive of mild traumatic brain injury: nausea, vomiting, visual disturbances, dizziness and/or vertigo, impaired memory and/or concentration.

Persistent post-traumatic headache attributed to traumatic injury to the head is a headache of greater than three months' duration caused by traumatic injury to the head and headache is reported to have developed within 7 days after one of the following: the injury to the head, regaining of consciousness following the injury to the head, or discontinuation of medication(s) that impair ability to sense or report headache following the injury to the head. In some cases, the headache persists for greater than three months after the injury to the head.

Diagnostic criteria for persistent headache attributed to moderate or severe traumatic injury to the head can include injury to the head associated with at least one of the following:

1. loss of consciousness for >30 minutes

2. Glasgow Coma Scale (GCS) score <13
3. post-traumatic amnesia1 lasting >24 hours
4. alteration in level of awareness for >24 hours
5. imaging evidence of a traumatic head injury such as intracranial haemorrhage and/or brain contusion.

Diagnostic criteria for persistent post-traumatic headache attributed to mild traumatic injury to the head can include injury to the head fulfilling both of the following:

1. associated with none of the following:

a) loss of consciousness for >30 minutes
b) Glasgow Coma Scale (GCS) score <13
c) post-traumatic amnesia lasting >24 hours
d) altered level of awareness for >24 hours
e) imaging evidence of a traumatic head injury such as intracranial haemorrhage and/or brain contusion

2. associated, immediately following the head injury, with one or more of the following symptoms and/or signs:

a) transient confusion, disorientation, or impaired consciousness
b) loss of memory for events immediately before or after the head injury
c) two or more other symptoms suggestive of mild traumatic brain injury: nausea, vomiting, visual disturbances, dizziness and/or vertigo, impaired memory and/or concentration.

Traumatic injury to the head is defined as a structural or functional injury resulting from the action of external forces on the head. These include striking the head with or the head striking an object, penetration of the head by a foreign body, forces generated from blasts or explosions, and other forces yet to be defined.

A "variable region" of an antibody refers to the variable region of the antibody light chain or the variable region of the antibody heavy chain, either alone or in combination. The variable regions of the heavy and light chain each consist of four framework regions (FR) connected by three complementarity determining regions (CDRs) also known as hypervariable regions. The CDRs in each chain are held together in close proximity by the FRs and, with the CDRs from the other chain, contribute to the formation of the antigen-binding site of antibodies. There are at least two techniques for determining CDRs: (1) an approach based on cross-species sequence variability (i.e., Kabat et al., Sequences of Proteins of Immunological Interest, (5th ed., 1991, National Institutes of Health, Bethesda MD)); and (2) an approach based on crystallographic studies of antigen-antibody complexes (Al-lazikani et al (1997) J. Molec. Biol. 273:927-948)). As used herein, a CDR may refer to CDRs defined by either approach or by a combination of both approaches.

A "constant region" of an antibody refers to the constant region of the antibody light chain or the constant region of the antibody heavy chain, either alone or in combination.

An epitope that "preferentially binds" or "specifically binds" (used interchangeably herein) to an antibody or a polypeptide is a term well understood in the art, and methods to determine such specific or preferential binding are also well known in the art. A molecule is said to exhibit "specific binding" or "preferential binding" if it reacts or associates more frequently, more rapidly, with greater duration and/or with greater affinity with a particular cell or substance than it does with alternative cells or substances. An antibody "specifically binds" or "preferentially binds" to a target if it binds with greater affinity, avidity, more readily, and/or with greater duration than it binds to other substances. For example, an antibody that specifically or preferentially binds to a CGRP epitope is an antibody that binds this epitope with greater affinity, avidity, more readily, and/or with greater duration than it binds to other CGRP epitopes or non-CGRP epitopes. It is also understood by reading this definition that, for example, an antibody (or moiety or epitope) that specifically or preferentially binds to a first target may or may not specifically or preferentially bind to a second target. As such, "specific binding" or "preferential binding" does not necessarily require (although it can include) exclusive binding. Generally, but not necessarily, reference to binding means preferential binding.

As used herein, "substantially pure" refers to material which is at least 50% pure (i.e., free from contaminants), more preferably at least 90% pure, more preferably at least 95% pure, more preferably at least 98% pure, and more preferably at least 99% pure.

A "host cell" includes an individual cell or cell culture that can be or has been a recipient for vector(s) for incorporation of polynucleotide inserts. Host cells include progeny of a single host cell, and the progeny may not necessarily be completely identical (in morphology or in genomic DNA complement) to the original parent cell due to natural, accidental, or deliberate mutation. A host cell includes cells transfected in vivo with a polynucleotide(s) of this invention.

The term "Fc region" is used to define a C-terminal region of an immunoglobulin heavy chain. The "Fc region" may be a native sequence Fc region or a variant Fc region. Although the boundaries of the Fc region of an immunoglobulin heavy chain might vary, the human IgG heavy chain Fc region is usually defined to stretch from an amino acid residue at position Cys226, or from Pro230, to the carboxyl-terminus thereof. The numbering of the residues in the Fc region is that of the EU index as in Kabat. Kabat et al., Sequences of Proteins of Immunological Interest, 5th Ed. Public Health Service, National Institutes of Health, Bethesda, Md., 1991. The Fc region of an immunoglobulin generally comprises two constant domains, CH2 and CH3.

As used herein, "Fc receptor" and "FcR" describe a receptor that binds to the Fc region of an antibody. The preferred FcR is a native sequence human FcR. Moreover, a preferred FcR is one which binds an IgG antibody (a gamma receptor) and includes receptors of the FcγRI, FcγRII, and FcγRIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. FcRs are reviewed in Ravetch and Kinet, 1991, Ann. Rev. Immunol., 9:457-92; Capel et al., 1994, Immunomethods, 4:25-34; and de Haas et al., 1995, J. Lab. Clin. Med., 126:330-41. "FcR" also includes the neonatal receptor, FcRn, which is responsible for the transfer of maternal IgGs to the fetus (Guyer et al., 1976, J. Immunol., 117:587; and Kim et al., 1994, J. Immunol., 24:249).

"Complement dependent cytotoxicity" and "CDC" refer to the lysing of a target in the presence of complement. The complement activation pathway is initiated by the binding of the first component of the complement system (C1q) to a molecule (e.g., an antibody) complexed with a cognate antigen. To assess complement activation, a CDC assay, e.g., as described in Gazzano-Santoro et al., J. Immunol. Methods, 202:163 (1996), may be performed.

A "functional Fc region" possesses at least one effector function of a native sequence Fc region. Exemplary "effector functions" include C1q binding; complement dependent cytotoxicity (CDC); Fc receptor binding; antibody-dependent cell-mediated cytotoxicity (ADCC); phagocytosis; down-regulation of cell surface receptors (e.g., B cell receptor; BCR), etc. Such effector functions generally require the Fc region to be combined with a binding domain (e.g., an antibody variable domain) and can be assessed using various assays known in the art for evaluating such antibody effector functions.

A "native sequence Fc region" comprises an amino acid sequence identical to the amino acid sequence of an Fc region found in nature. A "variant Fc region" comprises an amino acid sequence which differs from that of a native sequence Fc region by virtue of at least one amino acid modification, yet retains at least one effector function of the native sequence Fc region. Preferably, the variant Fc region has at least one amino acid substitution compared to a native sequence Fc region or to the Fc region of a parent polypeptide, e.g., from about one to about ten amino acid substitutions, and preferably from about one to about five amino acid substitutions in a native sequence Fc region or in the Fc region of the parent polypeptide. The variant Fc region herein will preferably possess at least about 80% sequence identity with a native sequence Fc region and/or with an Fc region of a parent polypeptide, and most preferably at least about 90% sequence identity therewith, more preferably at least about 95%, at least about 96%, at least about 97%, at least about 98%, at least about 99% sequence identity therewith.

As used herein "antibody-dependent cell-mediated cytotoxicity" and "ADCC" refer to a cell-mediated reaction in which nonspecific cytotoxic cells that express Fc receptors (FcRs) (e.g., natural killer (NK) cells, neutrophils, and macrophages) recognize bound antibody on a target cell and subsequently cause lysis of the target cell. ADCC activity of a molecule of interest can be assessed using an in vitro ADCC assay, such as that described in U.S. Patent No. 5,500,362 or 5,821,337. Useful effector cells for such assays include peripheral blood mononuclear cells (PBMC) and NK cells. Alternatively, or additionally, ADCC activity of the molecule of interest may be assessed in vivo, e.g., in an animal model such as that disclosed in Clynes et al., 1998, PNAS (USA), 95:652-656.

As used herein, "preventing" is an approach to stop (persistent) PTH from occurring or existing in a subject, who does not already have (persistent) PTH. As used herein, "treatment" is an approach for obtaining beneficial or desired clinical results. For purposes of this invention, beneficial or desired clinical results include, but are not limited to, one or more of the following: improvement in any aspect of a post-traumatic headache including lessening severity, alleviation of pain intensity, and other associated symptoms, reducing frequency of recurrence, increasing the quality of life of those suffering from the post-traumatic headache, and decreasing dose of other medications required to treat the post-traumatic headache.

"Reducing incidence" of post-traumatic headache means any of reducing severity (which can include reducing need for and/or amount of (e.g., exposure to) other drugs and/or therapies generally used for this condition, including, for example, ergotamine, dihydroergotamine, or triptans), duration, and/or frequency (including, for example, delaying or increasing time to next episodic attack in an individual). As is understood by those skilled in the art, individuals may vary in terms of their response to treatment, and, as such, for example, a "method of reducing incidence of post-traumatic headache in an individual" reflects administering the anti-CGRP antagonist antibody based on a reasonable expectation that such administration may likely cause such a reduction in incidence in that particular individual.

"Ameliorating" post-traumatic headache or one or more symptoms of post-traumatic headache means a lessening or improvement of one or more symptoms of post-traumatic headache as compared to not administering an anti-CGRP antagonist antibody. "Ameliorating" also includes shortening or reduction in duration of a symptom.

As used herein, "controlling post-traumatic headache" refers to maintaining or reducing severity or duration of one or more symptoms of post-traumatic headache or frequency of post-traumatic headache attacks in an individual (as compared to the level before treatment). For example, the duration or severity of head and/or neck pain, or frequency of attacks is reduced by at least about any of 10%, 20%, 30%, 40%, 50%, 60%, or 70% in the individual as compared to the level before treatment.

As used herein, a "headache hour" refers to an hour during which a subject experiences headache. Headache hours can be expressed in terms of whole hours (e.g., one headache hour, two headache hours, three headache hours, etc.) or in terms of whole and partial hours (e.g., 0.5 headache hours, 1.2 headache hours, 2.67 headache hours, etc.). One or more headache hours may be described with respect to a particular time interval. For example, "daily headache hours" may refer to the number of headache hours a subject experiences within a day interval (e.g., a 24-hour period). In another example, "weekly headache hours" may refer to the number of headache hours a subject experiences within a week interval (e.g., a 7-day period). As can be appreciated, a week interval may or may not correspond to a calendar week. In another example, "monthly headache hours" may refer to the number of headache hours a subject experiences within a month interval. As can be appreciated, a month interval (e.g., a period of 28, 29, 30, or 31 days) may vary in terms of number of days depending upon the particular month and may or may not correspond to a calendar month. In yet another example, "yearly headache hours" may refer to the number of headache hours a subject experiences within a year interval. As can be appreciated, a year interval (e.g., a period of 365 or 366 days) may vary in terms of number of days depending upon the particular year and may or may not correspond to a calendar year.

As used herein, a "headache day" refers to a day during which a subject experiences headache. Headache days can be expressed in terms of whole days (e.g., one headache day, two headache days, three headache days, etc.) or in terms of whole and partial days (e.g., 0.5 headache days, 1.2 headache days, 2.67 headache days, etc.). One or more headache days may be described with respect to a particular time interval. For example, "weekly headache days" may refer to the number of headache days a subject experiences within a week interval (e.g., a 7-day period). As can be appreciated, a week interval may or may not correspond to a calendar week. In another example, "monthly headache days" may refer to the number of headache days a subject experiences within a month interval. As can be appreciated, a month interval (e.g., a period of 28, 29, 30, or 31 days) may vary in terms of number of days depending upon the particular month and may or may not correspond to a calendar month. In yet another example, "yearly headache days" may refer to the number of headache days a subject experiences within a year interval. As can be appreciated, a year interval (e.g., a period of 365 or 366 days) may vary in terms of number of days depending upon the particular year and may or may not correspond to a calendar year.

As used therein, "delaying" the development of post-traumatic headache means to defer, hinder, slow, retard, stabilize, and/or postpone progression of the disease. This delay can be of varying lengths of time, depending on the history of the disease and/or individuals being treated. As is evident to one skilled in the art, a sufficient or significant delay can, in effect, encompass prevention, in that the individual does not develop post-traumatic headache. A method that "delays" development of the symptom is a method that reduces probability of developing the symptom in a given time frame and/or reduces extent of the symptoms in a given time frame, when compared to not using the method. Such comparisons are typically based on clinical studies, using a statistically significant number of subjects.

"Development" or "progression" of post-traumatic headache means initial manifestations and/or ensuing progression of the disorder. Development of post-traumatic headache can be detectable and assessed using standard clinical techniques as well known in the art. However, development also refers to progression that may be undetectable. For purpose of this disclosure, development or progression refers to the biological course of the symptoms. "Development" includes occurrence, recurrence, and onset. As used herein "onset" or "occurrence" of post-traumatic headache includes initial onset and/or recurrence.

As used herein, an "effective dosage" or "effective amount" of drug, compound, or pharmaceutical composition is an amount sufficient to effect beneficial or desired results. For prophylactic use, beneficial or desired results include results such as eliminating or reducing the risk, lessening the severity, or delaying the onset of the disease, including biochemical, histological and/or behavioral symptoms of the disease, its complications and intermediate pathological phenotypes presenting during development of the disease. For therapeutic use, beneficial or desired results include clinical results such as reducing pain intensity, duration, or frequency of post-traumatic headache attack, and decreasing one or more symptoms resulting from post-traumatic headache (biochemical, histological and/or behavioral), including its complications and intermediate pathological phenotypes presenting during development of the disease, increasing the quality of life of those suffering from the disease, decreasing the dose of other medications required to treat the disease, enhancing effect of another medication, and/or delaying the progression of the disease of patients. An effective dosage can be administered in one or more administrations. For purposes of this disclosure, an effective dosage of drug, compound, or pharmaceutical composition is an amount sufficient to accomplish prophylactic or therapeutic treatment either directly or indirectly. As is understood in the clinical context, an effective dosage of a drug, compound, or pharmaceutical composition may or may not be achieved in conjunction with another drug, compound, or pharmaceutical composition. Thus, an "effective dosage" may be considered in the context of administering one or more therapeutic agents, and a single agent may be considered to be given in an effective amount if, in conjunction with one or more other agents, a desirable result may be or is achieved.

An "individual" or a "subject" is a mammal, more preferably a human. Mammals also include, but are not limited to, farm animals, sport animals, pets, primates, horses, dogs, cats, mice and rats.

As used herein, "vector" means a construct, which is capable of delivering, and preferably expressing, one or more gene(s) or sequence(s) of interest in a host cell. Examples of vectors include, but are not limited to, viral vectors, naked DNA or RNA expression vectors, plasmid, cosmid or phage vectors, DNA or RNA expression vectors associated with cationic condensing agents, DNA or RNA expression vectors encapsulated in liposomes, and certain eukaryotic cells, such as producer cells.

As used herein, "expression control sequence" means a nucleic acid sequence that directs transcription of a nucleic acid. An expression control sequence can be a promoter, such as a constitutive or an inducible promoter, or an enhancer. The expression control sequence is operably linked to the nucleic acid sequence to be transcribed.

As used herein, "pharmaceutically acceptable carrier" or "pharmaceutical acceptable excipient" includes any material which, when combined with an active ingredient, allows the ingredient to retain biological activity and is non-reactive with the subject's immune system. Examples include, but are not limited to, any of the standard pharmaceutical carriers such as a phosphate buffered saline solution, water, emulsions such as oil/water emulsion, and various types of wetting agents. Preferred diluents for aerosol or parenteral administration are phosphate buffered saline or normal (0.9%) saline. Compositions comprising such carriers are formulated by well-known conventional methods (see, for example, Remington's Pharmaceutical Sciences, 18th edition, A. Gennaro, ed., Mack Publishing Co., Easton, PA, 1990; and Remington, The Science and Practice of Pharmacy 20th Ed. Mack Publishing, 2000).

The term "$k_{on}$", as used herein, is intended to refer to the rate constant for association of an antibody to an antigen.

The term "$k_{off}$", as used herein, is intended to refer to the rate constant for dissociation of an antibody from the antibody/antigen complex.

The term "$K_D$", as used herein, is intended to refer to the equilibrium dissociation constant of an antibody-antigen interaction.

A. Methods for preventing, treating, or reducing post-traumatic headache and/or at least one secondary symptom associated with post-traumatic headache

Disclosed herein are methods of preventing, treating, or reducing incidence of (persistent) post-traumatic headache in a subject. Also disclosed herein is a method of treating or reducing incidence of at least one secondary symptom associated with post-traumatic headache in a subject.

Also disclosed herein are methods for preventing, ameliorating, controlling, reducing incidence of, or delaying the development or progression of (persistent) post-traumatic headache in an individual or symptoms associated with (persistent) post-traumatic headache (e.g., diarrhea, light sensitivity, fever, stiff neck, nausea, cognitive impairment and/or vomiting) comprising administering to the individual an effective amount of an antibody that modulates the CGRP pathway or an anti-CGRP antagonist antibody in combination with at least one additional agent useful for preventing, treating, or reducing (persistent) post-traumatic headache.

Such additional agents include, but are not limited to, 5-HT agonists and NSAIDs. For example, the antibody and the at least one additional agent can be concomitantly administered, i.e., they can be given in close enough temporal proximity to allow their individual therapeutic effects to overlap. For example, the amount of 5-HT agonist or NSAID administered in combination with an anti-CGRP antibody should be sufficient to reduce the frequency of (persistent) post-traumatic headache relapse in patients or produce longer lasting efficacy compared to the administration of either one of these agents in the absence of the other.

Additional non-limiting examples of additional agents that may be administered in combination with an anti-CGRP antagonist antibody include one or more of:

(i) an opioid analgesic, e.g., morphine, heroin, hydromorphone, oxymorphone, levorphanol, levallorphan, methadone, meperidine, fentanyl, cocaine, codeine, dihydrocodeine, oxycodone, hydrocodone, propoxyphene, nalmefene, nalorphine, naloxone, naltrexone, buprenorphine, butorphanol, nalbuphine or pentazocine;
(ii) a nonsteroidal antiinflammatory drug (NSAID), e.g., aspirin, diclofenac, diflusinal, etodolac, fenbufen, fenoprofen, flufenisal, flurbiprofen, ibuprofen, indomethacin, ketoprofen, ketorolac, meclofenamic acid, mefenamic acid, nabumetone, naproxen, oxaprozin, phenylbutazone, piroxicam, sulindac, tolmetin or zomepirac, cyclooxygenase-2 (COX-2) inhibitors, celecoxib; rofecoxib; meloxicam; JTE-522; L-745,337; NS398; or a pharmaceutically acceptable salt thereof;
(iii) a barbiturate sedative, e.g., amobarbital, aprobarbital, butabarbital, butabital, mephobarbital, metharbital, methohexital, pentobarbital, phenobartital, secobarbital, talbutal, theamylal or thiopental or a pharmaceutically acceptable salt thereof;

(iv) a barbiturate analgesic, e.g., butalbital or a pharmaceutically acceptable salt thereof or a composition comprising butalbital.

(v) a benzodiazepine having a sedative action, e.g., chlordiazepoxide, clorazepate, diazepam, flurazepam, lorazepam, oxazepam, temazepam, or triazolam or a pharmaceutically acceptable salt thereof;

(vi) an $H_1$ antagonist having a sedative action, e.g., diphenhydramine, pyrilamine, promethazine, chlorpheniramine, or chlorcyclizine or a pharmaceutically acceptable salt thereof;

(vii) a sedative such as glutethimide, meprobamate, methaqualone or dichloralphenazone or a pharmaceutically acceptable salt thereof;

(viii) a skeletal muscle relaxant, e.g., baclofen, carisoprodol, chlorzoxazone, cyclobenzaprine, methocarbamol or orphrenadine or a pharmaceutically acceptable salt thereof;

(ix) an NMDA receptor antagonist, e.g., dextromethorphan ((+)-3-hydroxy-N-methylmorphinan) or its metabolite dextrorphan ((+)-3-hydroxy-N-methylmorphinan), ketamine, memantine, pyrroloquinoline quinone or cis-4-(phosphonomethyl)-2-piperidinecarboxylic acid or a pharmaceutically acceptable salt thereof;

(x) an alpha-adrenergic, e.g., doxazosin, tamsulosin, clonidine or 4-amino-6,7-dimethoxy-2-(5-methanesulfonamido-1,2,3,4-tetrahydroisoquinol-2-yl)-5-(2-pyridyl) quinazoline;

(xi) a tricyclic antidepressant, e.g., desipramine, imipramine, amytriptiline or nortriptiline;

(xii) an anticonvulsant, e.g., carbamazepine or valproate;

(xiii) a tachykinin (NK) antagonist, particularly an NK-3, NK-2 or NK-1 antagonist, e.g., ($\alpha$R,9R)-7-[3,5-bis(trifluoromethyl)benzyl]-8,9,10,11-tetrahydro-9-methyl-5-(4-methylphenyl)-7H-[1,4]diazocino[2,1-g][1,7]naphthridine-6-13-dione (TAK-637), 5-[[(2R,3S)-2-[(1R)-1-[3,5-bis(trifluoromethyl)phenyl]ethoxy-3-(4-fluorophenyl)-4-morpholinyl]methyl]-1,2-dihydro-3H-1,2,4-triazol-3-one (MK-869), lanepitant, dapitant or 3-[[2-methoxy-5-(trifluoromethoxy)phenyl]methylamino]-2-phenyl-piperidine (2S,3S);

(xiv) a muscarinic antagonist, e.g., oxybutin, tolterodine, propiverine, tropsium chloride or darifenacin;

(xv) a COX-2 inhibitor, e.g., celecoxib, rofecoxib or valdecoxib;

(xvi) a non-selective COX inhibitor (preferably with GI protection), e.g., nitroflurbiprofen (HCT-1026);

(xvii) a coal-tar analgesic, in particular paracetamol;

(xviii) a neuroleptic such as droperidol;

(xix) a vanilloid receptor agonist (e.g., resinferatoxin) or antagonist (e.g., capsazepine);

(xx) a beta-adrenergic such as propranolol;

(xxi) a local anaesthetic, such as mexiletine;

(xxii) a corticosteroid, such as dexamethasone;

(xxiii) a serotonin receptor agonist or antagonist;

(xxiv) a cholinergic (nicotinic) analgesic;

(xxv) Tramadol (trade mark);

(xxvi) a PDEV inhibitor, such as sildenafil, vardenafil or taladafil;

(xxvii) an alpha-2-delta ligand such as gabapentin or pregabalin;

(xxviii) a canabinoid; and

(xxix) an antidepressant, such as amitriptyline (Elavil), trazodone (Desyrel), and imipramine (Tofranil) or anticonvulsants such as phenytoin (Dilantin) or carbamazepine (Tegretol).

Those skilled in the art will be able to determine appropriate dosage amounts for particular agents to be used in combination with an anti-CGRP antibody. For example, sumatriptan may be administered in a dosage from about 0.01 to about 300 mg. In some cases, sumatriptan may be administered in a dosage from about 2 mg to about 300 mg, e.g., about 5 mg to about 250 mg, about 5 mg to about 200 mg, about 5 mg to about 100 mg, about 5 mg to about 50 mg, or about 5 mg to about 25 mg. When administered non-parenterally, the typical dosage of sumatriptan is from about 25 to about 100 mg with about 50 mg being generally preferred, e.g., about 45 mg, about 55 mg, or about 60 mg. When sumatriptan is administered parenterally, the preferred dosage is about 6 mg, e.g., about 5 mg, about 7 mg, or about 8 mg. However, these dosages may be varied according to methods standard in the art so that they are optimized for a particular patient or for a particular combination therapy. Further, for example, celecoxib may be administered in an amount of between 50 and 500 mg, e.g., about 50 mg to about 400 mg, about 50 mg to about 300 mg, about 50 mg to about 200 mg, about 50 mg to about 100 mg, about 100 mg to about 400 mg, or about 200 mg to about 300 mg.

In some embodiments, the amount of the monoclonal antibody administered on each of the plurality of days may be between 0.1 mg - 5000 mg, 1 mg - 5000 mg, 10 mg - 5000 mg, 100 mg - 5000 mg, 1000 mg - 5000 mg, 0.1 mg - 4000 mg, 1 mg - 4000 mg, 10 mg - 4000 mg, 100 mg - 4000 mg, 1000 mg - 4000 mg, 0.1 mg - 3000 mg, 1 mg - 3000 mg, 10 mg - 3000 mg, 100 mg - 3000 mg, 1000 mg - 3000 mg, 0.1 mg - 2000 mg, 1 mg - 2000 mg, 10 mg - 2000 mg, 100 mg - 2000 mg, 1000 mg - 2000 mg, 0.1 mg - 1000 mg, 1 mg -1000 mg, 10 mg - 1000 mg or 100 mg - 1000 mg. In some embodiments, the amount is between about 225 mg and about 1000 mg, e.g., about 675 mg or about 900 mg. An exemplary dosing regimen comprises administering an initial antibody dose of about 675 mg subcutaneously, followed by a monthly antibody dose of about 225 mg subcutaneously for about two months, e.g., about three months, four months, five months, six months, or 12 months. Yet another dosing regimen comprises administering an initial antibody dose of about 900 mg intravenously in an infusion over about 60 minutes, followed by doses of about 900 mg administered intravenously in an infusion over about 60 minutes every quarter for one year, two years, three years, four years, or five years. However, other dosage regimens may be useful, depending on the pattern of pharmacokinetic decay that the practitioner wishes to achieve. In some embodiments, the initial dose and one or more of the additional doses are administered the same way, e.g., subcutaneously or intravenously. In some embodiments, the one or more additional doses are administered in a different way than the initial dose, e.g., the initial dose may be administered intravenously and the one or more additional doses may be administered subcutaneously.

In some embodiments, the single dose may be an amount of antibody between 0.1 mg - 5000 mg, 1 mg - 5000 mg, 10 mg -5000 mg, 100 mg - 5000 mg, 1000 mg - 5000 mg, 0.1 mg - 4000 mg, 1 mg - 4000 mg, 10 mg - 4000 mg, 100 mg - 4000 mg, 1000 mg - 4000 mg, 0.1 mg - 3000 mg, 1 mg - 3000 mg, 10 mg - 3000 mg, 100 mg - 3000 mg, 1000 mg - 3000 mg, 0.1 mg - 2000 mg, 1 mg - 2000 mg, 10 mg - 2000 mg, 100 mg - 2000 mg, 1000 mg - 2000 mg, 0.1 mg - 1000 mg, 1 mg -1000 mg, 10 mg - 1000 mg or 100 mg - 1000 mg. In some embodiments, the single dose may be an amount of antibody between 225 mg and about 1000 mg, e.g., about 675 mg or about 900 mg.

In some embodiments, the single dose may be an amount of antibody between 0.1 mg - 5000 mg, 1 mg - 5000 mg, 10 mg -5000 mg, 100 mg - 5000 mg, 1000 mg - 5000 mg, 0.1 mg - 4000 mg, 1 mg - 4000 mg, 10 mg - 4000 mg, 100 mg - 4000 mg, 1000 mg - 4000 mg, 0.1 mg - 3000 mg, 1 mg - 3000 mg, 10 mg - 3000 mg, 100 mg - 3000 mg, 1000 mg - 3000 mg, 0.1 mg - 2000 mg, 1 mg - 2000 mg, 10 mg - 2000 mg, 100 mg - 2000 mg, 1000 mg - 2000 mg, 0.1 mg - 1000 mg, 1 mg -1000 mg, 10 mg - 1000 mg or 100 mg - 1000 mg. In some embodiments, the monthly dose may be an amount of antibody between about 225 mg and about 1000 mg, e.g., about 675 mg or about 900 mg. An exemplary dosing regimen comprises administering an initial antibody dose of about 675 mg subcutaneously, followed by a monthly antibody dose of about 225 mg subcutaneously for about two months, e.g., about three months, four months, five months, six months, or 12 months. Yet another dosing regimen comprises administering an initial antibody dose of about 900 mg intravenously in an infusion over about 60 minutes, followed by doses of about 900 mg administered intravenously in an infusion over about 60 minutes every quarter for one year, two years, three years, four years, or five years. However, other dosage regimens may be useful, depending on the pattern of pharmacokinetic decay that the practitioner wishes to achieve. In some embodiments, the initial dose and one or more of the additional doses are administered the same way, e.g., subcutaneously or intravenously. In some embodiments, the one or more additional doses are administered in a different way than the initial dose, e.g., the initial dose may be administered intravenously and the one or more additional doses may be administered subcutaneously.

With respect to all methods described herein, references to antibodies (e.g., monoclonal antibodies that modulate the CGRP pathway, anti-CGRP antagonist antibodies, monoclonal anti-CGRP antagonist antibodies) also include compositions comprising one or more of these agents. Accordingly, such a composition may be used according to a method referring to an antibody described herein. These compositions may further comprise suitable excipients, such as pharmaceutically acceptable excipients as described elsewhere herein. The present invention can be used alone or in combination with other conventional methods of treatment.

A monoclonal anti-CGRP antagonist antibody can be administered to an individual or subject in any therapeutic dose, via any suitable route and in any suitable formulation. It should be apparent to a person skilled in the art that the examples described herein are not intended to be limiting but to be illustrative of the techniques available. Accordingly, in some embodiments, an antibody described herein can be administered to a subject in accord with known methods, such as intravenous administration, e.g., as a bolus or by continuous infusion over a period of time, e.g., about 10 minutes, about 20 minutes, about 30 minutes, about 40 minutes, about 50 minutes, about 60 minutes, about 90 minutes, about 120 minutes, about 180 minutes, or about 240 minutes. The antibody described herein can also be administered to the subject by subcutaneous, intramuscular, intraperitoneal, intracerebrospinal, intra-articular, sublingually, intra-arterial, intrasynovial, via insufflation, intrathecal, oral, inhalation, intranasal (e.g., with or without inhalation), buccal, rectal, transdermal, intracardiac, intraosseous, intradermal, transmucosal, vaginal, intravitreal, peri-articular, local, epicutaneous, or topical routes. Administration can be systemic, e.g., intravenous administration, or localized. Commercially available nebulizers for liquid formulations, including jet nebulizers and ultrasonic nebulizers are useful for administration. Liquid formulations can be directly nebulized and lyophilized powder can be nebulized after reconstitution. Alternatively, an antibody described herein can be aerosolized using a fluorocarbon formulation and a metered dose inhaler, or inhaled as a lyophilized and milled powder.

In some embodiments, an antibody can be administered via site-specific or targeted local delivery techniques. Examples of site-specific or targeted local delivery techniques include various implantable depot sources of the antibody or local delivery catheters, such as infusion catheters, an indwelling catheter, or a needle catheter, synthetic grafts, adventitial wraps, shunts and stents or other implantable devices, site specific carriers, direct injection, or direct application. See e.g., PCT Publication No. WO 00/53211 and U.S. Patent No. 5,981,568.

Various formulations of an antibody may be used for administration. In some embodiments, an antibody may be administered neat. In some embodiments, antibody and a pharmaceutically acceptable excipient may be in various formulations. Pharmaceutically acceptable excipients are known in the art, and are relatively inert substances that facilitate administration of a pharmacologically effective substance. For example, an excipient can give form or consistency, or act as a diluent. Suitable excipients include but are not limited to stabilizing agents, wetting and emulsifying agents, salts for varying osmolarity, encapsulating agents, buffers, and skin penetration enhancers. Excipients as well as formulations for parenteral and nonparenteral drug delivery are set forth in Remington, The Science and Practice of Pharmacy 20th Ed. Mack Publishing (2000).

In some embodiments, these agents, may be formulated for administration by injection (e.g., intravenously, subcutaneously, intraperitoneally, intramuscularly, etc.). Accordingly, these agents can be combined with pharmaceutically acceptable vehicles such as saline, Ringer's solution, dextrose solution, and the like. The particular dosage regimen, i.e., dose, timing and repetition, will depend on the particular individual and that individual's medical history.

In some embodiments, these agents, may be formulated for peripheral administration. Such formulations can be administered peripherally via any suitable peripheral route, including intravenously and subcutaneously. An agent prepared for peripheral administration can include a substance, medicament, and/or antibody that is not delivered centrally, spinally, intrathecally, or directly into the CNS. Non-limiting examples of peripheral administration routes include a route which is oral, sublingual, buccal, topical, rectal, via inhalation, transdermal, subcutaneous, intravenous, intra-arterial, intramuscular, intracardiac, intraosseous, intradermal, intraperitoneal, transmucosal, vaginal, intravitreal, intra-articular, peri-articular, local, or epicutaneous.

Therapeutic formulations of the antibodies used in accordance with the present disclosure can be prepared for storage and/or use by mixing an antibody having the desired degree of purity with optional pharmaceutically acceptable carriers, excipients or stabilizers (Remington, The Science and Practice of Pharmacy 20th Ed. Mack Publishing (2000)), and can in some cases be in the form of lyophilized formulations or aqueous solutions. Acceptable carriers, excipients, or stabilizers are nontoxic to recipients at the dosages and concentrations employed. A therapeutic formulation of an antibody may comprise one or more pharmaceutically acceptable carriers, excipients or stabilizes with non-limiting examples of such species that include buffers such as phosphate, citrate, and other organic acids; salts such as sodium chloride; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens, such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids (e.g., at concentrations of 0.1 mM to 100 mM, 0.1 mM to 1 mM, 0.01 mM to 50 mM, 1 mM to 50 mM, 1 mM to 30 mM, 1 mM to 20 mM, 10 mM to 25 mM) such as glycine, glutamine, methionine, asparagine, histidine, arginine, or lysine; monosaccharides, disaccharides, and other carbohydrates including glucose, mannose, or dextrins; chelating agents (e.g., at concentrations of 0.001 mg/mL to 1 mg/mL, 0.001 mg/mL to 1 mg/mL, 0.001 mg/mL to 0.1 mg/mL, 0.001 mg/mL to 0.01 mg/mL, 0.01 mg/mL to 0.1 mg/mL) such as EDTA (e.g., disodium EDTA dihydrate); sugars (e.g., at concentrations of 1 mg/mL to 500 mg/mL, 10 mg/mL to 200 mg/mL, 10 mg/mL to 100 mg/mL, 50 mg/mL to 150 mg/mL) such as sucrose, mannitol, trehalose or sorbitol; salt-forming counter-ions such as sodium; metal complexes (e.g., Zn-protein complexes); and/or non-ionic surfactants (e.g., at concentrations of 0.01 mg/mL to 10 mg/mL, 0.01 mg/mL to 1 mg/mL, 0.1 mg/mL to 1 mg/mL, 0.01 mg/mL to 0.5 mg/mL) such as TWEEN[3] (e.g., polysorbate (e.g., polysorbate 20, polysorbate 40, polysorbate 60, polysorbate 80)), PLURONICS[3] or polyethylene glycol (PEG).

An antibody formulation may be characterized in terms of any of a variety of physical properties. For example, a liquid antibody formulation may have any suitable pH for therapeutic efficacy, safety and storage. For example, the pH of a liquid antibody formulation may be from pH 4 to about pH 9, from about pH 5 to about pH 8, from about pH 5 to about pH 7 or from about pH 6 to about pH 8. In some embodiments, a liquid antibody formulation may have a pH of about 3.0, 3.5, 4.0, 4.5, 5.0, 5.5, 6.0, 6.5, 7.0, 7.5, 8.0, 8.5, 9.0, 9.5, or about 10 or higher or lower.

In another example, a liquid antibody formulation may have any suitable viscosity for therapeutic efficacy, safety and storage. For example, the viscosity of a liquid antibody formulation may be from about 0.5 centipoise (cP) to about 100 cP, about 1 cP to about 50 cP, about 1 cP to about 20 cP, about 1 cP to about 15 cP, or about 5 cP to about 15 cP at 25ºC. In some embodiments, a liquid antibody formulation may have a viscosity of about 0.5 cP, 1 cP, 1.2 cP, 1.4 cP, 1.6 cP, 1.8 cP, 2.0 cP, 2.2 cP, 2.4 cP, 2.6 cP, 2.8 cP, 3.0 cP, 3.2 cP, 3.4 cP, 3.6 cP, 3.8 cP, 4.0 cP, 4.2 cP, 4.4 cP, 4.6 cP, 4.8 cP, 5.0 cP, 5.2 cP, 5.4 cP, 5.6 cP, 5.8 cP, 6.0 cP, 6.2 cP, 6.4 cP, 6.6 cP, 6.8 cP, 7.0 cP, 7.2 cP, 7.4 cP, 7.6 cP, 7.8 cP, 8.0 cP, 8.2 cP, 8.4 cP, 8.6 cP, 8.8 cP, 9.0 cP, 9.2 cP, 9.4 cP, 9.6 cP, 9.8 cP, 10.0 cP, 10.2 cP, 10.4 cP, 10.6 cP, 10.8 cP, 11.0 cP, 11.2 cP, 11.4 cP, 11.6 cP, 11.8 cP, 12.0 cP, 12.2 cP, 12.4 cP, 12.6 cP, 12.8 cP, 13.0 cP, 13.2 cP, 13.4 cP, 13.6 cP, 13.8 cP, 14.0 cP, 14.2 cP, 14.4 cP, 14.6 cP, 14.8 cP, or about 15.0 cP at 25ºC or the viscosity may be higher or lower.

In another example, a liquid antibody formulation may have any suitable conductivity for therapeutic efficacy, safety and storage. For example, the conductivity of a liquid antibody formulation may be from about 0.1 millisiemens per centimeter (mS/cm) to about 15 mS/cm, 0.1 mS/cm to 10 mS/cm, 0.1 mS/cm to 5 mS/cm, 0.1 mS/cm to 2 mS/cm or 0.1 mS/cm to 1.5 mS/cm. In some embodiments, a liquid antibody formulation may have a conductivity of 0.19 mS/cm, 0.59 mS/cm, 1.09 mS/cm, 1.19 mS/cm, 1.29 mS/cm, 1.39 mS/cm, 1.49 mS/cm, 1.59 mS/cm, 1.69 mS/cm, 1.79 mS/cm, 1.89 mS/cm, 1.99 mS/cm, 2.09 mS/cm, 2.19 mS/cm, 2.29 mS/cm, 2.39 mS/cm, 2.49 mS/cm, 2.59 mS/cm, 2.69 mS/cm, 2.79 mS/cm, 2.89 mS/cm, 2.99 mS/cm, 3.09 mS/cm, 3.19 mS/cm, 3.29 mS/cm, 3.39 mS/cm, 3.49 mS/cm, 3.59 mS/cm, 3.69 mS/cm, 3.79 mS/cm, 3.89 mS/cm, 3.99 mS/cm, 4.09 mS/cm, 4.19 mS/cm, 4.29 mS/cm, 4.39 mS/cm, 4.49 mS/cm, 4.59 mS/cm, 4.69 mS/cm, 4.79 mS/cm, 4.89 mS/cm, 4.99 mS/cm, 5.09 mS/cm, 6.09 mS/cm, 6.59 mS/cm, 7.09 mS/cm, 7.59 mS/cm, 8.09 mS/cm, 8.59 mS/cm, 9.09 mS/cm, 9.59 mS/cm, 10.09 mS/cm, 10.59 mS/cm, 11.09 mS/cm, 11.59 mS/cm, 12.09 mS/cm, 12.59 mS/cm, 13.09 mS/cm, 13.59 mS/cm, 14.09 mS/cm, 14.59 mS/cm, or about 15.09 mS/cm or the conductivity may be higher or lower.

In another example, a liquid antibody formulation may have any suitable osmolality for therapeutic efficacy, safety, and storage. For example, the osmolality of a liquid antibody formulation may be from about 50 milliosmole per kilogram (mOsm/kg) to about 5000 mOsm/kg, about 50 mOsm/kg to about 2000 mOsm/kg, about 50 mOsm/kg to about 1000 mOsm/kg, about 50 mOsm/kg to about 750 mOsm/kg, or about 50 mOsm/kg to about 500 mOsm/kg. In some embodiments, a liquid antibody formulation may have an osmolality of about 50 mOsm/kg, 60 mOsm/kg, 70 mOsm/kg, 80 mOsm/kg, 90 mOsm/kg, 100 mOsm/kg 120 mOsm/kg, 140 mOsm/kg, 160 mOsm/kg, 180 mOsm/kg, 200 mOsm/kg, 220 mOsm/kg, 240 mOsm/kg, 260 mOsm/kg, 280 mOsm/kg, 300 mOsm/kg, 320 mOsm/kg, 340 mOsm/kg, 360 mOsm/kg, 380 mOsm/kg, 400 mOsm/kg, 420 mOsm/kg, 440 mOsm/kg, 460 mOsm/kg, 480 mOsm/kg, 500 mOsm/kg, 520 mOsm/kg, 540 mOsm/kg, 560 mOsm/kg, 580 mOsm/kg, 600 mOsm/kg, 620 mOsm/kg, 640 mOsm/kg, 660 mOsm/kg, 680 mOsm/kg, 700 mOsm/kg, 720 mOsm/kg, 740 mOsm/kg, 760 mOsm/kg, 780 mOsm/kg, 800 mOsm/kg, 820 mOsm/kg, 840 mOsm/kg, 860 mOsm/kg, 880 mOsm/kg, 900 mOsm/kg, 920 mOsm/kg, 940 mOsm/kg, 960 mOsm/kg, 980 mOsm/kg, 1000 mOsm/kg, 1050 mOsm/kg, 1100 mOsm/kg, 1150 mOsm/kg, 1200 mOsm/kg, 1250 mOsm/kg, 1300 mOsm/kg, 1350 mOsm/kg, 1400 mOsm/kg, 1450 mOsm/kg, about 1500 mOsm/kg, or the osmolality may be higher or lower.

Liposomes containing antibody can be prepared by methods known in the art, such as described in Epstein, et al., Proc. Natl. Acad. Sci. USA 82:3688 (1985); Hwang, et al., Proc. Natl Acad. Sci. USA 77:4030 (1980); and U.S. Patent Nos. 4,485,045 and 4,544,545. Liposomes with enhanced circulation time are disclosed in U.S. Patent No. 5,013,556. Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington, The Science and Practice of Pharmacy 20th Ed. Mack Publishing (2000).

Sustained-release preparations may be prepared. Suitable examples of sustained-release preparations include semipermeable matrices of solid hydrophobic polymers containing the antibody, which matrices are in the form of shaped articles, e.g., films, or microcapsules. Examples of sustained-release matrices include polyesters, hydrogels (for example, poly(2-hydroxyethyl-methacrylate), or 'poly(v nylalcohol)), polylactides (U.S. Patent No. 3,773,919), copolymers of L-glutamic acid and 7 ethyl-L-glutamate, non-degradable ethylene-vinyl acetate, degradable lactic acid-glycolic acid copolymers such as the LUPRON DEPOT[3] (injectable microspheres composed of lactic acid-glycolic acid copolymer and leuprolide acetate), sucrose acetate isobutyrate, and poly-D-(-)-3-hydroxybutyric acid.

The formulations to be used for in vivo administration should generally be sterile. This is readily accomplished by, for example, filtration through sterile filtration membranes. Therapeutic antibody compositions are generally placed into a container having a sterile access port, for example, an intravenous solution bag or vial having a stopper pierceable by a hypodermic injection needle.

The compositions for use in the present invention may be in unit dosage forms such as tablets, pills, capsules, powders, granules, solutions or suspensions, or suppositories, for oral, parenteral or rectal administration, or administration by inhalation or insufflation. In some cases, a unit dosage form may be supplied in a prefilled receptacle (e.g., a prefilled syringe) useful in administering the unit dosage to a subject.

For preparing solid compositions such as tablets, the principal active ingredient can be mixed with a pharmaceutical carrier, e.g., conventional tableting ingredients such as corn starch, lactose, sucrose, sorbitol, talc, stearic acid, magnesium stearate, dicalcium phosphate, or gums, and other pharmaceutical diluents, e.g., water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing from about 0.1 mg to about 500 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer that serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

Suitable surface-active agents include, in particular, non-ionic agents, such as polyoxyethylenesorbitans (e.g., TWEEN[3] 20, 40, 60, 80, or 85) and other sorbitans (e.g., SPAN[3] 20, 40, 60, 80, or 85). Compositions with a surface-active agent will conveniently comprise between about 0.05 and about 5% surface-active agent, and can be between about 0.1% and about 2.5%. It will be appreciated that other ingredients may be added, for example mannitol or other pharmaceutically acceptable vehicles, if necessary.

Suitable emulsions may be prepared using commercially available fat emulsions, such as INTRALIPID[3], LIPOSYN[3], INFONUTROL[3], LIPOFUNDIN[3], and LIPIPHYSAN[3]. The active ingredient may be either dissolved in a pre-mixed emulsion composition or alternatively it may be dissolved in an oil (e.g., soybean oil, safflower oil, cottonseed oil, sesame oil, corn oil or almond oil) and an emulsion formed upon mixing with a phospholipid (e.g., egg phospholipids, soybean phospholipids, or soybean lecithin) and water. It will be appreciated that other ingredients may be added, for example glycerol or glucose, to adjust the tonicity of the emulsion. Suitable emulsions will typically contain up to 20% oil, for example, between 5 and 20%. The fat emulsion can comprise fat droplets between about 0.1 and 1.0 1m, particularly about 0.1 and 0.5 1m, and have a pH in the range of about pH 5.5 to about pH 8.0.

The emulsion compositions can be those prepared by mixing an antibody with INTRALIPID[3] or the components thereof (soybean oil, egg phospholipids, glycerol and water).

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as set out above. In some embodiments, the compositions are administered by the oral or nasal respiratory route for local or systemic effect. Compositions in preferably sterile pharmaceutically acceptable solvents may be nebulised by use of gases. Nebulised solutions may be breathed directly from the nebulising device or the nebulising device may be attached to a face mask, tent or intermittent positive pressure breathing machine. Solution, suspension or powder compositions may be administered, preferably orally or nasally, from devices which deliver the formulation in an appropriate manner.

In some embodiments, a formulation comprising a monoclonal anti-CGRP antagonist antibody may be prepared for any suitable route of administration with an antibody amount ranging from about 0.1 mg to about 3000 mg, about 1 mg to about 1000 mg, about 100 mg to about 1000 mg, or about 100 mg to about 500 mg. In some cases, a formulation comprising an antibody (e.g., monoclonal antibody that modulates the CGRP pathway, anti-CGRP antagonist antibody, monoclonal anti-CGRP antagonist antibody) described herein may comprise an antibody amount of, at most, or at least about 0.1 mg, 1 mg, 100 mg, 1 mg, 10 mg, 25 mg, 50 mg, 75 mg, 100 mg, 125 mg, 150 mg, 175 mg, 200 mg, 225 mg, 250 mg, 275 mg, 300 mg, 325 mg, 350 mg, 375 mg, 400 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 825 mg, 850 mg, 875 mg, 900 mg, 925 mg, 950 mg, 975 mg, 1000 mg, 1100 mg, 1200 mg, 1300 mg, 1400 mg, 1500 mg, 1600 mg, 1700 mg, 1800 mg, 1900 mg, 2000 mg, or about 3000 mg.

In some embodiments, a liquid formulation comprising a monoclonal anti-CGRP antagonist antibody may be prepared for any suitable route of administration with an antibody concentration ranging from about 0.1 mg/mL to about 500 mg/mL, about 0.1 mg/mL to about 375 mg/mL, about 0.1 mg/mL to about 250 mg/mL, about 0.1 to about 175 mg/mL, about 0.1 to 100 mg/mL, about 1 mg/mL to about 500 mg/mL, about 1 mg/mL to about 375 mg/mL, about 1 mg/mL to about 300 mg/mL, about 1 mg/mL to 250 mg/mL, about 1 mg/mL to 200 mg/mL, about 1 mg/mL to 150 mg/mL, about 1 mg/mL to about 100 mg/mL, about 10 mg/ mL to 500 mg/mL, about 10 mg/mL to about 375 mg/mL, about 10 mg/mL to 250 mg/mL, about 10 mg/mL to about 150 mg/mL, about 10 mg/mL to 100 mg/mL, about 100 mg/mL to 500 mg/mL, about 100 mg/mL to 450 mg/mL, about 100 mg/mL to 400 mg/mL, about 100 mg/mL to about 350 mg/mL, about 100 mg/mL to about 300 mg/mL, about 100 mg/mL to about 250 mg/mL, 100 mg/mL to 200 mg/mL, or about 100 mg/mL to about 150 mg/mL. In some embodiments, a liquid formulation may comprise an antibody described herein at a concentration of, of at most, of at least, or less than about 0.1, 0.5, 1, 5, 10, 15 20, 25, 30, 35, 40, 45, 50, 55, 60, 65, 70, 75, 80, 85, 90, 95, 100, 105 110, 115, 120, 125, 130, 135, 140, 145, 150, 155, 160, 165, 170, 175, 180, 185, 190, 195, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, or about 500 mg/mL.

An antibody formulation may comprise one or more components including the antibody and other species described elsewhere herein. The antibody and other components may be in any suitable amount and/or any suitable concentration for therapeutic efficacy of the antibody, safety and storage. In one example, an antibody formulation may be a solution comprising about 51.4 mg/mL antibody (e.g., antibody G1, another anti-CGRP antagonist antibody, or a monoclonal antibody that modulates the CGRP pathway), 16-20 mM histidine, 0.1 mg/mL methionine, 84 mg/mL trehalose dihydrate, 0.05 mg/mL disodium EDTA dihydrate, and 0.2 mg/mL polysorbate 80.

In another example, an antibody formulation may comprise about 200 mg/mL antibody (e.g., antibody G1, another anti-CGRP antagonist antibody, or a monoclonal antibody that modulates the CGRP pathway), 15 mM arginine, 78 mg/mL sucrose, 0.3 mg/mL EDTA, and 0.1 mg/mL polysorbate 80.

In another example, an antibody formulation may comprise about 175 mg/mL antibody (e.g., antibody G1, another anti-CGRP antagonist antibody, or a monoclonal antibody that modulates the CGRP pathway), 20 mM glycine, 88 mg/mL trehalose dihydrate, 0.015 mg/mL EDTA, and 0.25 mg/mL polysorbate 80.

In another example, an antibody formulation may comprise about 225 mg/mL antibody (e.g., antibody G1, another anti-CGRP antagonist antibody, or a monoclonal antibody that modulates the CGRP pathway), 23 mM asparagine, 84 mg/mL sorbitol, 0.1 mg/mL EDTA, and 0.15 mg/mL polysorbate 60.

In another example, an antibody formulation may comprise about 150 mg/mL antibody (e.g., antibody G1, another anti-CGRP antagonist antibody, or a monoclonal antibody that modulates the CGRP pathway), 17 mM asparagine, 74 mg/mL mannitol, 0.025 mg/mL EDTA, and 0.2 mg/mL polysorbate 80.

In another example, an antibody formulation may comprise about 100 mg/mL antibody (e.g., antibody G1, another anti-CGRP antagonist antibody, or a monoclonal antibody that modulates the CGRP pathway), 16 mM arginine, 87 mg/mL mannitol, 0.025 mg/mL EDTA, and 0.15 mg/mL polysorbate 20.

In another example, an antibody formulation may comprise about 250 mg/mL antibody (e.g., antibody G1, another anti-CGRP antagonist antibody, or a monoclonal antibody that modulates the CGRP pathway), 25 mM histidine, 74 mg/mL mannitol, 0.025 mg/mL EDTA, and 0.25 mg/mL polysorbate 20.

In another example, an antibody formulation may comprise about 50 mg/mL antibody (e.g., antibody G1, another anti-CGRP antagonist antibody, or a monoclonal antibody that modulates the CGRP pathway), 19 mM arginine, 84 mg/mL sucrose, 0.05 mg/mL EDTA, and 0.3 mg/mL polysorbate 80.

In another example, an antibody formulation may comprise about 125 mg/mL antibody (e.g., antibody G1, another anti-CGRP antagonist antibody, or a monoclonal antibody that modulates the CGRP pathway), 22 mM glycine, 79 mg/mL trehalose dihydrate, 0.15 mg/mL EDTA, and 0.15 mg/mL polysorbate 80.

In another example, an antibody formulation may be a solution comprising about 175 mg/mL antibody (e.g., antibody G1, another anti-CGRP antagonist antibody, or a monoclonal antibody that modulates the CGRP pathway), 20 mM histidine, 0.1 mg/mL methionine, 84 mg/mL trehalose dihydrate, 0.05 mg/mL disodium EDTA dihydrate, and 0.2 mg/mL polysorbate 80.

In another example, an antibody formulation may comprise about 200 mg/mL antibody (e.g., antibody G1, another anti-CGRP antagonist antibody, or a monoclonal antibody that modulates the CGRP pathway), 30 mM arginine, 78 mg/mL sucrose, 0.3 mg/mL EDTA, and 0.1 mg/mL polysorbate 80.

In another example, an antibody formulation may comprise about 175 mg/mL antibody (e.g., antibody G1, another anti-CGRP antagonist antibody, or a monoclonal antibody that modulates the CGRP pathway), 20 mM glycine, 88 mg/mL trehalose dihydrate, 0.015 mg/mL EDTA, and 0.15 mg/mL polysorbate 80.

In another example, an antibody formulation may comprise about 150 mg/mL antibody (e.g., antibody G1, another anti-CGRP antagonist antibody, or a monoclonal antibody that modulates the CGRP pathway), 20 mM histidine, 84 mg/mL sucrose, 0.05 mg/mL EDTA, and 0.2 mg/mL polysorbate 80.

In another example, an antibody formulation may comprise about 225 mg/mL antibody (e.g., antibody G1, another anti-CGRP antagonist antibody, or a monoclonal antibody that modulates the CGRP pathway), 23 mM histidine, 84 mg/mL sorbitol, 0.1 mg/mL EDTA, and 0.15 mg/mL polysorbate 60.

In another example, an antibody formulation may comprise about 150 mg/mL antibody (e.g., antibody G1, another anti-CGRP antagonist antibody, or a monoclonal antibody that modulates the CGRP pathway), 17 mM asparagine, 74 mg/mL mannitol, 0.3 mg/mL EDTA, and 0.2 mg/mL polysorbate 80.

In another example, an antibody formulation may comprise about 100 mg/mL antibody (e.g., antibody G1, another anti-CGRP antagonist antibody, or a monoclonal antibody that modulates the CGRP pathway), 16 mM arginine, 87 mg/mL mannitol, 0.025 mg/mL EDTA, and 0.25 mg/mL polysorbate 20.

In another example, an antibody formulation may comprise about 250 mg/mL antibody (e.g., antibody G1, another anti-CGRP antagonist antibody, or a monoclonal antibody that modulates the CGRP pathway), 25 mM histidine, 89 mg/mL mannitol, 0.025 mg/mL EDTA, and 0.25 mg/mL polysorbate 20.

In another example, an antibody formulation may comprise 125 mg/mL antibody (e.g., antibody G1, another anti-CGRP antagonist antibody, or a monoclonal antibody that modulates the CGRP pathway), 29 mM arginine, 84 mg/mL sucrose, 0.05 mg/mL EDTA, and 0.3 mg/mL polysorbate 80.

In another example, an antibody formulation may comprise 150 mg/mL antibody (e.g., antibody G1, another anti-CGRP antagonist antibody, or a monoclonal antibody that modulates the CGRP pathway), 25 mM asparagine, 84 mg/mL mannitol, 0.05 mg/mL EDTA, and 0.2 mg/mL polysorbate 80.

In another example, an antibody formulation may comprise 145 mg/mL antibody (e.g., antibody G1, another anti-CGRP antagonist antibody, or a monoclonal antibody that modulates the CGRP pathway), 22 mM histidine, 72 mg/mL trehalose dihydrate, 0.05 mg/mL EDTA, and 0.1 mg/mL polysorbate 80.

An antibody can be administered using any suitable method, including by injection (e.g., intravenously, subcutaneously, intraperitoneally, intramuscularly, etc.). Antibodies can also be administered via inhalation, as described herein. In some cases, an antibody may be administered nasally with or without inhalation. Generally, for administration of an antibody described herein, an initial candidate dosage can be about 2 mg/kg. For the purpose of the present invention, a typical daily dosage might range from about any of 3 μg/kg to 30 μg/kg to 300 μg/kg to 3 mg/kg, to 30 mg/kg to 100 mg/kg or more, depending on the factors mentioned above. For example, dosage of about 1 mg/kg, about 2.5 mg/kg, about 5 mg/kg, about 10 mg/kg, about 25 mg/kg, and about 30 mg/kg may be used. For repeated administrations over several days or longer, depending on the condition, the treatment is sustained until a desired suppression of symptoms occurs or until sufficient therapeutic levels are achieved, for example, to reduce pain. An exemplary dosing regimen comprises administering an initial dose of about 8.5 mg/kg, or about 10 mg/kg, followed by a maintenance dose of about 2.8 mg/kg of an antibody, or followed by a maintenance dose of about 2.8 mg/kg every other week. Another exemplary dosing regimen comprises administering a dose of about 100 mg, 125 mg, 150 mg, 200 mg, 225 mg, 250 mg, 275 mg, 300 mg, 350 mg, 400 mg, 450 mg, 500 mg, 550 mg, 600 mg, about 675 mg, or about 900 mg to a subject once per month intravenously in an infusion over about one hour, or subcutaneously. Another exemplary dosing regimen comprises administering an initial antibody dose of about 675 mg subcutaneously, followed by a monthly antibody dose of about 225 mg subcutaneously for about two months, e.g., about three months, four months, five months, six months, or 12 months. Yet another dosing regimen comprises administering an initial dose of about 900 mg intravenously in an infusion over about 60 minutes, followed by doses of about 900 mg administered intravenously in an infusion over about 60 minutes every quarter for one year, two years, three years, four years, or five years. However, other dosage regimens may be useful, depending on the pattern of pharmacokinetic decay that the practitioner wishes to achieve. For example, in some embodiments, dosing from about one to about four times a week is contemplated. The progress of this therapy is easily monitored by conventional techniques and assays. The dosing regimen (including the CGRP antagonist(s) used) can vary over time.

In some embodiments, the dose or amount of a monoclonal anti-CGRP antagonist antibody may range from about 0.1 μg to about 3000 mg, 1 mg to 1000 mg, 100 mg to 1000 mg, 100 mg to 500 mg, 0.1 mg to 5000 mg, 1 mg to 4000 mg, 250 mg to 1000 mg, 500 mg to 1000 mg, 100 mg to 900 mg, 400 mg to 900 mg, 10 mg to 3000 mg, 10 mg to 2000 mg, 100 mg to 2000 mg, 150 mg to 2000 mg, 200 mg to 2000 mg, 250 mg to 2000 mg, 300 mg to 2000 mg, 350 mg to 2000 mg, 400 mg to 2000 mg, 450 mg to 2000 mg, 500 mg to 2000 mg, 550 mg to 2000 mg, 600 mg to 2000 mg, 650 mg to 2000 mg, 700 mg to 2000 mg, 750 mg to 2000 mg, 800 mg to 2000 mg, 850 mg to 2000 mg, 900 mg to 2000 mg, 950 mg to 2000 mg, or 1000 mg to 2000 mg. In some embodiments, the dose or amount of an antibody may be, may be at most, may be less than, or may be at least about 0.1 μg, 1 μg, 100 μg, 1 mg, 10 mg, 25 mg, 50 mg, 75 mg, 100 mg, 125 mg, 150 mg, 175 mg, 200 mg, 225 mg, 250 mg, 275 mg, 300 mg, 325 mg, 350 mg, 375 mg, 400 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 825 mg, 850 mg, 875 mg, 900 mg, 925 mg, 950 mg, 975 mg, 1000 mg, 1100 mg, 1200 mg, 1300 mg, 1400 mg, 1500 mg, 1600 mg, 1700 mg, 1800 mg, 1900 mg, 2000 mg, or about 3000 mg. In some embodiments, the amount is between about 225 mg to about 1000 mg, e.g., about 675 mg or about 900 mg. An exemplary dosing regimen comprises administering an initial antibody dose of about 675 mg subcutaneously, followed by a monthly antibody dose of about 225 mg subcutaneously for about two months, e.g., about three months, four months, five months, six months, or 12 months. Yet another dosing regimen comprises administering an initial dose of about 900 mg intravenously in an infusion over about 60 minutes, followed by doses of about 900 mg administered intravenously in an infusion over about 60 minutes every quarter for one year, two years, three years, four years, or five years. However, other dosage regimens may be useful, depending on the pattern of pharmacokinetic decay that the practitioner wishes to achieve.

In some embodiments, the dose or amount of a monoclonal anti-CGRP antagonist antibody may range from about 0.1 to 500, 0.1 to 100, 0.1 to 50, 0.1 to 20, 0.1 to 10, 1 to 10, 1 to 7, 1 to 5 or 0.1 to 3 mg/kg of body weight. In some embodiments, the dose or amount of a monoclonal anti-CGRP antagonist antibody may be, may be at most, may be less than, or may be at least about 0.1, 0.2, 0.3, 0.4, 0.5, 0.6, 0.7, 0.8, 0.9, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5, 15.0, 15.5, 16.0, 16.5, 17.0, 17.5, 18.0, 18.5, 19.0, 19.5, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 110, 120, 130, 140, 150, 160, 170, 180, 190, 200, 225, 250, 275, 300, 325, 350, 375, 400, 425, 450, 475, or about 500 mg/kg of body weight.

In some embodiments, the frequency at which a dose or amount of a monoclonal anti-CGRP antagonist antibody is administered to a subject may vary. In some embodiments, a single dose of antibody may be given to a subject across therapy. In some embodiments, the frequency at which a dose or amount of an antibody is administered to a subject is constant (e.g., administered about once per month or about once per quarter). In some embodiments, the frequency at which a dose or amount of an antibody is administered to a subject is about every quarter for about one year, two years, three years, four years, or five years. In some embodiments, the frequency at which a dose or amount of an antibody described herein is administered to a subject is variable (e.g., an initial dose followed by a dose at once per month, followed by additional doses at about three months and about seven months). In some embodiments, the frequency at which an antibody is administered to a subject is, is at least, is less than, or is at most about one, two, three, four, five, or six time(s) per day. In some embodiments, the frequency at which an antibody (e.g., monoclonal antibody that modulates the CGRP pathway, anti-CGRP antagonist antibody, monoclonal anti-CGRP antagonist antibody) is administered to a subject is, is at least, is less than, or is at most about one, two, three, four, five, or six dose(s) per day.

In some embodiments, the frequency at which a dose or amount of a monoclonal anti-CGRP antagonist antibody is administered to a subject is, is at least, is less than, or is at most about one, two, three, four, five, six, seven, eight, nine, ten, eleven, twelve, thirteen, fourteen, fifteen, sixteen, seventeen, eighteen, nineteen, or twenty time(s) per every one, two, three, four, five, six, seven, eight, nine, ten, eleven, twelve, thirteen, fourteen, fifteen, sixteen, seventeen, eighteen, nineteen, twenty, twenty-one, twenty-two, twenty-three, twenty-four, twenty-five, twenty-six, twenty-seven, twenty-eight, twenty-nine, thirty, thirty-one, thirty-two, thirty-three, thirty-four, thirty-five, thirty-six, thirty-seven, thirty-eight, thirty-nine, forty, forty-one, forty-two, forty-three, forty-four, forty-five, forty-six, forty-seven, forty-eight, forty-nine, fifty, fifty-five, sixty, sixty-five, seventy, seventy-five, eighty, eighty-five, ninety, ninety-five, one-hundred, one-hundred twenty-five, one-hundred fifty, one-hundred eighty, or two-hundred day(s).

In some embodiments, the frequency at which a dose or amount of a monoclonal anti-CGRP antagonist antibody is administered to a subject is, is at least, is less than, or is at most about one, two, three, four, five, six, seven, eight, nine, ten, eleven, twelve, thirteen, fourteen, fifteen, sixteen, seventeen, eighteen, nineteen, or twenty time(s) per every one, two, three, four, five, six, seven, eight, nine, ten, eleven, twelve, thirteen, fourteen, fifteen, sixteen, seventeen, eighteen, nineteen, twenty, twenty-one, twenty-two, twenty-three, twenty-four, twenty-five, twenty-six, twenty-seven, twenty-eight, twenty-nine, thirty, thirty-one, thirty-two, thirty-three, thirty-four, thirty-five, thirty-six, thirty-seven, thirty-eight, thirty-nine, forty, forty-one, forty-two, forty-three, forty-four, forty-five, forty-six, forty-seven, forty-eight, forty-nine, fifty, fifty-five, sixty, sixty-five, seventy, seventy-five, eighty, eighty-five, ninety, ninety-five, or one-hundred week(s). In some embodiments, the frequency at which a monoclonal anti-CGRP antagonist antibody is administered to a subject is less than or about one, two, three, four, five, six, seven, eight, nine, ten, eleven, twelve, thirteen, fourteen, or fifteen dose(s) per week.

In some embodiments, the frequency at which a dose or amount of a monoclonal anti-CGRP antagonist antibody is administered to a subject is, is at least, is less than, or is at most about one, two, three, four, five, six, seven, eight, nine, ten, eleven, twelve, thirteen, fourteen, fifteen, sixteen, seventeen, eighteen, nineteen, or twenty time(s) per every month, every two months, every three months, every four months, every five months, every six months, every seven months, every eight months, every nine months, every ten months, every eleven months, every twelve months, every thirteen months, every fourteen months, every fifteen months, every sixteen months, every seventeen months, or every eighteen month(s). In some embodiments, the frequency at which a dose or amount of a monoclonal anti-CGRP antagonist antibody is administered to a subject is about one time per every one month. In some embodiments, the frequency at which a dose or amount of a monoclonal anti-CGRP antagonist antibody is administered to a subject is about one time per every three months. In some embodiments, the frequency at which a monoclonal anti-CGRP antagonist antibody is administered to a subject is less than about one, two, three, four, five, six, seven, eight, nine, ten, eleven, twelve, thirteen, fourteen, or fifteen dose(s) per month. In some embodiments, a dose or amount of an antibody may be administered (e.g., subcutaneously or intravenously in an infusion) to a subject one time, two times, three times, four times, five times, six times, seven times, eight times, nine times, ten times or more per month.

In some embodiments, an antibody in a dose or amount of about 50 mg, 100 mg 150 mg, 200 mg, 225 mg, 250 mg, 300 mg, 350 mg, 400 mg, 450 mg, 500 mg, 550 mg, 600 mg, 650 mg, 675 mg, 700 mg, 750 mg, 800 mg, 850 mg, 900 mg, 950 mg, 1000 mg, 1050 mg, 1100 mg, 1150 mg, 1200 mg, 1250 mg, 1300 mg, 1350 mg, 1400 mg, 1450 mg, 1500 mg, 1550 mg, 1600 mg, 1650 mg, 1700 mg, 1750 mg, 1800 mg, 1850 mg, 1900 mg, 1950 mg, 2000 mg, 2050 mg, 2100 mg, 2150 mg, 2200 mg, 2250 mg, 2300 mg, 2350 mg, 2400 mg, 2450 mg, 2500 mg, 2550 mg, 2600 mg, 2650 mg, 2700 mg, 2750 mg, 2800 mg, 2850 mg, 2900 mg, 2950 mg, 3000 mg, or more may be administered (e.g., subcutaneously or intravenously in an infusion) to a subject once per month. In some embodiments, an antibody in a dose or amount of between about 0.1 mg to 5000 mg, 1 mg to 4000 mg, 10 mg to 3000 mg, 10 mg to 2000 mg, 100 mg to 2000 mg, 150 mg to 2000 mg, 200 mg to 2000 mg, 250 mg to 2000 mg, 300 mg to 2000 mg, 350 mg to 2000 mg, 400 mg to 2000 mg, 450 mg to 2000 mg, 500 mg to 2000 mg, 550 mg to 2000 mg, 600 mg to 2000 mg, 650 mg to 2000 mg, 700 mg to 2000 mg, 750 mg to 2000 mg, 800 mg to 2000 mg, 850 mg to 2000 mg, 900 mg to 2000 mg, 950 mg to 2000 mg, or about 1000 mg to 2000 mg may be administered (e.g., subcutaneously or intravenously in an infusion) to a subject once per month. In some embodiments, between about 225 mg and about 1000 mg, e.g., about 225 mg of antibody are administered once per month. An exemplary dosing regimen comprises administering an initial antibody dose of about 675 mg subcutaneously, followed by a monthly antibody dose of about 225 mg subcutaneously for about two months, e.g., about three months, four months, five months, six months, or 12 months. Yet another dosing regimen comprises administering an initial dose of about 900 mg intravenously in an infusion over about 60 minutes, followed by doses of about 900 mg administered intravenously in an infusion over about 60 minutes every quarter for one year, two years, three years, four years, or five years. However, other dosage regimens may be useful, depending on the pattern of pharmacokinetic decay that the practitioner wishes to achieve.

In some embodiments, an antibody in a dose or amount of about 50 mg, 100 mg 150 mg, 200 mg, 225 mg, 250 mg, 300 mg, 350 mg, 400 mg, 450 mg, 500 mg, 550 mg, 600 mg, 650 mg, 675 mg, 700 mg, 750 mg, 800 mg, 850 mg, 900 mg, 950 mg, 1000 mg, 1050 mg, 1100 mg, 1150 mg, 1200 mg, 1250 mg, 1300 mg, 1350 mg, 1400 mg, 1450 mg, 1500 mg, 1550 mg, 1600 mg, 1650 mg, 1700 mg, 1750 mg, 1800 mg, 1850 mg, 1900 mg, 1950 mg, 2000 mg, 2050 mg, 2100 mg, 2150 mg, 2200 mg, 2250 mg, 2300 mg, 2350 mg, 2400 mg, 2450 mg, 2500 mg, 2550 mg, 2600 mg, 2650 mg, 2700 mg, 2750 mg, 2800 mg, 2850 mg, 2900 mg, 2950 mg, 3000 mg, or more may be administered (e.g., subcutaneously or intravenously in an infusion) to a subject every three months. In some embodiments, an antibody in a dose or amount of between about 0.1 mg to 5000 mg, 1 mg to 4000 mg, 10 mg to 3000 mg, 10 mg to 2000 mg, 100 mg to 2000 mg, 150 mg to 2000 mg, 200 mg to 2000 mg, 250 mg to 2000 mg, 300 mg to 2000 mg, 350 mg to 2000 mg, 400 mg to 2000 mg, 450 mg to 2000 mg, 500 mg to 2000 mg, 550 mg to 2000 mg, 600 mg to 2000 mg, 650 mg to 2000 mg, 700 mg to 2000 mg, 750 mg to 2000 mg, 800 mg to 2000 mg, 850 mg to 2000 mg, 900 mg to 2000 mg, 950 mg to 2000 mg, or 1000 mg to 2000 mg may be administered (e.g., subcutaneously or intravenously in an infusion) to a subject every three months. In some embodiments, between about 225 mg to about 1000 mg is administered once every three months or less, e.g., about 900 mg is administered every three months intravenously in an infusion. An exemplary dosing regimen comprises administering an initial antibody dose of about 675 mg subcutaneously, followed by a monthly antibody dose of about 225 mg subcutaneously for about two months, e.g., about three months, four months, five months, six months, or 12 months. Yet another dosing regimen comprises administering an initial dose of about 900 mg intravenously in an infusion over about 60 minutes, followed by doses of about 900 mg administered intravenously in an infusion over about 60 minutes every quarter for one year, two years, three years, four years, or five years. However, other dosage regimens may be useful, depending on the pattern of pharmacokinetic decay that the practitioner wishes to achieve.

In some embodiments, an antibody in a dose or amount of about 50 mg, 100 mg 150 mg, 200 mg, 225 mg, 250 mg, 300 mg, 350 mg, 400 mg, 450 mg, 500 mg, 550 mg, 600 mg, 650 mg, 675 mg, 700 mg, 750 mg, 800 mg, 850 mg, 900 mg, 950 mg, 1000 mg, 1050 mg, 1100 mg, 1150 mg, 1200 mg, 1250 mg, 1300 mg, 1350 mg, 1400 mg, 1450 mg, 1500 mg, 1550 mg, 1600 mg, 1650 mg, 1700 mg, 1750 mg, 1800 mg, 1850 mg, 1900 mg, 1950 mg, 2000 mg, 2050 mg, 2100 mg, 2150 mg, 2200 mg, 2250 mg, 2300 mg, 2350 mg, 2400 mg, 2450 mg, 2500 mg, 2550 mg, 2600 mg, 2650 mg, 2700 mg, 2750 mg, 2800 mg, 2850 mg, 2900 mg, 2950 mg, 3000 mg, or more may be administered (e.g., subcutaneously or intravenously in an infusion) to a subject every six months. In some embodiments, an antibody in a dose or amount of between about 0.1 mg to 5000 mg, 1 mg to 4000 mg, 10 mg to 3000 mg, 10 mg to 2000 mg, 100 mg to 2000 mg, 150 mg to 2000 mg, 200 mg to 2000 mg, 250 mg to 2000 mg, 300 mg to 2000 mg, 350 mg to 2000 mg, 400 mg to 2000 mg, 450 mg to 2000 mg, 500 mg to 2000 mg, 550 mg to 2000 mg, 600 mg to 2000 mg, 650 mg to 2000 mg, 700 mg to 2000 mg, 750 mg to 2000 mg, 800 mg to 2000 mg, 850 mg to 2000 mg, 900 mg to 2000 mg, 950 mg to 2000 mg, or 1000 mg to 2000 mg may be administered (e.g., subcutaneously or intravenously in an infusion) to a subject every six months. In some embodiments, between 225 mg to 1000 mg is administered once every six months or less. An exemplary dosing regimen comprises administering an initial antibody dose of about 675 mg subcutaneously, followed by a monthly antibody dose of about 225 mg subcutaneously for about two months, e.g., about three months, four months, five months, six months, or 12 months. Yet another dosing regimen comprises administering an initial dose of about 900 mg intravenously in an infusion over about 60 minutes, followed by doses of about 900 mg administered intravenously in an infusion over about 60 minutes every quarter for one year, two years, three years, four years, or five years. However, other dosage regimens may be useful, depending on the pattern of pharmacokinetic decay that the practitioner wishes to achieve.

In some embodiments, the frequency at which a dose or amount of a monoclonal anti-CGRP antagonist antibody is administered to a subject (e.g., subcutaneously or intravenously) is, is at least, is less than, or is at most one, two, three, four, five, six, seven, eight, nine, ten, eleven, twelve, thirteen, fourteen, fifteen, sixteen, seventeen, eighteen, nineteen, or twenty time(s) per every quarter. As can be appreciated, a "quarter" can refer to a time period of a quarter year or may also refer to a calendar quarter such as a time period of January 1 - March 31, April 1 - June 30, July 1 - September 30, or October 1 - December 31. In some cases, a "quarter" may refer to a time period of approximately three months.

In some embodiments, an antibody in a dose or amount of about 50 mg, 100 mg 150 mg, 200 mg, 225 mg, 250 mg, 300 mg, 350 mg, 400 mg, 450 mg, 500 mg, 550 mg, 600 mg, 650 mg, 675 mg, 700 mg, 750 mg, 800 mg, 850 mg, 900 mg, 950 mg, 1000 mg, 1050 mg, 1100 mg, 1150 mg, 1200 mg, 1250 mg, 1300 mg, 1350 mg, 1400 mg, 1450 mg, 1500 mg, 1550 mg, 1600 mg, 1650 mg, 1700 mg, 1750 mg, 1800 mg, 1850 mg, 1900 mg, 1950 mg, 2000 mg, 2050 mg, 2100 mg, 2150 mg, 2200 mg, 2250 mg, 2300 mg, 2350 mg, 2400 mg, 2450 mg, 2500 mg, 2550 mg, 2600 mg, 2650 mg, 2700 mg, 2750 mg, 2800 mg, 2850 mg, 2900 mg, 2950 mg, 3000 mg, or more may be administered (e.g., subcutaneously or intravenously in an infusion) to a subject every quarter. In some embodiments, an antibody in a dose or amount of between about 0.1 mg to 5000 mg, 1 mg to 4000 mg, 10 mg to 3000 mg, 10 mg to 2000 mg, 100 mg to 2000 mg, 150 mg to 2000 mg, 200 mg to 2000 mg, 250 mg to 2000 mg, 300 mg to 2000 mg, 350 mg to 2000 mg, 400 mg to 2000 mg, 450 mg to 2000 mg, 500 mg to 2000 mg, 550 mg to 2000 mg, 600 mg to 2000 mg, 650 mg to 2000 mg, 700 mg to 2000 mg, 750 mg to 2000 mg, 800 mg to 2000 mg, 850 mg to 2000 mg, 900 mg to 2000 mg, 950 mg to 2000 mg, or 1000 mg to 2000 mg may be administered (e.g., subcutaneously or intravenously in an infusion) to a subject every quarter. An exemplary dosing regimen comprises administering an initial antibody dose of about 675 mg subcutaneously, followed by a monthly antibody dose of about 225 mg subcutaneously for about two months, e.g., about three months, four months, five months, six months, or 12 months. Yet another dosing regimen comprises administering an initial dose of about 900 mg intravenously in an infusion over about 60 minutes, followed by doses of about 900 mg administered intravenously in an infusion over about 60 minutes every quarter for one year, two years, three years, four years, or five years. However, other dosage regimens may be useful, depending on the pattern of pharmacokinetic decay that the practitioner wishes to achieve.

In some embodiments, the frequency at which a dose or amount of a monoclonal anti-CGRP antagonist antibody is administered is, is at least, is less than, or is at most about one, two, three, four, five, six, seven, eight, nine, ten, eleven, twelve, thirteen, fourteen, fifteen, sixteen, seventeen, eighteen, nineteen, or twenty time(s) per every year, every two years, every three years, every four years, or every five years. In some embodiments, the frequency at which a monoclonal anti-CGRP antagonist antibody is administered to a subject is less than one, two, three, four, five, six, seven, eight, nine, ten, eleven, twelve, thirteen, fourteen, fifteen, sixteen, seventeen, eighteen, nineteen, twenty, twenty-one, twenty-two, twenty-three, twenty-four or twenty-five dose(s) per year.

In some embodiments, an antibody in a dose or amount of about 50 mg, 100 mg 150 mg, 200 mg, 225 mg, 250 mg, 300 mg, 350 mg, 400 mg, 450 mg, 500 mg, 550 mg, 600 mg, 650 mg, 675 mg, 700 mg, 750 mg, 800 mg, 850 mg, 900 mg, 950 mg, 1000 mg, 1050 mg, 1100 mg, 1150 mg, 1200 mg, 1250 mg, 1300 mg, 1350 mg, 1400 mg, 1450 mg, 1500 mg, 1550 mg, 1600 mg, 1650 mg, 1700 mg, 1750 mg, 1800 mg, 1850 mg, 1900 mg, 1950 mg, 2000 mg, 2050 mg, 2100 mg, 2150 mg, 2200 mg, 2250 mg, 2300 mg, 2350 mg, 2400 mg, 2450 mg, 2500 mg, 2550 mg, 2600 mg, 2650 mg, 2700 mg, 2750 mg, 2800 mg, 2850 mg, 2900 mg, 2950 mg, 3000 mg, or more may be administered to a subject every once per year. In some embodiments, an antibody in a dose or amount of between about 0.1 mg to 5000 mg, 1 mg to 4000 mg, 10 mg to 3000 mg, 10 mg to 2000 mg, 100 mg to 2000 mg, 150 mg to 2000 mg, 200 mg to 2000 mg, 250 mg to 2000 mg, 300 mg to 2000 mg, 350 mg to 2000 mg, 400 mg to 2000 mg, 450 mg to 2000 mg, 500 mg to 2000 mg, 550 mg to 2000 mg, 600 mg to 2000 mg, 650 mg to 2000 mg, 700 mg to 2000 mg, 750 mg to 2000 mg, 800 mg to 2000 mg, 850 mg to 2000 mg, 900 mg to 2000 mg, 950 mg to 2000 mg, or 1000 mg to 2000 mg may be administered to a subject every once per year. In some embodiments, between about 450 mg and about 2000 mg is administered once every year or less.

In some embodiments, the treating or reducing may comprise administering a monoclonal anti-CGRP antagonist antibody to a subject on a plurality of days. Two, three, four, five, six, seven, eight or more days of the plurality of days may be more than 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75 or more days apart. In some embodiments, two of the plurality of days are more than one, two, three, four, five, six, seven, eight, nine, ten, eleven, twelve, thirteen, fourteen, fifteen, sixteen, seventeen, eighteen, nineteen, twenty, twenty-one, twenty-two, twenty-three, twenty-four, twenty-five, twenty-six, twenty-seven, twenty-eight, twenty-nine, thirty or more days apart. Moreover, in some embodiments, the amount of antibody administered on a first day of the plurality of days may be different (e.g., higher or lower) than the amount of the antibody administered on a second day.

In some embodiments, an initial dose (e.g., a loading dose) of a monoclonal anti-CGRP antagonist antibody may be administered to a subject, followed by administration of one or more additional doses at desired intervals. In some embodiments, the initial dose and one or more of the additional doses are the same dose. In some embodiments, the one or more additional doses are a different dose than the initial dose. In some embodiments, the initial dose and one or more of the additional doses are administered the same way, i.e., subcutaneously or intravenously. In some embodiments, the one or more additional doses are administered in a different way than the initial dose, e.g., the initial dose may be administered intravenously and the one or more additional doses may be administered subcutaneously. In some embodiments, the frequency at which the one or more additional doses are administered is constant (e.g., every month or every three months). In some embodiments, the frequency at which the one or more additional doses are administered is variable (e.g., one additional dose administered at one month following the initial dose, followed by another additional dose at three months following the initial dose). Any desirable and/or therapeutic regimen of initial loading dose, additional doses, and frequency (e.g., including those described herein) of additional doses may be used. An exemplary regimen includes an initial loading dose of about 675 mg anti-CGRP antagonist antibody administered subcutaneously, followed by subsequent maintenance doses of about 225 mg of the antibody administered subcutaneously at one month intervals. Yet another exemplary regimen includes an initial dose of about 900 mg anti-CGRP antagonist antibody administered intravenously in an infusion over about 60 minutes, followed by subsequent maintenance doses of about 900 mg anti-CGRP antagonist antibody administered intravenously in an infusion over about 60 minutes at three month intervals.

In some embodiments, an initial dose of a monoclonal anti-CGRP antagonist antibody of about 0.1 µg, 1 µg, 100 µg, 1 mg, 10 mg, 25 mg, 50 mg, 75 mg, 100 mg, 125 mg, 150 mg, 175 mg, 200 mg, 225 mg, 250 mg, 275 mg, 300 mg, 325 mg, 350 mg, 375 mg, 400 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 825 mg, 850 mg, 875 mg, 900 mg, 925 mg, 950 mg, 975 mg, 1000 mg, 1500 mg, 2000 mg, or about 3000 mg may be administered to a subject followed by one or more additional doses of the antibody of about 0.1 μg, 1 μg, 100 μg, 1 mg, 10 mg, 25 mg, 50 mg, 75 mg, 100 mg, 125 mg, 150 mg, 175 mg, 200 mg, 225 mg, 250 mg, 275 mg, 300 mg, 325 mg, 350 mg, 375 mg, 400 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 825 mg, 850 mg, 875 mg, 900 mg, 925 mg, 950 mg, 975 mg, 1000 mg, 1500 mg, 2000 mg, or about 3000 mg. An exemplary regimen includes an initial loading dose of about 675 mg anti-CGRP antagonist antibody administered subcutaneously, followed by subsequent maintenance doses of about 225 mg of the antibody administered subcutaneously at one month intervals. Yet another exemplary regimen includes an initial dose of about 900 mg anti-CGRP antagonist antibody administered intravenously in an infusion over about 60 minutes, followed by subsequent maintenance doses of about 900 mg anti-CGRP antagonist antibody administered intravenously in an infusion over about 60 minutes at three month intervals.

In some embodiments, a dose or amount of monoclonal anti-CGRP antagonist antibody may be divided into sub-doses and administered as multiple sub-doses, depending, for example, on the route of administration and/or particular formulation administered. For example, in cases where a dose is administered subcutaneously, the subcutaneous dose may be divided into multiple sub-doses and each sub-dose administered at a different site in order to avoid, for example, a larger, single subcutaneous injection at a single site. For example, an intravenous dose of 900 mg may be divided into four sub-doses of 225 mg each. As another example, a subcutaneous dose of 675 mg may be divided into three sub-doses of 225 mg each and each 225 mg dose may be administered at a different site, which can help minimize the volume injected at each site. The division of sub-doses may be equal (e.g., three equal sub-doses) or may be unequal (e.g., three sub-doses, two of the sub-doses twice as large as the other sub-doses).

In some embodiments, the number of doses of antibody administered to a subject over the course of treatment may vary depending upon, for example, achieving reduced incidence of a post-traumatic headache and/or secondary symptom associated with a post-traumatic headache in the subject. For example, the number of doses administered over the course of treatment may be, may be at least, or may be at most 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or treatment may be given indefinitely. In some cases, treatment may be acute such that at most 1, 2, 3, 4, 5, or 6 doses are administered to a subject for treatment.

In some embodiments, a dose (or sub-dose) or amount of a monoclonal anti-CGRP antagonist antibody may be formulated in a liquid formulation and administered (e.g., via subcutaneous injection, via intravenous injection) to a subject. In such cases, the volume of liquid formulation comprising antibody may vary depending upon, for example, the concentration of antibody in the liquid formulation, the desired dose of antibody, and/or the route of administration used. For example, the volume of liquid formulation comprising an antibody described herein and administered (e.g., via an injection, such as, for example, a subcutaneous injection or an intravenous infusion) to a subject may be from about 0.001 mL to about 10.0 mL, about 0.01 mL to about 5.0 mL, about 0.1 mL to about 5 mL, about 0.1 mL to about 3 mL, about 0.5 mL to about 2.5 mL, or about 1 mL to about 2.5 mL. For example, the volume of liquid formulation comprising a monoclonal anti-CGRP antagonist antibody and administered (e.g., via an injection, such as, for example, a subcutaneous injection, or an intravenous infusion) to a subject may be, may be at least, may be less than, or may be at most about 0.001, 0.005, 0.01, 0.02, 0.03, 0.04, 0.05, 0.06, 0.07, 0.08, 0.09, 0.10, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, or about 10.0 mL.

In some embodiments, a dose (or sub-dose) or amount of a monoclonal anti-CGRP antagonist antibody may be supplied in prefilled receptacles useful in administering antibody to a subject. Such prefilled receptacles may be designed for self-administration or for administration by another. For example, a dose (or sub-dose) or amount of antibody described herein may be supplied as a liquid formulation in pre-filled syringes, pre-filled syringes with a needle safety device, injection pens, or auto-injectors. In such examples, the pre-filled syringes may be designed for self-administration or for administration by another. In some cases, the pre-filled syringes or auto-injectors may be designed for subcutaneous administration and/or intravenous administration.

For the purpose of the present invention, the appropriate dosage of an antibody may depend on the antibody (or compositions thereof) employed, the type and severity of the secondary symptom, the type and severity of the (persistent) post-traumatic headache or other condition to be treated, whether the agent is administered for preventive or therapeutic purposes, previous therapy, the patient's clinical history and response to the agent, and the discretion of the attending physician. Typically, the clinician will administer an antibody, until a dosage is reached that achieves the desired result. Dose and/or frequency can vary over course of treatment.

Empirical considerations, such as the half-life, generally will contribute to the determination of the dosage. For example, antibodies that are compatible with the human immune system, such as humanized antibodies or fully human antibodies, may be used to prolong half-life of the antibody and to prevent the antibody being attacked by the host's immune system. Frequency of administration may be determined and adjusted over the course of therapy, and is generally, but not necessarily, based on treatment and/or suppression and/or amelioration and/or delay of post-traumatic headache or other condition. Alternatively, sustained continuous release formulations of antibodies may be appropriate. Various formulations and devices for achieving sustained release are known in the art.

In one embodiment, dosages for a monoclonal anti-CGRP antagonist antibody may be determined empirically in individuals who have been given one or more administration(s) of the antibody. Individuals are given incremental dosages of an antibody. To assess efficacy of an antibody, an indicator of the disease can be followed.

Administration of a monoclonal anti-CGRP antagonist antibody can be continuous or intermittent, depending, for example, upon the recipient's physiological condition, whether the purpose of the administration is therapeutic or prophylactic, and other factors known to skilled practitioners. The administration of an antibody may be essentially continuous over a preselected period of time or may be in a series of spaced dose, e.g., either before, during, or after developing (persistent) post-traumatic headache; before; during; before and after; during and after; before and during; or before, during, and after developing (persistent) post-traumatic headache. Administration can be before, during and/or after any event likely to give rise to (persistent) post-traumatic headache.

In some embodiments, more than one antibody may be present. At least one, at least two, at least three, at least four, at least five different, or more antibodies can be present. Generally, those antibodies may have complementary activities that do not adversely affect each other. A monoclonal anti-CGRP antagonist antibody can also be used in conjunction with other CGRP antagonists or CGRP receptor antagonists. For example, one or more of the following CGRP antagonists may be used: an anti-sense molecule directed to a CGRP (including an anti-sense molecule directed to a nucleic acid encoding CGRP), a CGRP inhibitory compound, a CGRP structural analog, a dominant-negative mutation of a CGRP receptor that binds a CGRP, and an anti-CGRP receptor antibody. An antibody can also be used in conjunction with other agents that serve to enhance and/or complement the effectiveness of the agents.

Diagnosis or assessment of post-traumatic headache is well-established in the art. Assessment may be performed based on subjective measures, such as patient characterization of symptoms. In some embodiments, assessment of post-traumatic headache may be via headache hours, as described elsewhere herein. For example, assessment of post-traumatic headache may be in terms of daily headache hours, weekly headache hours, monthly headache hours and/or yearly headache hours. In some cases, headache hours may be as reported by the subject.

Treatment efficacy can be assessed by methods well-known in the art. For example, pain relief may be assessed. Accordingly, in some embodiments, pain relief is subjectively observed after 1, 2, or a few hours after administering an anti-CGRP antibody. In some embodiments, frequency of (persistent) post-traumatic headache attacks is subjectively observed after administering an anti-CGRP antibody.

In some embodiments, the composition for use in treating or reducing incidence of post-traumatic headache in a subject may reduce incidence of post-traumatic headache after a single administration of a monoclonal anti-CGRP antagonist antibody for an extended period of time. For example, incidence of (persistent) post-traumatic headache may be reduced for at least 0.5, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 or more days after a single administration.

In some embodiments, the composition for use in treating or reducing incidence of post-traumatic headache in a subject may reduce the number of headache hours experienced by a subject from a pre-administration level after administration of one or more doses of a monoclonal anti-CGRP antagonist antibody to the subject. For example, daily headache hours experienced by the subject after administering one or more doses of an antibody to the subject may be reduced by 0.5, 1, 2, 3, 4, 5, 6, 7, 8, 9, 10, 11, 12, 13, 14, 15, 16, 17, 18, 19, 20, 21, 22, 23, or 24 headache hours from a pre-administration level in the subject. In some cases, daily headache hours experienced by the subject after administering one or more doses of an antibody to the subject may be reduced by 0.5%, 1%, 5%, 10%, 15%, 20%, 25%, 30%, 35%, 40%, 45%, 50%, 55%, 60%, 65%, 70%, 75%, 80%, 85%, 90%, 95%, 99%, or more relative to a pre-administration level in the subject. In another example, weekly headache hours experienced by the subject after administering one or more doses of an antibody to the subject may be reduced by 0.5, 1, 5, 10, 15, 20, 25, 30, 35, 40, 45, 50, 55, 60, 65, 70, 75 or more headache hours from a pre-administration level in the subject. In some cases, weekly headache hours experienced by the subject after administering one or more doses of an antibody to the subject may be reduced by 0.5%, 1%, 5%, 10%, 15%, 20%, 25%, 30%, 35%, 40%, 45%, 50%, 55%, 60%, 65%, 70%, 75%, 80%, 85%, 90%, 95%, 99%, or more relative to a pre-administration level in the subject. In another example, monthly headache hours experienced by the subject after administering one or more doses of an antibody to the subject may be reduced by 0.5, 1, 5, 10, 15, 20, 25, 30, 35, 40, 45, 50, 55, 60, 65, 70, 75, 80, 85, 90, 95, 100, 105, 110, 115, 120, 125, or more headache hours from a pre-administration level. In some cases, weekly headache hours experienced by the subject after administering one or more doses of an antibody to the subject may be reduced by 0.5%, 1%, 5%, 10%, 15%, 20%, 25%, 30%, 35%, 40%, 45%, 50%, 55%, 60%, 65%, 70%, 75%, 80%, 85%, 90%, 95%, 99% or more relative to a pre-administration level in the subject.

In some embodiments, the composition for use in treating or reducing incidence of (persistent) post-traumatic headache in a subject may reduce the number of headache days experienced by a subject from a pre-administration level after administration of one or more doses of a monoclonal anti-CGRP antagonist antibody to the subject. For example, weekly headache days experienced by the subject after administering one or more doses of an antibody to the subject may be reduced by 0.5, 1, 1.5, 2, 2.5, 3, 3.5, 4, 4.5, 5, 5.5, 6, 6.5, or 7 headache days from a pre-administration level in the subject. In some cases, weekly headache days experienced by the subject after administering one or more doses of an antibody to the subject may be reduced by 0.5%, 1%, 5%, 10%, 15%, 20%, 25%, 30%, 35%, 40%, 45%, 50%, 55%, 60%, 65%, 70%, 75%, 80%, 85%, 90%, 95%, 99% or more relative to a pre-administration level in the subject. In another example, monthly headache days experienced by the subject after administering one or more doses of an antibody to the subject may be reduced by 0.5, 1, 2, 3, 4, 5, 6, 7, 8, 9, 10, 15, 20 or more headache days from a pre-administration level.

In some embodiments, a method may comprise administering to a subject one or more additional agent(s) simultaneously or sequentially with a monoclonal anti-CGRP antagonist antibody. In some embodiments, an additional agent may be an anti-headache medication such as an example anti-headache medication (e.g., 5-HT1 agonists, triptans, ergot alkaloids, opiates, β-adrenergic antagonists, NSAIDs) described elsewhere herein. In some embodiments, a therapeutic effect may be greater as compared to use of an antibody or one or more additional agent(s) alone. Accordingly, a synergistic effect between an antibody and the one or more additional agents may be achieved. In some embodiments, the one or more additional agent(s) may be taken by a subject prophylactically.

B. Anti-CGRP antagonist antibodies

The composition for use in the invention comprises an anti-CGRP antagonist antibody. An anti-CGRP antagonist antibody can refer to any antibody molecule that blocks, suppresses or reduces (including significantly) CGRP biological activity, including downstream pathways mediated by CGRP signaling, such as receptor binding and/or elicitation of a cellular response to CGRP.

An anti-CGRP antagonist antibody can exhibit any one or more of the following characteristics: (a) bind to CGRP; (b) block CGRP from binding to its receptor(s); (c) block or decrease CGRP receptor activation (including, but not limited to, cAMP activation); (d) inhibit CGRP biological activity or downstream pathways mediated by CGRP signaling function; (e) prevent, ameliorate, or treat any aspect of post-traumatic headache; (f) increase clearance of CGRP; and (g) inhibit (reduce) CGRP synthesis, production or release. Anti-CGRP antagonist antibodies are known in the art. See e.g., Tan et al., Clin. Sci. (Lond). 89:565-73, 1995; Sigma (Missouri, US), product number C7113 (clone #4901); Plourde et al., Peptides 14:1225-1229, 1993.

In some embodiments, the antibody reacts with CGRP in a manner that inhibits CGRP, and/or the CGRP pathway, including downstream pathways mediated by the CGRP signaling function. In some embodiments, the anti-CGRP antagonist antibody recognizes human CGRP. In some embodiments, the anti-CGRP antagonist antibody binds to both human α-CGRP and β-CGRP. In some embodiments, the anti-CGRP antagonist antibody binds human and rat CGRP. In some embodiments, the anti-CGRP antagonist antibody binds the C-terminal fragment having amino acids 25-37 of CGRP. In some embodiments, the anti-CGRP antagonist antibody binds a C-terminal epitope within amino acids 25-37 of CGRP.

The anti-CGRP antagonist antibody is a monoclonal antibody. In some embodiments, the anti-CGRP antagonist antibody is humanized. In some embodiments, the antibody is human. In some embodiments, the anti-CGRP antagonist antibody is antibody G1 (as described herein). The anti-CGRP antagonist antibody comprises six CDRs of antibody G1. In still other embodiments, the anti-CGRP antagonist antibody comprises the amino acid sequence of the heavy chain variable region shown in Figure 5 (SEQ ID NO:1) and the amino acid sequence of the light chain variable region shown in Figure 5 (SEQ ID NO:2).

QASQSVYHNTYLA (SEQ ID NO:84)

In some embodiments, the antibody comprises a modified constant region, such as a constant region that is immunologically inert described herein. In some embodiments, the constant region is modified as described in Eur. J. Immunol. (1999) 29:2613-2624; PCT Application No. PCT/GB99/01441; and/or UK Patent Application No. 9809951.8. In other embodiments, the antibody comprises a human heavy chain IgG2 constant region comprising the following mutations: A330P331 to S330S331 (amino acid numbering with reference to the wildtype IgG2 sequence). Eur. J. Immunol. (1999) 29:2613-2624. In some embodiments, the antibody comprises a constant region of IgG4 comprising the following mutations: E233F234L235 to P233V234A235. In still other embodiments, the constant region is aglycosylated for N-linked glycosylation. In some embodiments, the constant region is aglycosylated for N-linked glycosylation by mutating the oligosaccharide attachment residue (such as Asn297) and/or flanking residues that are part of the N-glycosylation recognition sequence in the constant region. In some embodiments, the constant region is aglycosylated for N-linked glycosylation. The constant region may be aglycosylated for N-linked glycosylation enzymatically or by expression in a glycosylation deficient host cell.

The binding affinity ($K_D$) of an anti-CGRP antagonist antibody to CGRP (such as human α-CGRP) can be about 0.02 to about 200 nM. In some embodiments, the binding affinity is any of about 200 nM, about 100 nM, about 50 nM, about 10 nM, about 1 nM, about 500 pM, about 100 pM, about 60 pM, about 50 pM, about 20 pM, about 15 pM, about 10 pM, about 5 pM, or about 2 pM. In some embodiments, the binding affinity is less than any of about 250 nM, about 200 nM, about 100 nM, about 50 nM, about 10 nM, about 1 nM, about 500 pM, about 100 pM, or about 50 pM.

One way of determining binding affinity of antibodies to CGRP is by measuring binding affinity of monofunctional Fab fragments of the antibody. To obtain monofunctional Fab fragments, an antibody (for example, IgG) can be cleaved with papain or expressed recombinantly. The affinity of an anti-CGRP Fab fragment of an antibody can be determined by surface plasmon resonance (Biacore3000™ surface plasmon resonance (SPR) system, Biacore, INC, Piscataway NJ) equipped with pre-immobilized streptavidin sensor chips (SA) using HBS-EP running buffer (0.01M HEPES, pH 7.4, 0.15 NaCl, 3 mM EDTA, 0.005% v/v Surfactant P20). Biotinylated human CGRP (or any other CGRP) can be diluted into HBS-EP buffer to a concentration of less than 0.5 μg/mL and injected across the individual chip channels using variable contact times, to achieve two ranges of antigen density, either 50-200 response units (RU) for detailed kinetic studies or 800-1,000 RU for screening assays. Regeneration studies have shown that 25 mM NaOH in 25% v/v ethanol effectively removes the bound Fab while keeping the activity of CGRP on the chip for over 200 injections. Typically, serial dilutions (spanning concentrations of 0.1-10x estimated $K_D$) of purified Fab samples are injected for 1 min at 100 μL/minute and dissociation times of up to 2 hours are allowed. The concentrations of the Fab proteins are determined by ELISA and/or SDS-PAGE electrophoresis using a Fab of known concentration (as determined by amino acid analysis) as a standard. Kinetic association rates ($k_{on}$) and dissociation rates ($k_{off}$) are obtained simultaneously by fitting the data globally to a 1:1 Langmuir binding model (Karlsson, R. Roos, H. Fagerstam, L. Petersson, B. (1994). Methods Enzymology 6. 99-110) using the BIAevaluation program. Equilibrium dissociation constant ($K_D$) values are calculated as $k_{off}/k_{on}$. This protocol is suitable for use in determining binding affinity of an antibody to any CGRP, including human CGRP, CGRP of another mammalian (such as mouse CGRP, rat CGRP, primate CGRP), as well as different forms of CGRP (such as α and β form). Binding affinity of an antibody is generally measured at 25°C, but can also be measured at 37°C.

Antibodies, including anti-CGRP antagonist antibodies, may be made by any method known in the art. The route and schedule of immunization of the host animal are generally in keeping with established and conventional techniques for antibody stimulation and production, as further described herein. General techniques for production of human and mouse antibodies are known in the art and are described herein.

It is contemplated that any mammalian subject including humans or antibody producing cells therefrom can be manipulated to serve as the basis for production of mammalian, including human, hybridoma cell lines. Typically, the host animal is inoculated intraperitoneally, intramuscularly, orally, subcutaneously, intraplantar, and/or intradermally with an amount of immunogen, including as described herein.

Hybridomas can be prepared from the lymphocytes and immortalized myeloma cells using the general somatic cell hybridization technique of Kohler, B. and Milstein, C. (1975) Nature 256:495-497 or as modified by Buck, D. W., et al., In Vitro, 18:377-381 (1982). Available myeloma lines, including but not limited to X63-Ag8.653 and those from the Salk Institute, Cell Distribution Center, San Diego, Calif., USA, may be used in the hybridization. Generally, the technique involves fusing myeloma cells and lymphoid cells using a fusogen such as polyethylene glycol, or by electrical means well known to those skilled in the art. After the fusion, the cells are separated from the fusion medium and grown in a selective growth medium, such as hypoxanthine-aminopterin-thymidine (HAT) medium, to eliminate unhybridized parent cells. Any of the media described herein, supplemented with or without serum, can be used for culturing hybridomas that secrete monoclonal antibodies. As another alternative to the cell fusion technique, EBV immortalized B cells may be used to produce monoclonal antibodies (e.g., monoclonal the anti-CGRP antibodies) of the subject invention. The hybridomas are expanded and subcloned, if desired, and supernatants are assayed for anti-immunogen activity by conventional immunoassay procedures (e.g., radioimmunoassay, enzyme immunoassay, or fluorescence immunoassay).

Hybridomas that may be used as source of antibodies encompass all derivatives, progeny cells of the parent hybridomas that produce monoclonal antibodies specific for CGRP, or a portion thereof.

Hybridomas that produce such antibodies may be grown in vitro or in vivo using known procedures. The monoclonal antibodies may be isolated from the culture media or body fluids, by conventional immunoglobulin purification procedures such as ammonium sulfate precipitation, gel electrophoresis, dialysis, chromatography, and ultrafiltration, if desired. Undesired activity if present, can be removed, for example, by running the preparation over adsorbents made of the immunogen attached to a solid phase and eluting or releasing the desired antibodies off the immunogen. Immunization of a host animal with a human CGRP, or a fragment containing the target amino acid sequence conjugated to a protein that is immunogenic in the species to be immunized, e.g., keyhole limpet hemocyanin, serum albumin, bovine thyroglobulin, or soybean trypsin inhibitor using a bifunctional or derivatizing agent, for example maleimidobenzoyl sulfosuccinimide ester (conjugation through cysteine residues), N-hydroxysuccinimide (through lysine residues), glutaradehyde, succinic anhydride, SOCl2, or R1 N=C=NR, where R and R1 are different alkyl groups, can yield a population of antibodies (e.g., monoclonal antibodies).

If desired, an antibody (e.g., monoclonal or polyclonal anti-CGRP antagonist antibody) of interest may be sequenced and the polynucleotide sequence may then be cloned into a vector for expression or propagation. The sequence encoding the antibody of interest may be maintained in vector in a host cell and the host cell can then be expanded and frozen for future use. In an alternative, the polynucleotide sequence may be used for genetic manipulation to "humanize" the antibody or to improve the affinity, or other characteristics of the antibody. For example, the constant region may be engineered to more resemble human constant regions to avoid immune response if the antibody is used in clinical trials and treatments in humans. It may be desirable to genetically manipulate the antibody sequence to obtain greater affinity to CGRP and greater efficacy in inhibiting CGRP. It will be apparent to one of skill in the art that one or more polynucleotide changes can be made to the anti-CGRP antagonist antibody and still maintain its binding ability to CGRP.

Humanizing a monoclonal antibody can comprise four general steps. These are: (1) determining the nucleotide and predicted amino acid sequence of the starting antibody light and heavy variable domains (2) designing the humanized antibody, i.e., deciding which antibody framework region to use during the humanizing process (3) the actual humanizing methodologies/techniques and (4) the transfection and expression of the humanized antibody. See, for example, U.S. Patent Nos. 4,816,567; 5,807,715; 5,866,692; 6,331,415; 5,530,101; 5,693,761; 5,693,762; 5,585,089; and 6,180,370.

A number of "humanized" antibody molecules comprising an antigen-binding site derived from a non-human immunoglobulin have been described, including chimeric antibodies having rodent or modified rodent V regions and their associated complementarity determining regions (CDRs) fused to human constant domains. See, for example, Winter et al., Nature 349:293-299 (1991), Lobuglio et al., Proc. Nat. Acad. Sci. USA 86:4220-4224 (1989), Shaw et al., J Immunol. 138:4534-4538 (1987), and Brown et al., Cancer Res. 47:3577-3583 (1987). Other references describe rodent CDRs grafted into a human supporting framework region (FR) prior to fusion with an appropriate human antibody constant domain. See, for example, Riechmann et al., Nature 332:323-327 (1988), Verhoeyen et al. Science 239:1534-1536 (1988), and Jones et al., Nature 321:522-525 (1986). Another reference describes rodent CDRs supported by recombinantly veneered rodent framework regions. See, for example, European Patent Publication No. 0519596. These "humanized" molecules are designed to minimize unwanted immunological response toward rodent anti-human antibody molecules which limits the duration and effectiveness of therapeutic applications of those moieties in human recipients. For example, the antibody constant region can be engineered such that it is immunologically inert (e.g., does not trigger complement lysis). See, e.g., PCT Publication No. PCT/GB99/01441; UK Patent Application No. 9809951.8. Other methods of humanizing antibodies that may also be utilized are disclosed by Daugherty et al., Nucl. Acids Res. 19:2471-2476 (1991) and in U.S. Patent Nos. 6,180,377; 6,054,297; 5,997,867; 5,866,692; 6,210,671; and 6,350,861; and in PCT Publication No. WO 01/27160.

In yet another alternative, fully human antibodies may be obtained by using commercially available mice that have been engineered to express specific human immunoglobulin proteins. Transgenic animals that are designed to produce a more desirable (e.g., fully human antibodies) or more robust immune response may also be used for generation of humanized or human antibodies. Examples of such technology are XENOMOUSE™ from Abgenix, Inc. (Fremont, CA) and HuMAb-Mouse® and TC Mouse™ from Medarex, Inc. (Princeton, NJ).

In an alternative, antibodies may be made recombinantly and expressed using any method known in the art. In another alternative, antibodies may be made recombinantly by phage display technology. See, for example, U.S. Patent Nos. 5,565,332; 5,580,717; 5,733,743; and 6,265,150; and Winter et al., Annu. Rev. Immunol. 12:433-455 (1994). Alternatively, the phage display technology (McCafferty et al., Nature 348:552-553 (1990)) can be used to produce human antibodies and antibody fragments in vitro, from immunoglobulin variable (V) domain gene repertoires from unimmunized donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B cell. Phage display can be performed in a variety of formats; for review see, e.g., Johnson, Kevin S. and Chiswell, David J., Current Opinion in Structural Biology 3:564-571 (1993). Several sources of V-gene segments can be used for phage display. Clackson et al., Nature 352:624-628 (1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Mark et al., J. Mol. Biol. 222:581-597 (1991), or Griffith et al., EMBO J. 12:725-734 (1993). In a natural immune response, antibody genes accumulate mutations at a high rate (somatic hypermutation). Some of the changes introduced will confer higher affinity, and B cells displaying high-affinity surface immunoglobulin are preferentially replicated and differentiated during subsequent antigen challenge. This natural process can be mimicked by employing the technique known as "chain shuffling." Marks, et al., Bio/Technol. 10:779-783 (1992)). In this method, the affinity of "primary" human antibodies obtained by phage display can be improved by sequentially replacing the heavy and light chain V region genes with repertoires of naturally occurring variants (repertoires) of V domain genes obtained from unimmunized donors. This technique allows the production of antibodies and antibody fragments with affinities in the pM-nM range. A strategy for making very large phage antibody repertoires (also known as "the mother-of-all libraries") has been described by Waterhouse et al., Nucl. Acids Res. 21:2265-2266 (1993). Gene shuffling can also be used to derive human antibodies from rodent antibodies, where the human antibody has similar affinities and specificities to the starting rodent antibody. According to this method, which is also referred to as "epitope imprinting", the heavy or light chain V domain gene of rodent antibodies obtained by phage display technique is replaced with a repertoire of human V domain genes, creating rodent-human chimeras. Selection on antigen results in isolation of human variable regions capable of restoring a functional antigen-binding site, i.e., the epitope governs (imprints) the choice of partner. When the process is repeated in order to replace the remaining rodent V domain, a human antibody is obtained (see PCT Publication No. WO 93/06213, published April 1, 1993). Unlike traditional humanization of rodent antibodies by CDR grafting, this technique provides completely human antibodies, which have no framework or CDR residues of rodent origin.

It is apparent that although the above discussion pertains to humanized antibodies, the general principles discussed are applicable to customizing antibodies for use, for example, in dogs, cats, primate, equines and bovines. It is further apparent that one or more aspects of humanizing an antibody described herein may be combined, e.g., CDR grafting, framework mutation and CDR mutation.

Antibodies may be made recombinantly by first isolating the antibodies and antibody producing cells from host animals, obtaining the gene sequence, and using the gene sequence to express the antibody recombinantly in host cells (e.g., CHO cells). Another method which may be employed is to express the antibody sequence in plants (e.g., tobacco) or transgenic milk. Methods for expressing antibodies recombinantly in plants or milk have been disclosed. See, for example, Peeters, et al. Vaccine 19:2756 (2001); Lonberg, N. and D. Huszar Int. Rev. Immunol 13:65 (1995); and Pollock, et al., J Immunol Methods 231:147(1999). Methods for making derivatives of antibodies, e.g., humanized, single chain, etc. are known in the art.

Immunoassays and flow cytometry sorting techniques such as fluorescence activated cell sorting (FACS) can also be employed to isolate antibodies that are specific for CGRP.

The antibodies can be bound to many different carriers. Carriers can be active and/or inert. Examples of well-known carriers include polypropylene, polystyrene, polyethylene, dextran, nylon, amylases, glass, natural and modified celluloses, polyacrylamides, agaroses and magnetite. The nature of the carrier can be either soluble or insoluble. Those skilled in the art will know of other suitable carriers for binding antibodies, or will be able to ascertain such, using routine experimentation. In some embodiments, the carrier comprises a moiety that targets the myocardium.

DNA encoding the monoclonal antibodies is readily isolated and sequenced using conventional procedures (e.g., by using oligonucleotide probes that are capable of binding specifically to genes encoding the heavy and light chains of the monoclonal antibodies). The hybridoma cells serve as a preferred source of such DNA. Once isolated, the DNA may be placed into expression vectors (such as expression vectors disclosed in PCT Publication No. WO 87/04462), which are then transfected into host cells such as E. coli cells, simian COS cells, Chinese hamster ovary (CHO) cells, or myeloma cells that do not otherwise produce immunoglobulin protein, to obtain the synthesis of monoclonal antibodies in the recombinant host cells. See, e.g., PCT Publication No. WO 87/04462. The DNA also may be modified, for example, by substituting the coding sequence for human heavy and light chain constant domains in place of the homologous murine sequences, Morrison et al., Proc. Nat. Acad. Sci. 81:6851 (1984), or by covalently joining to the immunoglobulin coding sequence all or part of the coding sequence for a non-immunoglobulin polypeptide. In that manner, "chimeric" or "hybrid" antibodies are prepared that have the binding specificity of an anti-CGRP monoclonal antibody herein.

Antibodies (e.g., anti-CGRP antagonist antibodies) and polypeptides derived from antibodies can be identified or characterized using methods known in the art, whereby reduction, amelioration, or neutralization of a CGRP biological activity is detected and/or measured. For example, anti-CGRP antagonist antibody can also be identified by incubating a candidate agent with CGRP and monitoring any one or more of the following characteristics: (a) bind to CGRP; (b) block CGRP from binding to its receptor(s); (c) block or decrease CGRP receptor activation (including cAMP activation); (d) inhibit CGRP biological activity or downstream pathways mediated by CGRP signaling function; (e) prevent, ameliorate, or treat any aspect of post-traumatic headache; (f) increase clearance of CGRP; and (g) inhibit (reduce) CGRP synthesis, production or release. In some embodiments, an anti-CGRP antagonist antibody or polypeptide is identified by incubating a candidate agent with CGRP and monitoring binding and/or attendant reduction or neutralization of a biological activity of CGRP. The binding assay may be performed with purified CGRP polypeptide(s), or with cells naturally expressing, or transfected to express, CGRP polypeptide(s). In one embodiment, the binding assay is a competitive binding assay, where the ability of a candidate antibody to compete with a known anti-CGRP antagonist for CGRP binding is evaluated. The assay may be performed in various formats, including the ELISA format. In other embodiments, an anti-CGRP antagonist antibody is identified by incubating a candidate agent with CGRP and monitoring binding and attendant inhibition of CGRP receptor activation expressed on the surface of a cell. Following initial identification, the activity of a candidate antibody (e.g., anti-CGRP antagonist antibody) can be further confirmed and refined by bioassays, known to test the targeted biological activities. Alternatively, bioassays can be used to screen candidates directly. For example, CGRP promotes a number of measurable changes in responsive cells. These include, but are not limited to, stimulation of cAMP in the cell (e.g., SK-N-MC cells). Antagonist activity may also be measured using animal models, such as measuring skin vasodilatation induced by stimulation of the rat saphenous nerve. Escott et al., Br. J. Pharmacol. 110: 772-776, 1993. Animal models of post-traumatic headaches may further be used for testing efficacy of antagonist antibodies or polypeptides. Reuter, et al., Functional Neurology (15) Suppl.3, 2000. Some of the methods for identifying and characterizing anti-CGRP antagonist antibody or polypeptide are described in detail in the Examples.

Antibodies, including anti-CGRP antagonist antibodies, may be characterized using methods well known in the art. For example, one method is to identify the epitope to which it binds, or "epitope mapping." There are many methods known in the art for mapping and characterizing the location of epitopes on proteins, including solving the crystal structure of an antibody-antigen complex, competition assays, gene fragment expression assays, and synthetic peptide-based assays, as described, for example, in Chapter 11 of Harlow and Lane, Using Antibodies, a Laboratory Manual, Cold Spring Harbor Laboratory Press, Cold Spring Harbor, New York, 1999. In an additional example, epitope mapping can be used to determine the sequence to which an anti-CGRP antagonist antibody binds. Epitope mapping is commercially available from various sources, for example, Pepscan Systems (Edelhertweg 15, 8219 PH Lelystad, The Netherlands). The epitope can be a linear epitope, i.e., contained in a single stretch of amino acids, or a conformational epitope formed by a three-dimensional interaction of amino acids that may not necessarily be contained in a single stretch. Peptides of varying lengths (e.g., at least 4-6 amino acids long) can be isolated or synthesized (e.g., recombinantly) and used for binding assays with an anti-CGRP antagonist antibody. In another example, the epitope to which the anti-CGRP antagonist antibody binds can be determined in a systematic screening by using overlapping peptides derived from the CGRP sequence and determining binding by the anti-CGRP antagonist antibody. According to the gene fragment expression assays, the open reading frame encoding CGRP is fragmented either randomly or by specific genetic constructions and the reactivity of the expressed fragments of CGRP with the antibody to be tested is determined. The gene fragments may, for example, be produced by PCR and then transcribed and translated into protein in vitro, in the presence of radioactive amino acids. The binding of the antibody to the radioactively labeled CGRP fragments is then determined by immunoprecipitation and gel electrophoresis. Certain epitopes can also be identified by using large libraries of random peptide sequences displayed on the surface of phage particles (phage libraries). Alternatively, a defined library of overlapping peptide fragments can be tested for binding to the test antibody in simple binding assays. In an additional example, mutagenesis of an antigen binding domain, domain swapping experiments and alanine scanning mutagenesis can be performed to identify residues required, sufficient, and/or necessary for epitope binding. For example, domain swapping experiments can be performed using a mutant CGRP in which various fragments of the CGRP polypeptide have been replaced (swapped) with sequences from a closely related, but antigenically distinct protein (such as another member of the neurotrophin protein family). By assessing binding of the antibody to the mutant CGRP, the importance of the particular CGRP fragment to antibody binding can be assessed.

Yet another method which can be used to characterize an antibody, including an anti-CGRP antagonist antibody, is to use competition assays with other antibodies known to bind to the same antigen, i.e., various fragments on CGRP, to determine if the anti-CGRP antagonist antibody binds to the same epitope as other antibodies. Competition assays are well known to those of skill in the art.

An expression vector can be used to direct expression of an antibody, including an anti-CGRP antagonist antibody. One skilled in the art is familiar with administration of expression vectors to obtain expression of an exogenous protein in vivo. See, e.g., U.S. Patent Nos. 6,436,908; 6,413,942; and 6,376,471. Administration of expression vectors includes local or systemic administration, including injection, oral administration, particle gun or catheterized administration, and topical administration. In another embodiment, the expression vector is administered directly to the sympathetic trunk or ganglion, or into a coronary artery, atrium, ventrical, or pericardium.

Targeted delivery of therapeutic compositions containing an expression vector, or subgenomic polynucleotides can also be used. Receptor-mediated DNA delivery techniques are described in, for example, Findeis et al., Trends Biotechnol. (1993) 11:202; Chiou et al., Gene Therapeutics: Methods and Applications of Direct Gene Transfer (J.A. Wolff, ed.) (1994); Wu et al., J. Biol. Chem. (1988) 263:621; Wu et al., J. Biol. Chem. (1994) 269:542; Zenke et al., Proc. Natl. Acad. Sci. USA (1990) 87:3655; Wu et al., J. Biol. Chem. (1991) 266:338. Therapeutic compositions containing a polynucleotide are administered in a range of about 100 ng to about 200 mg of DNA for local administration in a gene therapy protocol. Concentration ranges of about 500 ng to about 50 mg, about 1 $\mu$g to about 2 mg, about 5 $\mu$g to about 500 $\mu$g, and about 20 $\mu$g to about 100 $\mu$g of DNA can also be used during a gene therapy protocol. The therapeutic polynucleotides and polypeptides can be delivered using gene delivery vehicles. The gene delivery vehicle can be of viral or non-viral origin (see generally, Jolly, Cancer Gene Therapy (1994) 1:51; Kimura, Human Gene Therapy (1994) 5:845; Connelly, Human Gene Therapy (1995) 1:185; and Kaplitt, Nature Genetics (1994) 6:148). Expression of such coding sequences can be induced using endogenous mammalian or heterologous promoters. Expression of the coding sequence can be either constitutive or regulated.

Viral-based vectors for delivery of a desired polynucleotide and expression in a desired cell are well known in the art. Exemplary viral-based vehicles include, but are not limited to, recombinant retroviruses (see, e.g., PCT Publication Nos. WO 90/07936; WO 94/03622; WO 93/25698; WO 93/25234; WO 93/11230; WO 93/10218; WO 91/02805; U.S. Patent Nos. 5, 219,740 and 4,777,127; GB Patent No. 2,200,651; and EP Patent No. 0 345 242), alphavirus-based vectors (e.g., Sindbis virus vectors, Semliki forest virus (ATCC VR-67; ATCC VR-1247), Ross River virus (ATCC VR-373; ATCC VR-1246) and Venezuelan equine encephalitis virus (ATCC VR-923; ATCC VR-1250; ATCC VR 1249; ATCC VR-532)), and adeno-associated virus (AAV) vectors (see, e.g., PCT Publication Nos. WO 94/12649, WO 93/03769; WO 93/19191; WO 94/28938; WO 95/11984 and WO 95/00655). Administration of DNA linked to killed adenovirus as described in Curiel, Hum. Gene Ther. (1992) 3:147 can also be employed.

Non-viral delivery vehicles and methods can also be employed, including, but not limited to, polycationic condensed DNA linked or unlinked to killed adenovirus alone (see, e.g., Curiel, Hum. Gene Ther. (1992) 3:147); ligand-linked DNA (see, e.g., Wu, J. Biol. Chem. (1989) 264:16985); eukaryotic cell delivery vehicles cells (see, e.g., U.S. Patent No. 5,814,482; PCT Publication Nos. WO 95/07994; WO 96/17072; WO 95/30763; and WO 97/42338) and nucleic charge neutralization or fusion with cell membranes. Naked DNA can also be employed. Exemplary naked DNA introduction methods are described in PCT Publication No. WO 90/11092 and U.S. Patent No. 5,580,859. Liposomes that can act as gene delivery vehicles are described in U.S. Patent No. 5,422,120; PCT Publication Nos. WO 95/13796; WO 94/23697; WO 91/14445; and EP 0524968. Additional approaches are described in Philip, Mol. Cell Biol. (1994) 14:2411, and in Woffendin, Proc. Natl. Acad. Sci. (1994) 91:1581.

C. Antibody G1 and related antibodies, polypeptides, polynucleotides, vectors and host cells

This invention encompasses compositions, including pharmaceutical compositions, comprising antibody G1 for use in the invention. These compositions may further comprise suitable excipients, such as pharmaceutically acceptable excipients including buffers, which are well known in the art.

In some embodiments, the anti-CGRP antagonist antibodies used in the invention are characterized by any (one or more) of the following characteristics: (a) bind to CGRP; (b) block CGRP from binding to its receptor(s); (c) block or decrease CGRP receptor activation (including cAMP activation); (d) inhibit CGRP biological activity or downstream pathways mediated by CGRP signaling function; (e) prevent, ameliorate, or treat any aspect of post-traumatic headache; (f) increase clearance of CGRP; and (g) inhibit (reduce) CGRP synthesis, production or release.

In some embodiments, the invention uses any of the following, or compositions (including pharmaceutical compositions) comprising any of the following: (a) antibody G1; (b) a fragment or a region of antibody G1; (c) a light chain of antibody G1; (d) a heavy chain of antibody G1; (e) one or more variable region(s) from a light chain and/or a heavy chain of antibody G1; (f) six CDRs of antibody G1; (k) three CDRs from the light chain and three CDRs from the heavy chain, of antibody G1.

The CDR portions of antibody G1 (including Chothia and Kabat CDRs) are diagrammatically depicted in Figure 5. Determination of CDR regions is well within the skill of the art. It is understood that in some embodiments, CDRs can be a combination of the Kabat and Chothia CDR (also termed "combined CDRs" or "extended CDRs"). In some embodiments, the CDRs are the Kabat CDRs. In other embodiments, the CDRs are the Chothia CDRs. In other words, in embodiments with more than one CDR, the CDRs may be any of Kabat, Chothia, combination CDRs, or combinations thereof.

Disclosed herein is a polypeptide (which may or may not be an antibody) which comprises at least one CDR, at least two, at least three, or at least four, at least five, or all six CDRs that are substantially identical to at least one CDR, at least two, at least three, at least four, at least five or all six CDRs of G1 or its variants shown in Table 6. Also disclosed are antibodies which have at least two, three, four, five, or six CDR(s) that are substantially identical to at least two, three, four, five or six CDRs of G1 or derived from G1. The at least one, two, three, four, five, or six CDR(s) are at least about 85%, 86%, 87%, 88%, 89%, 90%, 95%, 96%, 97%, 98%, or 99% identical to at least one, two, three, four, five or six CDRs of G1 or its variants shown in Table 6. It is understood that binding specificity and/or overall activity is generally retained, although the extent of activity may vary compared to G1 or its variants shown in Table 6 (may be greater or lesser).

Also disclosed herein is a polypeptide (which may or may not be an antibody) which comprises an amino acid sequence of G1 or its variants shown in Table 6 that has any of the following: at least 5 contiguous amino acids, at least 8 contiguous amino acids, at least about 10 contiguous amino acids, at least about 15 contiguous amino acids, at least about 20 contiguous amino acids, at least about 25 contiguous amino acids, at least about 30 contiguous amino acids of a sequence of G1 or its variants shown in Table 6, wherein at least 3 of the amino acids are from a variable region of G1 (Figure 5) or its variants shown in Table 6. An exemplary polypeptide has contiguous amino acid (lengths described above) from both the heavy and light chain variable regions of G1.

The binding affinity ($K_D$) of an anti-CGRP antagonist antibody and polypeptide to CGRP (such as human α-CGRP) can be about 0.06 to about 200 nM. In some embodiments, the binding affinity is any of about 200 nM, 100 nM, about 50 nM, about 10 nM, about 1 nM, about 500 pM, about 100 pM, about 60 pM, about 50 pM, about 20 pM, about 15 pM, about 10 pM, about 5 pM, or about 2 pM. In some embodiments, the binding affinity is less than any of about 250 nM, about 200 nM, about 100 nM, about 50 nM, about 10 nM, about 1 nM, about 500 pM, about 100 pM, or about 50 pM.

Disclosed herein methods of making any of these antibodies or polypeptides. The antibodies used in this in-

vention can be made by procedures known in the art. The polypeptides can be produced by proteolytic or other degradation of the antibodies, by recombinant methods (i.e., single or fusion polypeptides) as described above or by chemical synthesis. Polypeptides of the antibodies, especially shorter polypeptides up to about 50 amino acids, are conveniently made by chemical synthesis. Methods of chemical synthesis are known in the art and are commercially available. For example, an antibody could be produced by an automated polypeptide synthesizer employing the solid phase method. See also, U.S. Patent Nos. 5,807,715; 4,816,567; and 6,331,415.

In another alternative, the antibodies can be made recombinantly using procedures that are well known in the art. In one embodiment, a polynucleotide comprises a sequence encoding the heavy chain and/or the light chain variable regions of antibody G1 shown in SEQ ID NO:9 and SEQ ID NO:10. In another embodiment, the polynucleotide comprising the nucleotide sequence shown in SEQ ID NO:9 and SEQ ID NO:10 are cloned into one or more vectors for expression or propagation. The sequence encoding the antibody of interest may be maintained in a vector in a host cell and the host cell can then be expanded and frozen for future use. Vectors (including expression vectors) and host cells are further described herein.

In some embodiments, the invention also uses single chain variable region fragments ("scFv") of antibodies such as G1. Single chain variable region fragments are made by linking light and/or heavy chain variable regions by using a short linking peptide. Bird et al. (1988) Science 242:423-426. An example of a linking peptide is (GGGGS)3 (SEQ ID NO:57) which bridges approximately 3.5 nm between the carboxy terminus of one variable region and the amino terminus of the other variable region. Linkers of other sequences have been designed and used. Bird et al. (1988). Linkers can in turn be modified for additional functions, such as attachment of drugs or attachment to solid supports. The single chain variants can be produced either recombinantly or synthetically. For synthetic production of scFv, an automated synthesizer can be used. For recombinant production of scFv, a suitable plasmid containing polynucleotide that encodes the scFv can be introduced into a suitable host cell, either eukaryotic, such as yeast, plant, insect or mammalian cells, or prokaryotic, such as E. coli. Polynucleotides encoding the scFv of interest can be made by routine manipulations such as ligation of polynucleotides. The resultant scFv can be isolated using standard protein purification techniques known in the art.

Other forms of single chain antibodies, such as diabodies are also encompassed. Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see e.g., Holliger, P., et al. (1993) Proc. Natl. Acad Sci. USA 90:6444-6448; Poljak, R. J., et al. (1994) Structure 2:1121-1123).

For example, bispecific antibodies, monoclonal antibodies that have binding specificities for at least two different antigens, can be prepared using the antibodies disclosed herein. Methods for making bispecific antibodies are known in the art (see, e.g., Suresh et al., 1986, Methods in Enzymology 121:210). Traditionally, the recombinant production of bispecific antibodies was based on the coexpression of two immunoglobulin heavy chain-light chain pairs, with the two heavy chains having different specificities (Millstein and Cuello, 1983, Nature 305, 537-539).

According to one approach to making bispecific antibodies, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2 and CH3 regions. It is preferred to have the first heavy chain constant region (CH1), containing the site necessary for light chain binding, present in at least one of the fusions. DNAs encoding the immunoglobulin heavy chain fusions and, if desired, the immunoglobulin light chain, are inserted into separate expression vectors, and are cotransfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In one approach, the bispecific antibodies are composed of a hybrid immunoglobulin heavy chain with a first binding specificity in one arm, and a hybrid immunoglobulin heavy chain-light chain pair (providing a second binding specificity) in the other arm. This asymmetric structure, with an immunoglobulin light chain in only one half of the bispecific molecule, facilitates the separation of the desired bispecific compound from unwanted immunoglobulin chain combinations. This approach is described in PCT Publication No. WO 94/04690, published March 3, 1994.

Heteroconjugate antibodies, comprising two covalently joined antibodies, can be used in the invention. Such antibodies have been used to target immune system cells to unwanted cells (U.S. Patent No. 4,676,980), and for treatment of HIV infection (PCT application publication Nos. WO 91/00360 and WO 92/200373; EP 03089). Heteroconjugate antibodies may be made using any convenient cross-linking methods. Suitable cross-linking agents and techniques are well known in the art, and are described in U.S. Patent No. 4,676,980.

Chimeric or hybrid antibodies also may be prepared in vitro using known methods of synthetic protein chemistry, including those involving cross-linking agents. For example, immunotoxins may be constructed using a disulfide exchange reaction or by forming a thioether bond. Examples of suitable reagents for this purpose include iminothiolate and methyl-4-mercaptobutyrimidate.

Humanized antibody comprising one or more CDRs of antibody G1 or its variants shown in Table 6, or one or more CDRs derived from antibody G1 or its variants shown in Table 6 can be made using any methods known in the art. For example, four general steps may be used to humanize a monoclonal antibody.

In some embodiments, the invention encompasses modifications to antibody G1, including functionally equivalent antibodies which do not significantly affect their properties and variants which have enhanced or decreased activity and/or affinity. For example, the amino acid sequence of antibody G1 may be mutated to obtain an antibody with the desired binding affinity to CGRP. Modification of polypeptides is routine practice in the art and need not be described in detail herein. Modification of polypeptides is exemplified in the Examples. Examples of modified polypeptides include polypeptides with conservative substitutions of amino acid residues, one or more deletions or additions of amino acids which do not significantly deleteriously change the functional activity, or use of chemical analogs.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue or the antibody fused to an epitope tag. Other insertional variants of the antibody molecule include the fusion to the N- or C-terminus of the antibody of an enzyme or a polypeptide which increases the serum half-life of the antibody.

Substitution variants have at least one amino acid residue in the antibody molecule removed and a different residue inserted in its place. The sites of greatest interest for substitutional mutagenesis include the hypervariable regions, but FR alterations are also contemplated. Conservative substitutions are shown in Table 1 under the heading of "conservative substitutions". If such substitutions result in a change in biological activity, then more substantial changes, denominated "exemplary substitutions" in Table 1, or as further described below in reference to amino acid classes, may be introduced and the products screened.

Table 1: Amino Acid Substitutions

| Original Residue | Conservative Substitutions | Exemplary Substitutions |
|---|---|---|
| Ala (A) | Val | Val; Leu; Ile |
| Arg (R) | Lys | Lys; Gln; Asn |
| Asn (N) | Gln | Gln; His; Asp, Lys; Arg |
| Asp (D) | Glu | Glu; Asn |
| Cys (C) | Ser | Ser; Ala |
| Gln (Q) | Asn | Asn; Glu |
| Glu (E) | Asp | Asp; Gln |
| Gly (G) | Ala | Ala |
| His (H) | Arg | Asn; Gln; Lys; Arg |
| Ile (I) | Leu | Leu; Val; Met; Ala; Phe; Norleucine |
| Leu (L) | Ile | Norleucine; Ile; Val; Met; Ala; Phe |
| Lys (K) | Arg | Arg; Gln; Asn |
| Met (M) | Leu | Leu; Phe; Ile |
| Phe (F) | Tyr | Leu; Val; Ile; Ala; Tyr |
| Pro (P) | Ala | Ala |
| Ser (S) | Thr | Thr |
| Thr (T) | Ser | Ser |
| Trp (W) | Tyr | Tyr; Phe |
| Tyr (Y) | Phe | Trp; Phe; Thr; Ser |
| Val (V) | Leu | Ile; Leu; Met; Phe; Ala; Norleucine |

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:

(1) Non-polar: Norleucine, Met, Ala, Val, Leu, Ile;

(2) Polar without charge: Cys, Ser, Thr, Asn, Gln;

(3) Acidic (negatively charged): Asp, Glu;

(4) Basic (positively charged): Lys, Arg;

(5) Residues that influence chain orientation: Gly, Pro; and

(6) Aromatic: Trp, Tyr, Phe, His.

Non-conservative substitutions are made by exchanging a member of one of these classes for another class.

Any cysteine residue not involved in maintaining the proper conformation of the antibody also may be substituted, generally with serine, to improve the oxidative stability of the molecule and prevent aberrant cross-linking. Conversely, cysteine bond(s) may be added to the antibody to improve its stability, particularly where the antibody is an antibody fragment such as an Fv fragment.

Amino acid modifications can range from changing or modifying one or more amino acids to complete redesign of a region, such as the variable region. Changes in the variable region can alter binding affinity and/or specificity.

Modifications also include glycosylated and nonglycosylated polypeptides, as well as polypeptides with other post-translational modifications, such as, for example, glycosylation with different sugars, acetylation, and phosphorylation. Antibodies are glycosylated at conserved positions in their constant regions (Jefferis and Lund, 1997, Chem. Immunol. 65:111-128; Wright and Morrison, 1997, TibTECH 15:26-32). The oligosaccharide side chains of the immunoglobulins affect the protein's function (Boyd et al., 1996, Mol. Immunol. 32:1311-1318; Wittwe and Howard, 1990, Biochem. 29:4175-4180) and the intramolecular interaction between portions of the glycoprotein, which can affect the conformation and presented three-dimensional surface of the glycoprotein (Hefferis and Lund, supra; Wyss and Wagner, 1996, Current Opin. Biotech. 7:409-416). Oligosaccharides may also serve to target a given glycoprotein to certain molecules based upon specific recognition structures. Glycosylation of antibodies has also been reported to affect antibody-dependent cellular cytotoxicity (ADCC). In particular, CHO cells with tetracycline-regulated expression of $\beta(1,4)$-N-acetylglucosaminyltransferase III (GnTIII), a glycosyltransferase catalyzing formation of bisecting GlcNAc, was reported to have improved ADCC activity (Umana et al., 1999, Mature Biotech. 17:176-180).

Glycosylation of antibodies is typically either N-linked or O-linked. N-linked refers to the attachment of the carbohydrate moiety to the side chain of an asparagine residue. The tripeptide sequences asparagine-X-serine, asparagine-X-threonine, and asparagine-X-cysteine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-acetylgalactosamine, galactose, or xylose to a hydroxyamino acid, most commonly serine or threonine, although 5-hydroxyproline or 5-hydroxylysine may also be used.

Addition of glycosylation sites to the antibody is conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original antibody (for O-linked glycosylation sites).

The glycosylation pattern of antibodies may also be altered without altering the underlying nucleotide sequence. Glycosylation largely depends on the host cell used to express the antibody. Since the cell type used for expression of recombinant glycoproteins, e.g., antibodies, as potential therapeutics is rarely the native cell, variations in the glycosylation pattern of the antibodies can be expected (see, e.g., Hse et al., 1997, J. Biol. Chem. 272:9062-9070).

In addition to the choice of host cells, factors that affect glycosylation during recombinant production of antibodies include growth mode, media formulation, culture density, oxygenation, pH, purification schemes and the like. Various methods have been proposed to alter the glycosylation pattern achieved in a particular host organism including introducing or overexpressing certain enzymes involved in oligosaccharide production (U.S. Patent Nos. 5,047,335; 5,510,261, and 5,278,299). Glycosylation, or certain types of glycosylation, can be enzymatically removed from the glycoprotein, for example using endoglycosidase H (Endo H), N-glycosidase F, endoglycosidase F1, endoglycosidase F2, endoglycosidase F3. In addition, the recombinant host cell can be genetically engineered to be defective in processing certain types of polysaccharides. These and similar techniques are well known in the art.

Other methods of modification include using coupling techniques known in the art, including, but not limited to, enzymatic means, oxidative substitution and chelation. Modifications can be used, for example, for attachment of labels for immunoassay. Modified G1 polypeptides can be made using established procedures in the art and can be screened using standard assays known in the art, some of which are described below and in the Examples.

In some embodiments of the invention, the antibody comprises a modified constant region, such as a constant region that is immunologically inert or partially inert, e.g., does not trigger complement mediated lysis, does not stimulate antibody-dependent cell mediated cytotoxicity (ADCC), or does not activate microglia; or have reduced activities (compared to the unmodified antibody) in any one or more of the following: triggering complement mediated lysis, stimulating antibody-dependent cell mediated cytotoxicity (ADCC), or activating microglia. Different modifications of the constant region may be used to achieve optimal level and/or combination of effector functions. See, for example, Morgan et al., Immunology 86:319-324 (1995); Lund et al., J. Immunology 157:4963-9 157:4963-4969 (1996); Idusogie et al., J. Immunology 164:4178-4184 (2000); Tao et al., J. Immunology 143: 2595-2601 (1989); and Jefferis et al., Immunological Reviews 163:59-76 (1998). In some embodiments, the constant region is modified as described in Eur. J. Immunol. (1999) 29:2613-2624; PCT Application No. PCT/GB99/01441; and/or UK Patent Application No. 9809951.8. In other embodiments, the antibody comprises a human heavy chain IgG2 constant region comprising the following mutations: A330P331 to S330S331 (amino acid numbering with reference to the wildtype IgG2 sequence). Eur. J. Immunol. (1999) 29:2613-2624. In still other embodiments, the constant region is aglycosylated for N-linked glycosylation. In some embodiments, the constant region is aglycosylated for N-linked glycosylation by mutating the glycosylated amino acid residue or flanking residues that are part of the N-glycosylation recognition sequence in the constant region. For example, N-glycosylation site N297 may be mutated to A, Q, K, or H. See, Tao et al., J. Immunology 143: 2595-2601 (1989); and Jefferis et al., Immunological Reviews 163:59-76 (1998). In some embodiments, the constant region is aglycosylated for N-linked glycosylation. The constant region may be aglycosylated for N-linked glycosylation enzymatically (such as removing carbohydrate by enzyme PNGase), or by expression in a glycosylation deficient host cell.

Other antibody modifications include antibodies that have been modified as described in PCT Publication No. WO 99/58572, published November 18, 1999. These antibodies comprise, in addition to a binding domain directed at the target molecule, an effector domain having an amino acid sequence substantially homologous to all or part of a constant domain of a human immunoglobulin heavy chain. These antibodies are capable of binding the target molecule without triggering significant complement dependent lysis, or cell-mediated destruction of the target. In some embodiments, the effector domain is capable of specifically binding FcRn and/or FcγRIIb. These are typically based on chimeric domains derived from two or more human immunoglobulin heavy chain $C_H2$ domains. Antibodies modified in this manner are particularly suitable for use in chronic antibody therapy, to avoid inflammatory and other adverse reactions to conventional antibody therapy.

In some embodiments, affinity matured embodiments are used in the invention. For example, affinity matured antibodies can be produced by procedures known in the art (Marks et al., 1992, Bio/Technology, 10:779-783; Barbas et al., 1994, Proc Nat. Acad. Sci, USA 91:3809-3813; Schier et al., 1995, Gene, 169:147-155; Yelton et al., 1995, J. Immunol., 155:1994-2004; Jackson et al., 1995, J. Immunol., 154(7):3310-9; Hawkins et al, 1992, J. Mol. Biol., 226:889-896; and WO2004/058184).

The following methods may be used for adjusting the affinity of an antibody and for characterizing a CDR. One way of characterizing a CDR of an antibody and/or altering (such as improving) the binding affinity of a polypeptide, such as an antibody, termed "library scanning mutagenesis". Generally, library scanning mutagenesis works as follows. One or more amino acid positions in the CDR are replaced with two or more (such as 3, 4, 5, 6, 7, 8, 9, 10, 11, 12, 13, 14, 15, 16, 17, 18, 19, or 20) amino acids using art recognized methods. This generates small libraries of clones (in some embodiments, one for every amino acid position that is analyzed), each with a complexity of two or more members (if two or more amino acids are substituted at every position). Generally, the library also includes a clone comprising the native (unsubstituted) amino acid. A small number of clones, e.g., about 20-80 clones (depending on the complexity of the library), from each library are screened for binding affinity to the target polypeptide (or other binding target), and candidates with increased, the same, decreased or no binding are identified. Methods for determining binding affinity are well-known in the art. Binding affinity may be determined using Biacore surface plasmon resonance analysis, which detects differences in binding affinity of about 2-fold or greater. Biacore is particularly useful when the starting antibody already binds with a relatively high affinity, for example a $K_D$ of about 10 nM or lower. Screening using Biacore surface plasmon resonance is described in the Examples, herein.

Binding affinity may be determined using Kinexa Biocensor, scintillation proximity assays, ELISA, ORIGEN immunoassay (IGEN), fluorescence quenching, fluorescence transfer, and/or yeast display. Binding affinity may also be screened using a suitable bioassay.

In some embodiments, every amino acid position in a CDR is replaced (in some embodiments, one at a time) with all 20 natural amino acids using art recognized mutagenesis methods (some of which are described herein). This generates small libraries of clones (in some embodiments, one for every amino acid position that is analyzed), each with a complexity of 20 members (if all 20 amino acids are substituted at every position).

In some embodiments, the library to be screened comprises substitutions in two or more positions, which may be in the same CDR or in two or more CDRs. Thus, the library may comprise substitutions in two or more positions in one CDR. The library may comprise substitution in two or more positions in two or more CDRs. The library may comprise substitution in 3, 4, 5, or more positions, said positions found in two, three, four, five or six CDRs. The substitution may be prepared using low redundancy codons. See, e.g., Table 2 of Balint et al., (1993) Gene 137(1):109-18.

Candidates with improved binding may be sequenced, thereby identifying a CDR substitution mutant which results in improved affinity (also termed an "improved" substitution). Candidates that bind may also be sequenced, thereby identifying a CDR substitution which retains binding.

Multiple rounds of screening may be conducted. For example, candidates (each comprising an amino acid substitution at one or more position of one or more CDR) with improved binding are also useful for the design of a second library containing at least the original and substituted amino acid at each improved CDR position (i.e., amino acid position in the CDR at which a substitution mutant showed improved binding). Preparation, and screening or selection of this library is discussed further below.

Library scanning mutagenesis also provides a means for characterizing a CDR, in so far as the frequency of clones with improved binding, the same binding, decreased binding or no binding also provide information relating to the importance of each amino acid position for the stability of the antibody-antigen complex. For example, if a position of the CDR retains binding when changed to all 20 amino acids, that position is identified as a position that is unlikely to be required for antigen binding. Conversely, if a position of CDR retains binding in only a small percentage of substitutions, that position is identified as a position that is important to CDR function. Thus, the library scanning mutagenesis methods generate information regarding positions in the CDRs that can be changed to many different amino acids (including all 20 amino acids), and positions in the CDRs which cannot be changed or which can only be changed to a few amino acids.

Candidates with improved affinity may be combined in a second library, which includes the improved amino acid, the original amino acid at that position, and may further include additional substitutions at that position, depending on the complexity of the library that is desired, or permitted using the desired screening or selection method. In addition, if desired, adjacent amino acid position can be randomized to at least two or more amino acids. Randomization of adjacent amino acids may permit additional conformational flexibility in the mutant CDR, which may in turn, permit or facilitate the introduction of a larger number of improving mutations. The library may also comprise substitution at positions that did not show improved affinity in the first round of screening.

The second library is screened or selected for library members with improved and/or altered binding affinity using any method known in the art, including screening using Biacore surface plasmon resonance analysis, and selection using any method known in the art for selection, including phage display, yeast display, and ribosome display.

In some embodiments, the invention also encompasses fusion proteins comprising one or more fragments or regions from antibodies (such as G1) or polypeptides. In one embodiment, a fusion polypeptide is provided that comprises at least 10 contiguous amino acids of the variable light chain region shown in SEQ ID NO:2 (Figure 5) and/or at least 10 amino acids of the variable heavy chain region shown in SEQ ID NO:1 (Figure 5). In other embodiments, a fusion polypeptide is provided that comprises at least about 10, at least about 15, at least about 20, at least about 25, or at least about 30 contiguous amino acids of the variable light chain region shown in SEQ ID NO:2 (Figure 5) and/or at least about 10, at least about 15, at least about 20, at least about 25, or at least about 30 contiguous amino acids of the variable heavy chain region shown in SEQ ID NO:1 (Figure 5). In another embodiment, the fusion polypeptide comprises a light chain variable region and/or a heavy chain variable region of G1, as shown in SEQ ID NO:2 and SEQ ID NO:1 of Figure 5. For purposes of this invention, an G1 fusion protein contains one or more G1 antibodies and another amino acid sequence to which it is not attached in the native molecule, for example, a heterologous sequence or a homologous sequence from another region. Exemplary heterologous sequences include, but are not limited to a "tag" such as a FLAG tag or a 6His tag (SEQ ID NO:56). Tags are well known in the art.

A G1 fusion polypeptide can be created by methods known in the art, for example, synthetically or recombinantly. Typically, the G1 fusion proteins of this invention are made by preparing an expressing a polynucleotide encoding them using recombinant methods described herein, although they may also be prepared by other means known in the art, including, for example, chemical synthesis.

In some aspects, compositions comprising antibodies derived from G1 conjugated (for example, linked) to an agent that facilitate coupling to a solid support (such as biotin or avidin) are used. For simplicity, reference will be made generally to G1 or antibodies with the understanding that these methods apply to any of the CGRP binding embodiments described herein. Conjugation generally refers to linking these components as described herein. The linking (which is generally fixing these components in proximate association at least for administration) can be achieved in any number of ways. For example, a direct reaction between an agent and an antibody is possible when each possesses a substituent capable of reacting with the other. For example, a nucleophilic group, such as an amino or sulfhydryl group, on one may be capable of reacting with a carbonyl-containing group, such as an anhydride or an acid halide, or with an alkyl group containing a good leaving group (e.g., a halide) on the other.

An antibody or polypeptide may be linked to a labeling agent (alternatively termed "label") such as a fluorescent molecule, a radioactive molecule or any others labels known in the art. Labels are known in the art which generally provide (either directly or indirectly) a signal.

Disclosed herein are isolated polynucleotides encoding the antibodies and polypeptides (including an antibody comprising the polypeptide sequences of the light chain and heavy chain variable regions shown in Figure 5), and vectors and host cells comprising the polynucleotide.

Polynucleotides complementary to any such sequences are also disclosed. Polynucleotides may be single-stranded (coding or antisense) or double-stranded, and may be DNA (genomic, cDNA or synthetic) or RNA molecules. RNA molecules include HnRNA molecules, which contain introns and correspond to a DNA molecule in a one-to-one manner, and mRNA molecules, which do not contain introns. Additional coding or non-coding sequences may, but need not, be present within a polynucleotide of the present invention, and a polynucleotide may, but need not, be linked to other molecules and/or support materials.

Polynucleotides may comprise a native sequence (i.e., an endogenous sequence that encodes an antibody or a portion thereof) or may comprise a variant of such a sequence. Polynucleotide variants contain one or more substitutions, additions, deletions and/or insertions such that the immunoreactivity of the encoded polypeptide is not diminished, relative to a native immunoreactive molecule. The effect on the immunoreactivity of the encoded polypeptide may generally be assessed as described herein. Variants preferably exhibit at least about 70% identity, more preferably at least about 80% identity and most preferably at least about 90% identity to a polynucleotide sequence that encodes a native antibody or a portion thereof.

Two polynucleotide or polypeptide sequences are said to be "identical" if the sequence of nucleotides or amino acids in the two sequences is the same when aligned for maximum correspondence as described below. Comparisons between two sequences are typically performed by comparing the sequences over a comparison window to identify and compare local regions of sequence similarity. A "comparison window" as used herein, refers to a segment of at least about 20 contiguous positions, usually 30 to about 75, 40 to about 50, in which a sequence may be compared to a reference sequence of the same number of contiguous positions after the two sequences are optimally aligned.

Optimal alignment of sequences for comparison may be conducted using the Megalign program in the Lasergene suite of bioinformatics software (DNASTAR, Inc., Madison, WI), using default parameters. This program embodies several alignment schemes described in the following references: Dayhoff, M.O. (1978) A model of evolutionary change in proteins - Matrices for detecting distant relationships. In Dayhoff, M.O. (ed.) Atlas of Protein Sequence and Structure, National Biomedical Research Foundation, Washington DC Vol. 5, Suppl. 3, pp. 345-358; Hein J., 1990, Unified Approach to Alignment and Phylogenes pp. 626-645 Methods in Enzymology vol. 183, Academic Press, Inc., San Diego, CA; Higgins, D.G. and Sharp, P.M., 1989, CABIOS 5:151-153; Myers, E.W. and Muller W., 1988, CABIOS 4:11-17; Robinson, E.D., 1971, Comb. Theor. 11:105; Santou, N., Nes, M., 1987, Mol. Biol. Evol. 4:406-425; Sneath, P.H.A. and Sokal, R.R., 1973, Numerical Taxonomy the Principles and Practice of Numerical Taxonomy, Freeman Press, San Francisco, CA; Wilbur, W.J. and Lipman, D.J., 1983, Proc. Natl. Acad. Sci. USA 80:726-730.

Preferably, the "percentage of sequence identity" is determined by comparing two optimally aligned sequences over a window of comparison of at least 20 positions, wherein the portion of the polynucleotide or polypeptide sequence in the comparison window may comprise additions or deletions (i.e., gaps) of 20 percent or less, usually 5 to 15 percent, or 10 to 12 percent, as compared to the reference sequences (which does not comprise additions or deletions) for optimal alignment of the two sequences. The percentage is calculated by determining the number of positions at which the identical nucleic acid bases or amino acid residue occurs in both sequences to yield the number of matched positions, dividing the number of matched positions by the total number of positions in the reference sequence (i.e., the window size) and multiplying the results by 100 to yield the percentage of sequence identity.

Variants may also, or alternatively, be substantially homologous to a native gene, or a portion or complement thereof. Such polynucleotide variants are capable of hybridizing under moderately stringent conditions to a naturally occurring DNA sequence encoding a native antibody (or a complementary sequence).

Suitable "moderately stringent conditions" include prewashing in a solution of 5 X SSC, 0.5% SDS, 1.0 mM EDTA (pH 8.0); hybridizing at 50°C-65°C, 5 X SSC, overnight; followed by washing twice at 65°C for 20 minutes with each of 2X, 0.5X, and 0.2X SSC containing 0. 1 % SDS.

As used herein, "highly stringent conditions" or "high stringency conditions" are those that: (1) employ low ionic strength and high temperature for washing, for example 0.015 M sodium chloride/0.0015 M sodium citrate/0.1 % sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50 mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 $\mu$g/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C. The skilled artisan will recognize how to adjust the temperature, ionic strength, etc. as necessary to accommodate factors such as probe length and the like.

It will be appreciated by those of ordinary skill in the art that, as a result of the degeneracy of the genetic code, there are many nucleotide sequences that encode a polypeptide as described herein. Some of these polynucleotides bear minimal homology to the nucleotide sequence of any native gene. Nonetheless, polynucleotides that vary due to differences in codon usage are specifically contemplated by the present invention. Further, alleles of the genes comprising the polynucleotide sequences provided herein are disclosed herein. Alleles are endogenous genes that are altered as a result of one or more mutations, such as deletions, additions and/or substitutions of nucleotides. The resulting mRNA and protein may, but need not, have an altered structure or function. Alleles may be identified using standard techniques (such as hybridization, amplification and/or database sequence comparison).

The polynucleotides can be obtained using chemical synthesis, recombinant methods, or PCR. Methods of chemical polynucleotide synthesis are well known in the art and need not be described in detail herein. One of skill in the art can use the sequences provided herein and a commercial DNA synthesizer to produce a desired DNA sequence.

For preparing polynucleotides using recombinant methods, a polynucleotide comprising a desired sequence can be inserted into a suitable vector, and the vector in turn can be introduced into a suitable host cell for replication and amplification, as further discussed herein. Polynucleotides may be inserted into host cells by any means known in the art. Cells are transformed by introducing an exogenous polynucleotide by direct uptake, endocytosis, transfection, F-mating or electroporation. Once introduced, the exogenous polynucleotide can be maintained within the cell as a non-integrated vector (such as a plasmid) or integrated into the host cell genome. The polynucleotide so amplified can be isolated from the host cell by methods well known within the art. See, e.g., Sambrook et al. (1989).

Alternatively, PCR allows reproduction of DNA sequences. PCR technology is well known in the art and is described in U.S. Patent Nos. 4,683,195, 4,800,159, 4,754,065 and 4,683,202, as well as PCR: The Polymerase Chain Reaction, Mullis et al. eds., Birkauswer Press, Boston (1994).

RNA can be obtained by using the isolated DNA in an appropriate vector and inserting it into a suitable host cell. When the cell replicates and the DNA is transcribed into RNA, the RNA can then be isolated using methods well known to those of skill in the art, as set forth in Sambrook et al., (1989), for example.

Suitable cloning vectors may be constructed according to standard techniques, or may be selected from a large number of cloning vectors available in the art. While the cloning vector selected may vary according to the host cell intended to be used, useful cloning vectors will generally have the ability to self-replicate, may possess a single target for a particular restriction endonuclease, and/or may carry genes for a marker that can be used in selecting clones containing the vector. Suitable examples include plasmids and bacterial viruses, e.g., pUC18, pUC19, Bluescript (e.g., pBS SK+) and its derivatives, mp18, mp19, pBR322, pMB9, ColE1, pCR1, RP4, phage DNAs, and shuttle vectors such as pSA3 and pAT28. These and many other cloning vectors are available from commercial vendors such as BioRad, Strategene, and Invitrogen.

Expression vectors generally are replicable polynucleotide constructs that contain a polynucleotide according to any of the various aspects of the invention. It is implied that an expression vector must be replicable in the host cells either as episomes or as an integral part of the chromosomal DNA. Suitable expression vectors include but are not limited to plasmids, viral vectors, including adenoviruses, adeno-associated viruses, retroviruses, cosmids, and expression vector(s) disclosed in PCT Publication No. WO 87/04462. Vector components may generally include, but are not limited to, one or more of the following: a signal sequence; an origin of replication; one or more marker genes; suitable transcriptional controlling elements (such as promoters, enhancers and terminator). For expression (i.e., translation), one or more translational controlling elements are also usually required, such as ribosome binding sites, translation initiation sites, and stop codons.

The vectors containing the polynucleotides of interest can be introduced into the host cell by any of a number of appropriate means, including electroporation, transfection employing calcium chloride, rubidium chloride, calcium phosphate, DEAE-dextran, or other substances; microprojectile bombardment; lipofection; and infection (e.g., where the vector is an infectious agent such as vaccinia virus). The choice of introducing vectors or polynucleotides will often depend on features of the host cell.

Any host cells capable of over-expressing heterologous DNAs can be used for the purpose of isolating the genes encoding the antibody, polypeptide or protein of interest. Non-limiting examples of mammalian host cells include but not limited to COS, HeLa, and CHO cells. See also PCT Publication No. WO 87/04462. Suitable non-mammalian host cells include prokaryotes (such as E. coli or B. subtillis) and yeast (such as S. cerevisae, S. pombe; or K. lactis). Preferably, the host cells express the cDNAs at a level of about 5 fold higher, more preferably 10 fold higher, even more preferably 20 fold higher than that of the corresponding endogenous antibody or protein of interest, if present, in the host cells. Screening the host cells for a specific binding to A$\beta$1-40 is effected by an immunoassay or FACS. A cell overexpressing the antibody or protein of interest can be identified.

D. Compositions

In some embodiments, compositions used in the invention comprise an effective amount of an anti-CGRP antagonist antibody. Examples of such compositions, as well as how to formulate, are also described in an earlier section and below. In one embodiment, the composition further comprises a CGRP antagonist. The composition comprises one or more anti-CGRP antagonist antibodies. In some embodiments, the anti-CGRP antagonist antibody recognizes human CGRP. In some embodiments, the anti-CGRP antagonist antibody is humanized. In some embodiments, the anti-CGRP antagonist antibody comprises a constant region that does not trigger an unwanted or undesirable immune response, such as antibody-mediated lysis or ADCC. In some embodiments, the anti-CGRP antagonist antibody comprises six CDRs from G1. In some embodiments, the anti-CGRP antagonist antibody is human.

It is understood that the compositions can comprise more than one antibody (e.g., more than one anti-CGRP antagonist antibody -- a mixture of anti-CGRP antagonist antibodies that recognize different epitopes of CGRP). Other exemplary compositions comprise more than one anti-CGRP antagonist antibodies that recognize the same epitope(s), or different species of anti-CGRP antagonist antibodies that bind to different epitopes of CGRP.

A composition can further comprise pharmaceutically acceptable carriers, excipients, or stabilizers (Remington: The Science and practice of Pharmacy 20th Ed. (2000) Lippincott Williams and Wilkins, Ed. K. E. Hoover). Acceptable carriers, excipients, or stabilizers are nontoxic to recipients at the dosages and concentrations employed. A therapeutic formulation of an antibody may comprise one or more pharmaceutically acceptable carriers, excipients or stabilizes with non-limiting examples of such species that include buffers such as phosphate, citrate, and other organic acids; salts such as sodium chloride; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens, such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids (e.g., at concentrations of 0.1 mM to 100 mM, 0.1 mM to 1 mM, 0.01 mM to 50 mM, 1 mM to 50 mM, 1 mM to 30 mM, 1 mM to 20 mM, 10 mM to 25 mM) such as glycine, glutamine, methionine, asparagine, histidine, arginine, or lysine; monosaccharides, disaccharides, and other carbohydrates including glucose, mannose, or dextrins; chelating agents (e.g., at concentrations of 0.001 mg/mL to 1 mg/mL, 0.001 mg/mL to 1 mg/mL, 0.001 mg/mL to 0.1 mg/mL, 0.001 mg/mL to 0.01 mg/mL) such as EDTA (e.g., disodium EDTA dihydrate); sugars (e.g., at concentrations of 1 mg/mL to 500 mg/mL, 10 mg/mL to 200 mg/mL, 10 mg/mL to 100 mg/mL, 50 mg/mL to 150 mg/mL) such as sucrose, mannitol, trehalose or sorbitol; salt-forming counter-ions such as sodium; metal complexes (e.g., Zn-protein complexes); and/or non-ionic surfactants (e.g., at concentrations of 0.01 mg/mL to 10 mg/mL, 0.01 mg/mL to 1 mg/mL, 0.1 mg/mL to 1 mg/mL, 0.01 mg/mL to 0.5 mg/mL) such as TWEEN™ (e.g., polysorbate (e.g., polysorbate 20, polysorbate 40, polysorbate 60, polysorbate 80)), PLURONICS™ or polyethylene glycol (PEG). Pharmaceutically acceptable excipients are further described herein.

An antibody (e.g., an anti-CGRP antagonist antibody) and compositions thereof can also be used in conjunction with other agents that serve to enhance and/or complement the effectiveness of the agents.

E. Kits

Kits can include one or more containers comprising an antibody described herein (e.g., an anti-CGRP antagonist antibody (such as a humanized antibody)) or polypeptide described herein and instructions for use in accordance with any of the methods described herein. Generally, these instructions comprise a description of administration of the antibody to treat, ameliorate or prevent post-traumatic headache according to any of the methods described herein. The kit may further comprise a description of selecting an individual suitable for treatment based on identifying whether that individual has post-traumatic headache or whether the individual is at risk of having post-traumatic headache. The instructions comprise a description of administering an antibody (e.g., anti-CGRP antagonist antibody) to an individual at risk of having post-traumatic headache.

The instructions relating to the use of an antibody (e.g., anti-CGRP antagonist antibody) generally include information as to dosage, dosing schedule, and route of administration for the intended treatment. The containers may be unit doses, bulk packages (e.g., multi-dose packages) or sub-unit doses. Instructions supplied in the kits are typically written instructions on a label or package insert (e.g., a paper sheet included in the kit), but machine-readable instructions (e.g., instructions carried on a magnetic or optical storage disk) are also acceptable.

The label or package insert indicates that the composition is used for treating, ameliorating and/or preventing post-traumatic headache. Instructions may be provided for practicing any of the methods described herein.

The kits are in suitable packaging. Suitable packaging includes, but is not limited to, vials, bottles, jars, flexible packaging (e.g., sealed Mylar or plastic bags), and the like. Also contemplated are packages for use in combination with a specific device, such as an inhaler, nasal administration device (e.g., an atomizer) or an infusion device such as a minipump. A kit may have a sterile access port (for example the container may be an intravenous solution bag or a vial having a stopper pierceable by a hypodermic injection needle). The container may also have a sterile access port (for example the container may be an intravenous solution bag or a vial having a stopper pierceable by a hypodermic injection needle). At least one active agent in the composition is an anti-CGRP antagonist antibody and/or a monoclonal antibody that modulates the CGRP pathway. The container may further comprise a second pharmaceutically active agent.

Kits may optionally provide additional components such as buffers and interpretive information. Normally, the kit comprises a container and a label or package insert(s) on or associated with the container.

The following Examples are provided to illustrate but not limit the invention.

Examples

Example 1: Generation and characterization of monoclonal antibodies directed against CGRP

Generation of anti-CGRP antibodies. To generate anti-CGRP antibodies that have cross-species reactivity for rat and human CGRP, mice were immunized with 25-100 $\mu$g of human $\alpha$-CGRP or $\beta$-CGRP conjugated to KLH in adjuvant (50 $\mu$l per footpad, 100 $\mu$l total per mouse) at various intervals. Immunization was generally performed as described in Geerligs HJ et al., 1989, J. Immunol. Methods 124:95-102; Kenney JS et al., 1989, J. Immunol. Methods 121:157-166; and Wicher K et al., 1989, Int. Arch. Allergy Appl. Immunol. 89:128-135. Mice were first immunized with 50 $\mu$g of human $\alpha$-CGRP or $\beta$-CGRP conjugated to KLH in CFA (complete Freund's adjuvant). After 21 days, mice were secondly immunized with 25 $\mu$g of human $\beta$-CGRP (for mice first immunized with human $\alpha$-CGRP) or $\alpha$-CGRP (for mice first immunized with human $\beta$-CGRP) conjugated to KLH in IFA (incomplete Freund's adjuvant). Twenty-three days later after the second immunization, third immunization was performed with 25 $\mu$g of rat $\alpha$-CGRP conjugated to KLH in IFA. Ten days later, antibody titers were tested using ELISA. Forth immunization was performed with 25 $\mu$g of the peptide (rat $\alpha$-CGRP-KLH) in IFA 34 days after the third immunization. Final booster was performed with 100 $\mu$g soluble peptide (rat $\alpha$-CGRP) 32 days after the forth immunization.

Splenocytes were obtained from the immunized mouse and fused with NSO myeloma cells at a ratio of 10:1, with polyethylene glycol 1500. The hybrids were plated out into 96-well plates in DMEM containing 20% horse serum and 2-oxaloacetate/pyruvate/insulin (Sigma), and hypoxanthine/aminopterin/thymidine selection was begun. On day 8, 100 $\mu$l of DMEM containing 20% horse serum was added to all the wells. Supernatants of the hybrids were screened by using antibody capture immunoassay. Determination of antibody class was done with class-specific second antibodies.

A panel of monoclonal antibody-producing cell lines was selected based on their binding to human and rat CGRP for further characterization. These antibodies and characteristics are shown below in Tables 2 and 3.

Purification and Fab fragment preparation. Monoclonal antibodies selected for further characterization were purified from supernatants of hybridoma cultures using protein A affinity chromatography. The supernatants were equilibrated to pH 8. The supernatants were then loaded to the protein A column MabSelect (Amersham Biosciences # 17-5199-02) equilibrated with PBS to pH 8. The column was washed with 5 column volumes of PBS, pH 8. The antibodies were eluted with 50 mM citrate-phosphate buffer, pH 3. The eluted antibodies were neutralized with 1 M Phosphate Buffer, pH 8. The purified antibodies were dialyzed with PBS, pH 7.4. The antibody concentrations were determined by SDS-PAGE, using a murine monoclonal antibody standard curve.

Fabs were prepared by papain proteolysis of the full antibodies using Immunopure Fab kit (Pierce # 44885) and purified by flow through protein A chromatography following manufacturer instructions. Concentrations were determined by ELISA and/or SDS-PAGE electrophoresis using a standard Fab of known concentration (determined by amino acid analysis), and by A280 using 1OD=0.6 mg/ml (or theoretical equivalent based on the amino acid sequence).

Affinity determination of the Fabs. Affinities of the anti-CGRP monoclonal antibodies were determined at either 25°C or 37°C using the BIACORE3000[3] surface plasmon resonance (SPR) system (Biacore, INC, Piscataway NJ) with the manufacture's own running buffer, HBS-EP (10 mM HEPES pH 7.4, 150 mM NaCl, 3 mM EDTA, 0.005% v/v polysorbate P20). Affinity was determined by capturing N-terminally biotinylated CGRP peptides (custom ordered from GenScript Corporation, New Jersey or Global Peptide Services, Colorado) via pre-immobilized streptavidin on SA chip and measuring binding kinetics of antibody Fab titrated across the CGRP surface. Biotinylated CGRP was diluted into HBS-EP and injected over the chip at a concentration of less than 0.001 mg/ml. Using variable flow time across the individual chip channels, two ranges of antigen density were achieved: <50 response units (RU) for detailed kinetic studies and about 800 RU for concentration studies and screening. Two- or three-fold serial dilutions typically at concentrations spanning 1 $\mu$M - 0.1 nM (aimed at 0.1-10x estimated $K_D$) of purified Fab fragments were injected for 1 minute at 100 $\mu$L/min and dissociation times of 10 minutes were allowed. After each binding cycle, surfaces were regenerated with 25 mM NaOH in 25% v/v ethanol, which was tolerated over hundreds of cycles. Kinetic association rate ($k_{on}$) and dissociation rate ($k_{off}$) were obtained simultaneously by fitting the data to a 1:1 Langmuir binding model (Karlsson, R. Roos, H. Fagerstam, L. Petersson, B. (1994). Methods Enzymology 6. 99-110) using the BIAevaluation program. Global equilibrium dissociation constants ($K_D$) or "affinities" were calculated from the ratio $K_D = k_{off}/k_{on}$. Affinities of the murine Fab fragments are shown in Tables 2 and 3.

Epitope mapping of the murine anti-CGRP antibodies. To determine the epitope that anti-CGRP antibodies bind on human $\alpha$-CGRP, binding affinities of the Fab fragments to various CGRP fragments were measured as described above by capturing N-terminally biotinylated CGRP fragments amino acids 19-37 and amino acids 25-37 on a SA sensor chip. Figure 1 shows their binding affinities measured at 25°C. As shown in Figure 1, all antibodies, except antibody 4901, bind to human $\alpha$-CGRP fragments 19-37 and 25-37 with affinity similar to their binding affinity to full length human $\alpha$-CGRP (1-37). Antibody 4901 binds to human $\alpha$-CGRP fragment 25-37 with six-fold lower affinity than binding to full length human $\alpha$-CGRP fragment, due mainly to a loss in off-rate. The data indicate that these anti-CGRP antibodies generally bind to the C-terminal end of CGRP.

Alanine scanning was performed to further characterize amino acids in human $\alpha$-CGRP involved in binding of anti-CGRP antibodies. Different variants of human $\alpha$-CGRP with single alanine substitutions were generated by peptide synthesis. Their amino acid sequences are shown in Table 4 along with all the other peptides used in the Biacore analysis. Affinities of Fab fragments of the anti-CGRP antibodies to these variants were determined using Biacore as described above. As shown in Figure 1, all 12 antibodies target a C-terminal epitope, with amino acid F37 being the most crucial residue. Mutation of F37 to alanine significantly lowered the affinity or even completely knocked out binding of the anti-CGRP antibodies to the peptide. The next most important amino acid residue is G33, however, only the high affinity antibodies (7E9, 8B6, 10A8, and 7D11) were affected by alanine replacement at this position. Amino acid residue S34 also plays a significant, but lesser, role in the binding of these four high affinity antibodies.

Table 2. Characteristics of the anti-CGRP monoclonal antibodies' binding to human $\alpha$-CGRP and their antagonist activity

| Antibodies | $K_D$ to human $\alpha$-CGRP at 25°C (nM) | $K_D$ to human $\alpha$-CGRP at 37°C (nM) | Cell-based blocking human $\alpha$-CGRP binding to its receptor at 25°C (measured by cAMP activation) | $IC_{50}$ (nM binding sites) at 25°C (room temp.) measured in radioligand binding assay. |
|---|---|---|---|---|
| 7E9 | 1.0 | 0.9 | Yes | 2.5 |
| 8B6 | 1.1 | 1.2 | Yes | 4.0 |
| 10A8 | 2.1 | 3.0 | Yes | n.d. |
| 7D11 | 4.4 | 5.4 | Yes | n.d. |
| 6H2 | 9.3 | 42 | Yes | 12.9 |
| 4901 | 61 | 139 | Yes | 58 |
| 14E10 | 80 | 179 | Yes | n.d. |
| 9B8 | 85 | 183 | No | n.d. |
| 13C2 | 94 | 379 | No | n.d. |
| 14A9 | 148 | 581 | No | n.d. |
| 6D5 | 210 | 647 | No | n.d. |
| 1C5 | 296 | 652 | No | n.d. |
| Note: Antibody 4901 is commercially available (Sigma, Product No. C7113). n.d. = not determined | | | | |

Table 3. Characteristics of the anti-CGRP monoclonal antibodies' binding to rat $\alpha$-CGRP and antagonist activity

| Antibodies | $K_D$ to rat $\alpha$-CGRP at 37°C (nM) | Cell-based blocking of binding of rat $\alpha$-CGRP to its receptor at 25°C (measured by cAMP activation) | In vivo blocking in saphenous nerve assay |
|---|---|---|---|
| 4901 | 3.4 | Yes | Yes |
| 7E9 | 47 | Yes | Yes |
| 6H2 | 54 | No | No |
| 8B6 | 75 | Yes | Yes |

(continued)

| Antibodies | $K_D$ to rat $\alpha$-CGRP at 37°C (nM) | Cell-based blocking of binding of rat $\alpha$-CGRP to its receptor at 25°C (measured by cAMP activation) | In vivo blocking in saphenous nerve assay |
|---|---|---|---|
| 7D11 | 218 | Yes | Yes |
| 10A8 | 451 | No | n.d. |
| 9B8 | 876 | No | n.d. |
| 14E10 | 922 | No | n.d. |
| 13C2 | > 1000 | No | n.d. |
| 14A9 | > 1000 | No | n.d. |
| 6D5 | > 1000 | No | n.d. |
| 1C5 | > 1000 | No | n.d. |
| "n.d." indicates no test was performed for the antibody. | | | |

Table 4. Amino acid sequences of human $\alpha$-CGRP fragments (SEQ ID NOS:15-40) and related peptides (SEQ ID NOS:41-47). All peptides are C-terminally amidated except SEQ ID NOS:36-40. Residues in bold indicate point mutations.

| CGRP | Amino acid sequence | SEQ ID NO |
|---|---|---|
| 137 (WT) | ACDTATCVTHRLAGLLSRSGGVVKNNFVPTNVGSKAF | 15 |
| 8-37 | VTHRLAGLLSRSGGVVKNNFVPTNVGSKAF | 16 |
| 19-37 | SGGVVKNNFVPTNVGSKAF | 17 |
| P29A (19-37) | SGGVVKNNFVATNVGSKAF | 18 |
| K35A (19-37) | SGGVVKNNFVPTNVGSAAF | 19 |
| K35E (19-37) | SGGVVKNNFVPTNVGSEAF | 20 |
| K35M (19-37) | SGGVVKNNFVPTNVGSMAF | 21 |
| K35Q (19-37) | SGGVVKNNFVPTNVGSQAF | 22 |
| F37A (19-37) | SGGVVKNNFVPTNVGSKAA | 23 |
| 25-38A | NNFVPTNVGSKAFA | 24 |
| 25-37 | NNFVPTNVGSKAF | 25 |
| F27A (25-37) | NNAVPTNVGSKAF | 26 |
| V28A (25-37) | NNFAPTNVGSKAF | 27 |
| P29A (25-37) | NNFVATNVGSKAF | 28 |
| T30A (25-37) | NNFVPANVGSKAF | 29 |
| N31A (25-37) | NNFVPTAVGSKAF | 30 |
| V32A (25-37) | NNFVPTNAGSKAF | 31 |
| G33A (25-37) | NNFVPTNVASKAF | 32 |
| S34A (25-37) | NNFVPTNVGAKAF | 33 |
| F37A (25-37) | NNFVPTNVGSKAA | 34 |
| 26-37 | NFVPTNVGSKAF | 35 |
| 19-37-COOH | SGGVVKNNFVPTNVGSKAF | 36 |
| 19-36-COOH | SGGVVKNNFVPTNVGSKA | 37 |

(continued)

| CGRP | Amino acid sequence | SEQ ID NO |
|---|---|---|
| 1-36-COOH | ACDTATCVTHRLAGLLSRSGGVVKNNFVPTNVGSKA | 38 |
| 1-19-COOH | ACDTATCVTHRLAGLLSRS | 39 |
| 1-13-COOH | ACDTATCVTHRLA | 40 |
| rat $\alpha$ (1-37) | SCNTATCVTHRLAGLLSRSGGVVKDNFVPTNVGSEAF | 41 |
| rat $\alpha$ (19-37) | SGGVVKDNFVPTNVGSEAF | 42 |
| human $\beta$ (1-37) | ACNTATCVTHRLAGLLSRSGGMVKSNFVPTNVGSKAF | 43 |
| rat $\beta$ (1-37) | SCNTATCVTHRLAGLLSRSGGVVKDNFVPTNVGSKAF | 44 |
| Human calcitonin (1-32) | CGNLSTCMLGTYTQDFNKFHTFPQTAIGVGAP | 45 |
| Human amylin (1-37) | KCNTATCATQRLANFLVHSSNNFGAILSSTNVGSNTY | 46 |
| Human adrenomedullin (1-52) | YRQSMNNFQGLRSFGCRFGTCTVQKLAHQIYQFTDK DKDNVAPRSKISPQGY | 47 |

Example 2: Screening of anti-CGRP antagonist antibodies using in vitro assays.

Murine anti-CGRP antibodies were further screened for antagonist activity in vitro using cell based cAMP activation assay and binding assay.

Antagonist activity measured by cAMP assay. Five microliters of human or rat $\alpha$-CGRP (final concentration 50 nM) in the presence or absence of an anti-CGRP antibody (final concentration 1-3000 nM), or rat $\alpha$-CGRP or human $\alpha$-CGRP (final concentration 0.1 nM-10 $\mu$M; as a positive control for c-AMP activation) was dispensed into a 384-well plate (Nunc, Cat. No. 264657). Ten microliters of cells (human SK-N-MC if human $\alpha$-CGRP is used, or rat L6 from ATCC if rat $\alpha$-CGRP is used) in stimulation buffer (20 mM HEPES, pH 7.4, 146 mM NaCl, 5 mM KCl, 1 mM CaCl$_2$, 1 mM MgCl$_2$, and 500 $\mu$M 3-Isobutyl-1-methylxanthine (IBMX)) were added into the wells of the plate. The plate was incubated at room temperature for 30 minutes.

After the incubation, cAMP activation was performed using HitHunter™ Enzyme Fragment Complementation Assay (Applied Biosystems) following manufacture's instruction. The assay is based on a genetically engineered $\beta$-galactosidase enzyme that consists of two fragments -termed Enzyme Acceptor (EA) and Enzyme Donor (ED). When the two fragments are separated, the enzyme is inactive. When the fragments are together they can recombine spontaneously to form active enzyme by a process called complementation. The EFC assay platform utilizes an ED-cAMP peptide conjugate in which cAMP is recognized by anti-cAMP. This ED fragment is capable of reassociation with EA to form active enzyme. In the assay, anti-cAMP antibody is optimally titrated to bind ED-cAMP conjugate and inhibit enzyme formation. Levels of cAMP in cell lysate samples compete with ED-cAMP conjugate for binding to the anti-cAMP antibody. The amount of free ED conjugate in the assay is proportional to the concentration of cAMP. Therefore, cAMP is measured by the formation of active enzyme that is quantified by the turnover of $\beta$-galactosidase luminescent substrate. The cAMP activation assay was performed by adding 10 $\mu$l of lysis buffer and anti-cAMP antibody (1:1 ratio) following by incubation at room temperature for 60 min. Then 10 $\mu$l of ED-cAMP reagent was added into each well and incubated for 60 minutes at room temperature. After the incubation, 20 $\mu$l of EA reagent and CL mixture (containing the substrate) (1:1 ratio) was added into each well and incubated for 1-3 hours or overnight at room temperature. The plate was read at 1 second/well on PMT instrument or 30 seconds/place on imager. The antibodies that inhibit activation of cAMP by $\alpha$-CGRP were identified (referred to as "yes") in Tables 2 and 3 above. Data in Tables 2 and 3 indicate that antibodies that demonstrated antagonist activity in the assay generally have high affinity. For example, antibodies having $K_D$ (determined at 25°C) of about 80 nM or less to human $\alpha$-CGRP or having $K_D$ (determined at 37°C) of about 47 nM or less to rat $\alpha$-CGRP showed antagonist activity in this assay.

Radioligand binding assay. Binding assay was performed to measure the IC$_{50}$ of anti-CGRP antibody in blocking the CGRP from binding to the receptor as described previously. Zimmermann et al., Peptides 16:421-4, 1995; Mallee et al., J. Biol. Chem. 277:14294-8, 2002. Membranes (25 $\mu$g) from SK-N-MC cells were incubated for 90 min at room temperature in incubation buffer (50 mM Tris-HCl, pH 7.4, 5 mM MgCl$_2$, 0.1% BSA) containing 10 pM $^{125}$I-human $\alpha$-CGRP in a total volume of 1 mL. To determine inhibition concentrations (IC$_{50}$), antibodies or unlabeled CGRP (as a control), from a about 100 fold higher stock solution were dissolved at varying concentrations in the incubation buffer and incubated at the same time with membranes and 10 pM $^{125}$I-human $\alpha$-CGRP. Incubation was terminated by filtration through a glass microfiber filter (GF/B, 1 $\mu$m) which had been blocked with 0.5% polyethylemimine. Dose response curves were plotted and Ki values were determined by using the equation: $K_i = IC_{50}/(1+([ligand]/K_D))$; where the equilibrium dissociation constant $K_D = 8$ pM for human $\alpha$-CGRP to CGRP1 receptor as present in SK-N-MC cells, and $B_{max} = 0.025$ pmol/mg protein. The reported $IC_{50}$ value (in terms of IgG molecules) was converted to binding sites (by multiplying it by 2) so that it could be compared with the affinities ($K_D$) determined by Biacore (see Table 2).

Table 2 shows the $IC_{50}$ of murine antibodies 7E9, 8B6, 6H2 and 4901. Data indicate that antibody affinity generally correlates with $IC_{50}$: antibodies with higher affinity (lower $K_D$ values) have lower $IC_{50}$ in the radioligand binding assay.

Example 3: Effect of anti-CGRP antagonist antibodies on skin vasodilatation induced by stimulation of rat saphenous nerve

To test antagonist activity of anti-CGRP antibodies, effect of the antibodies on skin vasodilatation by stimulation of rat saphenous nerve was tested using a rat model described previously. Escott et al., Br. J. Pharmacol. 110:772-776, 1993. In this rat model, electrical stimulation of saphenous nerve induces release of CGRP from nerve endings, resulting in an increase in skin blood flow. Blood flow in the foot skin of male Sprague Dawley rats (170-300 g, from Charles River Hollister) was measured after saphenous nerve stimulation. Rats were maintained under anesthesia with 2% isoflurane. Bretylium tosylate (30 mg/kg, administered i.v.) was given at the beginning of the experiment to minimize vasoconstriction due to the concomitant stimulation of sympathetic fibers of the saphenous nerve. Body temperature was maintained at 37°C by the use of a rectal probe thermostatically connected to a temperature controlled heating pad. Compounds including antibodies, positive control (CGRP 8-37), and vehicle (PBS, 0.01% Tween 20) were given intravenously through the right femoral vein, except for the experiment shown in Figure 3, the test compound and the control were injected through tail vein, and for experiments shown in Figures 2A and 2B, antibodies 4901 and 7D11 were injected intraperitoneally (IP). Positive control compound CGRP 8-37 (vasodilatation antagonist), due to its short half-life, was given 3-5 min before nerve stimulation at 400 nmol/kg (200 μl). Tan et al., Clin. Sci. 89:656-73, 1995. The antibodies were given in different doses (1 mg/kg, 2.5 mg/kg, 5 mg/kg, 10 mg/kg, and 25 mg/kg).

For experiments shown in Figures 2A and 2B, antibody 4901 (25 mg/kg), antibody 7D11 (25 mg/kg), or vehicle control (PBS with 0.01% Tween 20) was administered intraperitoneally (IP) 72 hours before the electrical pulse stimulation. For experiment shown in Figure 3, antibody 4901 (1 mg/kg, 2.5 mg/kg, 5 mg/kg, or 25 mg/kg) or vehicle control (PBS with 0.01% Tween 20) was administered intravenously 24 hours before the electrical pulse stimulation. After administration of the antibodies or vehicle control, the saphenous nerve of the right hindlimb was exposed surgically, cut proximally and covered with plastic wrap to prevent drying. A laser Doppler probe was placed over the medio-dorsal side of the hindpaw skin, which is the region innervated by the saphenous nerve. Skin blood flow, measured as blood cell flux, was monitored with a laser Doppler flow meter. When a stable base-line flux (less than 5% variation) was established for at least 5 minutes, the nerve was placed over platinum bipolar electrodes and electrically stimulated with 60 pulses (2 Hz, 10 V, 1 ms, for 30 seconds) and then again 20 minutes later. Cumulative change in skin blood flow was estimated by the area under the flux-time curve (AUC, which is equal to change in flux multiplied by change in time) for each flux response to electrical pulse stimulation. The average of the blood flow response to the two stimulations was taken. Animals were kept under anesthesia for a period of one to three hours.

As shown in Figure 2A and Figure 2B, blood flow increase stimulated by applying electronic pulses on saphenous nerve was inhibited by the presence of CGRP 8-37 (400 nmol/kg, administered i.v.), antibody 4901 (25 mg/kg, administered ip), or antibody 7D11 (25 mg/kg, administered ip) as compared to the control. CGRP 8-37 was administered 3-5 minutes before the saphenous nerve stimulation; and antibodies were administered 72 hours before the saphenous nerve stimulation. As shown in Figure 3, blood flow increase stimulated by applying electronic pulses on saphenous nerve was inhibited by the presence of antibody 4901 at different doses (1 mg/kg, 2.5 mg/kg, 5 mg/kg, and 25 mg/kg) administered intravenously at 24 hours before the saphenous nerve stimulation.

For experiments shown in Figures 4A and 4B, saphenous nerve was exposed surgically before antibody administration. The saphenous nerve of the right hindlimb was exposed surgically, cut proximally and covered with plastic wrap to prevent drying. A laser Doppler probe was placed over the medio-dorsal side of the hindpaw skin, which is the region innervated by the saphenous nerve. Skin blood flow, measured as blood cell flux, was monitored with a laser Doppler flow meter. Thirty to forty-five minutes after bretylium tosylate injection, when a stable base-line flux (less than 5% variation) was established for at least 5 minutes, the nerve was placed over platinum bipolar electrodes and electrically stimulated (2Hz, 10V, 1 ms, for 30 sec) and again 20 minutes later. The average of the blood flow flux response to these two stimulations was used to establish the baseline response (time 0) to electrical stimulation. Antibody 4901 (1 mg/kg or 10 mg/kg), antibody 7E9 (10 mg/kg), antibody 8B6 (10 mg/kg), or vehicle (PBS with 0.01% Tween 20) were then administered intravenously (i.v.). The nerve was subsequently stimulated (2Hz, 10V, 1 ms, for 30 sec) at 30 minutes, 60 minutes, 90 minutes, and 120 minutes after antibody or vehicle administration. Animals were kept under anesthesia for a period of approximately three hours. Cumulative change in skin blood flow was estimated by the area under the flux-time curve (AUC, which is equal to change in flux multiplied by change in time) for each flux response to electrical pulse stimulations.

As shown in Figure 4A, blood flow increase stimulated by applying electronic pulses on saphenous nerve was significantly inhibited by the presence of antibody 4901 1 mg/kg administered i.v., when electronic pulse stimulation was applied at 60 minutes, 90 minutes, and 120 minutes after the antibody administration, and blood flow increase stimulated by applying electronic pulses on saphenous nerve was significantly inhibited by the presence of antibody 4901 10 mg/kg administered i.v., when electronic pulse stimulation was applied at 30 minutes, 60 minutes, 90 minutes, and 120 minutes after the antibody administration. Figure 4B shows that blood flow increase stimulated by applying electronic pulses on saphenous nerve was significantly inhibited by the presence of antibody 7E9 (10 mg/kg, administered i.v.) when electronic pulse stimulation was applied at 30 min, 60 min, 90 min, and 120 min after antibody administration, and by the presence of antibody 8B6 (10 mg/kg, administered i.v.) when electronic pulse stimulation was applied at 30 min after antibody administration.

These data indicate that antibodies 4901, 7E9, 7D11, and 8B6 are effective in blocking CGRP activity as measured by skin vasodilatation induced by stimulation of rat saphenous nerve.

Example 4. Characterization of anti-CGRP antibody G1 and its variants

Amino acid sequences for the heavy chain variable region and light chain variable region of anti-CGRP antibody G1 are shown in Figure 5. The following methods were used for expression and characterization of antibody G1 and its variants.

Expression vector used. Expression of the Fab fragment of the antibodies was under control of an IPTG inducible lacZ promoter similar to that described in Barbas (2001) Phage display: a laboratory manual, Cold Spring Harbor, NY, Cold Spring Harbor Laboratory Press pg. 2.10. Vector pComb3X), however, modifications included addition and expression of the following additional domains: the human Kappa light chain constant domain and the CH1 constant domain of IgG2 human immunoglobulin, Ig gamma-2 chain C region, protein accession number P01859; Immunoglobulin kappa light chain (Homo sapiens), protein accession number CAA09181.

Small scale Fab preparation. From E. coli transformed (either using electroporation-competent TG1 cells or chemically-competent Top 10 cells) with a Fab library, single colonies were used to inoculate both a master plate (agar LB + carbenicillin (50 $\mu$g/mL) + 2% glucose) and a working plate (2 mL/well, 96-well/plate) where each well contained 1.5 mL LB + carbenicillin (50 $\mu$g/mL) + 2% glucose. A gas permeable adhesive seal (ABgene, Surrey, UK) was applied to the plate. Both plates were incubated at 30°C for 12-16 hours; the working plate was shaken vigorously. The master plate was stored at 4°C until needed, while the cells from the working plate were pelleted (4000 rpm, 4°C, 20 minutes) and resuspended in 1.0 mL LB + carbenicillin (50 $\mu$g/mL) + 0.5 mM IPTG to induce expression of Fabs by vigorous shaking for 5 hours at 30°C. Induced cells were centrifuges at 4000 rpm, 4°C for 20 minutes and resuspended in 0.6 mL Biacore HB-SEP buffer (10 mM HEPES pH 7.4, 150 mM NaCl, 3 mM EDTA, 0.005% v/v P20). Lysis of HB-SEP resuspended cells was accomplished by freezing (-80°C) and then thawing at 37°C. Cell lysates were centrifuged at 4000 rpm, 4°C for 1 hour to separate the debris from the Fab-containing supernatants, which were subsequently filtered (0.2 $\mu$m) using a Millipore MultiScreen Assay System 96-Well Filtration Plate and vacuum manifold. Biacore was used to analyze filtered supernatants by injecting them across CGRPs on the sensor chip. Affinity-selected clones expressing Fabs were rescued from the master plate, which provided template DNA for PCR, sequencing, and plasmid preparation.

Large scale Fab preparation. To obtain kinetic parameters, Fabs were expressed on a larger scale as follows. Erlenmeyer flasks containing 150 mL LB + carbenicillin (50 $\mu$g/mL) + 2% glucose were inoculated with 1 mL of a "starter" overnight culture from an affinity-selected Fab-expressing E. coli clone. The remainder of the starter culture (~3 mL) was used to prepare plasmid DNA (QIAprep mini-prep, Qiagen kit) for sequencing and further manipulation. The large culture was incubated at 30°C with vigorous shaking until an $OD_{600nm}$ of 1.0 was attained (typically 12-16 h). The cells were pelleted by centrifuging at 4000 rpm, 4°C for 20 minutes, and resuspended in 150 mL LB + carbenicillin (50 $\mu$g/mL) + 0.5 mM IPTG. After 5 hours expression at 30°C, cells were pelleted by centrifuging at 4000 rpm, 4°C for 20 minutes, resuspended in 10 mL Biacore HBS-EP buffer, and lysed using a single freeze (-80°C)/thaw (37°C) cycle. Cell lysates were pelleted by centrifuging at 4000rpm, 4°C for one hour, and the supernatant was collected and filtered (0.2um). Filtered supernatants were loaded onto Ni-NTA superflow sepharose (Qiagen, Valencia, CA) columns equilibrated with PBS, pH 8, then washed with 5 column volumes of PBS, pH 8. Individual Fabs eluted in different fractions with PBS (pH 8) + 300 mM Imidazole. Fractions containing Fabs were pooled and dialyzed in PBS, then quantified by ELISA prior to affinity characterization.

Full antibody preparation. For expression of full antibodies, heavy and light chain variable regions were cloned in mammalian expression vectors and transfected using lipofectamine into HEK 293 cells for transient expression. Antibodies were purified using protein A using standard methods.

Vector pDb.CGRP.hFcGI is an expression vector comprising the heavy chain of the G1 antibody, and is suitable for transient or stable expression of the heavy chain. Vector pDb.CGRP.hFcGI has nucleotide sequences corresponding to the following regions: the murine cytomegalovirus promoter region (nucleotides 7-612); a synthetic intron (nucleotides 613-1679); the DHFR coding region (nucleotides 688-1253); human growth hormone signal peptide (nucleotides 1899-1976); heavy chain variable region of G1 (nucleotides 1977-2621); human heavy chain IgG2 constant region containing the following mutations: A330P331 to S330S331 (amino acid numbering with reference to the wildtype IgG2 sequence; see Eur. J. Immunol. (1999) 29:2613-2624). Vector pDb.CGRP.hFcGI was deposited at the ATCC on July 15, 2005, and was assigned ATCC Accession No. PTA-6867.

Vector pEb.CGRP.hKGI is an expression vector comprising the light chain of the G1 antibody, and is suitable for transient expression of the light chain. Vector pEb.CGRP.hKGI has nucleotide sequences corresponding to the following regions: the murine cytomegalovirus promoter region (nucleotides 2-613); human EF-1 intron (nucleotides 614-1149); human growth hormone signal peptide (nucleotides 1160-1237); antibody G1 light chain variable region (nucleotides 1238-1558); human kappa chain constant region (nucleotides 1559-1882). Vector pEb.CGRP.hKGI was deposited at the ATCC on July 15, 2005, and was assigned ATCC Accession No. PTA-6866.

Biacore assay for affinity determination. Affinities of G1 monoclonal antibody and its variants were determined at either 25°C or 37°C using the BIACORE3000[3] surface plasmon resonance (SPR) system (Biacore, INC, Piscataway NJ). Affinity was determined by capturing N-terminally biotinylated CGRP or fragments via pre-immobilized streptavidin (SA sensor chip) and measuring the binding kinetics of antibody G1 Fab fragments or variants titrated across the CGRP or fragment on the chip. All Biacore assays were conducted in HBS-EP running buffer (10 mM HEPES pH 7.4, 150 mM NaCl, 3 mM EDTA, 0.005% v/v polysorbate P20). CGRP surfaces were prepared by diluting the N-biotinylated CGRP to a concentration of less than 0.001 mg/mL into HBS-EP buffer and injecting it across the SA sensor chip using variable contact times. Low capacity surfaces, corresponding to capture levels <50 response units (RU) were used for high-resolution kinetic studies, whereas high capacity surfaces (about 800 RU of captured CGRP) were used for concentration studies, screening, and solution affinity determinations. Kinetic data were obtained by diluting antibody G1 Fab serially in two- or three-fold increments to concentrations spanning 1uM-0.1nM (aimed at 0.1-10x estimated $K_D$). Samples were typically injected for 1 minute at 100 $\mu$L/min and dissociation times of at least 10 minutes were allowed. After each binding cycle, surfaces were regenerated with 25 mM NaOH in 25% v/v ethanol, which was tolerated over hundreds of cycles. An entire titration series (typically generated in duplicate) was fit globally to a 1:1 Langmuir binding model using the BIAevaluation program. This returned a unique pair of association and dissociation kinetic rate constants (respectively, $k_{on}$ and $k_{off}$) for each binding interaction, whose ratio gave the equilibrium dissociation constant ($K_D = k_{off}/k_{on}$). Affinities ($K_D$ values) determined in this way are listed in Tables 6 and 7.

High-resolution analysis of binding interactions with extremely slow offrates. For interactions with extremely slow offrates (in particular, antibody G1 Fab binding to human $\alpha$-CGRP on the chip at 25°C), affinities were obtained in a two-part experiment. The protocol described above was used with the following modifications. The association rate constant ($k_{on}$) was determined by injecting a 2-fold titration series (in duplicate) spanning 550 nM-1 nM for 30 seconds at 100 $\mu$L/min and allowing only a 30 second dissociation phase. The dissociation rate constant ($k_{off}$) was determined by injecting three concentrations (high, medium, and low) of the same titration series in duplicate for 30 seconds and allowing a 2-hour dissociation phase. The affinity ($K_D$) of each interaction was obtained by combining the $k_{on}$ and $k_{off}$ values obtained in both types of experiments, as shown in Table 5.

Determining solution affinity by Biacore. The solution affinity of antibody G1 for rat $\alpha$-CGRP and F37A (19-37) human $\alpha$-CGRP was measured by Biacore at 37°C. A high capacity CGRP chip surface was used (the high-affinity human $\alpha$-CGRP was chosen for detection purposes) and HBS-EP running buffer was flowed at 5 $\mu$L/min. Antibody G1 Fab fragment at a constant concentration of 5 nM (aimed to be at or below the expected $K_D$ of the solution-based interaction) was pre-incubated with competing peptide, either rat $\alpha$-CGRP or F37A (19-37) human $\alpha$-CGRP, at final concentrations spanning 1 nM to 1 $\mu$M in 3-fold serial dilutions. Antibody G1 Fab solutions in the absence or presence of solution-based competing peptide, were injected across CGRP on the chip and the depletion of binding responses detected at the chip surface as a result of solution competition was monitored. These binding responses were converted to "free Fab concentrations" using a calibration curve, which was constructed by titrating antibody G1 Fab alone (5, 2.5, 1.25, 0.625, 0.325 and 0 nM) across the CGRP on the chip. "Free Fab concentrations" were plotted against the concentration of competing solution-based peptide used to generate each data point and fit to a solution affinity model using the BIAevaluation software. The solution affinities determined (indirectly) in this way are shown in Tables 5 and 7 and were used to validate the affinities obtained when Fabs are injected directly across N-biotinylated CGRPs on a SA chip. The close agreement between the affinities determined by these two methods confirms that tethering an N-biotinylated version of the CGRP to the chip does not alter its native solution binding activity.

Table 5 below shows the binding affinities of antibody G1 to human $\alpha$-CGRP, human $\beta$-CGRP, rat $\alpha$-CGRP, and rat $\beta$-CGRP determined by Biacore, by flowing Fab fragments across N-biotinylated CGRPs on a SA chip. To better resolve the affinities of binding interactions with extremely slow offrates, affinities were also determined in a two-part experiment to complement this assay orientation, the solution affinity of the rat $\alpha$-CGRP interaction was also determined (as described above). The close agreement of the affinities measured in both assay orientations confirms that the binding affinity of the native rat $\alpha$-CGRP in solution is not altered when it is N-biotinylated and tethered to a SA chip.

Table 5. Binding affinities of antibody G1 Fabs titrated across CGRPs on the chip

| CGRP on chip | Temp. (°C) | $k_{on}$ (1/Ms) | $k_{off}$ (1/s) | $K_D$ (nM) |
|---|---|---|---|---|
| Human $\alpha$-CGRP | 25 | $1.86 \times 10^5$ | $7.80 \times 10^{-6}$ | 0.042 (7%, n=4)* |
| Human $\alpha$-CGRP | 37 | $5.78 \times 10^5$ | $3.63 \times 10^{-5}$ | 0.063 (4%, n=2)* |
| Human $\beta$-CGRP | 37 | $4.51 \times 10^5$ | $6.98 \times 10^{-5}$ | 0.155 |
| Rat $\alpha$-CGRP | 25 | $5.08 \times 10^4$ | $6.18 \times 10^{-5}$ | 1.22 (12%, n=2)* |
| Rat $\alpha$-CGRP | 37 | $1.55 \times 10^5$ | $3.99 \times 10^{-4}$ | 2.57* (Solution $K_D$=10 (50%, n=4)** |
| Rat $\beta$-CGRP | 37 | $5.16 \times 10^5$ | $7.85 \times 10^{-5}$ | 0.152 |

*Affinities for $\alpha$-CGRPs (rat and human) were determined in a high-resolution two-part experiment, in which the dissociation phase was monitored for 2 hours (the values for $k_{on}$, $k_{off}$, and $K_D$ represent the average of n replicate experiments with the standard deviation expressed as a percent variance). Affinities for $\beta$-CGRPs (rat and human) were determined by global analysis using only a 20-min dissociation phase, which was not accurate enough to quantify their extremely offrates (their offrates are likely slower than stated here and therefore their affinities are likely even higher). Antibody G1 Fab dissociated extremely slowly from all CGRPs (except $\alpha$-rat CGRP) with offrates that approached the resolution limit of the Biacore assay (especially at 25°C).
**Solution affinity determined by measuring the depletion of binding responses detected at CGRP on the chip for antibody G1 Fab pre-incubated with solution-based rat $\alpha$-CGRP competitor.

Table 6 below shows antibodies having the amino acid sequence variation as compared to antibody G1 and their affinities to both rat $\alpha$-CGRP and human $\alpha$-CGRP. All amino acid substitutions of the variants shown in Table 6 are described relative to the sequence of G1. The binding affinities of Fab fragments were determined by Biacore by flowing them across CGRPs on a SA chip.

Table 6. Amino acid sequences and binding affinity data for antibody G1 variants determined at 37°C by Biacore.

| Clone | L1 | L2 | H2 | HC-FW3 | $\alpha$-rat $k_{off}$ (1/s) | $\alpha$-rat $K_D$ (nM) | $\alpha$-human $k_{off}$ (1/s) | $\alpha$-human $K_D$ (nM) |
|---|---|---|---|---|---|---|---|---|
| G1 | | | | | $3.99 \times 10^{-4}$ | 2.57 | $3.63 \times 10^{-5}$ | 0.063 |
| M1 | | | | A100L | $1.10 \times 10^{-3}$ | | $1.73 \times 10^{-4}$ | |
| M2 | | | | L99A A100R | $2.6 \times 10^{-3}$ | 58 | $3.1 \times 10^{-4}$ | 3 |
| M3 | | | | L99A A100S | $2.0 \times 10^{-3}$ | 61 | $2.1 \times 10^{-4}$ | 1.7 |
| M4 | | | | L99A A100V | $1.52 \times 10^{-3}$ | 84.4 | $6.95 \times 10^{-5}$ | 0.43 |
| M5 | | | | L99A A100Y | $7.35 \times 10^{-4}$ | 40.8 | $3.22 \times 10^{-5}$ | 0.20 |
| M6 | | | | L99N | $7.84 \times 10^{-4}$ | 43.6 | $1.33 \times 10^{-4}$ | 0.83 |
| M7 | | | | L99N A100C | $9.18 \times 10^{-4}$ | 51.0 | $2.43 \times 10^{-4}$ | 1.52 |
| M8 | | | | L99N A100G | $7.45 \times 10^{-4}$ | 41.4 | $9.20 \times 10^{-5}$ | 0.58 |
| M9 | | | | L99N A100Y | n.d. | n.d. | $1.00 \times 10^{-5}$ | 0.06 |
| M10 | | | | L99S A100S | $1.51 \times 10^{-3}$ | 83.9 | $1.73 \times 10^{-4}$ | 1.08 |

(continued)

| Clone | L1 | L2 | H2 | HC-FW3 | $\alpha$-rat $k_{off}$ (1/s) | $\alpha$-rat $K_D$ (nM) | $\alpha$-human $k_{off}$ (1/s) | $\alpha$-human $K_D$ (nM) |
|---|---|---|---|---|---|---|---|---|
| M11 | | | | L99S A100T | $4.83 \times 10^{-3}$ | 268.3 | $2.83 \times 10^{-4}$ | 1.77 |
| M12 | | | | L99S A100V | $1.94 \times 10^{-3}$ | 107.8 | $1.01 \times 10^{-4}$ | 0.63 |
| M13 | | | | L99T A100G | $1.84 \times 10^{-3}$ | 102.2 | $1.86 \times 10^{-4}$ | 1.16 |
| M14 | | | | L99T A100K | n.d. | n.d. | $1.00 \times 10^{-5}$ | 0.06 |
| M15 | | | | L99T A100P | $1.15 \times 10^{-3}$ | 63.9 | $1.58 \times 10^{-5}$ | 0.10 |
| M16 | | | | L99T A100S | $9.96 \times 10^{-4}$ | 55.3 | $1.65 \times 10^{-4}$ | 1.03 |
| M17 | | | | L99T A100V | $2.06 \times 10^{-3}$ | 114.4 | $1.85 \times 10^{-4}$ | 1.16 |
| M18 | | | | L99V A100G | $1.22 \times 10^{-3}$ | 67.8 | $7.03 \times 10^{-5}$ | 0.44 |
| M19 | | | | L99V A100R | n.d. | n.d. | $1.00 \times 10^{-5}$ | 0.06 |
| M20 | R28W | | | L99R A100L | $1.44 \times 10^{-3}$ | 80.0 | $1.36 \times 10^{-4}$ | 0.85 |
| M21 | R28W | | | L99S | $\underline{6.95 \times 10^{-4}}$ | $\underline{15.2}$ | $\underline{1.42 \times 10^{-4}}$ | $\underline{1.23}$ |
| M22 | R28W | | | L99T | $1.10 \times 10^{-3}$ | 61.1 | $1.16 \times 10^{-4}$ | 0.73 |
| M23 | R28G | | | L99T A100V | $7.99 \times 10^{-4}$ | 44.4 | $1.30 \times 10^{-4}$ | 0.81 |
| M24 | R28L | | | L99T A100V | $1.04 \times 10^{-3}$ | 57.8 | $1.48 \times 10^{-4}$ | 0.93 |
| M25 | R28N | | | L99T A100V | $\underline{1.4 \times 10^{-3}}$ | $\underline{76}$ | $\underline{1.4 \times 10^{-4}}$ | $\underline{1.3}$ |
| M26 | R28N | | A57G | L99T A100V | $9.24 \times 10^{-4}$ | 51.3 | $1.48 \times 10^{-4}$ | 0.93 |
| M27 | R28N T30A | | | L99T A100V | $3.41 \times 10^{-3}$ | 189.4 | $3.57 \times 10^{-4}$ | 2.23 |
| M28 | R28N T30D | | E54R A57N | L99T A100V | $1.25 \times 10^{-3}$ | 69.4 | $9.96 \times 10^{-5}$ | 0.62 |
| M29 | R28N T30G | | | L99T A100V | $3.59 \times 10^{-3}$ | 199.4 | $3.80 \times 10^{-4}$ | 2.38 |
| M30 | R28N T30G | | E54K A57E | L99T A100V | $6.38 \times 10^{-3}$ | 354.4 | $5.90 \times 10^{-4}$ | 3.69 |
| M31 | R28N T30G | | E54K A57G | L99T A100V | $3.61 \times 10^{-3}$ | 200.6 | $3.47 \times 10^{-4}$ | 2.17 |
| M32 | R28N T30G | | E54K A57H | L99T A100V | $2.96 \times 10^{-3}$ | 164.4 | $2.71 \times 10^{-4}$ | 1.69 |

(continued)

| Clone | L1 | L2 | H2 | HC-FW3 | $\alpha$-rat $k_{off}$ (1/s) | $\alpha$-rat $K_D$ (nM) | $\alpha$-human $k_{off}$ (1/s) | $\alpha$-human $K_D$ (nM) |
|---|---|---|---|---|---|---|---|---|
| M33 | R28N | | E54K | L99T | $9.22 \times 10^{-3}$ | 512.2 | $7.50 \times 10^{-4}$ | 4.69 |
| | T30G | | A57N S58G | A100V | | | | |
| M34 | R28N T30G | | E54K A57N S58T | L99T A100V | $2.17 \times 10^{-3}$ | 120.6 | $6.46 \times 10^{-4}$ | 4.04 |
| M35 | R28N T30G | | E54K A57S | L99T A100V | $3.99 \times 10^{-3}$ | 221.7 | $3.39 \times 10^{-4}$ | 2.12 |
| M36 | R28N T30R | | | L99T A100V | $4.79 \times 10^{-3}$ | 266.1 | $2.39 \times 10^{-4}$ | 1.49 |
| M37 | R28N T30S | | A57G | L99T A100V | $1.45 \times 10^{-3}$ | 80.6 | $2.26 \times 10^{-4}$ | 1.41 |
| M38 | R28N T30W | | | L99T A100V | $5.11 \times 10^{-3}$ | 283.9 | $2.18 \times 10^{-4}$ | 1.36 |
| M39 | R28N | G50A L56T | A57N S58Y | L99T A100V | $9.95 \times 10^{-3}$ | 552.8 | $4.25 \times 10^{-4}$ | 2.66 |
| M40 | R28N | G50A L56T | E54K A57L | L99T A100V | 0.36 | 20000.0 | $1.28 \times 10^{-3}$ | 8.00 |
| M41 | R28N | G50A L56T | E54K A57N E64D | L99T A100V | $4.53 \times 10^{-3}$ | 251.7 | $2.10 \times 10^{-4}$ | 1.31 |
| M42 | R28N | G50A L56T | E54K A57N H61F | L99T A100V | $7.52 \times 10^{-3}$ | 417.8 | $4.17 \times 10^{-4}$ | 2.61 |
| M43 | R28N | G50A L56T | E54K A57N S58C | L99T A100V | $4.53 \times 10^{-3}$ | 251.7 | $2.63 \times 10^{-4}$ | 1.64 |
| M44 | R28N | G50A L56T | E54K A57N S58E | L99T A100V | $\underline{6.13 \times 10^{-3}}$ | $\underline{443}$ | $\underline{2.10 \times 10^{-4}}$ | $\underline{2.05}$ |
| M45 | R28N | G50A L56T | E54K A57N S58E E64D | L99T A100V | $\underline{5.58 \times 10^{-3}}$ | $\underline{259}$ | $\underline{2.11 \times 10^{-4}}$ | $\underline{1.85}$ |
| M46 | R28N | G50A L56T | E54K A57N S58E H61F | L99T A100V | $2.94 \times 10^{-3}$ | 163.3 | $5.39 \times 10^{-4}$ | 3.37 |
| M47 | R28N | G50A L56T | E54K A57N S58G | L99T A100V | $8.23 \times 10^{-3}$ | 457.2 | $3.32 \times 10^{-4}$ | 2.08 |
| M48 | R28N | G50A L56T | E54K A57N S58L | L99T A100V | 0.0343 | 1905.6 | $8.42 \times 10^{-4}$ | 5.26 |
| M49 | R28N | G50A L56T | E54K A57N S58Y H61F | L99T A100V | 0.0148 | 822.2 | $5.95 \times 10^{-4}$ | 3.72 |
| M50 | R28N | G50A L56T | E54K A57R | L99T A100V | $5.30 \times 10^{-3}$ | 294.4 | $4.06 \times 10^{-4}$ | 2.54 |
| M51 | R28N | L56I | E54K A57G | L99T A100V | $1.18 \times 10^{-3}$ | 65.6 | $1.31 \times 10^{-4}$ | 0.82 |
| M52 | R28N | L56I | E54K A57N | L99T A100V | $2.29 \times 10^{-3}$ | 127.2 | $2.81 \times 10^{-4}$ | 1.76 |
| | | | S58A | | | | | |

(continued)

| Clone | L1 | L2 | H2 | HC-FW3 | $\alpha$-rat $k_{off}$ (1/s) | $\alpha$-rat $K_D$ (nM) | $\alpha$-human $k_{off}$ (1/s) | $\alpha$-human $K_D$ (nM) |
|---|---|---|---|---|---|---|---|---|
| M53 | R28N | L56I | E54K A57N S58G | L99T A100V | $1.91 \times 10^{-3}$ | 106.1 | $3.74 \times 10^{-4}$ | 2.34 |
| M54 | R28N T30A | G50A | E54K A57N S58P | L99T A100V | $2.16 \times 10^{-3}$ | 120.0 | $1.79 \times 10^{-3}$ | 11.19 |
| M55 | R28N T30A | L56S | E54K A57N S58E E64D | L99T A100V | $5.85 \times 10^{-3}$ | 325.0 | $4.78 \times 10^{-4}$ | 2.99 |
| M56 | R28N T30D | L56S | E54K A57N H61F | L99T A100V | $9.35 \times 10^{-3}$ | 519.4 | $4.79 \times 10^{-4}$ | 2.99 |
| M57 | R28N T30D | L56S | E54K A57N S58E | L99T A100V | 0.0104 | 1,200 | $3.22 \times 10^{-4}$ | 3.08 |
| M58 | R28N T30D | L56S | E54K A57N S58I H61F | L99T A100V | No binding | n.d. | $1.95 \times 10^{-3}$ | 12.19 |
| M59 | R28N T30D | L56S | E54K A57N S58N H61F | L99T A100V | 0.0123 | 683.3 | $5.24 \times 10^{-4}$ | 3.28 |
| M60 | R28N T30D | L56S | E54K A57N S58R H61F | L99T A100V | 0.0272 | 1511.1 | $9.11 \times 10^{-4}$ | 5.69 |
| M61 | R28N T30G | A51H | E54Q A57N H61F | L99T A100V | $5.21 \times 10^{-3}$ | 289.4 | $4.59 \times 10^{-4}$ | 2.87 |
| M62 | R28N T30G | A51H L56T | E54K A57N S58E | L99T A100V | $5.75 \times 10^{-3}$ | 242 | $5.57 \times 10^{-4}$ | 5.86 |
| M63 | R28N T30G | G50A | E54K A57N S58T | L99T A100V | $2.65 \times 10^{-3}$ | 147.2 | $1.50 \times 10^{-3}$ | 9.38 |
| M64 | R28N T30G | G50A | E54K A57N S58V | L99T A100V | 0.0234 | 1300.0 | $1.32 \times 10^{-3}$ | 8.25 |
| M65 | R28N T30G | G50A L56I | E54K A57C | L99T A100V | $4.07 \times 10^{-3}$ | 226.1 | $8.03 \times 10^{-4}$ | 5.02 |
| M66 | R28N T30G | L56I | E54K A57E | L99T A100V | $5.11 \times 10^{-3}$ | 283.9 | $5.20 \times 10^{-4}$ | 3.25 |
| M67 | R28N T30G | L56I | E54K A57F | L99T A100V | $1.71 \times 10^{-3}$ | 95.0 | $8.20 \times 10^{-4}$ | 5.13 |
| M68 | R28N T30G | L56I | E54K A57N S58D E64D | L99T A100V | $6.76 \times 10^{-3}$ | 375.6 | $4.28 \times 10^{-4}$ | 2.68 |
| M69 | R28N T30G | L56I | E54K A57N S58E | L99T A100V | $1.81 \times 10^{-3}$ | 100.6 | $7.33 \times 10^{-4}$ | 4.58 |
| M70 | R28N T30G | L56I | E54K A57S | L99T A100V | $6.07 \times 10^{-3}$ | 337.2 | $5.59 \times 10^{-4}$ | 3.49 |
| M71 | R28N T30G | L56I | E54K A57Y | L99T A100V | $2.12 \times 10^{-3}$ | 117.8 | $1.28 \times 10^{-3}$ | 8.00 |
| M72 | R28N T30G | L56S | E54K | L99T A100V | $3.95 \times 10^{-3}$ | 219.4 | $4.00 \times 10^{-4}$ | 2.50 |
| M73 | R28N T30G | L56S | E54K A57N S58Y E64D | L99T A100V | $3.00 \times 10^{-3}$ | 166.7 | $2.55 \times 10^{-4}$ | 1.59 |

(continued)

| Clone | L1 | L2 | H2 | HC-FW3 | α-rat $k_{off}$ (1/s) | α-rat $K_D$ (nM) | α-human $k_{off}$ (1/s) | α-human $K_D$ (nM) |
|-------|-----|-----|-----|--------|------------|-----------|--------------|--------------|
| M74 | R28N T30G | L56S | E54K A57S | L99T A100V | $6.03 \times 10^{-3}$ | 335.0 | $5.97 \times 10^{-4}$ | 3.73 |
| M75 | R28N T30G | L56S | E54K A57V | L99T A100V | $1.87 \times 10^{-2}$ | 1038.9 | $1.16 \times 10^{-3}$ | 7.25 |
| M76 | R28N T30S | G50A L56T | A57G | L99T A100V | $1.16 \times 10^{-3}$ | 64.4 | $3.64 \times 10^{-4}$ | 2.28 |
| M77 | R28N T30S | G50A L56T | E54K A57D | L99T A100V | 0.0143 | 794.4 | $4.77 \times 10^{-4}$ | 2.98 |
| M78 | R28N T30S | G50A L56T | E54K A57N S58T | L99T A100V | 0.167 | 9277.8 | $1.31 \times 10^{-3}$ | 8.19 |
| M79 | R28N T30S | G50A L56T | E54K A57P | L99T A100V | 0.19 | 10555.6 | $1.29 \times 10^{-3}$ | 8.06 |
| M80 | R28N T30S | L56I | E54K A57N S58V | L99T A100V | 0.0993 | 5516.7 | $2.09 \times 10^{-3}$ | 13.06 |
| M81 | R28N T30S | L56S | E54K A57N S58E | L99T A100V | $4.29 \times 10^{-3}$ | 238.3 | $4.90 \times 10^{-4}$ | 3.06 |
| M82 | R28N T30V | A51H L56T | A57N | L99T A100V | $6.99 \times 10^{-3}$ | 388.3 | $8.77 \times 10^{-4}$ | 5.48 |
| M83 | R28N T30V | A51H L56T | E54K A57N S58M H61F | L99T A100V | No binding | n.d. | $9.33 \times 10^{-4}$ | 5.83 |
| M84 | R28N T30V | A51H L56T | E54N A57N | L99T A100V | $1.76 \times 10^{-2}$ | 977.8 | $1.08 \times 10^{-3}$ | 6.75 |

All CDRs including both Kabat and Chothia CDRs. Amino acid residues are numbered sequentially (see Figure 5). All clones have L3+H1+H3 sequences identical to G1.

$K_D = k_{off}/k_{on}$. All $k_{off}$ values were determined in a screening mode except those that are <u>underlined</u>, which were obtained by global analysis of a Fab concentration series (G1 was analyzed in a high-resolution mode). <u>Underlined</u> $K_D$ values were therefore determined experimentally by measuring $k_{on}$. Other $k_{on}$ values were estimated to be the same as M25.

n.d. = not determined

To determine the epitope on human α-CGRP that is recognized by antibody G1, Biacore assays described above were used. Human α-CGRP was purchased as an N-biotinylated version to enable its high-affinity capture via SA sensor chips. The binding of G1 Fab fragment to the human α-CGRP on the chip in the absence or presence of a CGRP peptide was determined. Typically, a 2000:1 mol peptide/Fab solution (e.g., 10 μM peptide in 50nM G1 Fab) was injected across human α-CGRP on the chip. Figure 6 shows the percentage of binding blocked by competing peptide. Data shown in Figure 6 indicate that peptides that block 100% binding of G1 Fab to human α-CGRP are 1-37 (WT), 8-37, 26-37, P29A (19-37), K35A (19-37), K35E (19-37), and K35M (19-37) of human α-CGRP; 1-37 of β-CGRP (WT); 1-37 of rat α-CGRP (WT); and 1-37 of rat β-CGRP (WT). All these peptides are amidated at the C-terminus. Peptides F37A (19-37) and 19-37 (the latter not amidated at the C-terminus) of human α-CGRP also blocked about 80% to 90% of binding of G1 Fab to human α-CGRP. Peptide 1-36 (not amidated at the C-terminus) of human α-CGRP blocked about 40% of binding of G1 Fab to human α-CGRP. Peptide fragment 19-36 (amidated at the C-terminus) of human α-CGRP; peptide fragments 1-13 and 1-19 of human α-CGRP (neither of which are amidated at the C-terminus); and human amylin, calcitonin, and adrenomedullin (all amidated at the C-terminus) did not compete with binding of G1 Fab to human α-CGRP on the chip. These data demonstrate that G1 targets a C-terminal epitope of CGRP and that both the identity of the most terminal residue (F37) and its amidation is important for binding.

Binding affinities of G1 Fab to variants of human α-CGRP (at 37°C) was also determined. Table 7 below shows the affinities as measured directly by titrating G1 Fab across N-biotinylated human α-CGRP and variants on the chip.

Data in Table 7 indicate that antibody G1 binds to a C-terminal epitope with F37 and G33 being the most important residues. G1 does not bind to CGRP when an extra amino acid residue (alanine) is added at the C-terminal (which is amidated).

Table 7. Binding affinities of G1 Fab to human α-CGRP and variants measured at 37°C (see Table 4 for their amino acid sequences)

| CGRP on chip | $k_{on}$ (1/Ms) | $k_{off}$ (1/s) | $K_D$ (nM) |
|---|---|---|---|
| 137 (WT) | $4.68 \times 10^5$ | $7.63 \times 10^{-5}$ | 0.16 (high resolution $K_D$ = 0.06) |
| 19-37 | $4.60 \times 10^5$ | $7.30 \times 10^{-5}$ | 0.16 |
| 25-37 | $3.10 \times 10^5$ | $8.80 \times 10^{-5}$ | 0.28 |
| F27A (25-37) | $3.25 \times 10^5$ | $1.24 \times 10^{-4}$ | 0.38 |
| V28A (25-37) | $3.32 \times 10^5$ | $9.38 \times 10^{-5}$ | 0.28 |
| P29A (25-37) | $2.26 \times 10^5$ | $1.78 \times 10^{-4}$ | 0.79 |
| T30A (25-37) | $1.79 \times 10^5$ | $8.41 \times 10^{-5}$ | 0.47 |
| N31A (25-37) | $2.17 \times 10^5$ | $1.14 \times 10^{-4}$ | 0.53 |
| V32A (25-37) | $2.02 \times 10^5$ | $3.46 \times 10^{-4}$ | 1.71 |
| G33A (25-37) | $2.07 \times 10^5$ | 0.0291 | 141 |
| S34A (25-37) | $2.51 \times 10^5$ | $7.64 \times 10^{-4}$ | 3.04 |
| K35A (19-37) | $2.23 \times 10^5$ | $2.97 \times 10^{-4}$ | 1.33 |
| K35E (19-37) | $5.95 \times 10^4$ | $5.79 \times 10^{-4}$ | 9.73 |
| K35M (19-37) | $2.63 \times 10^5$ | $1.34 \times 10^{-4}$ | 0.51 |
| K35Q (19-37) | $1.95 \times 10^5$ | $2.70 \times 10^{-4}$ | 1.38 |
| F37A (25-37) | $8.90 \times 10^4$ | $8.48 \times 10^{-3}$ | 95 (solution $K_D$ = 172 nM) |
| 38A (25-38A) | - | - | No binding detected |

The above data indicate that the epitope that antibody G1 binds is on the C-terminal end of human α-CGRP, and amino acids 33 and 37 on human α-CGRP are important for binding of antibody G1. Also, the amidation of residue F37 is important for binding.

Example 5: Effect of anti-CGRP antagonist antibody G1 on skin vasodilatation induced by stimulation of rat saphenous nerve

To test antagonist activity of anti-CGRP antibody G1, effect of the antibody on skin vasodilatation by stimulation of rat saphenous nerve was tested using a rat model described in Example 3. Briefly, rats were maintained anesthesia with 2% isoflurane. Bretylium tosylate (30 mg/kg, administered i.v.) was given at the beginning of the experiment to minimize vasoconstriction due to the concomitant stimulation of sympathetic fibers of the saphenous nerve. Body temperature was maintained at 37°C by the use of a rectal probe thermostatically connected to a temperature controlled heating blanket. The saphenous nerve of the right hindlimb was exposed surgically, cut proximally and covered with plastic wrap to prevent drying. A laser Doppler probe was placed over the medio-dorsal side of the hindpaw skin, which is the region innervated by the saphenous nerve. Skin blood flow, measured as blood cell flux, was monitored with a laser Doppler flow meter. In experiments to determine effects of antibody within two hours of injection thirty to forty-five minutes after bretylium tosylate injection, when a stable base-line flux (less than 5% variation) was established for at least 5 minutes, the nerve was placed over platinum bipolar electrodes and electrically stimulated (2Hz, 10V, 1 ms, for 30 seconds) and again 20 minutes later. The average of the blood flow flux response to these two stimulations was used to establish the baseline response (time 0) to electrical stimulation. Antibody G1 (1 mg/kg or 10 mg/kg) or vehicle (PBS with 0.01% Tween 20 equal volume to 10 mg/kg G1) were then administered intravenously (i.v.). The nerve was subsequently stimulated (2Hz, 10V, 1 ms, for 30 seconds) at 30 minutes, 60 minutes, 90 minutes, and 120 minutes after the antibody administration. Animals were kept under anesthesia for a period of approximately three hours. Cumulative change in skin blood flow was estimated by the area under the flux-time curve (AUC, which is equal to change in flux multiplied by change in time) for each flux response to electrical pulse stimulations.

As shown in Figure 7, blood flow increase stimulated by applying electronic pulses on saphenous nerve was significantly inhibited by the presence of antibody G1 at 1 mg/kg (administered i.v.) as compared to the vehicle, when the saphenous nerve was electrically stimulated at 90 min after the antibody administration. Blood flow increase stimulated by applying electronic pulses on saphenous nerve was significantly inhibited by the presence of antibody G1 at 10 mg/kg (administered i.v.) as compared to the vehicle, when the saphenous nerve was electrically stimulated at 90 minutes and 120 minutes after antibody administration.

In experiments to determine effects of the antibodies at longer time points in the saphenous assay, rats were injected i.v. with the indicated doses of antibody 24 hours or 7 days prior to preparing the animal for saphenous nerve stimulation as described above. In these experiments it was impossible to establish a baseline response in individual rats to electrical pulse stimulation prior to dosing, so treated groups were compared to animals dosed with vehicle (PBS, 0.01% Tween 20) at 24 hours or 7 days.

As shown in Figures 8A and 8B, blood flow increases in the dorso-medial hindpaw skin evoked by saphenous nerve stimulation were significantly inhibited in the groups of animals dosed with either 10 mg/kg or 3 mg/kg G1 at either 24 hours or 7 days prior to stimulation as compared to vehicle groups dosed at the same time points.

Figure 8C represents a curve fit analysis applied to the dose response data represented in figures 8A and 8B to determine the dose required for 50% maximal effect ($EC_{50}$). The $EC_{50}$ at 24 hours is 1.3 mg/kg and the $EC_{50}$ at 7 days is slightly lower (0.8 mg/kg).

Example 6: Acute effect of anti-CGRP antagonist antibody mu7E9 in a dural artery (closed cranial window) assay

Closed Cranial Window Model: The purpose of this experiment was to determine the acute effect of anti-CGRP antagonist antibodies and compare it with the acute effect of the CGRP receptor antagonist BIBN4096BS. Experiments were carried out as previously described (Williamson et al., Cephalalgia 17(4):518-24 (1997)) with the following modifications. Sprague Dawley rats (300-400g) were anesthetized with 70 mg/kg i.p. pentobarbital. Anesthesia was maintained with 20 mg/kg/hr i.v. pentobarbital. Rats were cannulated through the jugular vein for delivery of all drugs. Blood pressure was monitored with a probe (mikro-tip catheter, Millar Instruments) threaded through the femoral artery into the abdominal aorta. The rats were tracheotomized and breathing rate was maintained at 75 breaths per minute at a volume of 3.5 mL. After fixating the head in a stereotactic instrument and removing the scalp, a 2x6 mm window in the left parietal area just lateral to the sagittal suture was made by thinning the bone with a dental drill. Using a micromanipulator, a platinum bipolar electrode was lowered onto the surface and covered with heavy mineral oil. Lateral to the electrode window another window of 5x6 mm was created and filled with heavy mineral oil through which the diameter of a branch of the middle meningeal artery (MMA) was continuously monitored with a CCD camera and a video dimension analyzer (Living Systems). The rats were rested for no less than 45 minutes after the preparation. A baseline response to electrical stimulation was established (15 V, 10 hz, 0.5 ms pulses, 30 seconds) and then rats were dosed i.v. with experimental compound (10 mg/kg mu7E9, 300 μg/kg BIBN4096BS, or PBS 0.01%, Tween 20). Additional electrical stimulations were done at 5 (BIBN4096BS), 30, 60, 90, and 120 minutes after dosing. All data was recorded using chart software (ADInstruments).

As shown in Figure 9, mu7E9 at 10 mg/kg significantly blocks MMA dilation evoked by electrical field stimulation within 60 minutes after dosing and maintains the effect throughout the duration of the assay (120 minutes). For comparison BIBN4096BS blocks MMA dilation within 5 minutes of dosing but the effect has completely disappeared by 90 minutes. The magnitude of the block is comparable between BIBN4096BS and mu7E9.

Example 7: Chronic effect of anti-CGRP antagonist antibody G1 in a dural artery (closed cranial window) assay

The purpose of this experiment was to determine if the anti CGRP antibody could still block electrically stimulated MMA dilation 7 days after dosing. Preparation of the rats was identical to the above described acute experiment (Example 6) with the following exceptions. Rats were injected i.v. (10 mg/kg, 3 mg/kg, or 1 mg/kg G1) 7 days prior to creating the closed cranial window prep and stimulation. It was impossible to establish a baseline dilation response to electrical stimulation prior to dosing as in the acute experiment so the antibody groups were compared to dilation of the MMA in a vehicle (PBS, 0.01% Tween 20) dosed control group. After the rats were allowed to rest for no less than 45 minutes the dura was electrically stimulated at 30 minute intervals. Stimulations were at 2.5V, 5V, 10V, 15V, and 20V, all at 10 Hz, 0.5 ms pulses for 30 seconds.

As shown in Figure 10, G1 at 10 mg/kg and 3 mg/kg significantly blocked MMA dilation evoked by electrical stimulation in the range of 10 to 20 volts. This data demonstrates that G1 can block electrically stimulated MMA dilation up to 7 days after dosing.

Example 8: Morphine withdrawal hot flush model

The morphine withdrawal rat model is an established rodent model for menopausal hot flush mechanisms (Sipe et al., Brain Res. 1028(2):191-202 (2004); Merchenthaler et al., Maturitas 30:307-316 (1998); Katovich et al., Brain Res. 494:85-94 (1989); Simpkins et al., Life Sciences 32:1957-1966 (1983)). Basically the rats are addicted to morphine by implanting morphine pellets under the skin. Upon addiction the animals are injected with naloxone (opioid antagonist) which sends them into withdrawal immediately. This withdrawal is accompanied by a skin temperature increase, a core body temperature decrease, an increase in heart rate and an increase in serum luteinizing hormone. These are all similar in magnitude and timing to what occurs in human hot flush (Simpkins et al., Life Sciences 32:1957-1966 (1983)). Furthermore, if rats are treated with estradiol prior to inducing withdrawal, the symptoms of hot flush are reduced (Merchenthaler et al., Maturitas 30:307-316 (1998)). This is why the morphine withdrawal model is believed to mimic clinical hot flush.

Ovariectomized rats were ordered from Charles River Laboratories. Not less than 7 days post ovariectomy morphine dependency was created by implanting a morphine pellet (75 mg morphine base) subcutaneously. Two days later, two more pellets were implanted. The following day rats were injected intravenously with either 10 mg/kg 4901 [**] or vehicle (PBS, 0.01% tween). Two days after the second pelleting the rats were anesthetized with ketamine (90 mg/kg) and lightly restrained. A surface temperature thermocouple was taped to the base of the tail and a rectal thermocouple is used to measure core temperature. Data was recorded using Chart software (ADInstruments). After recording 15 minutes of stable baseline temperature, naloxone (1mg/kg) was injected subcutaneously. Temperature was recorded continuously for the next 60 minutes. The results are shown in Figures 11A and 11B.

Example 9: Non-clinical Toxicology and Pharmacokinetics

Anti-CGRP antagonist antibody G1 was well-tolerated in 1-month IV repeat-dose toxicity studies in Sprague-Dawley (SD) rats and cynomolgus monkeys and no target organ toxicity was determined in either of these studies. A no adverse event level (NOAEL) of 100 mg/kg/week was established for both the rat and monkey studies. This dose level corresponded to systemic exposure with a maximum concentration (Cmax) of 2,570 and 3,440 $\mu$g/mL and areas under the curve (AUC(0-168h)) of 194,000 $\mu$g·h/mL and 299,000 $\mu$g·h/mL (Day 22) in rats and monkeys, respectively.

In a 3-month IV/SC rat study, no target organ toxicities were identified and G1 was well-tolerated up to the highest tested dose, 300 mg/kg. In a 3-month monkey study, perivascular inflammation of the ciliary artery, as the result of the deposition of immune complexes was observed at ≥100 mg/kg. This finding was attributed to the monkey's immunogenic response to a humanized antibody and was not considered to be clinically relevant. The highest tested dose of 300 mg/kg in this monkey study are at least 10-fold greater than the highest anticipated clinical dose of 2,000 mg or 29 mg/kg on a mg/kg basis (assuming an average subject weight of 70 kg).

Example 10: Clinical Pharmacokinetics

The PK of antibody G1 following single IV exposure was examined in four randomized, placebo-controlled, double-blind studies examining doses between 10 and 2,000 mg. Maximum plasma concentrations (Cmax) were reached shortly after the end of the 1-hour IV infusion. Median time to Cmax (Tmax) ranged from 1.0 to 3.0 hours, followed by a multiphasic decline. Cmax and total exposure increased approximately linearly with escalating doses of G1. Terminal half-life (t½) ranged from 36.4 to 48.3 days. There is no evidence of G1 metabolism in the liver, the primary mode of metabolism is by proteosomic degradation.

One study defined the pharmacokinetics of 30 mg and 300 mg doses given twice, two weeks apart. Maximum concentrations and area under the concentration-time profile increased with increasing dose. The apparent terminal half-life (t½) after the second dose was 41.2 days (30 mg) and 50.0 days (300 mg) (arithmetic mean). The plasma accumulation ratios of G1 after two IV doses administered 15 days apart were 1.5 (30 mg) and 1.4 (300 mg).

Example 11: Clinical Safety and Pharmacokinetics

In six studies, antibody G1 was administered to 118 healthy males and females, while 57 male and female subjects received placebo. The study included single IV doses ranging from 0.2 mg up to 2,000 mg, two IV doses of up to 300 mg given once every 14 days, and SC administration of 225 and 900 mg. The six studies included: two IV single dose escalation PK and pharmacodynamics (PD) studies in healthy males (studies B0141001 and B0141002); a two-cohort, placebo controlled cross-over study to examine the acute effects of IV administration of antibody G1 on capsaicin flare response in healthy volunteers (B0141006); a parallel group repeat dose study of antibody G1 in healthy male and female volunteers (B0141007); a single dose study evaluating the safety and tolerability of doses up to 2,000 mg administered IV to healthy female volunteers (B0141008), and a study comparing the relative safety and bioavailability between IV and SC administration (G1-SC-IV).

The six studies are summarized below in Table 8. Of the five IV studies (B0141001, B0141002, B0141006, B0141007 and B0141008), three had virtually identical designs and assessments. Study B014100 tested doses of 0.2 mg, 1 mg, and 3 mg given as a single one-hour IV infusion. The study had a parallel design. Participants were confined in the clinic for seven days after the infusion, with multiple assessments on each of these days. After discharge, patients were reassessed one week after discharge (day 14), and then one, two, and three months after the infusion. Study B0141002 tested doses ranging from 10 mg to 1000 mg as a single administration. Finally, Study B0141008 tested doses of 300 mg, 1000 mg, 1500 mg, or 2000 mg. Study B0141006 was distinct from the others since it also aimed to integrate pharmacodynamic readouts through measuring capsaicin flare inhibition up to one week after IV infusion of antibody G1.

For the IV studies, adverse events (AEs) profiles were reported for the first dosed period only. Study B0141007 tested multiple doses of antibody G1 at either 30 or 300 mg IV given two weeks apart, using a parallel design. Each eligible subject was assigned a randomization sequence via an interactive Web-based system that contained the treatment assignment. The randomization schema was developed by the lead statistician. Participants in all studies were generally healthy men and women (from 18 to 65 years of age); all participants signed informed consent forms. All studies were approved by investigation review boards (IRBs). AEs were defined as any untoward medical occurrence in clinical study participants, with or without causal relationship to study drug. AEs observed after administration of the study drug or placebo were termed "treatment-emergent" AE (TEAEs) regardless of potential causality with the study drug. All subjects experiencing TEAEs were followed at appropriate time intervals until the event had resolved or until the event had stabilized and/or reached a new baseline. All TEAEs were ranked as being mild, moderate, or severe. Serious AEs (SAEs) were defined a priori as any untoward medical occurrence that at any dose resulted in death, was life threatening (i.e., the subject was at immediate risk of death at the time of the event), required inpatient hospitalization or prolongation of existing hospitalization, resulted in persistent or significant disability/incapacity (e.g., a substantial disruption of the subject's ability to carry out normal life functions), resulted in a congenital anomaly/birth defect, or any other medically important event. Treatment-related AE (TRAEs) were to be considered when one of the following situations was present: 1) a plausible temporal relationship between the onset of the AE and administration of the investigational product could be identified; 2) the AE could not be readily explained by the patient's clinical state, intercurrent illness, or concomitant therapies; and 3) the AE abated on discontinuation of the investigational product or dose reduction.

Blood pressure, pulse rate and oral temperature were measured at screening, pre-dose, immediately after the end of the infusion and multiple times during the patients' confinements in the clinics, as well as at all clinic visits. Laboratory tests included serum chemistries, hematology, and urinalysis. Hematology, chemistry, coagulation, and urine safety laboratory tests were performed at multiple study times. ECGs were recorded at screening, pre-dose on Day 1, immediately after the end of the infusion and five other times during the first day, as well as in all clinic visits. QTcF values were derived using Fridericia's (QTcF) heart rate correction formula. Absolute values and changes from baseline for the ECG parameters QT interval, heart rate, QTcF interval, PR interval and QRS interval were assessed by cohort, treatment, and time post-dose. In addition to the safety assessments described above, Protocol B014008 included complete ophthalmic assessments at baseline and at three time points after dosing (Day 28, Day 84, and Day 168).

Clinical data and vital signs were summarized using descriptive tables and summary statistics. Laboratory and other safety data were summarized as a function of any change (values outside of the reference range), as well as any clinical relevant changes, which were defined a priori. Summary tables were stratified by dose and data were pooled across studies. In addition, comparisons for consolidated data for all antibody G1 exposures were contrasted with placebo. Placebo was also contrasted with antibody G1 doses of 100 mg and higher (100 mg, 300 mg, 1000 mg, 1500 mg, and 2000 mg), and with antibody G1 doses of 1000 mg and higher (1000 mg, 1500 mg, and 2000 mg).

In the IV/SC study (G1-SC-IV), thirty-six subjects were randomized to receive a single administration of antibody G1 (225 or 900 mg) or placebo, delivered as either a subcutaneous (SC) bolus injection or a 1-hour IV infusion. Subjects were confined in the clinical research unit for seven days after dosing, and returned to the clinic periodically for additional outpatient visits up to Study Day 90. ECGs were performed extensively on Day 1 (pre-dose, Hours 1, 6, 12), Day 3, and Day 7 while the subjects were confined and once at the completion of the study (Day 90). Vital signs, including temperature, blood pressure and heart rate, were collected pre-dose, Days 1, 3, 7, and 90.

Table 8

| Study | Study population | Treatment with Antibody G1 |
|---|---|---|
| B0141001 | Healthy adult men (n = 24) | Single intravenous (IV) infusion of 0.2, 1 or 3 mg in cohorts of eight (six/cohort, active treatment; two in placebo cohort) |
| B0141002 | Healthy adult men (n = 40) | Single IV infusion of 10, 30, 100, 300 or 1000 mg in cohorts of eight (six/cohort, active treatment; 10 in placebo cohort) |

(continued)

| Study | Study population | Treatment with Antibody G1 |
|---|---|---|
| B0141006 | Healthy adult men (n = 12) | Two cohorts modified cross-over, placebo or 300 mg IV infusion in cohorts of six. In the first period, all participants received placebo. For the second period (included herein), 12 participants received placebo and 11 received 300 mg. |
| B0141007 | Healthy adult men and women (n = 21) | Two IV infusions two weeks apart at 30 or 300 mg in cohorts of 10 or 11 (six/cohort, active treatment; nine in placebo cohort) |
| B0141008 | Healthy adult women (n = 31) | Single IV infusion of 300, 1000, 1500, or 2000 mg (five in 2000 mg cohort; six/cohort remaining treatment groups; eight in placebo cohort) |
| G1-SC-IV | Thirty-six subjects (n = 36) | Single subcutaneous (SC) bolus injection or single IV infusion of 225 or 900 mg |

Across the broad range of dosages evaluated in the five IV studies (0.2 to 2,000 mg), IV antibody G1 was acceptably tolerated. Table 9 summarizes the overall adverse event (AE) rate by dose for the IV studies. Based on these tolerability results, overt safety concerns have not emerged. Across all trials in the IV studies, participants receiving placebo reported an average of 1.3 treatment emergent adverse events (TEAEs). These are all reported events, regardless of the investigator's opinion of relationship to study drug. Across all IV G1 doses, the rate was 1.4 TEAEs/subject. Subjects receiving G1 doses of 100 mg or higher had an average of 1.5 TEAEs; those receiving doses of 1,000 mg or higher had an average of 1.6 TEAEs.

Table 9

| | Subjects Evaluated for AE | Number of AEs - n (N) | Subjects with AE - n (N) | Subject with Serious AE-n (N) | Subject with Severe AE-n(N) | Subjects discontinued for AEs - n (N) | Dose reduced or temporary discontinuations - n (N) |
|---|---|---|---|---|---|---|---|
| Placebo | 45 | 57 (11) | 23 (8) | 0 | 0 | 2(1) | 0 |
| 0.2mg | 6 | 5 (0) | 2 (0) | 0 | 0 | 0 | 0 |
| 1mg | 6 | 1 (1) | 3 (0) | 0 | 0 | 0 | 0 |
| 3mg | 6 | 10 (2) | 4 (1) | 0 | 0 | 0 | 0 |
| 10mg | 6 | 5 (1) | 4 (1) | 0 | 0 | 0 | 0 |
| 30mg | 12 | 21 (11) | 8 (5) | 0 | 0 | 0 | 0 |
| 100mg | 6 | 5 (1) | 4 (1) | 0 | 0 | 0 | 0 |
| 300mg | 29 | 47 (10) | 20 (7) | 1 (1) | 1 (1) | 0 | 0 |
| 1000mg | 12 | 17 (4) | 8 (4) | 0 | 0 | 0 | 0 |
| 1500mg | 6 | 8 (0) | 3 (0) | 0 | 1 (0) | 0 | 0 |
| 2000mg | 5 | 12 (2) | 4 (1) | 0 | 0 | 0 | 1 (1) |
| AE = adverse events; n= any event, treatment related or not; (N) = considered treatment related by investigator. Note: For protocol B0141006 (placebo and 300 mg), only data for the first active treatment period was included, due to its cross-over nature | | | | | | | |

In the IV studies, treatment-related adverse events (TRAEs, or AEs that might be related to the therapy according to the primary investigator) were reported in 21.2% of subjects receiving IV G1, compared to 17.7% in those receiving placebo. At doses of 100 mg of G1 or higher, TRAEs occurred in 22.4% of participants. At doses of 1,000 mg or higher, TRAEs occurred in 21.7% of participants. Antibody G1 does not appear to be associated with any clinically relevant patterns of change in vital signs (systolic and diastolic blood pressure [BP], temperature and heart rate [HR]), electrocardiogram (ECG) abnormalities (including QTcB and QTcF), infusion site reactions, or clinical laboratory findings. There were limited effects on liver function tests (aspartate aminotransferase [AST], alanine aminotransferase [ALT], total bilirubin, and alkaline phosphatase) with a grade 1 increase in total bilirubin in one subject receiving placebo (Study B0141001), and a grade 1 increase in ALT in one subject receiving placebo (Study B0141002). Clinically significant liver function abnormalities were not seen among subjects receiving any of the studied doses of G1. There was no evidence of differences between G1 and placebo in hematological tests assessing renal function, electrolytes, or in urine tests.

In the IV/SC study (G1-SC-IV), safety and tolerability were comparable between SC and IV routes of delivery. Mean heart rate and blood pressure (diastolic and systolic) were not affected by antibody G1 treatment, nor were there any meaningful changes in any cardiovascular parameter after treatment with SC antibody G1. A summary of TRAEs observed during the SC study is shown below in Table 10.

Table 10

|  | 900 mg (N=6) | 225 mg (N=6) | Placebo (N=6) |
|---|---|---|---|
| GI disorders | 2 (33.3%) | 0 | 1 (16.7%) |
| CNS | 0 | 1 (16.7%) | 0 |
| Infections and Infestations | 0 | 0 | 0 |
| Musculoskeletal and connective tissue | 0 | 0 | 0 |
| Respiratory | 0 | 0 | 0 |
| Reproductive and Breast Disorders | 0 | 0 | 0 |
| Injuries | 0 | 0 | 0 |
| Pregnancy | 0 | 0 | 0 |
| Renal | 1 (16.7%) | 0 | 0 |
| Vascular | 0 | 0 | 0 |

In the single dose studies (B0141001, B0141002, B0141006, and B0141008), pharmacokinetic (PK) parameters were calculated for doses ranging from 30 mg to 2,000 mg. Group mean terminal half-life ($t_{1/2}$) ranged from approximately 40 to 48 days. $C_{max}$ and total exposure (assessed by $AUC_{inf}$) increased with increasing dose. The increase in $AUC_{inf}$ appeared to be approximately dose proportional between 30 and 1,000 mg and appeared to be greater than dose proportional between 1,000 and 2,000 mg. The volume of distribution was low, between 6-10 L.

In the two dose study (B0141007), the apparent terminal half-life after a second dose was between 41 and 50 days. Plasma concentrations accumulated after the second dose, with an accumulation ratio of approximately 1.5. Moreover, in the IV/SC study (G1-SC-IV), pharmacokinetic assessments indicated G1 had a similar terminal half-life when delivered SC as IV.

Example 12: Non-clinical safety

Two studies assessing the safety of antibody G1 were conducted in cynomolgus monkeys. In the first study, safety of a single dose of antibody G1 was evaluated. In the second study, safety of repeated dosing of antibody G1 was evaluated. Each of the studies and their results are further described in detail below. For both the single and repeat-dose studies, antibody G1 was formulated as a 51.4 mg/mL solution in 20 mM histidine, 84 mg/mL trehalose dihydrate, 0.2 mg/mL polysorbate 80, 0.05 mg/mL disodium EDTA dihydrate and 0.1 mg/mL L-methionine, pH ~5.5. Vehicle was formulated identically without antibody G1. Additionally, in both studies, blood samples were taken periodically for analysis of antibody G1 plasma concentration using a validated ELISA method.

Data were first aggregated in summary tables and figures using GraphPad Prism (version 6.0) and Excel 2010 (Microsoft). For the single exposure study, telemetry data were analyzed using ANOVA. Analysis was performed using SAS Release 8.2. In order to normalize the QT interval over a range of R-R intervals, Individual Animal Correction Factors (IACFs) were generated for each animal by relating each RR-interval with its associated QT-interval. The linear regression of this QT/RR-interval relationship was determined for the data set. The slope of this linear regression was used as the IACF for the associated animal across all treatments. This IACF was used to calculate the corrected QT-interval (QTc) using the following equation:

$$QT\text{-}I(c) = QT \text{ interval corrected for heart rate} = QT\text{-}I - [(RR - 300)*(IACF)].$$

For the multiple-dose study, one-way ANOVA was also used to analyze data. If the ANOVA was significant ($P \leq 0.05$), Dunnett's post-test was used for in-between group comparisons. For each gender, the treated group was compared with the control (vehicle) group at the 5% two-tailed probability level.

*Single-dose telemetry study*

Eight adult male cynomolgus monkeys (Charles River Primates) were surgically instrumented with telemeters and allowed to recover for at least two weeks. Implants (DSI TL11M2-D70-PCT) and receivers (RMC-1) were manufactured by Data Sciences International.

Animals were acclimated to telemetry data acquisition cages at least overnight prior to dosing. During acclimation, pre-study recording of hemodynamic parameters was conducted to verify that the transducers and equipment were functioning correctly. During telemetered data acquisition, animals were housed individually in cages equipped with telemetry receivers. On non-collection days, animals were housed in cages without telemetry receivers. Animals were maintained on a 12-hours light, 12-hours dark day cycle, with ad libitum water and fed with certified primate diet.

For the first phase of the study, animals (8 males) were administered vehicle only, and telemetry data were collected beginning ~1 hour pre-dose through 22 hours post-dose. Six days after vehicle administration, the same animals received a single IV administration of antibody G1 (100 mg/kg, an ~10-fold greater dose than the pharmacological EC50 in cynomolgus monkeys). Telemetered electrocardiographic and hemodynamic data were again continuously recorded from all animals. In addition, these animals were monitored for ~24 hours on days 3, 7, 10, and 14 after receiving their single dose of antibody G1. Telemetered ECG and blood pressure signals were transmitted via the implanted radio-telemetry devices to receivers mounted in each cage. The acquired signals were passed through a data exchange matrix (DSI model DEM) and on to a PC-based data acquisition system (DSI software Ponemah P3 version 3.4); the data analysis software was Emka Technologies version 2.4.0.20 (Emka Technologies). The analog/digital sampling rate was 1,000 Hz for telemetered ECG data and 500 Hz for blood pressure data. Data were logged as one minute means.

Group mean systolic blood pressure (SBP) was similar before and after treatment with antibody G1 throughout the first day after dosing and on subsequent days (animals telemetered on days 3, 7, 10 and 14 at identical time intervals as day 1). At hours 1-4 post-dosing when antibody G1 blood concentrations were at maximal levels (mean concentration of 3,500 $\mu$g/mL at 4 hours), mean SBP was 111 mmHg compared with 113 mmHg at the same time interval following vehicle administration. Furthermore, SBP was 110 mmHg on days 3 and 7, 109 mmHg on day 10 and 110 mmHg on day 14 after antibody G1 administration. Similar SBP data were recorded for other time intervals. Since this was a crossover designed study, the treated animals served as their own controls. When the data were analyzed as differences in blood pressure after antibody G1 administration compared with vehicle treatment, there are minor statistically significant reductions in SBP at the latter time interval on days 7, 10, and 14.

Following treatment with antibody G1, diastolic blood pressure (DBP) was noted to be around 3 mmHg lower than the mean values obtained after vehicle administration. From hours 5-22, the group mean for the vehicle and antibody G1 group were similar. The same trend was seen on other days, when a slight decrease in the DBP (ranging from 2.62-3.5 mmHg) occurred in the first interval measured, with a few changes of similar magnitude seen sporadically on days 7-10 in the 7-22 hour interval. Similar to what was seen for the DBP, minor decreases in the heart rate were seen during the first assessment (hours 1-4) relative to vehicle treatment. Differences were undetectable during the intermediate assessments and were once more seen between hours 18-22 on all days.

Moreover, with respect to ECG findings, there were no statistically significant changes in QTc interval at any time point, relative to vehicle treatment. Although statistically significant changes in RR, PR, RS and QT were seen over the 14 day period when compared with vehicle, they were all minor in absolute value.

*Repeat-dose safety study*

The repeat-dose safety study included 48 adult, gender-matched (6 per gender per group) antibody G1-naïve cynomolgus monkeys (Charles River Primates). Animals received vehicle or antibody G1 as an intravenous injection once weekly for 14 weeks at doses of 10 mg/kg, 100 mg/kg, or 300 mg/kg. In each group, two animals of each gender were allowed to recover for an additional 4 months following the end of dosing.

ECG and blood pressure measurements were recorded once during the pre-study phase, twice after steady-state was achieved (prior to dosing and 4 hours post-dose on Day 85) and once ~1 week after the end of dosing (day 103 of the recovery phase). Animals were anesthetized with ketamine and ECGs were recorded using eight leads. Measurement of ECGs (including heart rate) was done with the captured data using the Life Science Suite Ponemah Physiology Platform software system via DSI, using leads I, II, aVF, CG4RL and CV4LL, as standard. A heart rate correction for the QT interval (QTc) was calculated using the Bazett formula.

Blood pressure was recorded prior to the first dose, after 12 weeks of dosing (13 doses) and approximately 1 week after the end of dosing. No significant changes were noted in SBP or DBP in any of the treated groups of animals relative to vehicle-treated animals. Group mean heart rates were relatively consistent across the dose groups and time points measured, with no statistical differences measured. Plasma concentrations of antibody G1 were measured during the first week of dosing and at the time of blood pressure and ECG assessments, demonstrating accumulation with repeated, weekly dosing.

Moreover, with respect to ECG findings, there were no significant differences in QTc interval across all doses and time points. Additionally, no significant or relevant ECG changes were seen for any of the ECG parameters assessed over the course of the study.

In summary, antibody G1 was very well tolerated in both studies, with no clinically significant changes noted in any hemodynamic parameter, nor any relevant changes noted in any ECG parameter. In cynomolgus monkeys, cardiovascular and hemodynamic parameters do not appear to be affected by long-term inhibition of CGRP with antibody G1.

Example 13: Phase 2 study

A Phase 2, Multicenter, Randomized, Double-Blind, Placebo-Controlled, Parallel-Group Study Comparing the Efficacy and Safety of 4 Dose Regimens of Subcutaneous Administration of antibody G1 (TEV-48125) Versus Placebo for the Treatment of Post Traumatic Headache

*Primary Endpoint:*

The primary endpoint is the mean change from baseline (28-day run-in period) in the number of headache hours of any severity during the 28-day period after the last ($3^{rd}$) dose of study drug.

*Secondary Endpoints:*

- Proportion of patients reaching at least 50% reduction in the monthly headache days of any severity during 3 months of treatment with study drug

- Mean change from baseline (28-day run-in period) in the number of headache hours of any severity during the 28-day period after the $1^{st}$ dose of study drug

- Mean change from baseline (28-day run-in period) in the use of any acute headache medications during the 28-day period after the last ($3^{rd}$) dose of study drug

- Mean change from baseline (day 0) in disability score, as measured by the 6-item Headache Impact Test (HIT-6) at 28 days after administration of the last ($3^{rd}$) dose of study drug

*Sample Size*

150 patients (30 per arm).

*Study arms*

- Arm 1. Placebo

- Arm 2. TEV-48125 SC 225 mg monthly

- Arm 3. TEV-48125 SC 675 mg monthly

- Arm 4. TEV-48125 IV 675 mg one dose

- Arm 5. TEV-48125 IV 1000 mg one dose

*Inclusion criteria*

Participants with a history of Chronic Post Traumatic headache as per the ICHD-3.

*Exclusion Criteria:*

- Current enrollment in or discontinuation within the last 30 days from, a clinical trial involving any investigational drug or device.

- Current use or any prior exposure to any calcitonin-gene-related peptide (CGRP) antibody, any antibody to the CGRP receptor, or antibody to nerve growth factor (NGF).

- Failed more than 4 adequate trials of preventive medication

- Known hypersensitivity to multiple drugs, monoclonal antibodies or other therapeutic proteins.

- A history or presence of other medical illness that indicates a medical problem that would preclude study participation.

- Evidence of significant active or unstable psychiatric disease, in the opinion of the investigator.

- Women who are pregnant or nursing

**Table 11: Study Procedures and Assessments**

| Study period | Pretreatment period (incl. screening visit and run-in period) | Double-blind treatment period | | | | Follow-up period |
|---|---|---|---|---|---|---|
| Visit number | V1[a] | V2[b] | V3 | V4 | V5[c] | V6[d] |
| Month number | Month -1 | Month 0 | Month 1 | Month 2 | Month 3 | Month 9.5 |
| Procedures and assessments [e] | Screening days -28 to -1 | Baseline dose 1 day 0 (+3 days) | Dose 2 day 28 (±3 days) | Dose 3 day 56 (±3 days) | EOT or early withdrawal day 84 (±3 days) | Final visit day 281 (±15 days) |
| Informed consent | X | | | | | |
| Medical and psychiatric history | X | | | | | |
| Prior medication history | X | | | | | |
| Inclusion and exclusion criteria | X | X | | | | |
| Physical examination, including weight and height[f] | X | X | | | X | |
| 12-lead ECG[g] | X | X | X | X | X | |
| Vital signs measurement [h] | X | X | X | X | X | |
| Adverse events[i] | X | X | X | X | X | X |

(continued)

| Study period | Pretreatment period (incl. screening visit and run-in period) | Double-blind treatment period | | | | Follow-up period |
|---|---|---|---|---|---|---|
| Visit number | V1[a] | V2[b] | V3 | V4 | V5[c] | V6[d] |
| Month number | Month -1 | Month 0 | Month 1 | Month 2 | Month 3 | Month 9.5 |
| Procedures and assessments [e] | Screening days -28 to -1 | Baseline dose 1 day 0 (+3 days) | Dose 2 day 28 (±3 days) | Dose 3 day 56 (±3 days) | EOT or early withdrawal day 84 (±3 days) | Final visit day 281 (±15 days) |
| Concomitant medication inquiry | X | X | X | X | X | X |
| Clinical laboratory tests[j] | | | | | | |
| Serum/urine β-HCG test[k] | X | X | | | X | |
| FSH[l] | X | | | | | |
| Electronic headache diary[m] | X | X | X | X | X | |
| Blood samples for plasma drug concentration | | X | X | X | X | X |
| Blood samples for serum ADA concentration | | X | X | | X | X |
| Blood sample for pharmacogenomic analysis | | X | | | | |
| Blood collection for further biomarker analysis | | X | | X | X | |
| Urine collection for further biomarker analysis | | X | | X | X | |
| DISABILITY (TBD) | | X | | | X | |
| COGNITION (TBD) | | X | | | X | |
| QOL (TBD) | | X | | | X | |
| PGIC questionnaire | | | X | X | X | |
| Administration of study drug | | X | X | X | | |
| **Injection** site assessments [o] | | X | X | X | | |

Example 14: Phase 2 Study

A Phase 2, multicenter, randomized, double-blind, placebo-controlled, parallel-group study comparing the efficacy and safety of two dose regimens of TEV-48125 (one subcutaneous and one intravenous dose regimens) versus placebo for the treatment of persistent post-traumatic headache (PPTH).

**Study Population**

The study population will be composed of male and female patients, aged 18 to 70 years, inclusive, with a history of Persistent Post-Traumatic Headaches (as defined by International Classification of Headache Disorders, third revision [ICHD-3] criteria (IHS 2013).

This will include patients with persistent headache attributed to mild traumatic injury of the head and patients with persistent headache attributed to whiplash.

Traumatic injury to the head is defined as a structural or functional injury resulting from the action of external forces on the head. These include striking the head with or the head striking an object, penetration of the head by a foreign body, forces generated from blasts or explosions, and other forces yet to be defined.

The duration of post-traumatic amnesia is defined as the time between head injury and recovery of memory of current events and those occurring in the last 24 hours.

PPTH is a headache attributed to mild head injury with a Glasgow Coma Scale score (GCS) of 13 to 15, loss of consciousness less than 30 minutes and duration of post-traumatic amnesia of less than 24 hours defined as the time between head injury and recovery of memory of current events. Also, two or more other symptoms suggestive of mild traumatic brain injury: nausea, vomiting, visual disturbances, dizziness and/or vertigo, impaired memory and/or concentration.

Persistent headache attributed to whiplash is a headache has developed within 7 days after the whiplash and with greater than three months' duration. Whiplash, associated at the time with neck pain and/or headache. Whiplash is defined as sudden and inadequately restrained acceleration/deceleration movements of the head with flexion/extension of the neck. Whiplash may occur after either high or low impact forces.

**Study Endpoints**

**Primary endpoint:** The primary endpoint is the mean change from baseline (28-day run-in period) in the monthly average number of headache days of at least moderate severity during the four weeks period after the administration of study drug.

**Secondary endpoints:** The secondary endpoints are:

- proportion of patients reaching at least 50% reduction in the monthly average headache days of any severity during the 12 week period of treatment with study drug
- mean change from baseline (28-day run-in period) in the number of headache days of any severity during the 5 to 8 week period after the 1st dose of study drug
- mean change from baseline (28-day run-in period) in the number of headache days of any severity during the 12 week period after the 1st dose of study drug
- mean change from baseline (day 0) in disability score, as measured by the 6-item Headache Impact Test (HIT-6) at 12 weeks after administration of first study drug.

**Exploratory Endpoints:** The exploratory endpoints are as follows:

- mean change from baseline (28-day run-in period) in the number of headache days of at least moderate severity during the 12 week period after the first dose of study drug
- proportion of patients reaching at least 75% reduction and total (100%) reduction in the monthly average number of headache days of any severity during the 12-week period after the first dose of study drug
- proportion of patients reaching at least 50% reduction, at least 75% reduction, in the number of headache days of any severity during the four week period after the first dose of study drug relative to the baseline period who sustain this level of response over the 12 week period after the first dose of study drug
- mean change from baseline (28-day run-in period) in the monthly average number of headache days of any severity during the 12-week period after the first dose of study drug
- mean change from baseline (28-day run-in period) in the monthly average number of headache days of at least moderate severity during the 4-week period after the first dose of study drug
- proportion of patients reaching at least 50% reduction, at least 75% reduction and total (100%) reduction, in the monthly average number of headache days of at least moderate severity during the eight week period after the first dose of study drug relative to the baseline period
- mean change from baseline (28-day run-in period) only for patients with whiplash, in the monthly average number of neck pain days of any severity during the 12 week period after the first dose of study drug

- mean change from baseline (28-day run-in period) in the use of any acute headache medications (triptans and ergot compounds) during the 12 week period after the administration of first study drug
- mean change from baseline (28-day run-in-period) in the use of opioids, during the 12 week period after the first dose of study drug
- mean change from baseline (day 0) in disability score, as measured by the 6-item Headache Impact Test (HIT-6) at four weeks after administration of first study drug
- mean change from baseline (day 0) in the health status, as measured by the 12-Item Short-Form Health Survey (SF-12) physical and mental health, at four weeks after first dose of study drug administration
- mean change from baseline (day 0) in the assessment of patient satisfaction, as measured by the Patient Global Impression of Change (PGIC) scale, at 4, 8, and 12 weeks after administration of the first dose of study drug
- mean change from baseline (day 0), as measured by the SCAT-3 "Sports concussion assessment tool-3rd" to end of evaluation period week 12 after the first dose of study drug.

General Design and Methodology:

See Table 12 for study procedures and assessments.

A Multicenter, Randomized, Proof of Concept, Double-Blind, Placebo-Controlled, Parallel Group Study Comparing the Efficacy and Safety of two Dose Regimens of TEV-48125 (1 Subcutaneous and 1 intravenous) Administration Versus Placebo with a diagnosis of persistent post traumatic headache (PTH). The study will consist of a screening visit, a 28-day run-in period, and a double-blind treatment period lasting approximately 12 weeks.

Patients will complete a screening visit (visit 1) after providing written informed consent, and eligible patients will enter a run-in period lasting approximately four weeks (28 days) during which they will enter baseline persistent post-traumatic headache (PPTH) attacks information into an electronic headache diary device daily. Patients will return to the study center after completing the run-in period (visit 2). Patients meeting eligibility requirements at the screening visit and following the 28-day run-in period were randomized to one of three treatment groups:

one treatment group received first dose of TEV-48125 900 mg IV in an infusion during one hour, quarterly;
one treatment group received a first dose of TEV-48125 a 675 mg SC followed by 225 mg SC on the following two months; and
one treatment group received three monthly doses of placebo.

Randomization will be performed using electronic interactive response technology (IRT).

Blinded treatment will be administered IV and SC the first month and SC the second and third month for a total of three doses. First treatment administration will occur at visit 2 and additional doses will be administered at visits 3 and 4. Patients will return to the study center approximately every four weeks for blinded treatment administered SC for safety and efficacy assessments and blood and urine sampling for pharmacokinetic, immunogenicity, biomarkers, and pharmacogenomics (unless prohibited by local regulations) analyses. Final study assessments will be performed at the final visit for this study (visit 5), approximately 12 weeks after administration of study drug.

Method of Blinding and Randomization:

The sponsor, investigators, study staff (except for staff involved in bioanalytical analyses) and patients will be blinded to treatment assignment. A computer-generated master randomization list will be provided to drug packaging facilities. Packaging vendor(s) will package active and placebo into single-visit kits according to Good Manufacturing Practice procedures. Kits will be identical in appearance and contain one vial with active drug or placebo and prefilled syringes (active or placebo). Adequate kit supply for upcoming study visits will be managed by IRT and kept (refrigerated at 2°C to 8°C) on site.

At the end of the screening period, patients will be randomized if they have at least 6 or more headache days of at least moderate severity, during the run in period and at least 85% of diary compliance.

This study is a randomized study with stratification based on gender, the severity of traumatic injury to the head (mild, moderate or severe) according to the IHS Classification. Each patient will undergo randomization in a 1:1:1 ratio within the stratum to which he or she belongs to receive TEV-48125 or placebo, as assigned by the IRT. The IRT will manage initial drug supply, maintenance of adequate study drug supplies on site, and study randomization centrally.

Study Drug Dose, Mode of Administration, and Administration Rate:

Prefilled vials (active or placebo) will be contained in uniquely numbered kits and stored (refrigerated at 2°C to 8°C) on site. Active vials for IV administration (10 mL) will contain TEV-48125 at a concentration of 150 mg/mL, and placebo vials (10 mL) will contain the same vehicle and excipients as those for active infusion and injection. Active syringes will contain 150 mg/mL of TEV-48125 and placebo syringes will contain the same vehicle and excipients as those for active injections. Prefilled syringes (active or placebo) and one vial will be contained in uniquely numbered kits.

Study drug will be administered by qualified study personnel and will retrieve the appropriately numbered kit and extract a volume of the vial contents and add it to 500 mL of normal saline solution, or a SC injections and administered as follows:

- Patients randomized to receive TEV-48125 will receive 900 mg of TEV-48125 as a 1-hour IV infusion and 3(three) 1.5 mL placebo injections SC at visit 2, and single 1.5 mL, placebo injections SC at visits 3 and 4.
- Patients randomized to receive TEV 41825 will receive 675 mg as three active injections (225 mg/1.5 mL) SC and placebo as a 1-hour IV infusion at visit 2, and single 1.5 mL, 225 mg active injections SC at visits 3 and 4.

Patients randomized to receive placebo will receive placebo as a 1-hour IV infusion and three 1.5 mL placebo injections SC at visit 2, and single 1.5 mL placebo injection SC at visits 3 and 4.

Investigational Product: TEV-48125
Placebo: Same vehicle and excipients as those for active

**Duration of Patient Participation:** Patient participation will last for approximately 16 weeks (including a run-in period lasting approximately four week and a 12 week double-blind treatment period). Patients are expected to complete the entire duration of the study.

**Criteria for Inclusion:** Patients may be included in the study if they meet all of the following criteria:

a. Participants with a diagnosis of Persistent Post Traumatic headache as per the ICHD-3 (beta version) criteria
b. Traumatic injury to the head has occurred defined as a structural or functional injury resulting from the action of external forces on the head. These include striking the head with or the head striking an object, penetration of the head by a foreign body, forces generated from blasts or explosions, and other forces yet to be defined.
c. Headache is reported to have developed within seven days after one of the following:

1. mild traumatic injury to the head
2. regaining of consciousness following the injury to the head
3. discontinuation of medication(s) that impair ability to sense or report headache following the injury to the head

d. Headache persists for greater than 3 months after the injury to the head Persistent post-traumatic headache (PPTH) attributed to whiplash has developed within 7 days after the whiplash and with greater than 3 months' duration. There will be no more than 30% of patients with PPTH with whiplash with no evidence of head injury.
e. The patient signs and dates the informed consent document.
f. Males or females aged 18 to 70 years, inclusive
g. The patient has at least six or more headache days of at least moderate severity during the run-in period.
h. The patient is in good health as determined by a medical history, medical examination, ECG, serum chemistry, hematology, urinalysis, and serology.
i. All subjects must be of non-childbearing potential, defined as:

◦ women surgically sterile by documented complete hysterectomy, bilateral oophorectomy, or bitubal ligations or confirmed to be postmenopausal (at least one year since last menses and follicle stimulating hormone [FSH] above 35 U/L);
◦ men surgically sterile by documented vasectomy; or
◦ if of childbearing potential, subjects must meet any of the following criteria:

▪ Subjects must simultaneously use two forms of highly effective contraception methods with their partners during the entire study period and for 7.5 months after the last dose of study drug.
▪ Sexual abstinence is only considered a highly effective method if defined as refraining from heterosexual intercourse in the defined period. The reliability of sexual abstinence needs to be evaluated in relation to the duration of the clinical study and the preferred and usual lifestyle of the subject. Periodic abstinence (e.g., calendar, ovulation, symptothermal, post-ovulation methods), declaration of abstinence for the duration of a study, and withdrawal are not acceptable methods of contraception.

j. Female subjects of childbearing potential must have negative serum beta human chorionic gonadotropin (β-HCG) pregnancy test at screening (confirmed by serum β-HCG pregnancy test at check-in).

k. The patient, if a man, is surgically sterile, or, if capable of producing offspring, has exclusively same-sex partners or is currently using an approved method of birth control and agrees to continued use of this method for the duration of the study. Acceptable methods of contraception include abstinence, female partner's use of steroidal contraceptive (oral, implanted or injected) in conjunction with a barrier method, female partner's use of an intrauterine device, or if female partner is surgically sterile. In addition, male patients may not donate sperm for the duration of the study.

l. The patient must be willing and able to comply with study restrictions and to remain at the clinic for the required duration during the study period, and willing to return to the clinic for the follow-up evaluation as specified in this protocol.

**Criteria for Exclusion:** Patients will be excluded from participating in this study if they meet any of the following criteria:

a. Previous history of brain imaging showing evidence of intracranial hemorrhage, subdural or epidural hematomas and subarachnoid hemorrhages as a consequence of the traumatic head injury.

b. PPTH attributed to craniotomy

c. Patient has another headache disorder not attributable to head trauma

d. Patients with previous history (before the headache trauma) of any type of headache that has >6 headache days per month

e. Patient is using medications containing opioids (including codeine) and barbiturates containing analgesics on average more than 10 days per month.

f. Current enrollment or previous participation, within the last 30 days from, in a clinical trial involving any investigational drug or device, or 5.5 half-lives, whatever is longer

g. Current use or any prior exposure to any calcitonin-gene-related peptide (CGRP) antibody, any antibody to the CGRP receptor, or antibody to nerve growth factor (NGF).

h. Known hypersensitivity to multiple drugs, monoclonal antibodies or other therapeutic proteins.

i. A history or presence of other medical illness that indicates a medical problem that would preclude study participation.

j. Evidence of significant active or unstable psychiatric disease, in the opinion of the investigator.

k. The patient is a pregnant or lactating woman. (Any woman becoming pregnant during the study will be withdrawn from the study.)

l. The patient has previously participated in a Teva sponsored clinical study with study drug.

m. Clinically significant hematological, cardiac, renal, endocrine, pulmonary, gastrointestinal, genitourinary, neurologic, hepatic, or ocular disease, and patient has any clinically significant uncontrolled medical condition (treated or untreated) at the discretion of the investigator.

n. The patient cannot participate or successfully complete the study, in the opinion of their healthcare provider or the investigator, for any of the following reasons:

◦ mentally or legally incapacitated or unable to give consent for any reason

◦ in custody due to an administrative or a legal decision, under tutelage, or being admitted to a sanitarium or social institution

◦ unable to be contacted in case of emergency

◦ has any other condition, which, in the opinion of the investigator, makes the patient inappropriate for inclusion in the study

**Measures and Time Points:**

**Primary Efficacy Measure and Time Point:** The primary efficacy endpoint for this study will be derived from persistent post-traumatic headache days data (i.e., occurrence of headaches days, duration of the headaches, severity of headaches, and acute headache-specific medication use) collected daily using an electronic headache diary device.

Eligible patients will receive training on the use of the electronic headache diary device and will be informed of compliance requirements at screening. Patients will complete electronic headache diary entries with questions about the previous day daily, beginning on the day after the screening visit through the EOT/early withdrawal visit. The electronic headache diary device will allow entry of headache information for up to 24 hours after a given day.

**Secondary Efficacy Measures and Time Points:** Secondary efficacy endpoints will be derived from headache days data (i.e., occurrence, duration of headache, severity of headaches, and acute headache-specific medication use collected daily using an electronic headache diary device. In addition, patient perception of improvement will be evaluated by the Headache Impact Test (HIT-6) after 8 weeks of first dose of study drug administration

**Exploratory Efficacy Measures and Time Points:** The following exploratory efficacy measures will be assessed:

- exploratory efficacy endpoints derived from PTH days data (i.e., occurrence headache days, duration, severity of headache, and acute headache-specific medication use, which are collected daily using an electronic headache diary device
- Headache Impact Test (HIT-6 questionnaire)
- SF-12 physical and mental health
- Patient Global Impression of Change (PGIC) questionnaire
- SCAT-3 "Sports concussion assessment tool-3rd

**Safety Measures and Time Points:**

Safety and tolerability will be assessed using the following measures:

- inquiries about adverse events
- inquiries about concomitant medication usage
- safety laboratory tests (serum chemistry, hematology, coagulation, and urinalysis)
- serum/urine $\beta$-HCG test (women of childbearing potential only)
- 12-lead ECGs
- vital signs measurements (systolic and diastolic blood pressure, pulse, and oral temperature. Oxygen saturation should be measured in cases of suspected anaphylaxis. Respiratory rate will also be measured in these cases (but not as a standard vital sign)
- physical examinations, including body weight
- hypersensitivity reaction assessment
- eC-SSRS

**Pharmacokinetics/Biomarkers/Immunogenicity and Biomarker Substudy Measures and Time Points:** Pharmacokinetic Measures and Time Points: Blood samples for pharmacokinetics analysis of TEV-48125 will be collected from all patients for the purpose of a population pharmacokinetic modeling approach and pharmacokinetic/pharmacodynamic relationship assessment. The pharmacodynamic parameters will be the efficacy responses.

The actual date and time of each blood sample, as well as the date and time of dosing prior to each sample, will be recorded in the case report form. TEV-48125 plasma concentration will be measured using a validated assay.

Immunogenicity Measures and Time Points: Blood samples for immunogenicity will be collected.

Biomarker Measures and Time Points: Biomarker blood and urine samples will be collected from all patients, and a blood sample will be collected from patients at visit 2 or at any visit thereafter (unless prohibited by local regulation).

**Allowed and Disallowed Medications Before and During the Study:**

No more than 2 medications regardless of the indication and no restrictions in abortive agents except opioids and barbiturates (refer to exclusion criteria)

**Statistical Considerations:**

**Sample Size Rationale:** No prospective calculations of statistical power have been made. A sample size of 75 patients (25 patients per treatment arm group) is chosen based on clinical and practical considerations.

**Analysis of Primary Endpoint:** The primary efficacy endpoint, the mean change from baseline (28-day run-in period) in the monthly average number of headache days of at least moderate severity during the four weeks period after the administration of study drug, will be analyzed using an analysis of covariance (ANCOVA) method. The model will include treatment, gender, and the severity (mild, moderate, or severe) of traumatic injury to the head as fixed effects. Ninety five percent confidence intervals will be constructed for the least squares mean differences between each TEV-48125 group and the placebo group.

Analysis of Secondary and Exploratory Endpoints: The same analysis used for the primary efficacy endpoint will be performed for the continuous secondary and exploratory efficacy endpoints. For the proportion of responders defined as 50% or more reduction from baseline in the monthly average headache days, Cochran-Mantel-Haenszel test will be used.

**Multiple Comparisons and Multiplicity:** There will be no multiple comparisons.

**Safety Analyses:**

All adverse events will be coded using the Medical Dictionary for Regulatory Activities. Each patient will be counted only once in each preferred term or system organ class category for the analyses of safety. Summaries will be presented for all adverse events (overall and by severity), adverse events determined by the investigator to be related to study drug (defined as related or with missing relationship) (overall and by severity), serious adverse events, and adverse events causing withdrawal from the study. Patient listings of serious adverse events and adverse events leading to withdrawal will be presented.

Local tolerability findings will be listed and summarized descriptively.

Changes in laboratory and vital signs measurement data will be summarized descriptively. All values will be compared with pre-specified boundaries to identify potentially clinically significant changes or values, and such values will be listed.

The use of concomitant medications will be summarized by therapeutic class using descriptive statistics. Concomitant medications will include all medications taken while the patient is treated with study drug.

Safety data will be summarized descriptively overall and by treatment group. For continuous variables, descriptive statistics (n, mean, SD, median, minimum, and maximum) will be provided for actual values and changes from baseline to each time point. For categorical variables, patient counts and percentages will be provided. Descriptive summaries of serious adverse events, patient withdrawals due to adverse events, and potentially clinically significant abnormal values (clinical laboratory or vital signs) based on predefined criteria will also be provided.

If any patient dies during the study, a listing of deaths will be provided, and all relevant information will be discussed in the patient narrative included in the clinical study report.

**Immunogenicity Analysis:** Summary of immunogenicity results will be provided, and the incidence of immunogenicity will be calculated. The impact of immunogenicity on the pharmacokinetic profile, drug efficacy, and clinical safety will be evaluated. This analysis will be reported separately.

**Biomarker Analysis:** Biomarker analysis will include logistic regression, receiver operating characteristic curves, and summary statistics. Results will be reported separately. Measurements will be made using validated assays.

**Table 12: Study Procedures and Assessments**

| Study period | Pretreatment period (incl. screening visit and run-in period) | Double-blind treatment period | | | | Follow-up period |
|---|---|---|---|---|---|---|
| Visit number | V1[a)] | V2[b)] | V3 | V4 | V5[c)] | V6[d)] |
| Month number | Month -1 | Month 0 | Month 1 | Month 2 | Month 3 | Month 9.5 |
| Procedures and assessments [e)] | Screening days -28 to -1 | Baseline dose 1 day 0 (+3 days) | Dose 2 day 28 (±3 days) | Dose 3 day 56 (±3 days) | EOT or early withdrawal day 84 (±3 days) | Final visit day 281 (±15 days) |
| Informed consent | X | | | | | |
| Medical and psychiatric history | X | | | | | |
| Prior medication history | X | | | | | |
| Inclusion and exclusion criteria | X | X | | | | |

(continued)

| Study period | Pretreatment period (incl. screening visit and run-in period) | Double-blind treatment period | | | | Follow-up period |
|---|---|---|---|---|---|---|
| Visit number | V1[a)] | V2[b)] | V3 | V4 | V5[c)] | V6[d)] |
| Month number | Month -1 | Month 0 | Month 1 | Month 2 | Month 3 | Month 9.5 |
| Procedures and assessments [e)] | Screening days -28 to -1 | Baseline dose 1 day 0 (+3 days) | Dose 2 day 28 ($\pm$3 days) | Dose 3 day 56 ($\pm$3 days) | EOT or early withdrawal day 84 ($\pm$3 days) | Final visit day 281 ($\pm$15 days) |
| Physical examination, including weight and height[f)] | X | X | | | X | |
| 12-lead ECG[g)] | X | X | X | X | X | |
| Vital signs measurement [h)] | X | X | X | X | X | |
| Adverse events[i)] | X | X | X | X | X | X |
| Concomitant medication inquiry | X | X | X | X | X | X |
| Clinical laboratory tests[j)] | X | X | X | X | X | |
| Serum/urine $\beta$-HCG test[k)] | X | X | | | X | |
| FSH[l)] | X | | | | | |
| Electronic headache diary[m)] | X | X | X | X | X | |
| Blood samples for plasma drug concentration | | X | X | X | X | X |
| Blood samples for serum ADA concentration | | X | X | | X | X |
| Blood sample for pharmacogenomic analysis[n)] | | X | | | | |

(continued)

| Study period | Pretreatment period (incl. screening visit and run-in period) | Double-blind treatment period | | | | Follow-up period |
|---|---|---|---|---|---|---|
| Visit number | V1[a] | V2[b] | V3 | V4 | V5[c] | V6[d] |
| Month number | Month -1 | Month 0 | Month 1 | Month 2 | Month 3 | Month 9.5 |
| Procedures and assessments[e] | Screening days -28 to -1 | Baseline dose 1 day 0 (+3 days) | Dose 2 day 28 (±3 days) | Dose 3 day 56 (±3 days) | EOT or early withdrawal day 84 (±3 days) | Final visit day 281 (±15 days) |
| Blood collection for further biomarker analysis | | X | | X | X | |
| Urine collection for further biomarker analysis | | X | | X | X | |
| HIT 6 questionnaire | | X | | | X | |
| | | X | | | X | |
| SF 12 questionnaire | | X | | | X | |
| PGIC questionnaire | | | X | X | X | |
| Administration of study drug | | X | X | X | | |
| Injection site assessments [o] | | X | X | X | | |

Example 15: Development of Headache-Related Behaviors in a Clinically Relevant Rat Model of Posttraumatic Headache are mediated by CGRP

**Materials and Methods**

Animals

Male Sprague-Dawley rats (Taconic, USA) weighing 250-300g at time of arrival were used in all studies. Animals were housed in pairs under a constant 12 hour light/dark (lights on at 07:00h) cycle at room temperature. Food and water were available ad libitum. In all experiments animals were randomly assigned to either sham or mild closed head injury (mCHI) groups as well as for the different treatments and group were tested in a blinded fashion. All experiments were approved and conducted in compliance with the institutional Animal Care and Use Committee of the Beth Israel Deaconess Medical Centre and Harvard Medical School and were in compliance with the ARRIVE (Animal Research: Reporting of In Vivo Experiments) guidelines.

Experimental Mild Closed Head Injury

Experimental mCHI was induced using the weight-drop concussive device as described previously (Marmarou et al., J of Neurosurgery 1994;80(2):291-300; Mychasiuk et al., J Neurotrauma 2014;31 (8):749-757). Briefly, rats were anesthetized with 3% isoflurane and placed chest down directly under a weight-drop concussive head trauma device. The device consisted of a hollow cylindrical tube (inner diameter 2.54 cm) placed vertically over the rat's head. To induce a head trauma, a 250 g weight was dropped through the tube from a height of 80 cm, striking the center of the head. A foam sponge (thickness 3.81 cm, density 1.1 $g/cm^3$) was placed under the animals to support the head while allowing some anterior-posterior motion without any rotational movement at the moment of impact. Immediately after the impact animals were returned to their home cages for recovery. All animals regained consciousness within 2 minutes of injury and were neurologically assessed in the early hours and days post-injury for any behavioral abnormalities suggestive of neurological impairment. Sham animals were anesthetized but not subjected to the weight drop. All animals subjected to PTH behavioral assessments did not display any major neurological deficits.

Behavioral Testing

Open Field Monitoring System

Activity Monitor SOF-811 (Med Associates, Vermont, USA) was used to measure locomotor activity in an open field environment. The system consists of a two areas setup and evaluates the movement of animals in the horizontal (X-Y axis) and vertical (Z-axis) planes. Each plane was monitored by 16 beams spaced 2.54 cm apart, and there was an infrared emitter and detector per beam located on each side of each arena to monitor movement across the width of the arena. Data were sent from the 3 sets of detector-emitters to a central computer displaying a range of pre-selected outputs including total distance moved and vertical activity (rearing) during 60 second intervals and were analysed as a total over 20 minutes. Each arena was lit with a single white LED bulb on a dimmer switch to maintain a homogenous lighting across the arenas (80 lux). The arenas were cleaned with mild detergent and dried to remove odour cues between successive rats. Testing was conducted at baseline (24 hours prior to head trauma) and then at 48 hours, 72 hours, 7 and 14 days post mCHI.

Novel Object Recognition

The novel object recognition test is designed to evaluate deficits in recognition memory in rodents, as a measure of the severity of the brain injury. The procedure used for the novel-object recognition test was similar to that described previously (King et al., Neuropharmacology 2004;47(2):195-204; Moriarty et al., Behavioural brain research 2016;303:61-70), with some modifications. The apparatus was constructed of Perspex arena (45 cm$^2$). The "familiar" objects used were two identical shapes composed of "Lego DUPLO" building blocks. A third object, the "novel object" consisted of a plastic bottle covered with green tape. Animals were habituated to the arena in the absence of objects for 30 minutes on the day before the test day. The test day comprised three stages: habituation (5 minute exposure to arena in absence of objects), exposure 1 (5 minute familiarisation to identical objects) and exposure 2 (5 minute exposure to one familiar and one novel object). Habituation and exposure 1 were separated by a 5 minute inter-trial interval and exposures 1 and 2 by a further 5 minute interval. Exploration of an object was defined as any behaviour directed toward it (i.e., sniffing, rearing, leaning or climbing). The three test stages were recorded for subsequent analysis and object exploration was manually rated in a blind manner. The discrimination ratio for each object was calculated as time spent exploring either object/time spent exploring both objects. A Preference Index above 0.5 (i.e., 50%) indicates novel object preference, below 0.5-familiar object preference and 0.5 itself- no preference.

Von Frey Testing

The method used was previously used to study headache-related behaviours (Edelmayer et al., Methods Mol Biol 2012;851:109-120; Oshinsky, Headache 2007;47(7):1026-1036; Yan et al., Mol Pain 2012;8:6, Zhao et al., Pain 2014;155(7):1392-1400). Briefly, animals were placed in a transparent flat-bottomed acrylic holding apparatus (20.4cm x 8.5cm). The apparatus was large enough to enable to the animals to escape the stimulus. Animals were habituated to the arena for 15 minutes prior to testing. In order to determine if animals developed pericranial mechanical hypersensitivity following mCHI the skin region, including the midline area above the eyes and 2 cm posterior, was stimulated with different von Frey filaments (0.6g-10g) (18011 Semmes-Weinstein Anesthesiometer Kit). Development of hindpaw hypersensitivity was tested by stimulating the mid-dorsal part of the hindpaw. Changes in tactile skin sensitivity were evaluated similar to previous studies (Levy et al., Brain, Behavior, and Immunity 2012;26(2):311-317) by recording four behavioural responses adapted from Vos et al. (Journal of Neuroscience: the official journal of the Society for Neuroscience 1994;14(5 Pt 1):2708-2723) as follows: 0) No response: rat did not display any response to stimulation 1) Detection: rat turned its head towards stimulating object and latter is explored usually by sniffing; 2) Withdrawal: rat turned its head away or pulled it briskly away from stimulating object (usually followed by scratching or grooming of stimulated region); 3) Escape/Attack: rat turned its body briskly in the holding apparatus in order to escape stimulation or attacked (biting and grabbing movements) the stimulating object. Starting with the lowest weight, each filament was applied three times with an intra-application interval of 5 seconds and the behaviour that was observed at least twice was recorded. For statistical analysis the score recorded was based on the most aversive behaviour noted. The force that elicited three consecutive withdrawal responses was considered the response threshold. To evaluate pain behaviour in addition to changes in threshold, for each rat, at each time point, a cumulative response score was determined by combining the individual scores (0-3) for each one of the VF filaments tested. All tests were conducted and evaluated in a blinded manner. Responses to von Frey stimuli were tested at baseline and also 48 hours, 72 hours as well at 7 and 14 days post mCHI.

Conditioned Place Preference using sumatriptan

The method employed was adapted from previously published reports (Griggs et al., Neuroreport 2015;26(9):522-527). Two CPP boxes (Med Associates, Vermont, USA), consisting of two separate chambers, were used to assess chamber preference before and after the drug-conditioning phase. Sumatriptan was chosen for the initial characterization experiment as it has previously demonstrated efficacy with CPP in a pre-clinical migraine model (De Felice et al., Annals of neurology 2013;74(2):257-265). On the pre-conditioning day (Day 6 post mCHI) sham or mCHI rats were placed into CPP boxes with access to both chambers for a period of 20 minutes and time spent in each chamber was recorded. Chambers were discriminated between based on visual (walls) and tactile (floors) cues. On the morning of the conditioning day (Day 7 post mCHI), animals received a vehicle control (saline i.p.), and 2 hours later were confined to the vehicle-paired chamber for 20 minutes. In the afternoon, 4 hours after vehicle injection, animals received sumatriptan, and 2 hours later were confined to the opposite chamber (sumatriptan-paired chamber) for 20 minutes. A 2 hour period was chosen between administration of sumatriptan and chamber confinement as data suggests that optimal efficacy of sumatriptan is observed 2 hours post administration (Tfelt-Hansen et al., Headache 1998;38(10):748-755). 24 hours later (Day 8 following mCHI) animals were placed into the CPP apparatus with free access to both chambers for 15 minutes and time spent in each chamber was recorded.

GTN-evoked mechanical hypersensitivity

Low dose GTN (100 $\mu$g/kg) was administered on Day 15 and 30 post mCHI to investigate if any increased susceptibility to common headache triggers existed in mCHI animals following resolution of the cephalic pain hypersensitivity 2 weeks post mCHI. Development of pericranial and hindpaw mechanical hypersensitivity was assessed as above at 1 hour and 4 hours post-GTN administration. Acute administration of sumatriptan or chronic blockade of CGRP action using a murine mAb were investigated for their ability to attenuate the GTN-induced pain hypersensitivity.

GTN induced conditioned place aversion

Conditioned place aversion to GTN was tested using the two CPP boxes as indicated above. The protocol included a pre-condition day (Day 13 post mCHI), followed by a conditioning day (Day 14) and a post conditioning day 24 hours later. On the morning of the conditioning day, animals were first confined for 20 minutes to one chamber, prior to GTN administration (pre-GTN-paired chamber). Animals were then administered with GTN and 4 hours later were confined to the opposite chamber (GTN-paired chamber) for 20 minutes. On the post-conditioning day animals had again free access to both chambers. Aversion to GTN was determined by calculating difference scores between the times spent in the different chambers during the pre-conditioning and post-conditioning days in animals treated with the murine anti-CGRP mAb or the control IgG.

Drugs

Sumatriptan (Sigma, USA) was freshly dissolved in 0.9% saline and administered intra-peritoneal (i.p.) at a dose of 1 mg/kg in a volume of 1 ml/kg. All behavioural testing was conducted 2 hours after sumatriptan administration. Drug dose and times of administration were based on the pharmacokinetics of the drugs as well as in-house pilot work and published studies demonstrating their efficacy in animal models of trigeminal pain (Oshinsky et al., Headache 2012;52(9):1336-1349; Winner et al., Mayo Clin Proc 2003;78(10):1214-1222). A murine-specific mAb targeting CGRP and its control IgG were provided by Teva Pharmaceuticals and were administered i.p. at a dose of 30 mg/kg in a volume of 0.54 ml/100g (Kopruszinski et al., Cephalalgia: an International Journal of Headache 2016). The first administration was delivered immediately after mCHI induction and every 6 days subsequently. GTN (American Reagent, USA) was freshly dissolved in 0.9% saline and administered intra-peritoneal (i.p.) at a dose of 100 $\mu$g/kg in a volume of 1 ml/kg. The final vehicle concentration for the GTN was 0.6% propylene glycol, 0.6% ethanol and 0.9% saline. Previous work has shown no effect on mechanical thresholds with 6% propylene glycol and ethanol vehicle concentration (Pradhan et al., Pain 2014;155(2):269-274).

Data Analysis

Statistical analyses were conducted using Statview (SAS Institute, New York, NY, USA). Normality and homogeneity of variance were assessed using Shapiro-Wilk and Levene tests, respectively. Two-way repeated measures analysis of variance (ANOVA) was performed to determine main effects of time, head trauma, and drug treatment, or their interaction, on locomotor activity in the open field or the development of pericranial hypersensitivity using the von Frey apparatus. Fisher's LSD post hoc test or Student's unpaired 2-tailed t-tests were used, as appropriate, to assess differences between the groups and across time points. Data are presented as mean $\pm$ standard error of the mean. $p < 0.05$ was considered statistically significant.

**Results**

Reduced vertical rearing activity but no evidence of cognitive deficits in mCHI/concussed animals

Following mCHI, animals displayed reduced vertical exploratory (rearing) activity in the open field (RM ANOVA time: $F_{(4, 56)} = 7.03$, $p < 0.01$, injury $F_{(1, 14)} = 21.31$, $p < 0.01$). Student's unpaired two-tailed t-tests revealed that the mCHI group displayed consistently decreased vertical exploratory activity compared to the sham group at 48 hours, 72 hours, and Day 7 post mCHI ($p < 0.01$) (Figure 12A), indicating a mild traumatic brain injury. At these time points, however, no changes were detected in total distance travelled, centre zone exploration or thigmotaxis (all $p > 0.05$). There was also no evidence of major cognitive deficits in mCHI animals at 7 days post-injury as measured by the novel object recognition test ($p > 0.05$) (Figure 12B).

Development of tactile hypersensitivity in mCHI animals

Using von Frey filaments, a time dependent development of cephalic tactile hypersensitivity was identified in mCHI, but not in sham animals. Repeated measures ANOVA revealed a significant effect of time ($F_{(4, 48)} = 7.54$, $p < 0.001$) and a time x injury interaction ($F_{(4, 48)} = 5.71$, $p < 0.001$). Student's unpaired two-tailed t-tests revealed that response thresholds were significantly reduced and nociceptive scores in the mCHI group were significantly increased at 72 hours and Day 7 (both $p < 0.05$) compared to the sham group (Figures 13A and 13C). At any of the time point tested, mCHI did not lead to development of hindpaw mechanical hypersensitivity (Figures 13B and 13D).

Effects of acute sumatriptan treatment and chronic CGRP blockade on mCHI-induced cephalic tactile hypersensitivity

Overall, acute sumatriptan treatment at 72h post-CHI attenuated cephalic tactile hypersensitivity. (One-way ANOVA $F_{(2, 17)} = 8.79$, $p < 0.05$) (Figures 14A and 14B). The ability of chronic blockade of CGRP using injections of a blocking mAb, or control IgG, starting immediately after mCHI and every 6 days subsequently was tested on the development of mCHI-related cephalic tactile hypersensitivity. Overall, CGRP blockade attenuated mCHI-induced mechanical hypersensitivity (RM ANOVA time: $F_{(3, 39)} = 17.76$, $p < 0.001$). Student's unpaired two-tailed t-tests revealed that anti-CGRP mAb treated group had a significantly increased response threshold and reduced nociceptive score compared to the control IgG-treated group at Day 7 post mCHI (Figures 14C and 14D, $p < 0.05$). Chronic treatment with the anti-CGRP mAb did not have any effect on mechanical response threshold in sham animals in agreement with the current finding of Kopruszinski et al., who also employed an anti-CGRP mAb (Kopruszinski et al., Cephalalgia: an International Journal of Headache 2016).

Sumatriptan related conditioned Place Preference in mCHI animals

To investigate the aversive nature of PTH pain in mCHI animals, the CPP paradigm was used to determine whether the anti-migraine drug sumatriptan could produce CPP in mCHI rats but not in sham controls at 7 days post-head injury. Difference scores (postconditioning - preconditioning time difference for each chamber) for individual rats indicate that mCHI rats displayed significantly increased time ($p < 0.01$) in the sumatriptan chamber compared to sham controls (Figure 15B), suggesting that systemic sumatriptan treatment alleviated the aversive, headache-like effect of mCHI.

Development of mechanical hypersensitivity following administration of GTN to asymptomatic mCHI animals

By Day 14 post-mCHI, no significant differences in cephalic tactile sensitivity existed between sham and mCHI groups. To determine if rats subjected to mCHI developed enhanced tactile pain sensitivity to a migraine-triggering event, following the resolution of the acute behavioural symptoms, we tested changes in cephalic and extra-cephalic responses to mechanical stimulation following the administration of the common migraine trigger GTN on Days 15 and 30 post-injury. On Day 15, administration of GTN resulted in renewed and pronounced cephalic mechanical hypersensitivity in mCHI animals (RM ANOVA time: $F_{(1, 14)} = 38.68$, $p < 0.001$, time x GTN treatment $F_{(2, 14)} = 5.18$, $p < 0.05$), but not in sham animals. Fisher's LSD post-hoc tests revealed that the mCHI group displayed a significantly reduced cephalic response threshold and increased nociceptive score at 1h (p<0.01) and 4h (p<0.001) post GTN and compared to sham controls (Figures 16A and 16B). On Day 30 post mCHI, GTN also produced pronounced cephalic hypersensitivity (RM ANOVA treatment: $F_{(1, 13)}$=9.7, p<0.05, time x GTN treatment: $F_{(2, 26)}$=3.24, p<0.05). Fisher's LSD post-hoc tests revealed that the mCHI group displayed a significantly reduced cephalic response threshold and at 1 h and 4 h post-GTN (p<0.05,) compared to sham controls and an increased nociceptive score at 4h post-GTN (Figures 16C and 16D, p<0.05).

GTN administration also induced hindpaw mechanical hypersensitivity that was however limited to reduced mechanical thresholds, selectively in mCHI animals on Day 15 (RM ANOVA time: $F_{(2, 26)}$=8.27, p<0.01, and GTN treatment: $F_{(1, 14)}$=8.12, p<0.05) and Day 30 (RM ANOVA time: $F_{(2, 26)}$=37.04, p<0.05, and GTN treatment: $F_{(2, 26)}$=3.24, p<0.05) post-injury. Fisher's LSD post-hoc tests revealed that the mCHI group displayed a significantly reduced mechanical thresholds at 4 h post GTN (p<0.05) on Day 15 and at 1 h post GTN on Day 30, compared to sham controls (Figures 17A and 17C, p<0.05).

Effects of acute sumatriptan treatment and chronic CGRP blockade on GTN-evoked mechanical hypersensitivity in mCHI animals

On day 15 post mCHI, acute sumatriptan treatment attenuated the GTN-evoked delayed mechanical cephalic hypersensitivity (at 4 h) when administered 1 h after GTN. Student's unpaired two-tailed t-tests revealed that acute sumatriptan treatment significantly reduced both the decrease in response threshold, and the increase in nociceptive score compared to vehicle treated animals (p<0.001 and p<0.05, Figures 18A and 18B). Sumatriptan treatment did not attenuate the GTN-evoked hindpaw hypersensitivity in mCHI animals. Chronic treatment with the anti-CGRP mAb prevented GTN-induced mechanical hypersensitivity on Day 15 post-mCHI. Student's unpaired two-tailed t-tests revealed that the anti-CGRP mAb group displayed a significantly increased response threshold and a reduced nociceptive score compared to the control IgG group (p<0.05, p<0.01 respectively, Figures 18C and 18D). When compared to treatment with the control IgG, anti-CGRP mAb treatment was also effective at attenuating the GTN-induced reduced mechanical thresholds at 30 days post-injury (p<0.05, Student's unpaired two-tailed t-tests). Anti-CGRP mAb treatment, however, was ineffective in blocking the GTN-evoked hindpaw hypersensitivity in mCHI animals.

Conditioned Place Aversion to GTN

Using the conditioned place aversion paradigm, GTN administration following mCHI was tested to see if it could induce ongoing pain-like behaviour that is responsive to anti-CGRP mAb treatments. Administration of GTN to mCHI animals on Day 14 post mCHI resulted in a conditioned place aversion, that is reduced time in the GTN-paired chamber, in animals treated with the control IgG, suggesting GTN-evoked pain in mCHI animals at a times when the cephalic pain hypersensitivity was already resolved. There was no evidence of a similar GTN-evoked conditioned place aversion in mCHI animals treated with the anti-CGRP mAb (Figures 19A and 19B), suggesting that GTN-evoked pain in mCHI animals is CGRP-dependent.

Effect of chronic treatment with anti-CGRP mAb on PTH-related behaviours

The main findings of the study are: 1) Concussion via mCHI was associated with an acute phase of reduced rearing (i.e., exploratory activity), but not with major motor or cognitive deficits, suggesting a mild transient form of traumatic brain injury (Kilbourne et al., J Neurotrauma 2009;26(12):2233-2243), 2) Concussed animals developed cephalic tactile pain hypersensitivity that was resolved by 2 weeks post-injury, and which was attenuated by acute treatment with sumatriptan and prolonged inhibition of CGRP via a blocking mAb, 3) Animals subjected to mCHI displayed spontaneous/ongoing pain-like behaviour in the CPP paradigm when using systemic sumatriptan treatment as an analgesic treatment, and 4) Following resolution of the headache/pain behaviours, administration of the headache trigger GTN resulted in a renewed and pronounced cephalic hypersensitivity as well as conditioned place aversion that was inhibited by sumatriptan and anti-CGRP mAb treatments.

Currently, there are no evidence-based treatment guidelines for PTH management, thus it is often treated similar to the primary headache disorder it resembles, with studies showing the efficacy of sumatriptan in some PTH patients with migrainous features (Abend et al., J Child Neurol 2008;23(4):438-440; Lucas, Curr Pain Headache Rep 2015;19(10):48). However, because a significant proportion of patients do not respond to triptan treatment (Visser et al., Headache 1996;36(8):471-475), other therapy options are needed. Based on the hypothesized role of CGRP in migraine and headache (Pietrobon et al., Annu Rev Physiol 2013;75:365-391; Russo, Annual review of pharmacology and toxicology 2015;55:533-552), a promising emerging therapy strategy targets CGRP or its receptor using blocking mAbs (Mitsikostas et al., BMC Med 2015;13:279). Phase IIb trials of one such mAb, TEV-48125, have demonstrated favourable results in terms of safety, efficacy and tolerability of this agent (Bigal et al., Lancet Neurol 2015;14(11):1081-1090; Bigal et al., Lancet Neurol 2015;14(11):1091-1100). The results from the current study indicate that inhibiting CGRP via a rodent-specific neutralising mAb can reduce the headache/pain symptoms evoked by mCHI suggesting the involvement of CGRP in mediating the development of PTH pain. Treatment with the anti-CGRP mAb was also effective in attenuating the GTN-related cephalic mechanical hypersensitivity in mCHI animals and attenuated conditioned place aversion to GTN, pointing to CGRP also as a mediator of the hyperalgesic priming-like effect evoked by the head trauma, in particular the headache-like allodynia and ongoing pain induced by GTN. As the mAb is unlikely to cross the blood-brain-barrier (due to its large molecular weight), its mechanism of action in mCHI animals likely involves a peripheral site, potentially the interruption of mCHI-evoked meningeal or periosteal inflammatory response. Because the anti-CGRP mAb treatment was ineffective in ameliorating the development of GTN-evoked hindpaw mechanical sensitization, we proposed that this response in mCHI animals is centrally mediated.

**Deposit of Biological Material**

The following materials have been deposited with the American Type Culture Collection, 10801 University Boulevard, Manassas, Virginia 20110-2209, USA (ATCC):

| Material | Antibody No. | ATCC Accession No. | Date of Deposit |
|---|---|---|---|
| pDb.CGRP.hFcGI | G1 heavy chain | PTA-6867 | July 15, 2005 |
| pEb.CGRP.hKGI | G1 light chain | PTA-6866 | July 15, 2005 |

Vector pEb.CGRP.hKGI is a polynucleotide encoding the G1 light chain variable region and the light chain kappa constant region; and vector pDb.CGRP.hFcGI is a polynucleotide encoding the G1 heavy chain variable region and the heavy chain IgG2 constant region containing the following mutations: A330P331 to S330S331 (amino acid numbering with reference to the wildtype IgG2 sequence; see Eur. J. Immunol. (1999) 29:2613-2624).

These deposits were made under the provisions of the Budapest Treaty on the International Recognition of the Deposit of Microorganisms for the Purpose of Patent Procedure and the Regulations thereunder (Budapest Treaty). This assures maintenance of a viable culture of the deposit for 30 years from the date of deposit. The deposit will be made available by ATCC under the terms of the Budapest Treaty, and subject to an agreement between Rinat Neuroscience Corp. and ATCC, which assures permanent and unrestricted availability of the progeny of the culture of the deposit to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 USC Section 122 and the Commissioner's rules pursuant thereto (including 37 CFR Section 1.14 with particular reference to 886 OG 638).

The assignee of the present application has agreed that if a culture of the materials on deposit should die or be lost or destroyed when cultivated under suitable conditions, the materials will be promptly replaced on notification with another of the same. Availability of the deposited material is not to be construed as a license to practice the invention in contravention of the rights granted under the authority of any government in accordance with its patent laws.

**Antibody Sequences**

G1 heavy chain variable region amino acid sequence (SEQ ID NO:1)

EVQLVESGGGLVQPGGSLRLSCAASGFTFSNYWISWVRQAPGKGLEWVAEIRSESDA
SATHYAEAVKGRFTISRDNAKNSLYLQMNSLRAEDTAVYYCLAYFDYGLAIQNYWGQG
TLVTVSS

G1 light chain variable region amino acid sequence (SEQ ID NO:2)

EIVLTQSPATLSLSPGERATLSCKASKRVTTYVSWYQQKPGQAPRLLIYGASNRYLGIP
ARFSGSGSGTDFTLTISSLEPEDFAVYYCSQSYNYPYTFGQGTKLEIK

G1 CDR H1 (extended CDR) (SEQ ID NO:3)
GFTFSNYWIS

G1 CDR H2 (extended CDR) (SEQ ID NO:4)
EIRSESDASATHYAEAVKG

G1 CDR H3 (SEQ ID NO:5)
YFDYGLAIQNY

G1 CDR L1 (SEQ ID NO:6)
KASKRVTTYVS

G1 CDR L2 (SEQ ID NO:7)
GASNRYL

G1 CDR L3 (SEQ ID NO:8)
SQSYNYPYT

G1 heavy chain variable region nucleotide sequence (SEQ ID NO:9)

GAAGTTCAGCTGGTTGAATCCGGTGGTGGTCTGGTTCAGCCAGGTGGTTCCCTGC

GTCTGTCCTGCGCTGCTTCCGGTTTCACCTTCTCCAACTACTGGATCTCCTGGGTT

CGTCAGGCTCCTGGTAAAGGTCTGGAATGGGTTGCTGAAATCCGTTCCGAATCCG

ACGCGTCCGCTACCCATTACGCTGAAGCTGTTAAAGGTCGTTTCACCATCTCCCGT

GACAACGCTAAGAACTCCCTGTACCTGCAGATGAACTCCCTGCGTGCTGAAGACAC

CGCTGTTTACTACTGCCTGGCTTACTTTGACTACGGTCTGGCTATCCAGAACTACT

GGGGTCAGGGTACCCTGGTTACCGTTTCCTCC

G1 light chain variable region nucleotide sequence (SEQ ID NO:10)

GAAATCGTTCTGACCCAGTCCCCGGCTACCCTGTCCCTGTCCCCAGGTGAACGTGCT

ACCCTGTCCTGCAAAGCTTCCAAACGGGTTACCACCTACGTTTCCTGGTACCAGCAGA

AACCCGGTCAGGCTCCTCGTCTGCTGATCTACGGTGCTTCCAACCGTTACCTCGGTAT

CCCAGCTCGTTTCTCCGGTTCCGGTTCCGGTACCGACTTCACCCTGACCATCTCCTCC

CTGGAACCCGAAGACTTCGCTGTTTACTACTGCAGTCAGTCCTACAACTACCCCTACA

CCTTCGGTCAGGGTACCAAACTGGAAATCAAA

G1 heavy chain full antibody amino acid sequence (including modified IgG2 as described herein) (SEQ ID NO:11)

EVQLVESGGGLVQPGGSLRLSCAASGFTFSNYWISWVRQAPGKGLEWVAEIRSESDA
SATHYAEAVKGRFTISRDNAKNSLYLQMNSLRAEDTAVYYCLAYFDYGLAIQNYWGQG
TLVTVSSASTKGPSVFPLAPCSRSTSESTAALGCLVKDYFPEPVTVSWNSGALTSGVH
TFPAVLQSSGLYSLSSVVTVPSSNFGTQTYTCNVDHKPSNTKVDKTVERKCCVECPPC
PAPPVAGPSVFLFPPKPKDTLMISRTPEVTCVVVDVSHEDPEVQFNWYVDGVEVHNAK
TKPREEQFNSTFRVVSVLTVVHQDWLNGKEYKCKVSNKGLPSSIEKTISKTKGQPREP
QVYTLPPSREEMTKNQVSLTCLVKGFYPSDIAVEWESNGQPENNYKTTPPMLDSDGS
FFLYSKLTVDKSRWQQGNVFSCSVMHEALHNHYTQKSLSLSPGK

G1 light chain full antibody amino acid sequence (SEQ ID NO:12)


EIVLTQSPATLSLSPGERATLSCKASKRVTTYVSWYQQKPGQAPRLLIYGASNRYLGIP
ARFSGSGSGTDFTLTISSLEPEDFAVYYCSQSYNYPYTFGQGTKLEIKRTVAAPSVFIF
PPSDEQLKSGTASVVCLLNNFYPREAKVQWKVDNALQSGNSQESVTEQDSKDSTYS
LSSTLTLSKADYEKHKVYACEVTHQGLSSPVTKSFNRGEC

G1 heavy chain full antibody nucleotide sequence (including modified IgG2 as described herein) (SEQ ID NO:13)


GAAGTTCAGCTGGTTGAATCCGGTGGTGGTCTGGTTCAGCCAGGTGGTTCCCTGC
GTCTGTCCTGCGCTGCTTCCGGTTTCACCTTCTCCAACTACTGGATCTCCTGGGTT
CGTCAGGCTCCTGGTAAAGGTCTGGAATGGGTTGCTGAAATCCGTTCCGAATCCG
ACGCGTCCGCTACCCATTACGCTGAAGCTGTTAAAGGTCGTTTCACCATCTCCCGT
GACAACGCTAAGAACTCCCTGTACCTGCAGATGAACTCCCTGCGTGCTGAAGACA
CCGCTGTTTACTACTGCCTGGCTTACTTTGACTACGGTCTGGCTATCCAGAACTAC
TGGGGTCAGGGTACCCTGGTTACCGTTTCCTCCGCCTCCACCAAGGGCCCATCTG
TCTTCCCACTGGCCCCATGCTCCCGCAGCACCTCCGAGAGCACAGCCGCCCTGG

GCTGCCTGGTCAAGGACTACTTCCCAGAACCTGTGACCGTGTCCTGGAACTCTGG
CGCTCTGACCAGCGGCGTGCACACCTTCCCAGCTGTCCTGCAGTCCTCAGGTCTC
TACTCCCTCAGCAGCGTGGTGACCGTGCCATCCAGCAACTTCGGCACCCAGACCT
ACACCTGCAACGTAGATCACAAGCCAAGCAACACCAAGGTCGACAAGACCGTGGA
GAGAAAGTGTTGTGTGGAGTGTCCACCTTGTCCAGCCCCTCCAGTGGCCGGACCA
TCCGTGTTCCTGTTCCCTCCAAAGCCAAAGGACACCCTGATGATCTCCAGAACCCC
AGAGGTGACCTGTGTGGTGGTGGACGTGTCCCACGAGGACCCAGAGGTGCAGTT
CAACTGGTATGTGGACGGAGTGGAGGTGCACAACGCCAAGACCAAGCCAAGAGA
GGAGCAGTTCAACTCCACCTTCAGAGTGGTGAGCGTGCTGACCGTGGTGCACCAG
GACTGGCTGAACGGAAAGGAGTATAAGTGTAAGGTGTCCAACAAGGGACTGCCAT
CCAGCATCGAGAAGACCATCTCCAAGACCAAGGGACAGCCAAGAGAGCCACAGGT
GTATACCCTGCCCCCATCCAGAGAGGAGATGACCAAGAACCAGGTGTCCCTGACC
TGTCTGGTGAAGGGATTCTATCCATCCGACATCGCCGTGGAGTGGGAGTCCAACG
GACAGCCAGAGAACAACTATAAGACCACCCCTCCAATGCTGGACTCCGACGGATC
CTTCTTCCTGTATTCCAAGCTGACCGTGGACAAGTCCAGATGGCAGCAGGGAAAC
GTGTTCTCTTGTTCCGTGATGCACGAGGCCCTGCACAACCACTATACCCAGAAGAG
CCTGTCCCTGTCTCCAGGAAAGTAA

G1 light chain full antibody nucleotide sequence (SEQ ID NO:14)

GAAATCGTTCTGACCCAGTCCCCGGCTACCCTGTCCCTGTCCCCAGGTGAACGTG
CTACCCTGTCCTGCAAAGCTTCCAAACGGGTTACCACCTACGTTTCCTGGTACCAG
CAGAAACCCGGTCAGGCTCCTCGTCTGCTGATCTACGGTGCTTCCAACCGTTACCT
CGGTATCCCAGCTCGTTTCTCCGGTTCCGGTTCCGGTACCGACTTCACCCTGACC
ATCTCCTCCCTGGAACCCGAAGACTTCGCTGTTTACTACTGCAGTCAGTCCTACAA
CTACCCCTACACCTTCGGTCAGGGTACCAAACTGGAAATCAAACGCACTGTGGCT
GCACCATCTGTCTTCATCTTCCCTCCATCTGATGAGCAGTTGAAATCCGGAACTGC
CTCTGTTGTGTGCCTGCTGAATAACTTCTATCCGCGCGAGGCCAAAGTACAGTGGA
AGGTGGATAACGCCCTCCAATCCGGTAACTCCCAGGAGAGTGTCACAGAGCAGGA
CAGCAAGGACAGCACCTACAGCCTCAGCAGCACCCTGACCCTGAGCAAAGCAGAC
TACGAGAAACACAAAGTCTACGCCTGCGAAGTCACCCATCAGGGCCTGAGTTCTC
CAGTCACAAAGAGCTTCAACCGCGGTGAGTGCTAA

Amino acid sequence comparison of human and rat CGRP (human α-CGRP (SEQ ID NO:15); human β-CGRP (SEQ ID NO:43); rat α-CGRP (SEQ ID NO:41); and rat β-CGRP (SEQ ID NO:44)):

NH2-ACDTATCVTHRLAGLLSRSGGVVKNNFVPTNVGSKAF-CONH2 (human α-CGRP)

NH2-ACNTATCVTHRLAGLLSRSGGMVKSNFVPTNVGSKAF-CONH2 (human β-CGRP)

NH2-SCNTATCVTHRLAGLLSRSGGVVKDNFVPTNVGSEAF-CONH2 (rat α-CGRP)

NH2-SCNTATCVTHRLAGLLSRSGGVVKDNFVPTNVGSKAF-CONH2 (rat β-CGRP)

Light chain variable region LCVR17 amino acid sequence (SEQ ID NO:58)

DIQMTQSPSSLSASVGDRVTITCRASQDIDNYLNWYQQKPGKAPKLLIYYTSEYHSGV
PSRFSGSGSGTDFTFTISSLQPEDIATYYCQQGDALPPTFGQGTKLEIK

Heavy chain variable region HCVR22 amino acid sequence (SEQ ID NO:59)

QVQLVQSGAEVKKPGASVKVSCKASGYTFGNYWMQWVRQAPGQGLEWMGAIYEGT
GDTRYIQKFAGRVTMRDTSTSTVYMELSSLRSEDTAVYYCARLSDYVSGFSYWGQG
TLVTVSS

Light chain variable region LCVR18 amino acid sequence (SEQ ID NO:60)

DIQMTQSPSSLSASVGDRVTITCRASQDIDNYLNWYQQKPGKAPKLLIYYTSEYHSGV
PSRFSGSGSGTDFTFTISSLQPEDIATYYCQQGDALPPTFGQGTKLEIK

Heavy chain variable region HCVR23 amino acid sequence (SEQ ID NO:61)

QVQLVQSGAEVKKPGASVKVSCKASGYTFGNYWMQWVRQAPGQGLEWMGAIYEGT
GKTVYIQKFAGRVTMRDTSTSTVYMELSSLRSEDTAVYYCARLSDYVSGFSYWGQG
TLVTVSS

Light chain variable region LCVR19 amino acid sequence (SEQ ID NO:62)

DIQMTQSPSSLSASVGDRVTITCRASKDISKYLNWYQQKPGKAPKLLIYYTSGYHSGVP
SRFSGSGSGTDFTLTISSLQPEDFATYYCQQGDALPPTFGGGTKVEIK

Heavy chain variable region HCVR24 amino acid sequence (SEQ ID NO:63)

QVQLVQSGAEVKKPGSSVKVSCKASGYTFGNYWMQWVRQAPGQGLEWMGAIYEGT
GKTVYIQKFADRVTITADKSTSTAYMELSSLRSEDTAVYYCARLSDYVSGFGYWGQGT
TVTVSS

Light chain variable region LCVR20 amino acid sequence (SEQ ID NO:64)

DIQMTQSPSSLSASVGDRVTITCRASRPIDKYLNWYQQKPGKAPKLLIYYTSEYHSGVP
SRFSGSGSGTDFTFTISSLQPEDIATYYCQQGDALPPTFGQGTKLEIK

Heavy chain variable region HCVR25 amino acid sequence (SEQ ID NO:65)

QVQLVQSGAEVKKPGASVKVSCKASGYTFGNYWMQWVRQAPGQGLEWMGAIYEGT
GKTVYIQKFAGRVTMTRDTSTSTVYMELSSLRSEDTAVYYCARLSDYVSGFGYWGQG
TLVTVSS

Light chain variable region LCVR21 amino acid sequence (SEQ ID NO:66)

DIQMTQSPSSLSASVGDRVTITCRASQDIDKYLNWYQQKPGKAPKLLIYYTSGYHSGV
PSRFSGSGSGTDFTLTISSLQPEDFATYYCQQGDALPPTFGGGTKVEIK

Heavy chain variable region HCVR26 amino acid sequence (SEQ ID NO:67)

QVQLVQSGAEVKKPGSSVKVSCKASGYTFGNYWMQWVRQAPGQGLEWMGAIYEGT
GKTVYIQKFAGRVTITADKSTSTAYMELSSLRSEDTAVYYCARLSDYVSGFGYWGQGT
TVTVSS

Light chain variable region LCVR27 amino acid sequence (SEQ ID NO:68)

QVLTQSPSSLSASVGDRVTINCQASQSVYHNTYLAWYQQKPGKVPKQLIYDASTLASG
VPSRFSGSGSGTDFTLTISSLQPEDVATYYCLGSYDCTNGDCFVFGGGTKVEIKR

Heavy chain variable region HCVR28 amino acid sequence (SEQ ID NO:69)

EVQLVESGGGLVQPGGSLRLSCAVSGIDLSGYYMNWVRQAPGKGLEWVGVIGINGAT
YYASWAKGRFTISRDNSKTTVYLQMNSLRAEDTAVYFCARGDIWGQGTLVTVSS

Light chain variable region LCVR29 amino acid sequence (SEQ ID NO:70)

QVLTQSPSSLSASVGDRVTINCQASQSVYDNNYLAWYQQKPGKVPKQLIYSTSTLASG
VPSRFSGSGSGTDFTLTISSLQPEDVATYYCLGSYDCSSGDCFVFGGGTKVEIKR

Heavy chain variable region HCVR30 amino acid sequence (SEQ ID NO:71)

EVQLVESGGGLVQPGGSLRLSCAVSGLDLSSYYMQWVRQAPGKGLEWVGVIGINDN
TYYASWAKGRFTISRDNSKTTVYLQMNSLRAEDTAVYFCARGDIWGQGTLVTVSS

Light chain variable region LCVR31 amino acid sequence (SEQ ID NO:72)

QVLTQSPSSLSASVGDRVTINCQASQSVYDNNYLAWYQQKPGKVPKQLIYSTSTLASG
VPSRFSGSGSGTDFTLTISSLQPEDVATYYCLGSYDCSSGDCFVFGGGTKVEIKR

Heavy chain variable region HCVR32 amino acid sequence (SEQ ID NO:73)

EVQLVESGGGLVQPGGSLRLSCAVSGLDLSSYYMQWVRQAPGKGLEWVGVIGINDN
TYYASWAKGRFTISRDNSKTTVYLQMNSLRAEDTAVYFCARGDIWGQGTLVTVSS

Light chain variable region LCVR33 amino acid sequence (SEQ ID NO:74)

QVLTQTPSPVSAAVGSTVTINCQASQSVYHNTYLAWYQQKPGQPPKQLIYDASTLASG
VPSRFSGSGSGTQFTLTISGVQCNDAAAYYCLGSYDCTNGDCFVFGGGTEVVVKR

Heavy chain variable region HCVR34 amino acid sequence (SEQ ID NO:75)

QSLEESGGRLVTPGTPLTLTCSVSGIDLSGYYMNWVRQAPGKGLEWIGVIGINGATYY
ASWAKGRFTISKTSSTTVDLKMTSLTTEDTATYFCARGDIWGPGTLVTVSS

Light chain variable region LCVR35 amino acid sequence (SEQ ID NO:76)

QVLTQSPSSLSASVGDRVTINCQASQSVYHNTYLAWYQQKPGKVPKQLIYDASTLASG
VPSRFSGSGSGTDFTLTISSLQPEDVATYYCLGSYDCTNGDCFVFGGGTKVEIKR

Heavy chain variable region HCVR36 amino acid sequence (SEQ ID NO:77)

EVQLVESGGGLVQPGGSLRLSCAVSGIDLSGYYMNWVRQAPGKGLEWVGVIGINGAT
YYASWAKGRFTISRDNSKTTVYLQMNSLRAEDTAVYFCARGDIWGQGTLVTVSS

Light chain variable region LCVR37 amino acid sequence (SEQ ID NO:78)

QSVLTQPPSVSAAPGQKVTISCSGSSSNIGNNYVSWYQQLPGTAPKLLIYDNNKRPSG
IPDRFSGSKSGTSTTLGITGLQTGDEADYYCGTWDSRLSAVVFGGGTKLTVL

Heavy chain variable region HCVR38 amino acid sequence (SEQ ID NO:79)

QVQLVESGGGVVQPGRSLRLSCAASGFTFSSFGMHWVRQAPGKGLEWVAVISFDGS
IKYSVDSVKGRFTISRDNSKNTLFLQMNSLRAEDTAVYYCARDRLNYYDSSGYYHYKY
YGMAVWGQGTTVTVSS

**Claims**

**1.** A composition comprising a monoclonal anti-CGRP antagonist antibody for use in treating or reducing incidence of post-traumatic headache in a subject, wherein the monoclonal antibody has a CDR H1 as set forth in SEQ ID NO:3; a CDR H2 as set forth in SEQ ID NO:4; a CDR H3 as set forth in SEQ ID NO:5; a CDR L1 as set forth in SEQ ID NO:6; a CDR L2 as set forth in SEQ ID NO:7; and a CDR L3 as set forth in SEQ ID NO:8.

2. The composition for use according to claim 1, wherein the composition:

(a) comprises 225 mg to 1000 mg of the monoclonal antibody;
(b) is administered monthly, for example wherein:

(i) the composition is administered monthly for three months or more; and/or
(ii) the dose of the monoclonal antibody administered to the subject is 225 mg to 675 mg; and/or

(c) is administered as a single dose; for example wherein the dose of the monoclonal antibody administered to the subject is 675 mg to 1000 mg.

3. The composition for use according to claim 1 or 2, wherein the treating or reducing comprises reduction in the number of headache hours of any severity, reducing the number of monthly headache days of any severity, reducing the use of any acute headache medications, reducing a 6-item Headache Impact Test (HIT-6) disability score, or any combination thereof; for example wherein the number of monthly headache days is reduced by at least 50% from a pre-administration level in the subject.

4. The composition for use according to any one of the previous claims, wherein:

(a) the composition is administered subcutaneously or intravenously;
(b) the composition is administered utilizing a pre-filled syringe comprising a dose of the monoclonal antibody;
(c) the monoclonal antibody is formulated at a concentration of at least 150 mg/mL; and/or
(d) the monoclonal antibody is administered in a volume of less than 2 mL.

5. The composition for use according to any one of the previous claims, wherein the composition is administered to the subject with a second agent simultaneously or sequentially with the monoclonal antibody; in particular wherein monthly use of the second agent by the subject is decreased by at least 15% after administering the monoclonal antibody.

6. The composition for use according to any one of the previous claims, wherein:

(a) the subject is human;
(b) the monoclonal antibody is human or humanized; and/or
(c) an IgG1, IgG2, IgG3, or IgG4 antibody.

7. The composition for use according to any one of claims 1-5, wherein the monoclonal antibody comprises a heavy chain variable region as set forth in SEQ ID NO:1 and a light chain variable region as set forth in SEQ ID NO:2.

8. The composition for use according to claim 7, wherein the monoclonal antibody comprises a heavy chain produced by the expression vector with ATCC Accession No. PTA-6867 and a light chain produced by the expression vector with ATCC Accession No. PTA-6866.

**Patentansprüche**

1. Zusammensetzung, umfassend einen monoklonalen Anti-CGRP-Antagonisten-Antikörper, zur Verwendung bei der Behandlung oder Verringerung der Inzidenz von posttraumatischem Kopfschmerz bei einem Individuum, wobei der monoklonale Antikörper eine CDR H1 gemäß SEQ ID NO: 3, eine CDR H2 gemäß SEQ ID NO: 4, eine CDR H3 gemäß SEQ ID NO: 5, eine CDR L1 gemäß SEQ ID NO: 6, eine CDR L2 gemäß SEQ ID NO: 7 und eine CDR L3 gemäß SEQ ID NO: 8 aufweist.

2. Zusammensetzung zur Verwendung nach Anspruch 1, wobei die Zusammensetzung:

(a) 225 mg bis 1000 mg des monoklonalen Antikörpers umfasst,

(b) monatlich verabreicht wird, wobei zum Beispiel:

(i) die Zusammensetzung monatlich drei Monate lang oder länger verabreicht wird, und/oder
(ii) die Dosis des an das Individuum verabreichten monoklonalen Antikörpers 225 mg bis 675 mg beträgt, und/oder

(c) als Einzeldosis verabreicht wird, wobei zum Beispiel die Dosis des an das Individuum verabreichten monoklonalen Antikörpers 675 mg bis 1000 mg beträgt.

3. Zusammensetzung zur Verwendung nach Anspruch 1 oder 2, wobei die Behandlung oder Verringerung das Verringern der Anzahl an Kopfschmerzstunden jeglichen Schweregrads, das Verringern der Anzahl an monatlichen Kopfschmerztagen jeglichen Schweregrads, das Verringern der Verwendung jeglicher akuter Kopfschmerzmedikamente, das Verringern eines HIT-6 (6-Punkte-Headache Impact Test)-Beeinträchtigungswerts oder eine beliebige Kombination davon umfasst, wobei zum Beispiel die Anzahl an monatlichen Kopfschmerztagen gegenüber dem Vor-Verabreichungsniveau bei dem Individuum um mindestens 50% verringert wird.

4. Zusammensetzung zur Verwendung nach einem der vorhergehenden Ansprüche, wobei:

(a) die Zusammensetzung subkutan oder intravenös verabreicht wird,
(b) die Zusammensetzung unter Verwendung einer Fertigspritze, die eine Dosis des monoklonalen Antikörpers umfasst, verabreicht wird,
(c) der monoklonale Antikörper in einer Konzentration von mindestens 150 mg/ml formuliert ist, und/oder
(d) der monoklonale Antikörper in einem Volumen von weniger als 2 ml verabreicht wird.

5. Zusammensetzung zur Verwendung nach einem der vorhergehenden Ansprüche, wobei die Zusammensetzung an das Individuum mit einem zweiten Mittel gleichzeitig oder nacheinander mit dem monoklonalen Antikörper verabreicht wird, wobei insbesondere die monatliche Verwendung des zweiten Mittels durch das Individuum nach der Verabreichung des monoklonalen Antikörpers um mindestens 15% verringert ist.

6. Zusammensetzung zur Verwendung nach einem der vorhergehenden Ansprüche, wobei:

(a) das Individuum menschlich ist,
(b) der monoklonale Antikörper human oder humanisiert ist, und/oder
(c) ein IgG1-, IgG2-, IgG3- oder IgG4-Antikörper ist.

7. Zusammensetzung zur Verwendung nach einem der Ansprüche 1-5, wobei der monoklonale Antikörper eine variable Region der schweren Kette gemäß SEQ ID NO: 1 und eine variable Region der leichten Kette gemäß SEQ ID NO: 2 umfasst.

8. Zusammensetzung zur Verwendung nach Anspruch 7, wobei der monoklonale Antikörper eine durch den Expressionsvektor mit der ATCC-Zugangsnr. PTA-6867 produzierte schwere Kette und eine durch den Expressionsvektor mit der ATCC-Zugangsnr. PTA-6866 produzierte leichte Kette umfasst.

**Revendications**

1. Composition comprenant un anticorps antagoniste monoclonal anti-CGRP pour une utilisation dans le traitement ou la réduction de l'incidence d'une céphalée post-traumatique chez un sujet, dans laquelle l'anticorps monoclonal a une CDR H1 telle que présentée dans SEQ ID NO : 3 ; une CDR H2 telle que présentée dans SEQ ID NO : 4 ; une CDR H3 telle que présentée dans SEQ ID NO : 5 ; une CDR L1 telle que présentée dans SEQ ID NO : 6 ; une CDR L2 telle que présentée dans SEQ ID NO : 7 ; et une CDR L3 telle que présentée dans SEQ ID NO : 8.

2. Composition pour une utilisation selon la revendication 1, dans laquelle la composition :

(a) comprend 225 mg à 1000 mg de l'anticorps monoclonal ;
(b) est administrée mensuellement, par exemple de telle sorte que :

(i) la composition est administrée mensuellement pendant trois mois ou plus ; et/ou

(ii) la dose de l'anticorps monoclonal administrée au sujet est de 225 mg à 675 mg ; et/ou

(c) est administrée sous forme d'une dose unique ; par exemple dans laquelle la dose de l'anticorps monoclonal administrée au sujet est de 675 mg à 1000 mg.

**3.** Composition pour une utilisation selon la revendication 1 ou 2, dans laquelle le traitement ou la réduction comprend la réduction du nombre d'heures de céphalée de toute gravité, la réduction du nombre de jours de céphalée mensuels de toute gravité, la réduction de l'utilisation de tous médicaments des céphalées aigües, la réduction d'une note d'handicap à l'échelle d'impact des céphalées (Headache Impact Test (HIT-6)) à 6 items, ou toute combinaison de celles-ci ; par exemple dans laquelle le nombre de jours de céphalées mensuels est réduit d'au moins 50 % par rapport à un niveau avant administration chez le sujet.

**4.** Composition pour une utilisation selon l'une quelconque des revendications précédentes, dans laquelle :

(a) la composition est administrée par voie sous-cutanée ou intraveineuse ;
(b) la composition est administrée par utilisation d'une seringue pré-remplie comprenant une dose de l'anticorps monoclonal ;
(c) l'anticorps monoclonal est formulé à une concentration d'au moins 150 mg/ml ; et/ou
(d) l'anticorps monoclonal est administré en un volume inférieur à 2 ml.

**5.** Composition pour une utilisation selon l'une quelconque des revendications précédentes, dans laquelle la composition est administrée au sujet avec un second agent en même temps que l'anticorps monoclonal ou après celui-ci ; en particulier dans laquelle l'utilisation mensuelle du second agent par le sujet diminue d'au moins 15 % après administration de l'anticorps monoclonal.

**6.** Composition pour une utilisation selon l'une quelconque des revendications précédentes, dans laquelle :

(a) le sujet est humain ;
(b) l'anticorps monoclonal est humain ou humanisé ; et/ou
(c) un anticorps IgG1, IgG2, IgG3 ou IgG4.

**7.** Composition pour une utilisation selon l'une quelconque des revendications 1 à 5, dans laquelle l'anticorps monoclonal comprend une région variable de chaîne lourde telle que présentée dans SEQ ID NO:1 et une région variable de chaîne légère telle que présentée dans SEQ ID NO:2.

**8.** Composition pour une utilisation selon la revendication 7, dans laquelle l'anticorps monoclonal comprend une chaîne lourde produite par le vecteur d'expression portant le numéro d'accession ATCC PTA-6867 et une chaîne légère produite par le vecteur d'expression portant le numéro d'accession ATCC PTA-6866.

## Figure 1

EP 3 353 202 B1

# Figure 2A

Figure 2B

EP 3 353 202 B1

Figure 3

Figure 4A

EP 3 353 202 B1

Figure 4B

EP 3 353 202 B1

# Figure 5

**Bold=Kabat CDR**

<u>Underline=Chothia CDR</u>

G1 Heavy chain

```
1         5          10         15         20         25    H1  30
E V Q L V E S G G G L V Q P G G S L R L S C A A S G F T F S

31        35         40         45         50    H2        60
N Y W I S W V R Q A P G K G L E W V A R I R S E S D A S A T

61        65         70         75         80            90
H Y A E A V K G R F T I S R D N A K N S L Y L Q M N S L R A

91        95         100       105   H3    110        115       120
E D T A V Y Y C L A Y F D Y G L A I Q N Y W G Q G T L V T V

121 122
S S
```

G1 Light chain

```
1         5          10         15         20         25    L1  30
E I V L T Q S P A T L S L S P G E R A T L S C K A S K R V T

31        35         40         45         50    L2        60
T Y V S W Y Q Q K P G Q A P R L L I Y G A S N R Y L G I P A

61        65         70         75         80            90
R F S G S G S G T D F T L T I S S L E P E D F A V Y Y C S Q

91    L3   95         100       105   107
S Y N Y P Y T F G Q G T K L E I K
```

EP 3 353 202 B1

92

Figure 6

# Figure 7

EP 3 353 202 B1

Figure 8A

EP 3 353 202 B1

# Figure 8B

**Saphenous Assay
7-day**

Figure 8C

Saphenous Assay
G1 Antibody
Dose Response

EP 3 353 202 B1

# Figure 9

EP 3 353 202 B1

## Figure 10

# Figure 11A

Core Temp

EP 3 353 202 B1

# Figure 11B

**Tall surface**

Veh (N=11)
4901 10mg/kg (N=12)

EP 3 353 202 B1

FIGs. 12A and 12B

## cephalic

## Hindpaw

**FIGs. 13A to 13D**

FIGs. 14A to 14D

EP 3 353 202 B1

A

one
chamber

opposite
chamber

20
min

20
min

← 2 H →

← 2 H →

← 2 H →

**vehicle**

**Suma**

B

mCHI

✳✳

200

Sham

Difference score (Sec)

0

-200

-400

☐ Saline Paired
■ Sumatriptan Paired

-600

**FIGs. 15A and 15B**

FIGs. 16A to 16D

FIGs. 17A to 17D

FIGs. 18A to 18D

**FIGs. 19A and 19B**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 9901441 W
- GB 9809951 A
- US 4816567 A
- WO 9958572 A
- US 5545807 A
- US 5545806 A
- US 5569825 A
- US 5625126 A
- US 5633425 A
- US 5661016 A
- US 5750373 A
- US 5500362 A
- US 5821337 A
- WO 0053211 A
- US 5981568 A
- US 4485045 A
- US 4544545 A
- US 5013556 A
- US 3773919 A
- US 5807715 A
- US 5866692 A
- US 6331415 B
- US 5530101 A
- US 5693761 A
- US 5693762 A
- US 5585089 A
- US 6180370 B
- EP 0519596 A
- US 6180377 B
- US 6054297 A
- US 5997867 A
- US 6210671 B
- US 6350861 B
- WO 0127160 A
- US 5565332 A
- US 5580717 A
- US 5733743 A
- US 6265150 B
- WO 9306213 A
- WO 8704462 A
- US 6436908 B
- US 6413942 B
- US 6376471 B

- WO 9007936 A
- WO 9403622 PCT
- WO 9325698 PCT
- WO 9325234 PCT
- WO 9311230 PCT
- WO 9310218 PCT
- WO 9102805 PCT
- US 5219740 A
- US 4777127 A
- GB 2200651 A
- EP 0345242 A
- WO 9412649 A
- WO 9303769 PCT
- WO 9319191 PCT
- WO 9428938 PCT
- WO 9511984 PCT
- WO 9500655 PCT
- US 5814482 A
- WO 9507994 A
- WO 9617072 PCT
- WO 9530763 PCT
- WO 9742338 PCT
- WO 9011092 A
- US 5580859 A
- US 5422120 A
- WO 9513796 A
- WO 9423697 PCT
- WO 9114445 PCT
- EP 0524968 A
- WO 9404690 A
- US 4676980 A
- WO 9100360 A
- WO 92200373 PCT
- EP 03089 A
- US 5047335 A
- US 5510261 A
- US 5278299 A
- WO 2004058184 A
- US 4683195 A
- US 4800159 A
- US 4754065 A
- US 4683202 A

**Non-patent literature cited in the description**

- **RUSSELL et al.** *Physiol Rev,* 2014, vol. 94, 1099-1142
- **RIECHERS et al.** Handbook of Clinical Neurology. 2015, vol. 128, 567-578

- **DAIUTOLO et al.** *Department of Neurosurgery Faculty Papers,* 2014
- **BIGAL et al.** *CNS Drugs,* 2014, vol. 28 (5), 389-399
- **WARE et al.** *Med Care,* 1996, vol. 4, 220-233
- **HURST et al.** *J Manipulative Physiol Ther,* 2004, vol. 27, 26-35
- **MCCRORY et al.** *British Journal of Sports Medicine,* 2013, vol. 47, 263-266
- *Eur. J. Immunol.,* 1999, vol. 29, 2613-2624
- **SAMBROOK et al.** Molecular Cloning: A Laboratory Manual. Cold Spring Harbor Press, 1989
- Oligonucleotide Synthesis. 1984
- Methods in Molecular Biology. Humana Press
- Cell Biology: A Laboratory Notebook. Academic Press, 1998
- Animal Cell Culture. 1987
- **J.P. MATHER ; P.E. ROBERTS.** Introduction to Cell and Tissue Culture. Plenum Press, 1998
- Cell and Tissue Culture: Laboratory Procedures. J. Wiley and Sons, 1993
- Methods in Enzymology. Academic Press, Inc,
- Handbook of Experimental Immunology
- Gene Transfer Vectors for Mammalian Cells. 1987
- Current Protocols in Molecular Biology. 1987
- PCR: The Polymerase Chain Reaction. 1994
- Current Protocols in Immunology. 1991
- Short Protocols in Molecular Biology. Wiley and Sons, 1999
- **C.A. JANEWAY ; P. TRAVERS.** *Immunobiology,* 1997
- **P. FINCH.** *Antibodies,* 1997
- Antibodies: a practical approach. IRL Press, 1988
- Monoclonal antibodies: a practical approach. Oxford University Press, 2000
- Using antibodies: a laboratory manual. Cold Spring Harbor Laboratory Press, 1999
- The Antibodies. Harwood Academic Publishers, 1995
- **KOHLER ; MILSTEIN.** *Nature,* 1975, vol. 256, 495
- **MCCAFFERTY et al.** *Nature,* 1990, vol. 348, 552-554
- **VAUGHAN et al.** *Nature Biotechnology,* 1996, vol. 14, 309-314
- **SHEETS et al.** *PNAS,* 1998, vol. 95, 6157-6162
- **HOOGENBOOM ; WINTER.** *J. Mol. Biol.,* 1991, vol. 227, 381
- **MARKS et al.** *J. Mol. Biol.,* 1991, vol. 222, 581
- **COLE et al.** Monoclonal Antibodies and Cancer Therapy. Alan R. Liss, 1985, 77
- **BOERNER et al.** *J. Immunol.,* 1991, vol. 147 (1), 86-95
- Cephalalgia. The International Classification of Headache Disorders, 2013, vol. 33, 629-808
- **KABAT et al.** Sequences of Proteins of Immunological Interest. National Institutes of Health, 1991
- **AI-LAZIKANI et al.** *J. Molec. Biol.,* 1997, vol. 273, 927-948
- **RAVETCH ; KINET.** *Ann. Rev. Immunol.,* 1991, vol. 9, 457-92
- **CAPEL et al.** *Immunomethods,* 1994, vol. 4, 25-34
- **DE HAAS et al.** *J. Lab. Clin. Med.,* 1995, vol. 126, 330-41
- **GUYER et al.** *J. Immunol.,* 1976, vol. 117, 587
- **KIM et al.** *J. Immunol.,* 1994, vol. 24, 249
- **GAZZANO-SANTORO et al.** *J. Immunol. Methods,* 1996, vol. 202, 163
- **CLYNES et al.** *PNAS,* 1998, vol. 95, 652-656
- Remington's Pharmaceutical Sciences. Mack Publishing Co, 1990
- Remington, The Science and Practice of Pharmacy. Mack Publishing, 2000
- **EPSTEIN et al.** *Proc. Natl. Acad. Sci. USA,* 1985, vol. 82, 3688
- **HWANG et al.** *Proc. Natl Acad. Sci. USA,* 1980, vol. 77, 4030
- **TAN et al.** *Clin. Sci.,* 1995, vol. 89, 565-73
- **PLOURDE et al.** *Peptides,* 1993, vol. 14, 1225-1229
- **KARLSSON, R. ; ROOS, H. ; FAGERSTAM, L. ; PETERSSON, B.** *Methods Enzymology,* 1994, vol. 6, 99-110
- **KOHLER, B. ; MILSTEIN, C.** *Nature,* 1975, vol. 256, 495-497
- **BUCK, D. W. et al.** *In Vitro,* 1982, vol. 18, 377-381
- **WINTER et al.** *Nature,* 1991, vol. 349, 293-299
- **LOBUGLIO et al.** *Proc. Nat. Acad. Sci. USA,* 1989, vol. 86, 4220-4224
- **SHAW et al.** *J Immunol.,* 1987, vol. 138, 4534-4538
- **BROWN et al.** *Cancer Res.,* 1987, vol. 47, 3577-3583
- **RIECHMANN et al.** *Nature,* 1988, vol. 332, 323-327
- **VERHOEYEN et al.** *Science,* 1988, vol. 239, 1534-1536
- **JONES et al.** *Nature,* 1986, vol. 321, 522-525
- **DAUGHERTY et al.** *Nucl. Acids Res.,* 1991, vol. 19, 2471-2476
- **WINTER et al.** *Annu. Rev. Immunol.,* 1994, vol. 12, 433-455
- **MCCAFFERTY et al.** *Nature,* 1990, vol. 348, 552-553
- **JOHNSON, KEVIN S. ; CHISWELL, DAVID J.** *Current Opinion in Structural Biology,* 1993, vol. 3, 564-571

- **CLACKSON et al.** *Nature,* 1991, vol. 352, 624-628
- **MARK et al.** *J. Mol. Biol.,* 1991, vol. 222, 581-597
- **GRIFFITH et al.** *EMBO J.,* 1993, vol. 12, 725-734
- **MARKS et al.** *Bio/Technol.,* 1992, vol. 10, 779-783
- **WATERHOUSE et al.** *Nucl. Acids Res.,* 1993, vol. 21, 2265-2266
- **PEETERS et al.** *Vaccine,* 2001, vol. 19, 2756
- **LONBERG, N. ; D. HUSZAR.** *Int. Rev. Immunol,* 1995, vol. 13, 65
- **POLLOCK et al.** *J Immunol Methods,* 1999, vol. 231, 147
- **MORRISON et al.** *Proc. Nat. Acad. Sci.,* 1984, vol. 81, 6851
- **ESCOTT et al.** *Br. J. Pharmacol.,* 1993, vol. 110, 772-776
- **REUTER et al.** *Functional Neurology,* 2000, vol. 15 (3
- Harlow and Lane, Using Antibodies, a Laboratory Manual. Cold Spring Harbor Laboratory Press, 1999
- **FINDEIS et al.** *Trends Biotechnol.,* 1993, vol. 11, 202
- **CHIOU et al.** Gene Therapeutics: Methods and Applications of Direct Gene Transfer. 1994
- **WU et al.** *J. Biol. Chem.,* 1988, vol. 263, 621
- **WU et al.** *J. Biol. Chem.,* 1994, vol. 269, 542
- **ZENKE et al.** *Proc. Natl. Acad. Sci. USA,* 1990, vol. 87, 3655
- **WU et al.** *J. Biol. Chem.,* 1991, vol. 266, 338
- **JOLLY.** *Cancer Gene Therapy,* 1994, vol. 1, 51
- **KIMURA.** *Human Gene Therapy,* 1994, vol. 5, 845
- **CONNELLY.** *Human Gene Therapy,* 1995, vol. 1, 185
- **KAPLITT.** *Nature Genetics,* 1994, vol. 6, 148
- **CURIEL.** *Hum. Gene Ther.,* 1992, vol. 3, 147
- **WU.** *J. Biol. Chem.,* 1989, vol. 264, 16985
- **PHILIP.** *Mol. Cell Biol.,* 1994, vol. 14, 2411
- **WOFFENDIN.** *Proc. Natl. Acad. Sci.,* 1994, vol. 91, 1581
- **BIRD et al.** *Science,* 1988, vol. 242, 423-426
- **HOLLIGER, P. et al.** *Proc. Natl. Acad Sci. USA,* 1993, vol. 90, 6444-6448
- **POLJAK, R. J. et al.** *Structure,* 1994, vol. 2, 1121-1123
- **SURESH et al.** *Methods in Enzymology,* 1986, vol. 121, 210
- **MILLSTEIN ; CUELLO.** *Nature,* 1983, vol. 305, 537-539
- **JEFFERIS ; LUND.** *Chem. Immunol.,* 1997, vol. 65, 111-128
- **WRIGHT ; MORRISON.** *TibTECH,* 1997, vol. 15, 26-32
- **BOYD et al.** *Mol. Immunol.,* 1996, vol. 32, 1311-1318
- **WITTWE ; HOWARD.** *Biochem.,* 1990, vol. 29, 4175-4180
- **WYSS ; WAGNER.** *Current Opin. Biotech.,* 1996, vol. 7, 409-416
- **UMANA et al.** *Mature Biotech.,* 1999, vol. 17, 176-180
- **HSE et al.** *J. Biol. Chem.,* 1997, vol. 272, 9062-9070
- **MORGAN et al.** *Immunology,* 1995, vol. 86, 319-324
- **LUND et al.** *J. Immunology,* 1996, vol. 157, 4963-4969
- **IDUSOGIE et al.** *J. Immunology,* 2000, vol. 164, 4178-4184
- **TAO et al.** *J. Immunology,* 1989, vol. 143, 2595-2601
- **JEFFERIS et al.** *Immunological Reviews,* 1998, vol. 163, 59-76
- **MARKS et al.** *Bio/Technology,* 1992, vol. 10, 779-783
- **BARBAS et al.** *Proc Nat. Acad. Sci,* 1994, vol. 91, 3809-3813
- **SCHIER et al.** *Gene,* 1995, vol. 169, 147-155
- **YELTON et al.** *J. Immunol.,* 1995, vol. 155, 1994-2004
- **JACKSON et al.** *J. Immunol.,* 1995, vol. 154 (7), 3310-9
- **HAWKINS et al.** *J. Mol. Biol.,* 1992, vol. 226, 889-896
- **BALINT et al.** *Gene,* 1993, vol. 137 (1), 109-18
- A model of evolutionary change in proteins - Matrices for detecting distant relationships. **DAYHOFF, M.O.** Atlas of Protein Sequence and Structure. National Biomedical Research Foundation, 1978, vol. 5, 345-358
- Unified Approach to Alignment and Phylogenes. **HEIN J.** Methods in Enzymology. Academic Press, Inc, 1990, vol. 183, 626-645
- **HIGGINS, D.G. ; SHARP, P.M.** *CABIOS,* 1989, vol. 5, 151-153
- **MYERS, E.W. ; MULLER W.** *CABIOS,* 1988, vol. 4, 11-17
- **ROBINSON, E.D.** *Comb. Theor.,* 1971, vol. 11, 105
- **SANTOU, N. ; NES, M.** *Mol. Biol. E,* 1987, vol. 4, 406-425
- **SNEATH, P.H.A. ; SOKAL, R.R.** Numerical Taxonomy the Principles and Practice of Numerical Taxonomy. Freeman Press, 1973
- **WILBUR, W.J. ; LIPMAN, D.J.** *Proc. Natl. Acad. Sci. USA,* 1983, vol. 80, 726-730
- PCR: The Polymerase Chain Reaction. Birkauswer Press, 1994

- Remington: The Science and practice of Pharmacy. Lippincott Williams and Wilkins, 2000
- **GEERLIGS HJ et al.** *J. Immunol. Methods,* 1989, vol. 124, 95-102
- **KENNEY JS et al.** *J. Immunol. Methods,* 1989, vol. 121, 157-166
- **WICHER K et al.** *Int. Arch. Allergy Appl. Immunol.,* 1989, vol. 89, 128-135
- **ZIMMERMANN et al.** *Peptides,* 1995, vol. 16, 421-4
- **MALLEE et al.** *J. Biol. Chem.,* 2002, vol. 277, 14294-8
- **TAN et al.** *Clin. Sci.,* 1995, vol. 89, 656-73
- **BARBAS.** Phage display: a laboratory manual. Cold Spring Harbor Laboratory Press, 2001, 2.10
- **WILLIAMSON et al.** *Cephalalgia,* 1997, vol. 17 (4), 518-24
- **SIPE et al.** *Brain Res.,* 2004, vol. 1028 (2), 191-202
- **MERCHENTHALER et al.** *Maturitas,* 1998, vol. 30, 307-316
- **KATOVICH et al.** *Brain Res.,* 1989, vol. 494, 85-94
- **SIMPKINS et al.** *Life Sciences,* 1983, vol. 32, 1957-1966
- **MARMAROU et al.** *J of Neurosurgery,* 1994, vol. 80 (2), 291-300
- **MYCHASIUK et al.** *J Neurotrauma,* 2014, vol. 31 (8), 749-757
- **KING et al.** *Neuropharmacology,* 2004, vol. 47 (2), 195-204
- **MORIARTY et al.** *Behavioural brain research,* 2016, vol. 303, 61-70
- **EDELMAYER et al.** *Methods Mol Biol,* 2012, vol. 851, 109-120
- **OSHINSKY.** *Headache,* 2007, vol. 47 (7), 1026-1036
- **YAN et al.** *Mol Pain,* 2012, vol. 8, 6
- **ZHAO et al.** *Pain,* 2014, vol. 155 (7), 1392-1400
- **LEVY et al.** *Brain, Behavior, and Immunity,* 2012, vol. 26 (2), 311-317
- **VOS et al.** *Journal of Neuroscience: the official journal of the Society for Neuroscience,* 1994, vol. 14 (5), 2708-2723
- **GRIGGS et al.** *Neuroreport,* 2015, vol. 26 (9), 522-527
- **DE FELICE et al.** *Annals of neurology,* 2013, vol. 74 (2), 257-265
- **TFELT-HANSEN et al.** *Headache,* 1998, vol. 38 (10), 748-755
- **OSHINSKY et al.** *Headache,* 2012, vol. 52 (9), 1336-1349
- **WINNER et al.** *Mayo Clin Proc,* 2003, vol. 78 (10), 1214-1222
- **KOPRUSZINSKI et al.** *Cephalalgia: an International Journal of Headache,* 2016
- **PRADHAN et al.** *Pain,* 2014, vol. 155 (2), 269-274
- **KILBOURNE et al.** *J Neurotrauma,* 2009, vol. 26 (12), 2233-2243
- **ABEND et al.** *J Child Neurol,* 2008, vol. 23 (4), 438-440
- **LUCAS.** *Curr Pain Headache Rep,* 2015, vol. 19 (10), 48
- **VISSER et al.** *Headache,* 1996, vol. 36 (8), 471-475
- **PIETROBON et al.** *Annu Rev Physiol,* 2013, vol. 75, 365-391
- **RUSSO.** *Annual review of pharmacology and toxicology,* 2015, vol. 55, 533-552
- **MITSIKOSTAS et al.** *BMC Med,* 2015, vol. 13, 279
- **BIGAL et al.** *Lancet Neurol,* 2015, vol. 14 (11), 1081-1090
- **BIGAL et al.** *Lancet Neurol,* 2015, vol. 14 (11), 1091-1100